# EUROPÄISCHE PATENTSCHRIFT

(11) **EP 3 775 099 B1**
(45) Veröffentlichungstag und Bekanntmachung des Hinweises auf die Patenterteilung: **24.08.2022**
(21) Anmeldenummer: 19713814.2
(22) Anmeldetag: 01.04.2019
(51) Int. Cl.: C09K 11/06, C07C 211/00, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 04.04.2018 EP 18165764
(43) Veröffentlichungstag der Anmeldung: 17.02.2021
(73) Patentinhaber: Merck Patent GmbH, 64293 Darmstadt (DE)
(72) Erfinder: PARHAM, Amir, 60486 FRANKFURT AM MAIN (DE); KROEBER, Jonas, 60311 FRANKFURT AM MAIN (DE); GROSSMANN, Tobias, 75387 NEUBULACH (DE); JATSCH, Anja, 60489 FRANKFURT AM MAIN (DE); EICKHOFF, Christian, 68259 MANNHEIM (DE); EHRENREICH, Christian, 64285 DARMSTADT (DE); ENGELHART, Jens, 64285 DARMSTADT (DE)
(86) Internationale Anmeldenummer: PCT/EP2019/058174
(87) Internationale Veröffentlichungsnummer: WO 2019/192954

(56) Entgegenhaltungen:
- WO-A1-2017/148565
- KR-A- 20140 043 224
- KR-A- 20170 134 163
- US-A1- 2016 043 317
- US-A1- 2016 176 801
- US-A1- 2017 194 569

## Beschreibung

Die vorliegende Anmeldung betrifft Fluorenyl-Verbindungen enthaltend mindestens eine Aminogruppe. Die Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten, insbesondere Lebensdauer, Effizienz und Betriebsspannung. In diesen Punkten konnte noch keine vollständig zufriedenstellende Lösung gefunden werden.

Weiterhin werden Materialien mit einer hohen Glasübergangstemperatur, einer geringen Neigung zur Kristallisation und einem hohen Brechungsindex gesucht, insbesondere zur Verwendung in lochtransportierenden und in emittierenden Schichten von OLEDs.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Verbindungen gesucht, insbesondere lochtransportierende Verbindungen und Verbindungen, die als Matrixmaterial, insbesondere für phosphoreszierende Emitter, in einer emittierenden Schicht dienen können.

Verbindungen enthaltend eine oder mehrere Fluorenylgruppen, die direkt oder über Spacergruppen an eine Aminogruppe gebunden sind, sind im Stand der Technik als Verbindungen zur Verwendung in OLEDs bekannt, insbesondere zur Verwendung als lochtransportierende Verbindungen und unter anderem in US20160176801, KR20170134163, US20170194569, US20160043317, KR20140043224 und WO2017148565 beschrieben.

Es besteht jedoch weiterhin Bedarf an alternativen Verbindungen, die zur Verwendung in elektronischen Vorrichtungen geeignet sind. Es besteht auch Verbesserungsbedarf bezüglich der Leistungsdaten bei Verwendung in elektronischen Vorrichtungen, insbesondere bezüglich Lebensdauer, Betriebsspannung und Effizienz.

Nun wurde gefunden, dass sich bestimmte Verbindungen der oben genannten Strukturklasse hervorragend zur Verwendung in elektronischen Vorrichtungen eignen, insbesondere zur Verwendung in OLEDs, nochmals insbesondere darin zur Verwendung als Lochtransportmaterialien und zur Verwendung als Matrixmaterialien für phosphoreszierende Emitter. Die Verbindungen führen bevorzugt zu hoher Lebensdauer, hoher Effizienz und geringer Betriebsspannung der Vorrichtungen. Weiterhin bevorzugt weisen die Verbindungen eine geringe Kristallisationsneigung, eine hohe Glasübergangstemperatur und einen hohen Brechungsindex auf.

### Gegenstand der vorliegenden Anmeldung ist damit eine Verbindung einer Formel (I-A-4)

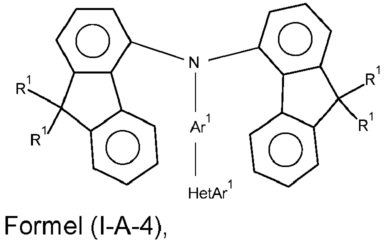

wobei die freien Positionen an den Fluorenylgruppen jeweils mit einem Rest R² substituiert sein können und wobei weiterhin gilt:
R¹ ist bei jedem Auftreten gleich oder verschieden gewählt aus geradkettigen Alkylgruppen mit 1 bis 10 C-Atomen, und verzweigten oder cyclischen Alkylgruppen mit 3 bis 10 C-Atomen, wobei zwei oder mehr Reste R¹ miteinander verknüpft sein können und einen Ring bilden können, und wobei ein oder mehrere H-Atome in den Alkylgruppen durch D ersetzt sein können;
Ar¹ ist gewählt aus aromatischen Ringsystemen mit 6 bis 20 aromatischen Ringatomen, die mit einem oder mehreren Resten R⁴ substituiert sein können;
HetAr¹ ist gewählt aus heteroaromatischen Ringsystemen mit 13 bis 40 aromatischen Ringatomen, die mit einem oder mehreren Resten R⁵ substituiert sein können;
R², R⁴ sind bei jedem Auftreten gleich oder verschieden gewählt aus H, D, F, C(=O)R¹¹, CN, Si(R¹¹)₃, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste gewählt aus Resten R², und zwei oder mehr Reste gewählt aus Resten R⁴ jeweils miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹- , NR¹¹, P(=O)(R¹¹), -O-, -S-, SO oder SO₂ ersetzt sein können;
R⁵ ist bei jedem Auftreten gleich oder verschieden gewählt aus H, D, F, C(=O)R¹¹, CN, Si(R¹¹)3, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste gewählt aus Resten R⁵ jeweils miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R¹¹ substituiert sein können; und wobei eine oder mehrere CH2-Gruppen in den genannten Alkyl-, Alkoxy-, Alkenyl- und Alkinylgruppen durch - R¹¹C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können;
R¹¹ ist bei jedem Auftreten gleich oder verschieden gewählt aus H, D, F, C(=O)R²¹, CN, Si(R²¹)₃, N(R²¹)₂, P(=O)(R²¹)₂, OR²¹, S(=O)R²¹, S(=O)₂R²¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R²¹ substituiert sein können; und wobei eine oder mehrere CH₂-Gruppen in den genannten Alkyl-, Alkoxy-, Alkenyl- und Alkinylgruppen durch - R²¹C=CR²¹-, -C=C-, Si(R²¹)₂, C=O, C=NR²¹, -C(=O)O-, -C(=O)NR²¹-, NR²¹ P(=O)(R²¹), -O-, -S-, SO oder SO₂ ersetzt sein können;
R²¹ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R³ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;
Der Kreis in den Sechsringen der Formel (I-A-4) bedeutet, dass der betreffende Ring aromatisch ist.

Die folgenden Definitionen gelten für die chemischen Gruppen, die in der vorliegenden Anmeldungen verwendet werden. Sie gelten, soweit keine spezielleren Definitionen angegeben sind.

Unter einer Arylgruppe im Sinne dieser Erfindung wird entweder ein einzelnder aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome, von denen keines ein Heteroatom ist.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied, dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus N, O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroatomatisches Ringsystem" umfasst.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

R¹ ist bevorzugt bei jedem Auftreten gleich oder verschieden, bevorzugt gleich, gewählt aus geradkettigen Alkylgruppen mit 1 bis 10 C-Atomen und verzweigten oder cyclischen Alkylgruppen mit 3 bis 10 C-Atomen, wobei zwei oder mehr Reste R¹ miteinander verknüpft sein können und einen Ring bilden können, und wobei ein oder mehrere H-Atome in den Alkylgruppen durch D ersetzt sein können. Besonders bevorzugt ist R¹ bei jedem Auftreten gleich oder verschieden, bevorzugt gleich, gewählt aus Methyl, n-Octyl und Cyclopentyl; ganz besonders bevorzugt ist R¹ gleich Methyl.

Ar¹ ist bevorzugt gewählt aus aromatischen Ringsystemen mit 6 bis 20 aromatischen Ringatomen, die mit einem oder mehreren Resten R⁴ substituiert sein können. Besonders bevorzugte Gruppen Ar¹ sind gewählt aus Benzol, Biphenyl, Terphenyl, Naphthalin, Phenyl-Naphthalin, Fluoren, Indenofluoren, die jeweils mit einem oder mehreren Resten R⁴ substituiert sein können. Ganz besonders bevorzugt ist Ar¹ Benzol oder Naphthalin, die jeweils mit einem oder mehreren Resten R³ substituiert sein können, am stärksten Benzol, das jeweils mit einem oder mehreren Resten R⁴ substituiert sein kann. Wenn Ar¹ Benzol ist, ist R⁴ bevorzugt gewählt aus H, Methyl und Phenyl.

Bevorzugte Gruppen Ar¹ sind in der folgenden Tabelle gezeigt:

| | | |
|---|---|---|
| 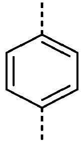 | 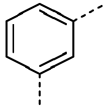 | 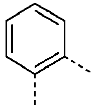 |
| Ar¹-1 | Ar¹-2 | Ar¹-3 |
| 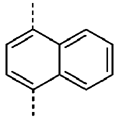 | 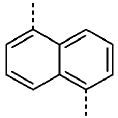 | 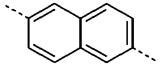 |
| Ar¹-4 | Ar¹-5 | Ar¹-6 |
| 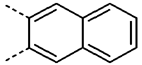 | 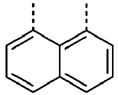 | 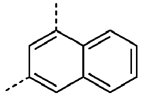 |
| Ar¹-7 | Ar¹-8 | Ar¹-9 |
| 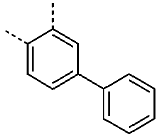 | 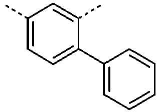 | 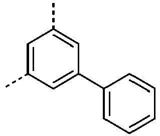 |
| Ar¹-10 | Ar¹-11 | Ar¹-12 |
| 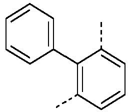 | 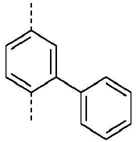 | 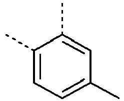 |
| Ar¹-13 | Ar¹-14 | Ar¹-15 |
| 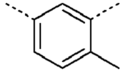 | 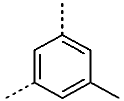 | 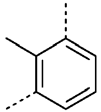 |
| Ar¹-16 | Ar¹-17 | Ar¹-18 |
| 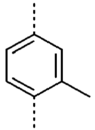 | 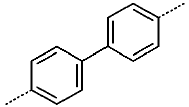 | 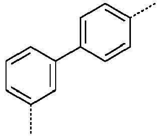 |
| Ar¹-19 | Ar¹-20 | Ar¹-21 |
| 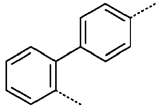 | 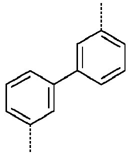 | 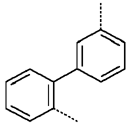 |
| Ar¹-22 | Ar¹-23 | Ar¹-24 |
| 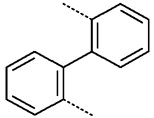 | 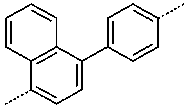 | 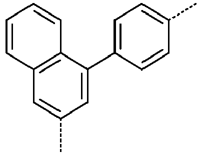 |
| Ar¹-25 | Ar¹-26 | Ar¹-27 |
| 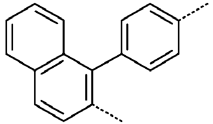 | 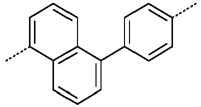 | 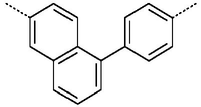 |
| Ar¹-28 | Ar¹-29 | Ar¹-30 |
| 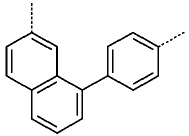 | 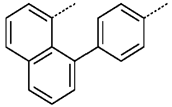 | 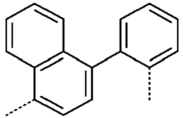 |
| Ar¹-31 | Ar¹-32 | Ar¹-33 |
| 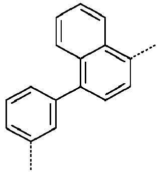 | 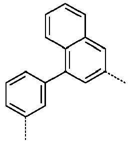 | 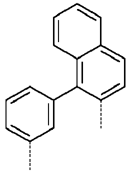 |
| Ar¹-34 | Ar¹-35 | Ar¹-36 |
| 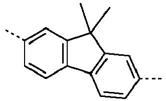 | 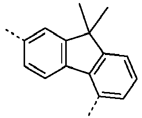 | 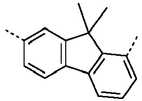 |
| Ar¹-37 | Ar¹-38 | Ar¹-39 |
| 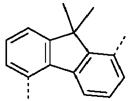 | | |
| Ar¹-40 | | |

HetAr¹ ist bevorzugt gewählt aus Dibenzofuran, Benzonaphthofuran, Dibenzothiophen, Benzonaphthothiophen, Carbazol, das über eines seiner C-Atome gebunden ist, Carbazol, das über sein N-Atom gebunden ist, Benzocarbazol, das über eines seiner C-Atome gebunden ist, und
Benzocarbazol, das über sein N-Atom gebunden ist, besonders bevorzugt Dibenzofuran, Dibenzothiophen und Carbazol, wobei Carbazol bevorzugt über eines seiner C-Atome gebunden ist, und wobei die genannten Gruppen mit einem oder mehreren Resten R⁵ substituiert sein können.

Die Gruppe -Ar¹-HetAr¹ in Formel (I-A-4) entspricht bevorzugt der folgenden Formel (H-1) oder (H-2) 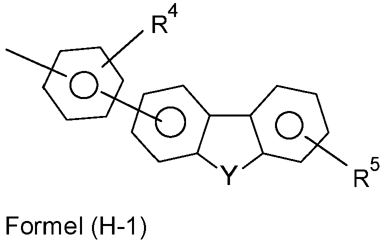 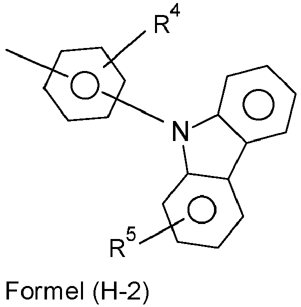 wobei Y gleich O, S oder NR⁵ ist, besonders bevorzugt gleich O, S, oder N-Ph, wobei Ph eine Phenylgruppe ist, die mit einem oder mehreren Resten R¹¹ substituiert sein kann; und
wobei R⁴ und R⁵ definiert sind wie oben, und bevorzugt H oder Phenyl sind, besonders bevorzugt H;
und wobei die Gruppe über die freie Bindung an das Stickstoffatom in Formel (I-A-4) gebunden ist.
R², R⁴, R⁵ sind bevorzugt bei jedem Auftreten gleich oder verschieden gewählt aus H, D, F, CN, Si(R¹¹)3, N(R¹¹)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R¹¹ substituiert sein können. Ganz besonders bevorzugt sind R², R⁴ bei jedem Auftreten gleich oder verschieden gewählt aus H, aromatischen Ringsystemen mit 6 bis 40 aromatischen Ringatomen, und heteroaromatischen Ringsystemen mit 5 bis 40 aromatischen Ringatomen, die jeweils mit einem oder mehreren Resten R¹¹ substituiert sein können Ganz besonders bevorzugt ist R⁵ bei jedem Auftreten gleich oder verschieden gewählt aus H und aromatischen Ringsystemen mit 6 bis 40 aromatischen Ringatomen, die mit einem oder mehreren Resten R¹¹ substituiert sein können. Ganz besonders bevorzugt sind R², R⁴, R⁵ bei jedem Auftreten gleich oder verschieden gewählt aus H und Phenyl, insbesondere H.
R¹¹ ist bevorzugt bei jedem Auftreten gleich oder verschieden gewählt aus H, D, F, CN, Si(R²¹)₃, N(R²¹)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R²¹ substituiert sein können.

Erfindungsgemäß sind die Fluorengruppen in Formel (I-A-4) in 4-Position an das N gebunden.

Dabei sind die Positionen an den Fluorengruppen wie folgt definiert: 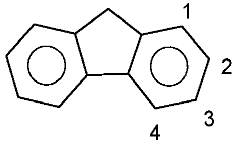 Weiterhin entspricht die Gruppe -Ar¹-HetAr¹ bevorzugt der Formel (H-1) oder (H-2), besonders bevorzugt der Formel (H-1).

Die folgenden Verbindungen sind bevorzugte Ausführungsformen der Formel (I-A-4)

| | | |
|---|---|---|
| 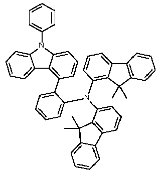 | 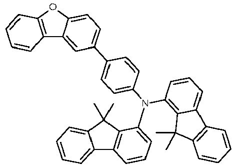 | 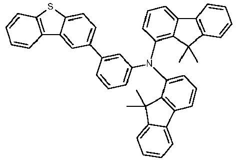 |
| #1 | #2 | #3 |
| 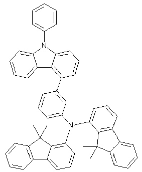 | 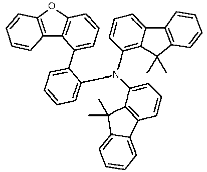 | 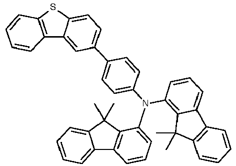 |
| #4 | #5 | #6 |
| 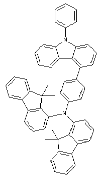 | 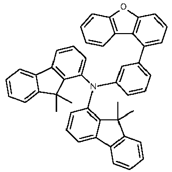 | 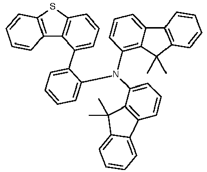 |
| #7 | #8 | #9 |
| 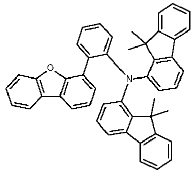 | 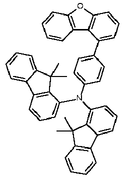 | 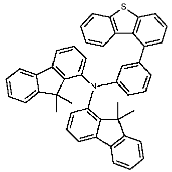 |
| #10 | #11 | #12 |
| 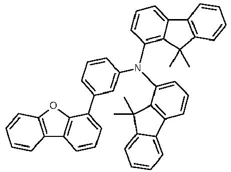 | 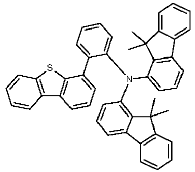 | 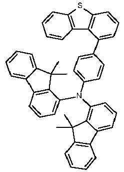 |
| #13 | #14 | #15 |
| 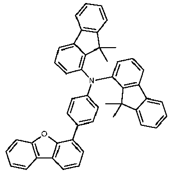 | 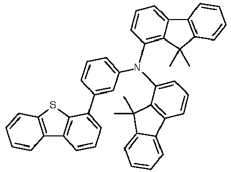 | 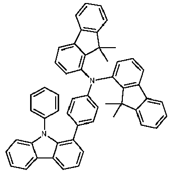 |
| #16 | #17 | #18 |
| 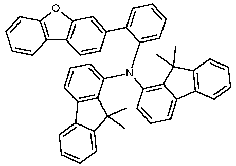 | 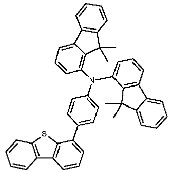 | 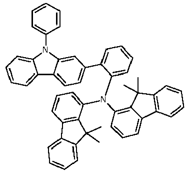 |
| #19 | #20 | #21 |
| 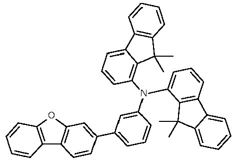 | 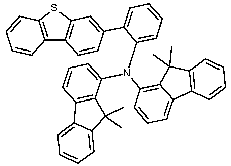 | 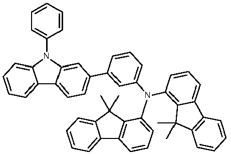 |
| #22 | #23 | #24 |
| 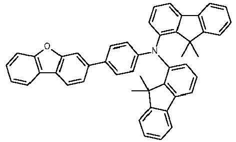 | 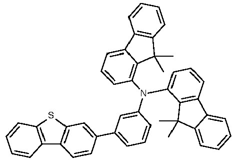 | 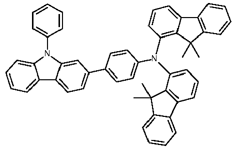 |
| #25 | #26 | #27 |
| 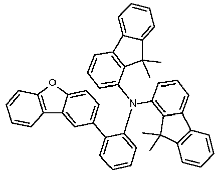 | 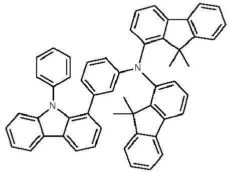 | 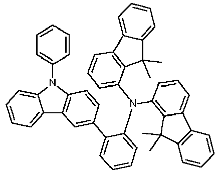 |
| #28 | #29 | #30 |
| 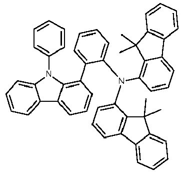 | 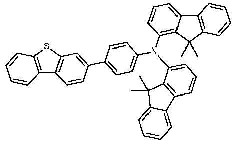 | 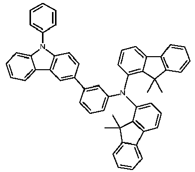 |
| #31 | #32 | #33 |
| 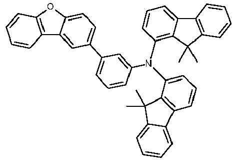 | 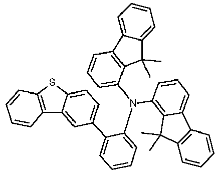 | 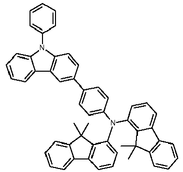 |
| #34 | #35 | #36 |
| 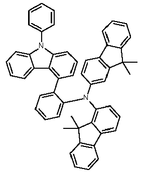 | 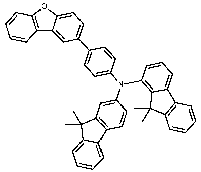 | 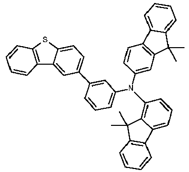 |
| #37 | #38 | #39 |
| 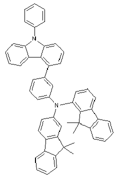 | 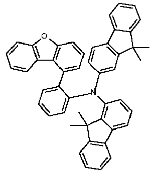 | 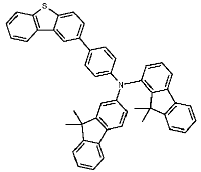 |
| #40 | #41 | #42 |
| 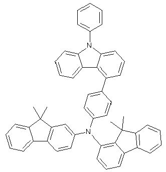 | 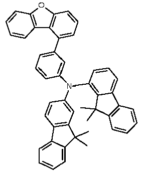 | 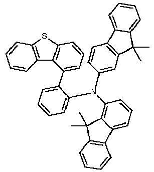 |
| #43 | #44 | #45 |
| 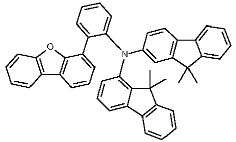 | 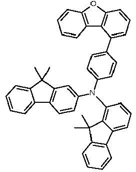 | 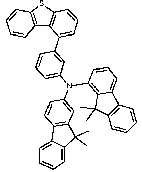 |
| #46 | #47 | #48 |
| 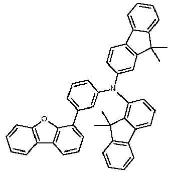 | 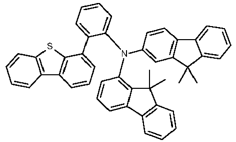 | 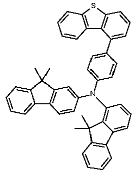 |
| #49 | #50 | #51 |
| 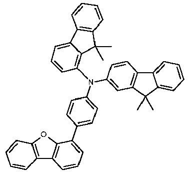 | 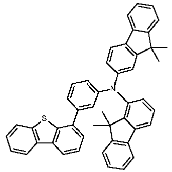 | 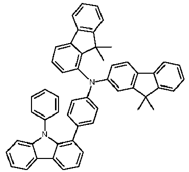 |
| #52 | #53 | #54 |
| 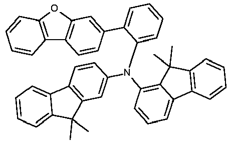 | 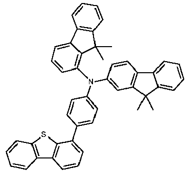 | 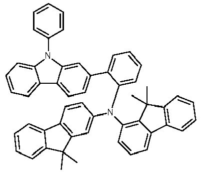 |
| #55 | #56 | #57 |
| 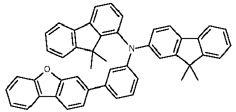 | 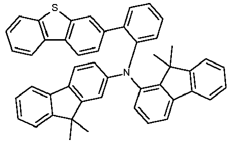 | 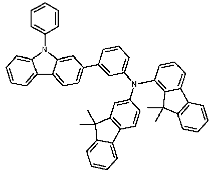 |
| #58 | #59 | #60 |
| 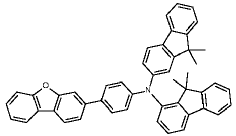 | 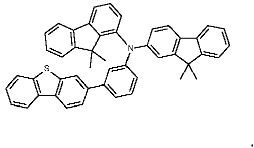 | 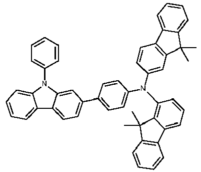 |
| #61 | #62 | #63 |
| 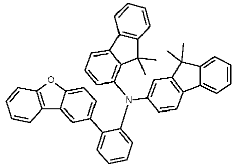 | 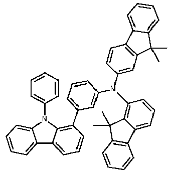 | 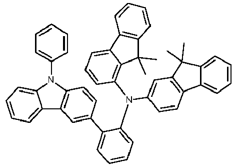 |
| #64 | #65 | #66 |
| 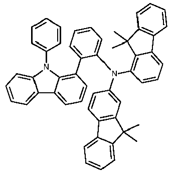 | 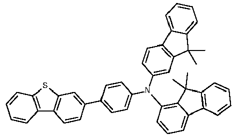 | 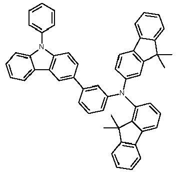 |
| #67 | #68 | #69 |
| 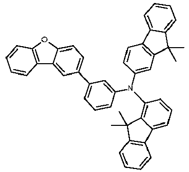 | 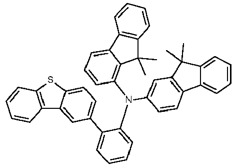 | 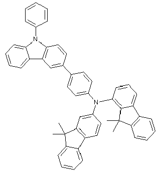 |
| #70 | #71 | #72 |
| 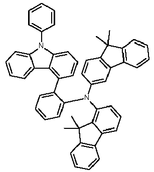 | 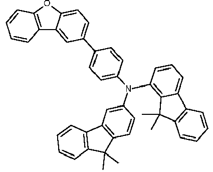 | 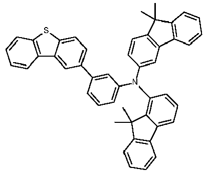 |
| #73 | #74 | #75 |
| 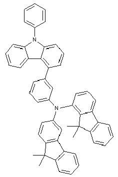 | 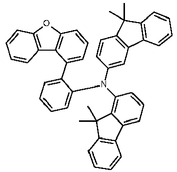 | 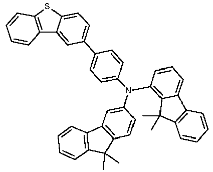 |
| #76 | #77 | #78 |
| 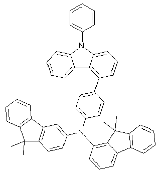 | 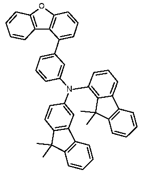 | 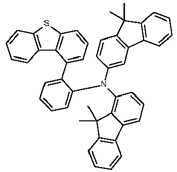 |
| #79 | #80 | #81 |
| 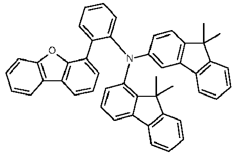 | 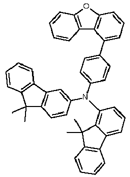 | 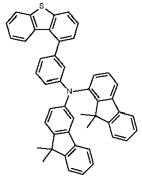 |
| #82 | #83 | #84 |
| 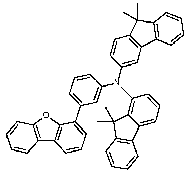 | 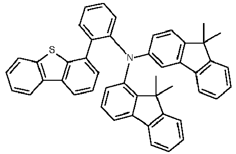 | 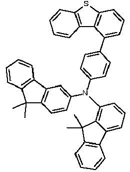 |
| #85 | #86 | #87 |
| 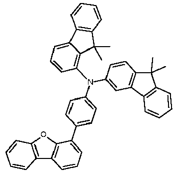 | 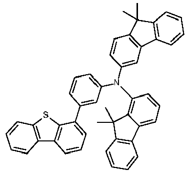 | 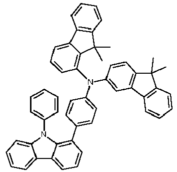 |
| #88 | #89 | ##90 |
| 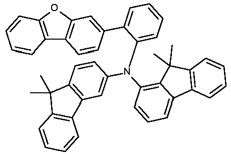 | 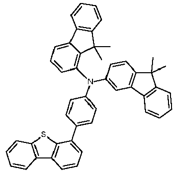 | 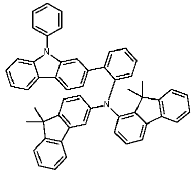 |
| #91 | #92 | #93 |
| 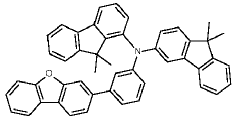 | 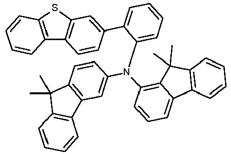 | 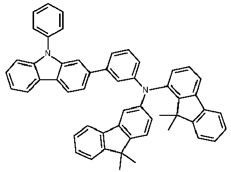 |
| #94 | #95 | #96 |
| 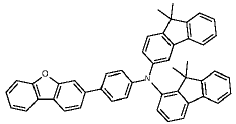 | 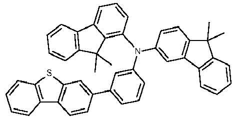 | 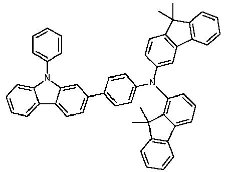 |
| #97 | #98 | #99 |
| 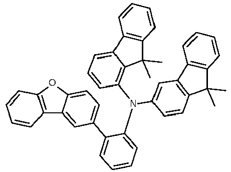 | 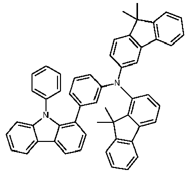 | 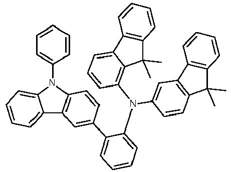 |
| #100 | #101 | #102 |
| 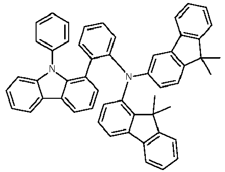 | 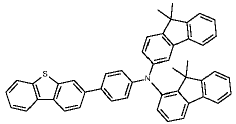 | 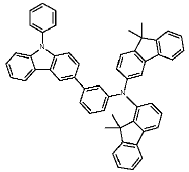 |
| #103 | #104 | #105 |
| 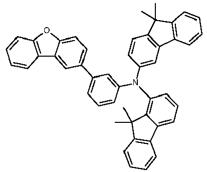 | 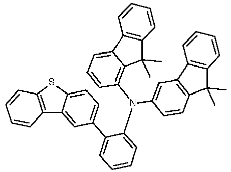 | 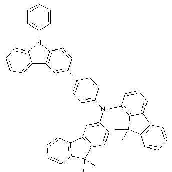 |
| #106 | #107 | #108 |
| 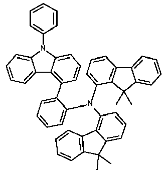 | 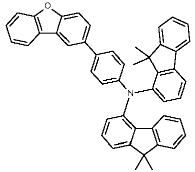 | 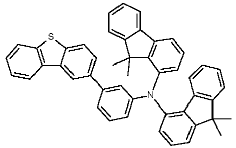 |
| #109 | #110 | #111 |
| 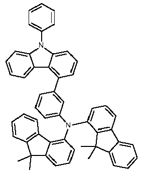 | 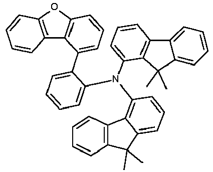 | 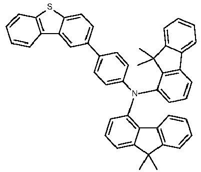 |
| #112 | #113 | #114 |
| 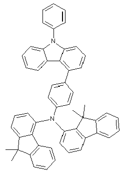 | 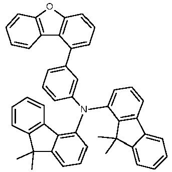 | 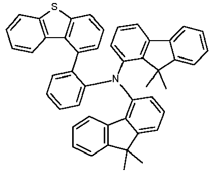 |
| #115 | #116 | #117 |
| 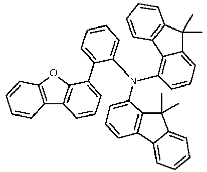 | 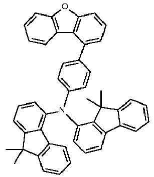 | 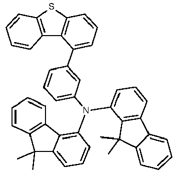 |
| #118 | #119 | #120 |
| 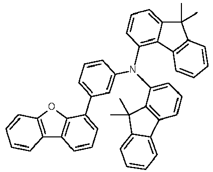 | 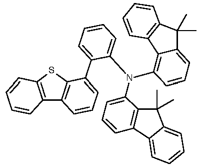 | 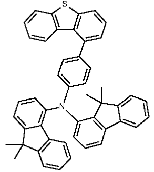 |
| #121 | #122 | #123 |
| 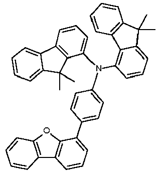 | 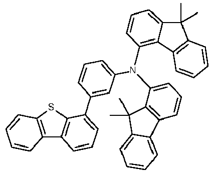 | 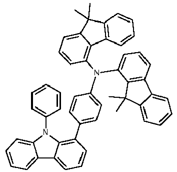 |
| #124 | #125 | #126 |
| 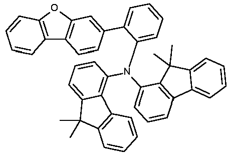 | 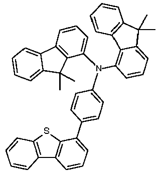 | 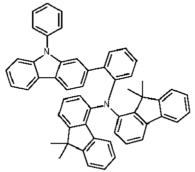 |
| #127 | #128 | #129 |
| 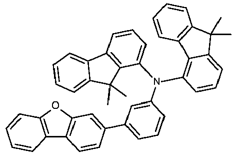 | 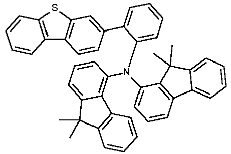 | 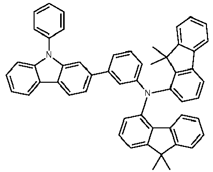 |
| #130 | #131 | #132 |
| 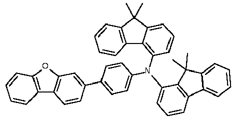 | 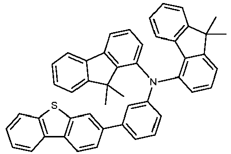 | 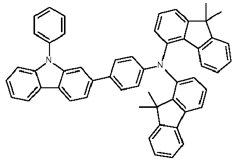 |
| #133 | #134 | #135 |
| 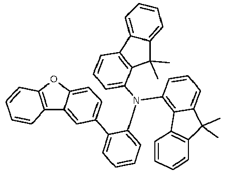 | 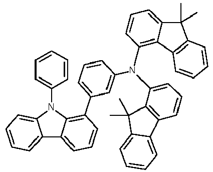 | 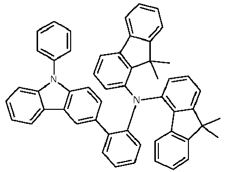 |
| #136 | #137 | #138 |
| 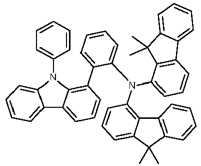 | 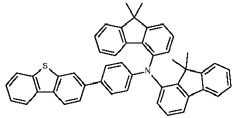 | 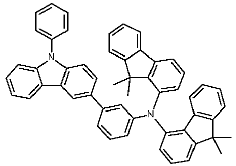 |
| #139 | #140 | #141 |
| 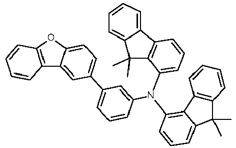 | 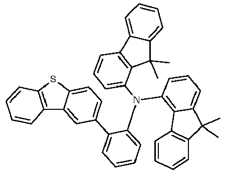 | 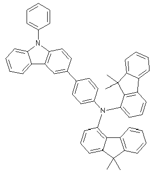 |
| #142 | #143 | #144 |
| 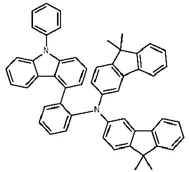 | 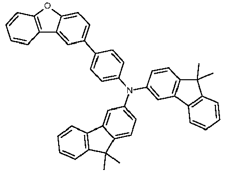 | 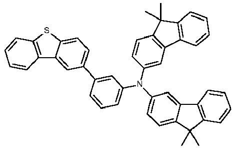 |
| #145 | #146 | #147 |
| 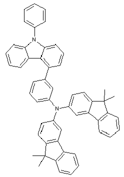 | 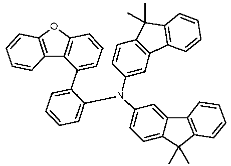 | 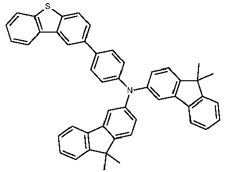 |
| #148 | #149 | #150 |
| 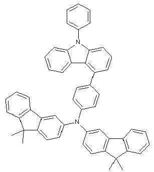 | 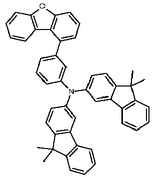 | 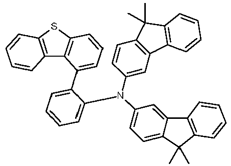 |
| #151 | #152 | #153 |
| 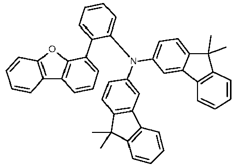 | 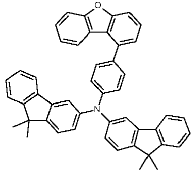 | 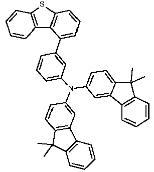 |
| #154 | #155 | #156 |
| 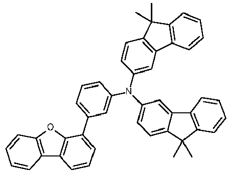 | 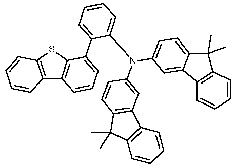 | 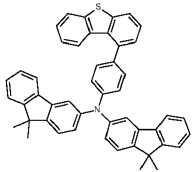 |
| #157 | #158 | #159 |
| 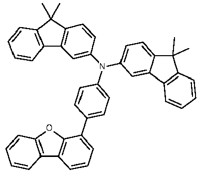 | 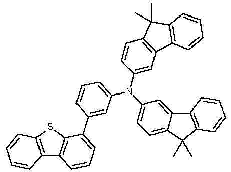 | 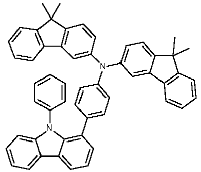 |
| #160 | #161 | #162 |
| 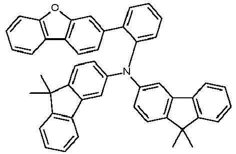 | 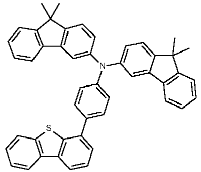 | 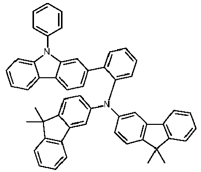 |
| #163 | #164 | #165 |
| 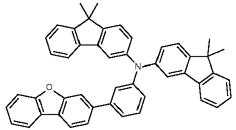 | 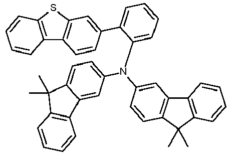 | 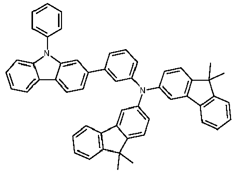 |
| #166 | #167 | #168 |
| 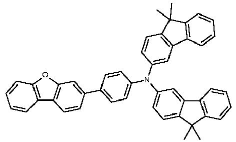 | 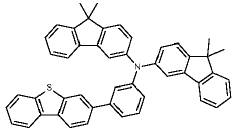 | 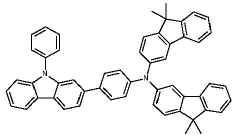 |
| #169 | #170 | #171 |
| 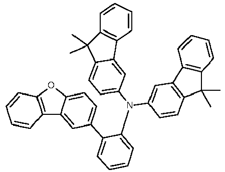 | 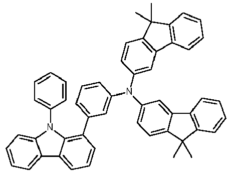 | 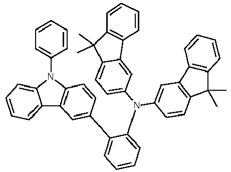 |
| #172 | #173 | #174 |
| 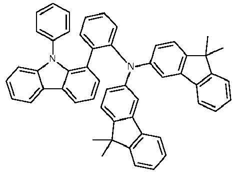 | 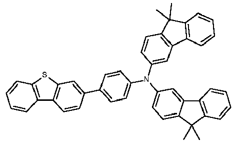 | 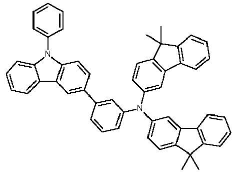 |
| #175 | #176 | #177 |
| 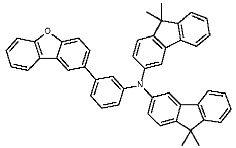 | 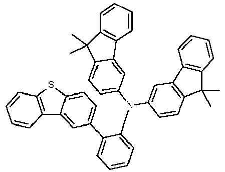 | 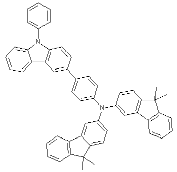 |
| #178 | #179 | #180 |
| 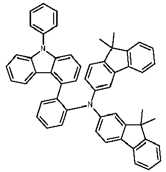 | 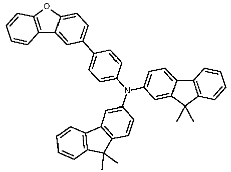 | 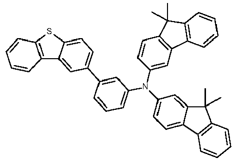 |
| #181 | #182 | #183 |
| 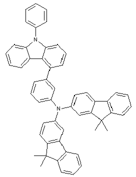 | 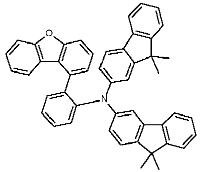 | 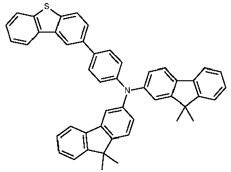 |
| #184 | #185 | #186 |
| 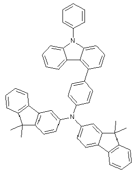 | 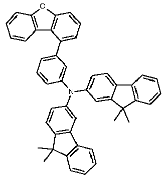 | 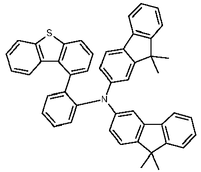 |
| #187 | #188 | #189 |
| 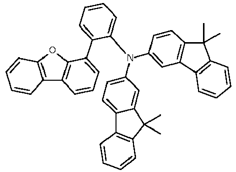 | 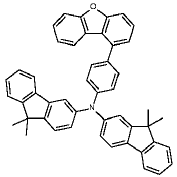 | 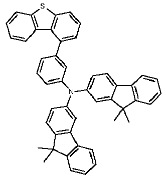 |
| #190 | #191 | #192 |
| 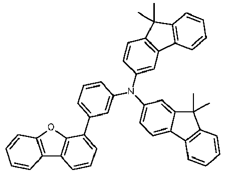 | 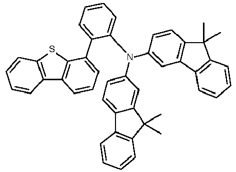 | 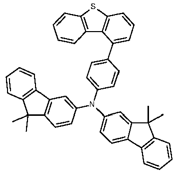 |
| #193 | #194 | #195 |
| 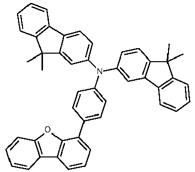 | 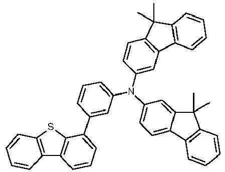 | 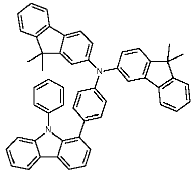 |
| #196 | #197 | #198 |
| 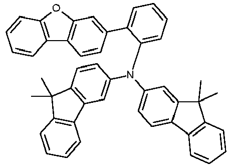 | 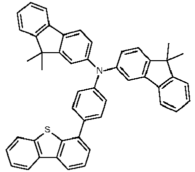 | 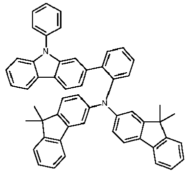 |
| #199 | #200 | #201 |
| 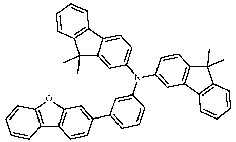 | 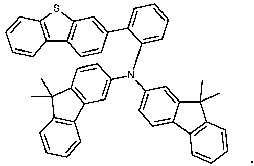 | 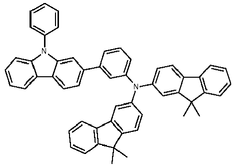 |
| #202 | #203 | #204 |
| 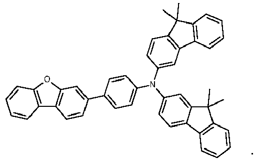 | 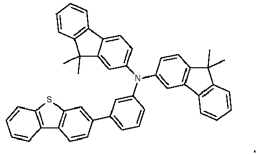 | 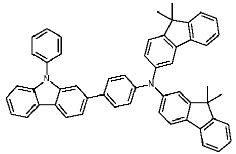 |
| #205 | #206 | #207 |
| 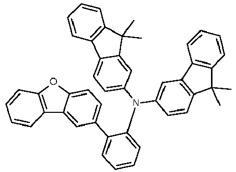 | 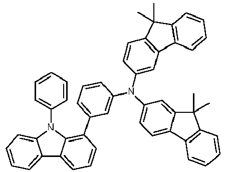 | 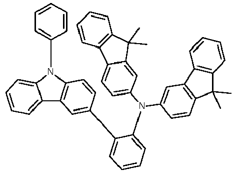 |
| #208 | #209 | #210 |
| 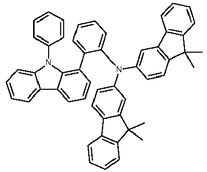 | 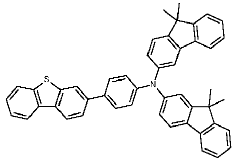 | 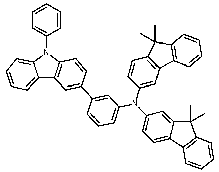 |
| #211 | #212 | #213 |
| 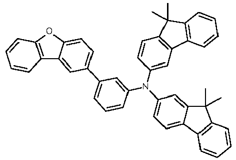 | 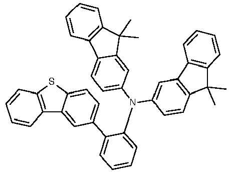 | 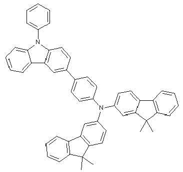 |
| #214 | #215 | #216 |
| 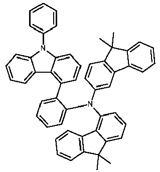 | 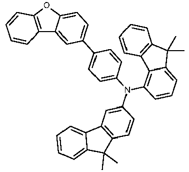 | 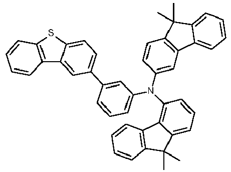 |
| #217 | #218 | #219 |
| 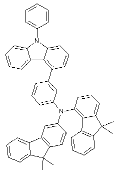 | 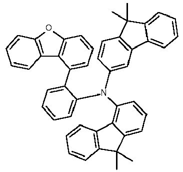 | 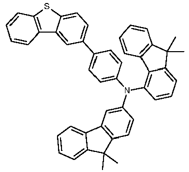 |
| #220 | #221 | #222 |
| 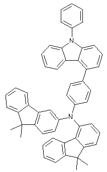 | 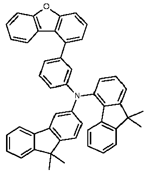 | 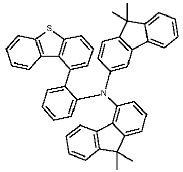 |
| #223 | #224 | #225 |
| 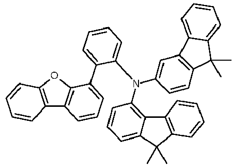 | 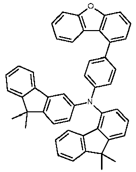 | 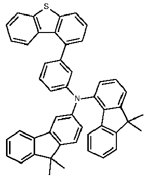 |
| #226 | #227 | #228 |
| 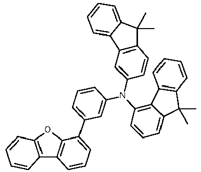 | 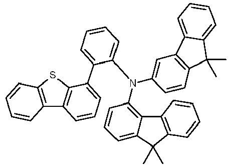 | 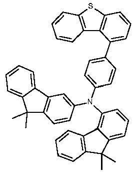 |
| #229 | #230 | #231 |
| 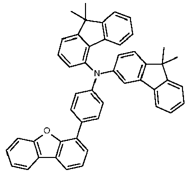 | 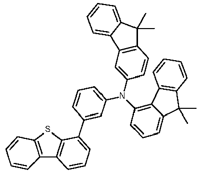 | 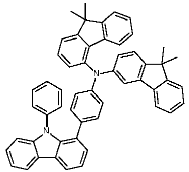 |
| #232 | #233 | #234 |
| 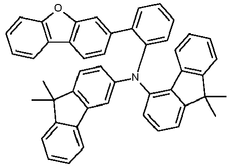 | 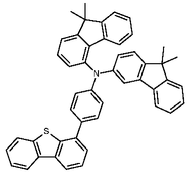 | 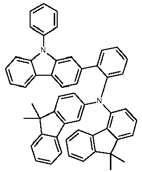 |
| #235 | #236 | #237 |
| 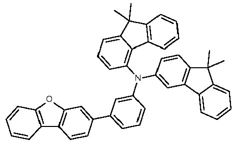 | 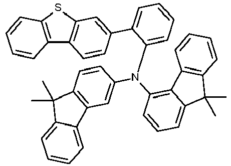 | 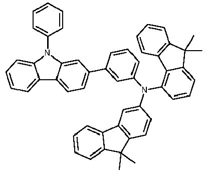 |
| #238 | #239 | #240 |
| 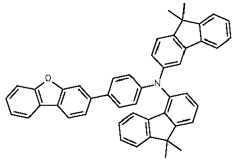 | 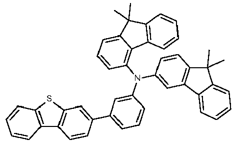 | 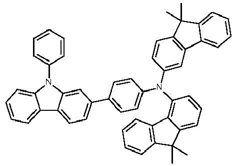 |
| #241 | #242 | #243 |
| 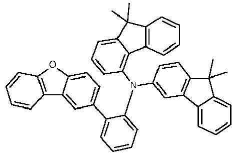 | 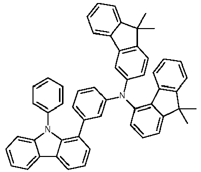 | 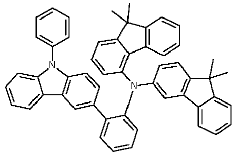 |
| #244 | #245 | #246 |
| 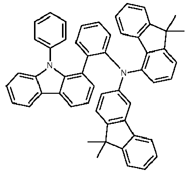 | 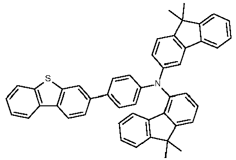 | 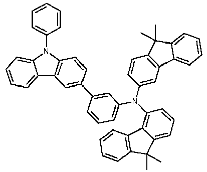 |
| #247 | #248 | #249 |
| 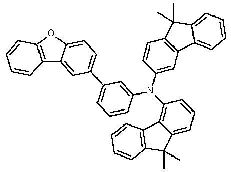 | 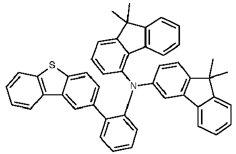 | 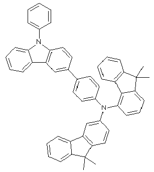 |
| #250 | #251 | #252 |
| 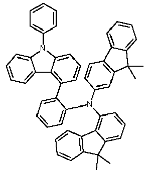 | 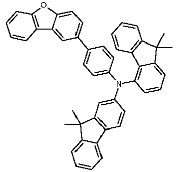 | 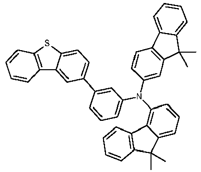 |
| #253 | #254 | #255 |
| 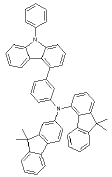 | 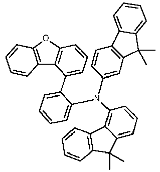 | 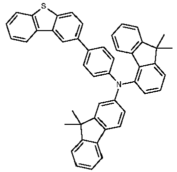 |
| #256 | #257 | #258 |
| 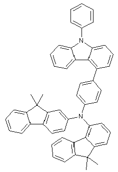 | 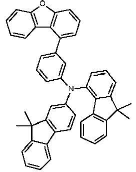 | 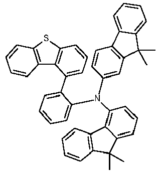 |
| #259 | #260 | #261 |
| 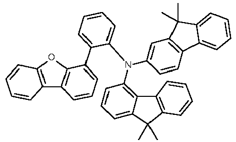 | 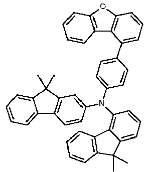 | 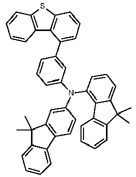 |
| #262 | #263 | #264 |
| 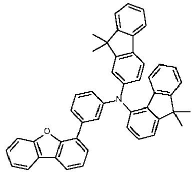 | 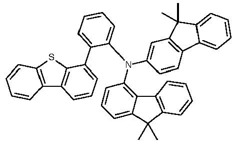 | 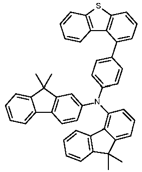 |
| #265 | #266 | #267 |
| 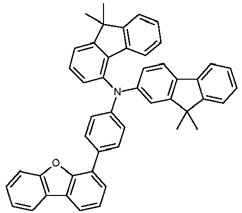 | 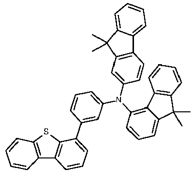 | 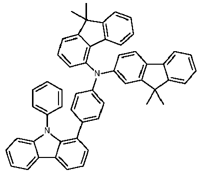 |
| #268 | #269 | #270 |
| 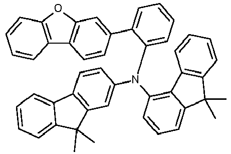 | 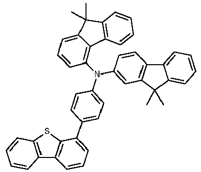 | 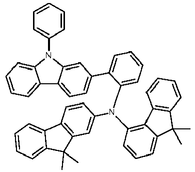 |
| #271 | #272 | #273 |
| 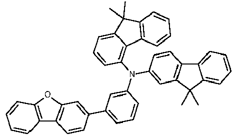 | 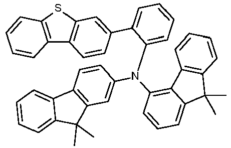 | 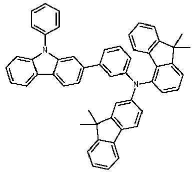 |
| #274 | #275 | #276 |
| 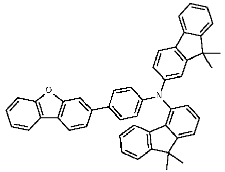 | 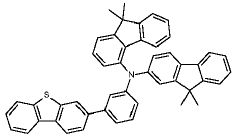 | 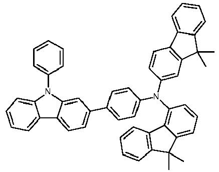 |
| #277 | #278 | #279 |
| 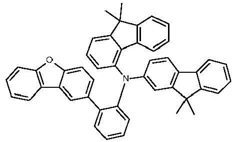 | 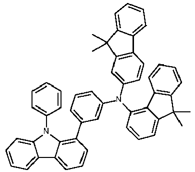 | 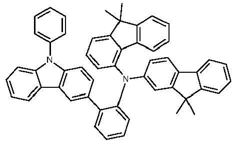 |
| #280 | #281 | #282 |
| 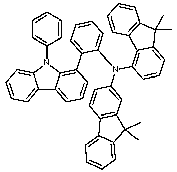 | 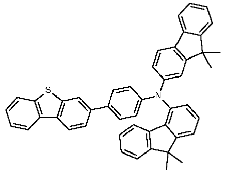 | 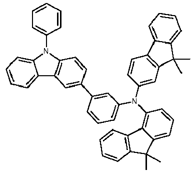 |
| #283 | #284 | #285 |
| 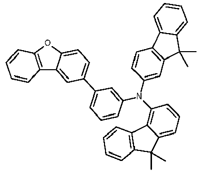 | 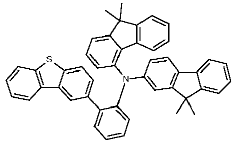 | 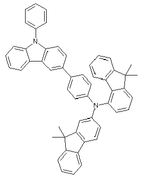 |
| #286 | #287 | #288 |
| 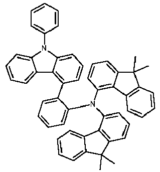 | 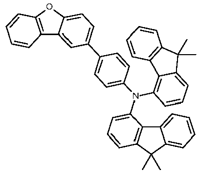 | 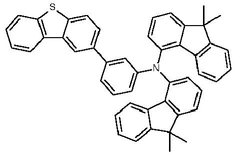 |
| 289 | 290 | 291 |
| 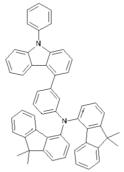 | 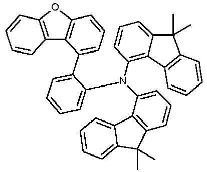 | 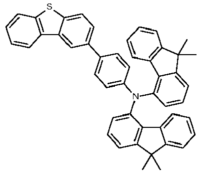 |
| 292 | 293 | 294 |
| 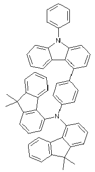 | 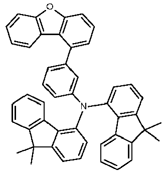 | 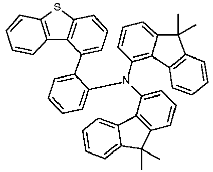 |
| 295 | 296 | 297 |
| 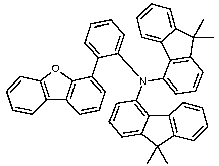 | 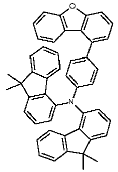 | 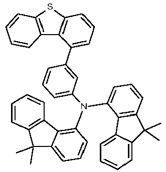 |
| 298 | 299 | 300 |
| 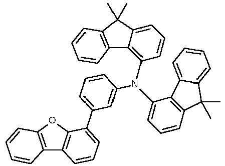 | 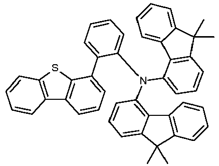 | 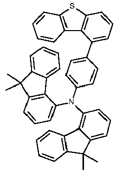 |
| 301 | 302 | 303 |
| 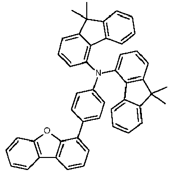 | 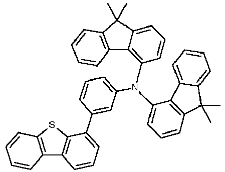 | 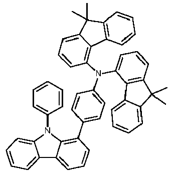 |
| 304 | 305 | 306 |
| 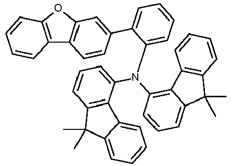 | 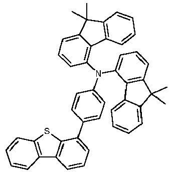 | 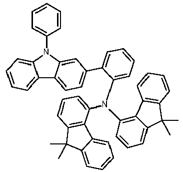 |
| 307 | 308 | 309 |
| 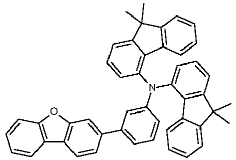 | 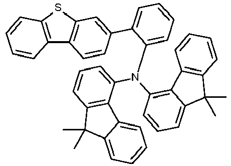 | 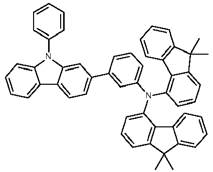 |
| 310 | 311 | 312 |
| 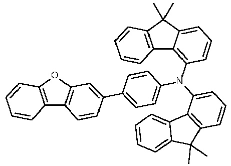 | 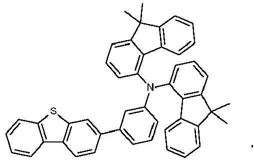 | 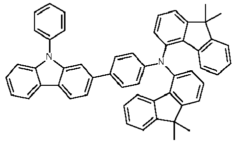 |
| 313 | 314 | 315 |
| 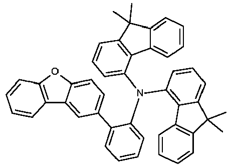 | 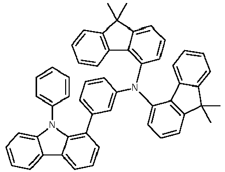 | 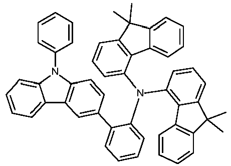 |
| 316 | 317 | 318 |
| 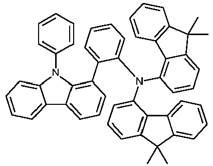 | 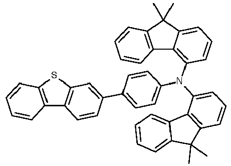 | 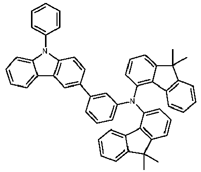 |
| 319 | 320 | 321 |
| 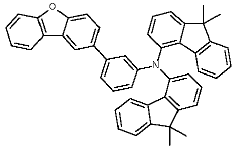 | 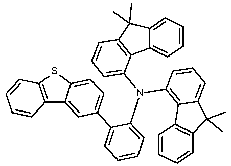 | 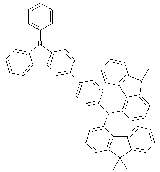 |
| 322 | 323 | 324 |
| 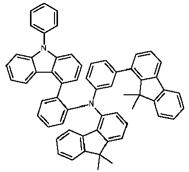 | 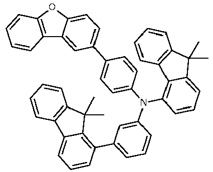 | 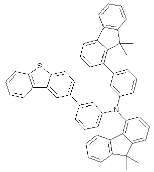 |
| #325 | #326 | #327 |
| 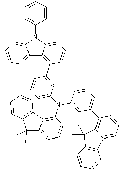 | 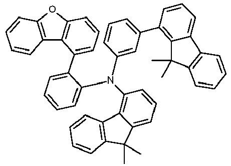 | 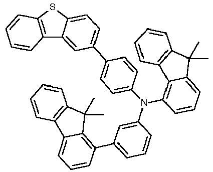 |
| #328 | #329 | #330 |
| 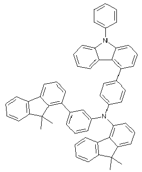 | 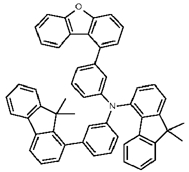 | 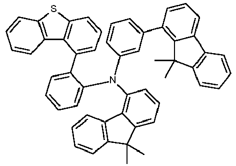 |
| #331 | #332 | #333 |
| 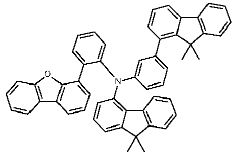 | 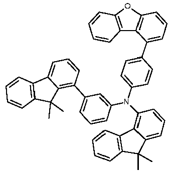 | 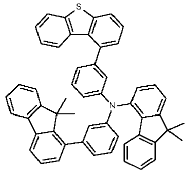 |
| #334 | #335 | #336 |
| 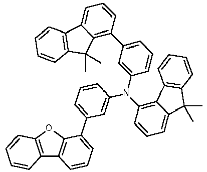 | 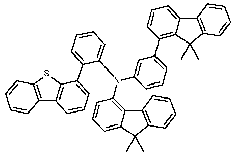 | 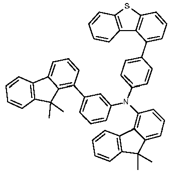 |
| #337 | #338 | #339 |
| 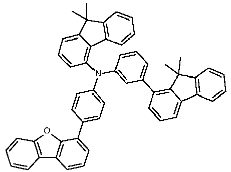 | 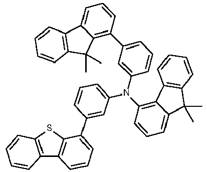 | 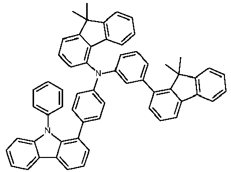 |
| #340 | #341 | #342 |
| 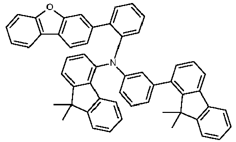 | 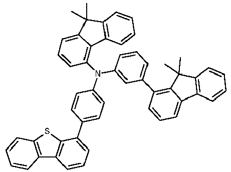 | 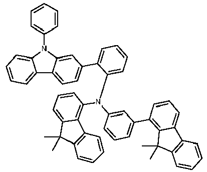 |
| #343 | #344 | #345 |
| 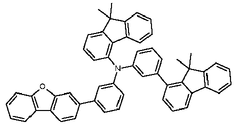 | 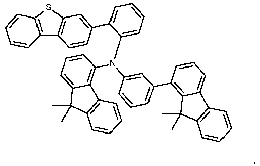 | 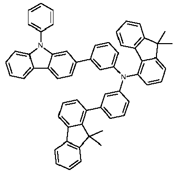 |
| #346 | #347 | #348 |
| 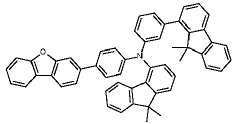 | 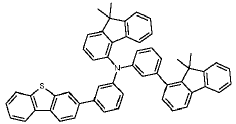 | 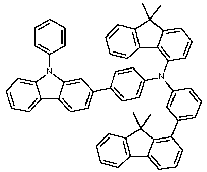 |
| #349 | #350 | #351 |
| 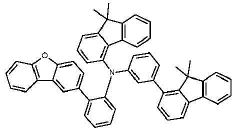 | 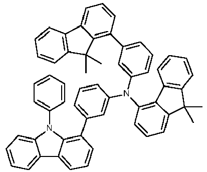 | 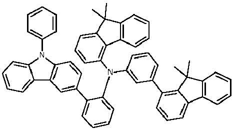 |
| #352 | #353 | #354 |
| 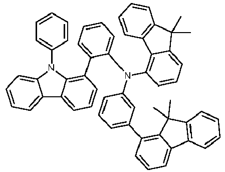 | 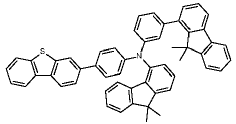 | 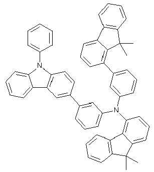 |
| #355 | #356 | #357 |
| 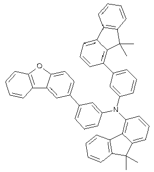 | 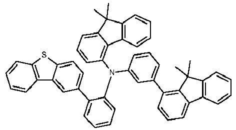 | 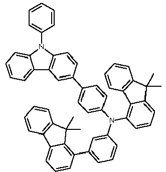 |
| #358 | #359 | #360 |
| 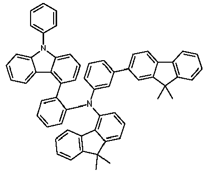 | 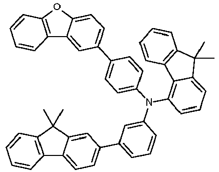 | 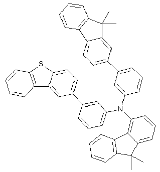 |
| #361 | #362 | #363 |
| 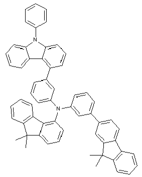 | 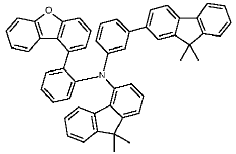 | 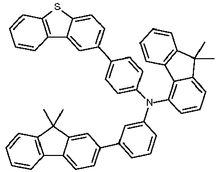 |
| #364 | #365 | #366 |
| 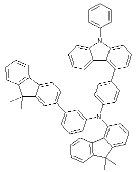 | 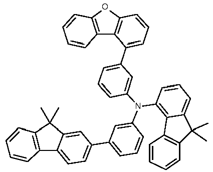 | 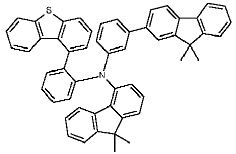 |
| #367 | #368 | #369 |
| 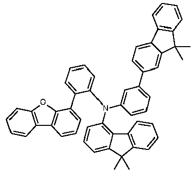 | 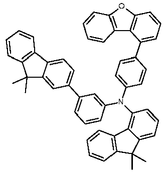 | 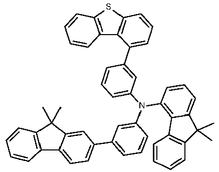 |
| #370 | #371 | #372 |
| 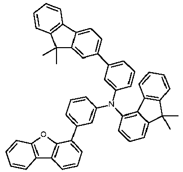 | 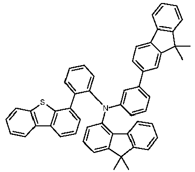 | 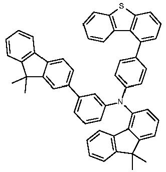 |
| #373 | #374 | #375 |
| 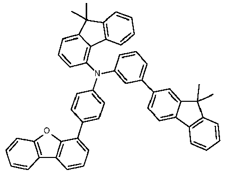 | 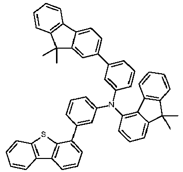 | 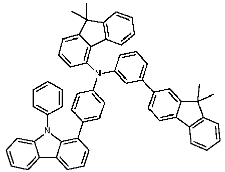 |
| #376 | #377 | #378 |
| 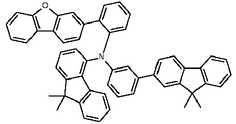 | 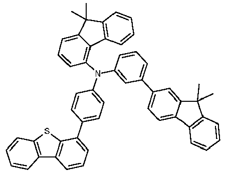 | 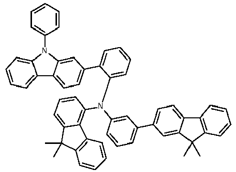 |
| #379 | #380 | #381 |
| 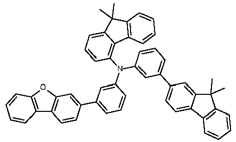 | 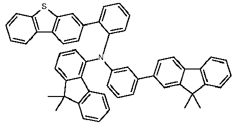 | 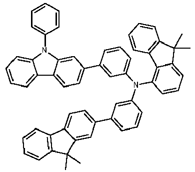 |
| #382 | #383 | #384 |
| 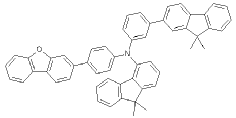 | 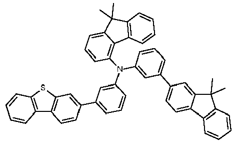 | 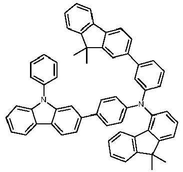 |
| #385 | #386 | #387 |
| 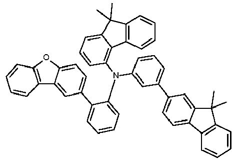 | 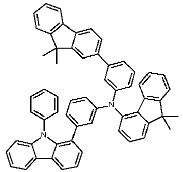 | 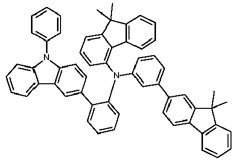 |
| #388 | #389 | #390 |
| 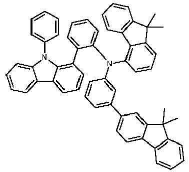 | 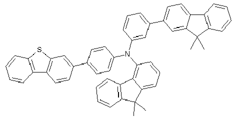 | 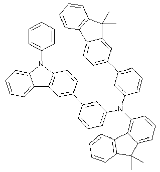 |
| #391 | #392 | #393 |
| 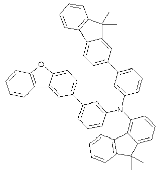 | 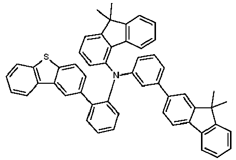 | 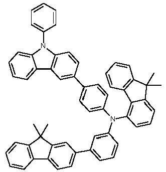 |
| #394 | #395 | #396 |
| 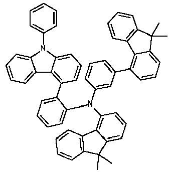 | 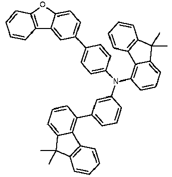 | 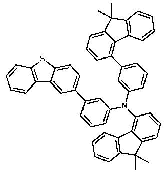 |
| #397 | #398 | #399 |
| 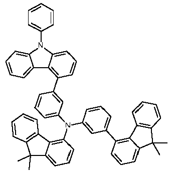 | 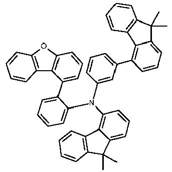 | 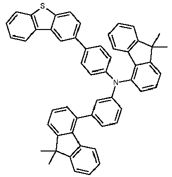 |
| #400 | #401 | #402 |
| 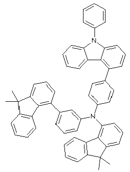 | 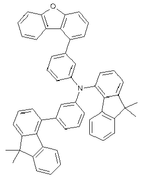 | 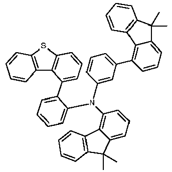 |
| #403 | #404 | #405 |
| 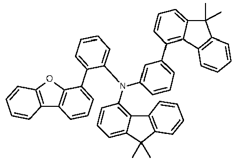 | 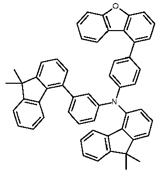 | 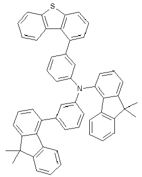 |
| #406 | #407 | #408 |
| 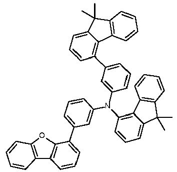 | 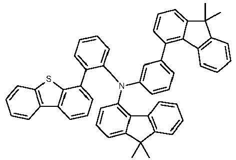 | 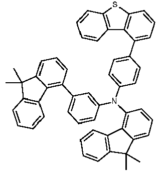 |
| #409 | #410 | #411 |
| 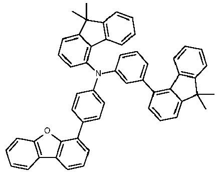 | 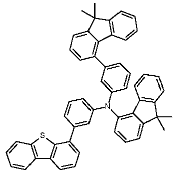 | 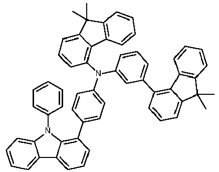 |
| #412 | #413 | #414 |
| 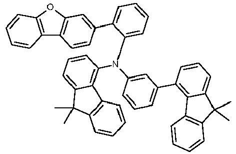 | 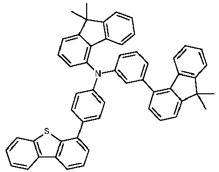 | 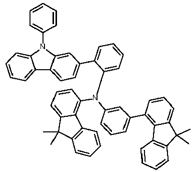 |
| #415 | #416 | #417 |
| 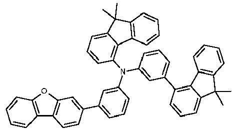 | 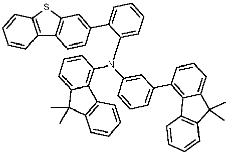 | 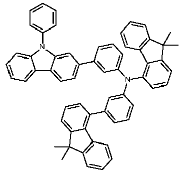 |
| #418 | #419 | #420 |
| 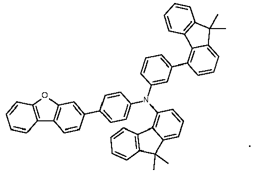 | 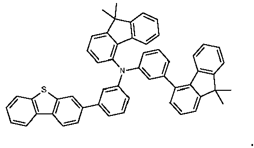 | 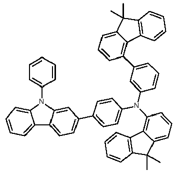 |
| #421 | #422 | #423 |
| 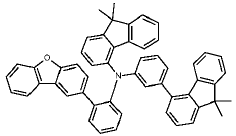 | 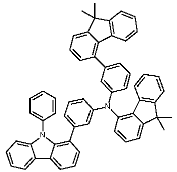 | 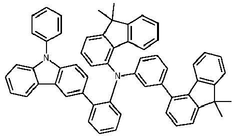 |
| #424 | #425 | #426 |
| 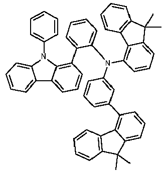 | 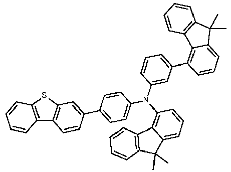 | 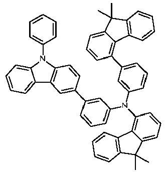 |
| #427 | #428 | #429 |
| 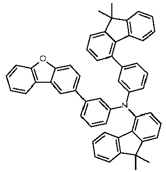 | 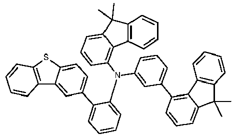 | 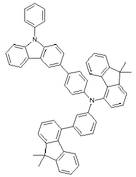 |
| #430 | #431 | #432 |
| 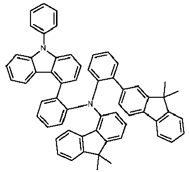 | 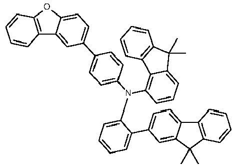 | 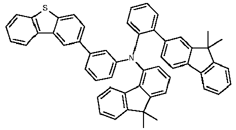 |
| #433 | #434 | #435 |
| 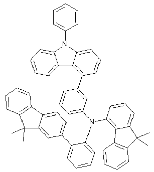 | 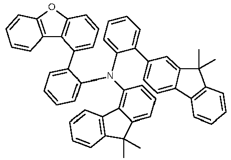 | 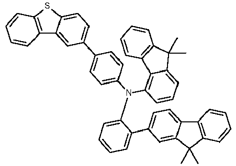 |
| #436 | #437 | #438 |
| 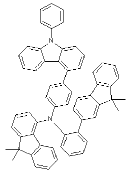 | 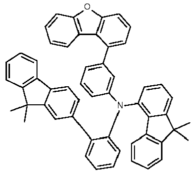 | 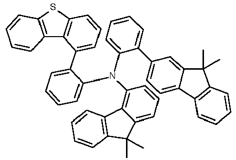 |
| #439 | #440 | #441 |
| 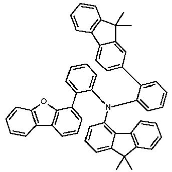 | 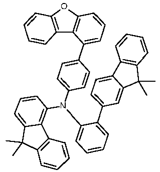 | 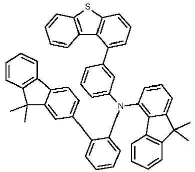 |
| #442 | #443 | #444 |
| 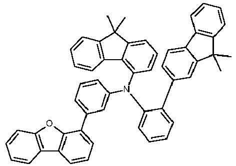 | 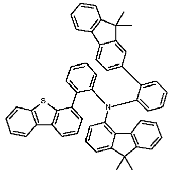 | 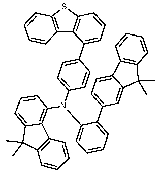 |
| #445 | #446 | #447 |
| 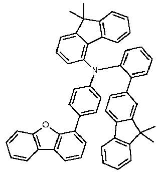 | 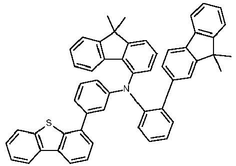 | 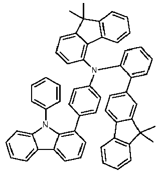 |
| #448 | #449 | #450 |
| 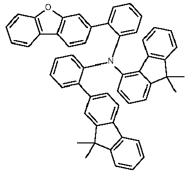 | 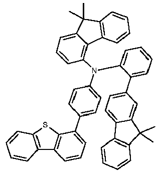 | 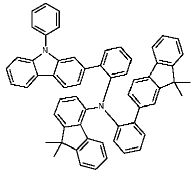 |
| #451 | #452 | #453 |
| 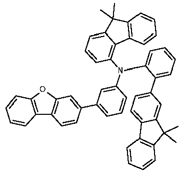 | 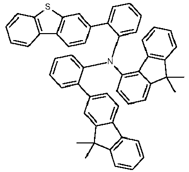 | 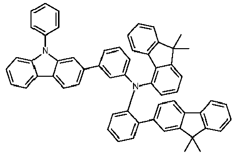 |
| #454 | #455 | #456 |
| 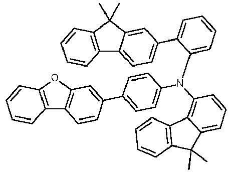 | 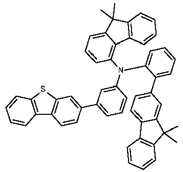 | 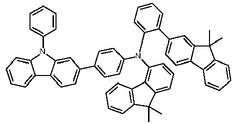 |
| #457 | #458 | #459 |
| 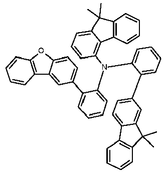 | 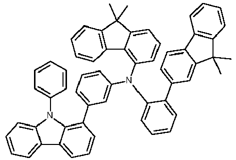 | 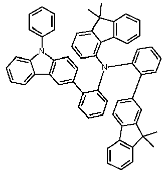 |
| #460 | #461 | #462 |
| 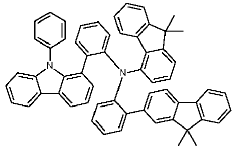 | 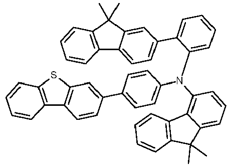 | 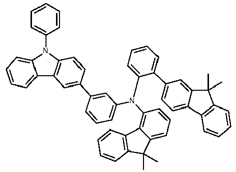 |
| #463 | #464 | #465 |
| 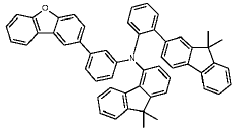 | 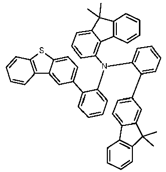 | 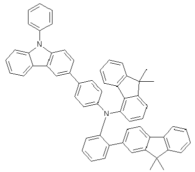 |
| #466 | #467 | #468 |
| 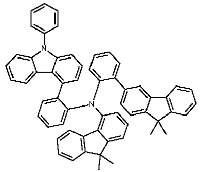 | 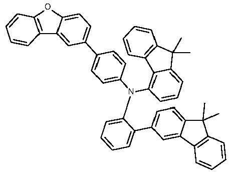 | 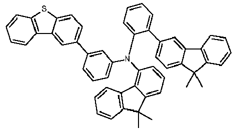 |
| #469 | #470 | #471 |
| 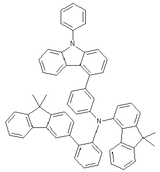 | 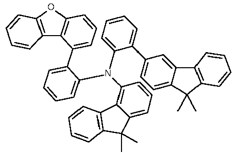 | 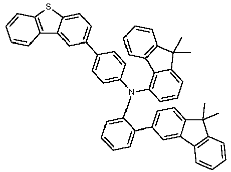 |
| #472 | #473 | #474 |
| 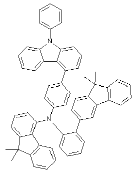 | 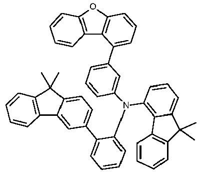 | 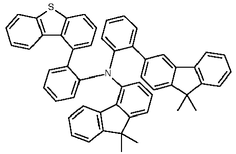 |
| #475 | #476 | #477 |
| 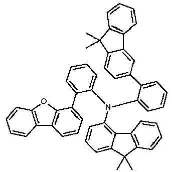 | 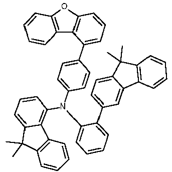 | 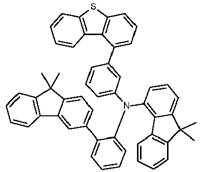 |
| #478 | #479 | #480 |
| 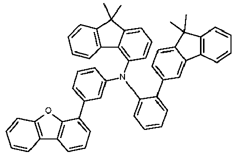 | 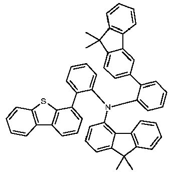 | 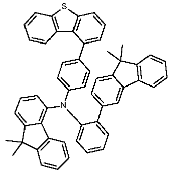 |
| #481 | #482 | #483 |
| 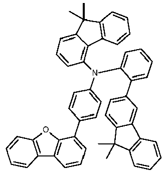 | 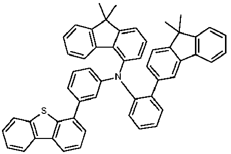 | 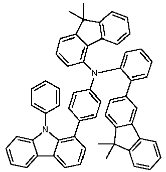 |
| #484 | #485 | #486 |
| 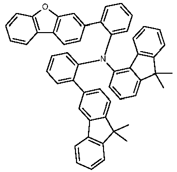 | 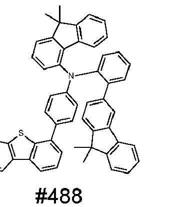 | 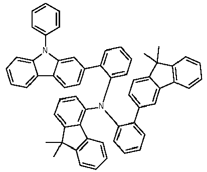 |
| #487 | #488 | #489 |
| 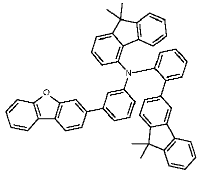 | 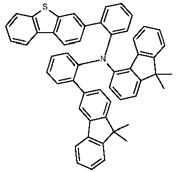 | 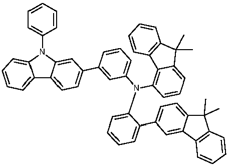 |
| #490 | #491 | #492 |
| 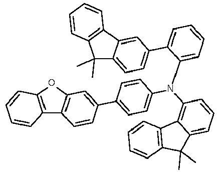 | 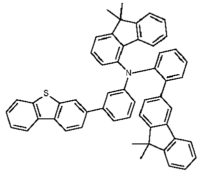 | 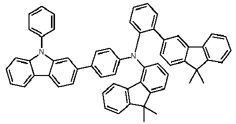 |
| #493 | #494 | #495 |
| 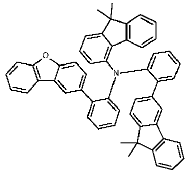 | 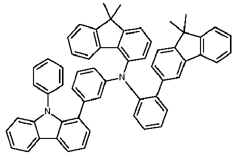 | 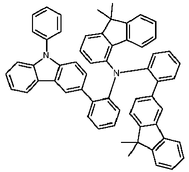 |
| #496 | #497 | #498 |
| 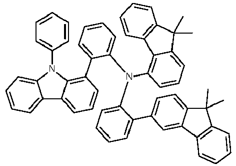 | 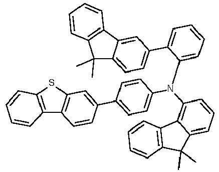 | 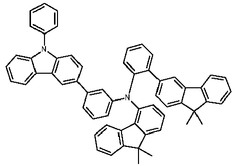 |
| #499 | #500 | #501 |
| 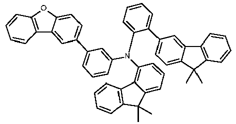 | 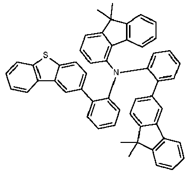 | 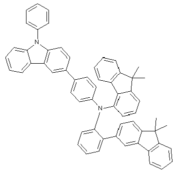 |
| #502 | #503 | #504 |
| 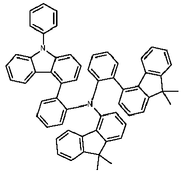 | 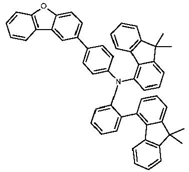 | 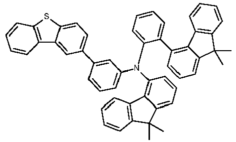 |
| #505 | #506 | #507 |
| 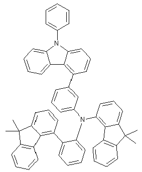 | 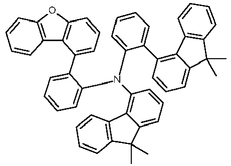 | 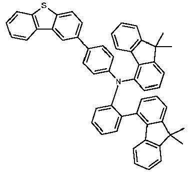 |
| #508 | #509 | #510 |
| 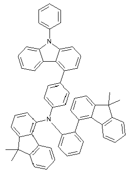 | 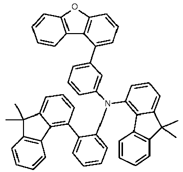 | 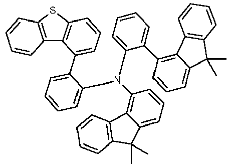 |
| #511 | #512 | #513 |
| 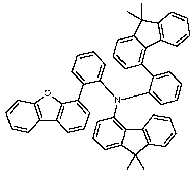 | 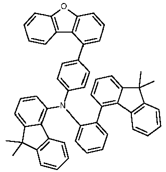 | 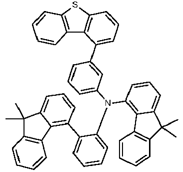 |
| #514 | #515 | #516 |
| 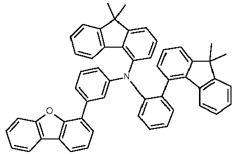 | 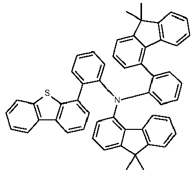 | 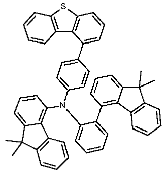 |
| #517 | #518 | #519 |
| 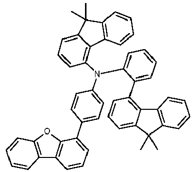 | 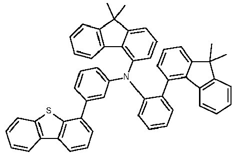 | 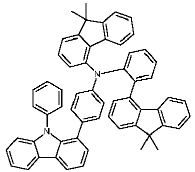 |
| #520 | #521 | #522 |
| 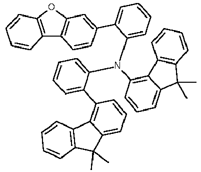 | 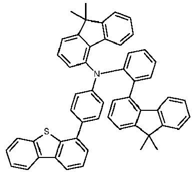 | 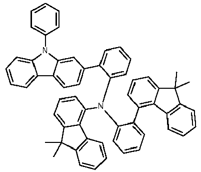 |
| #523 | #524 | #525 |
| 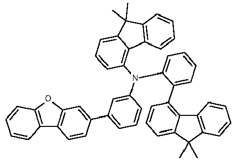 | 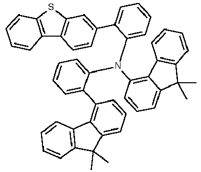 | 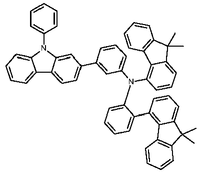 |
| #526 | #527 | #528 |
| 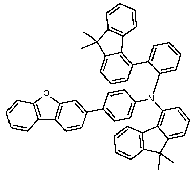 | 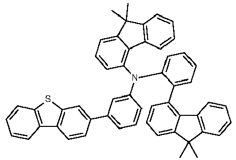 | 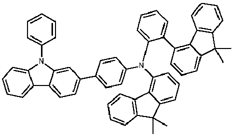 |
| #529 | #530 | #531 |
| 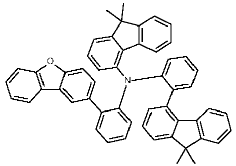 | 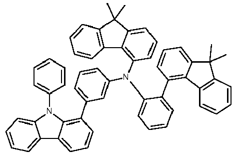 | 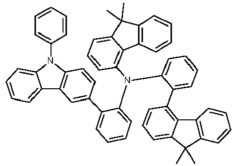 |
| #532 | #533 | #534 |
| 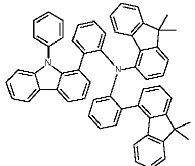 | 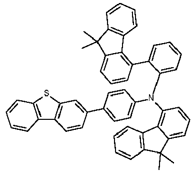 | 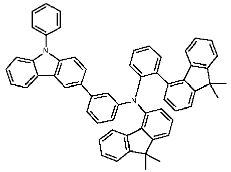 |
| #535 | #536 | #537 |
| 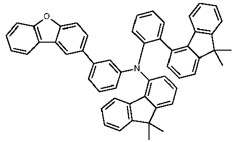 | 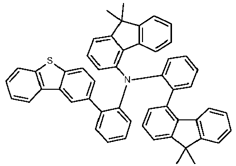 | 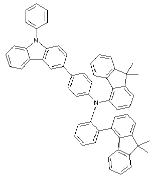 |
| #538 | #539 | #540 |
| 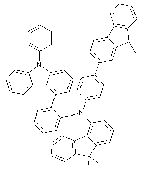 | 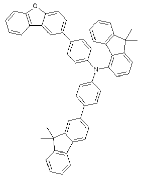 | 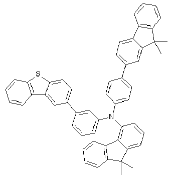 |
| #541 | #542 | #543 |
| 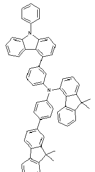 | 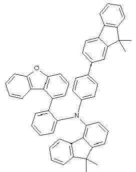 | 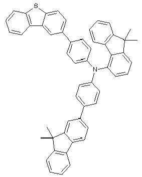 |
| #544 | #545 | #546 |
| 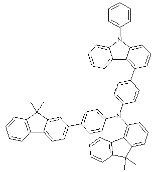 | 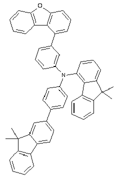 | 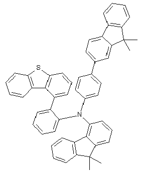 |
| #547 | #548 | #549 |
| 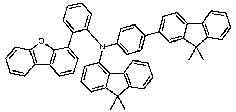 | 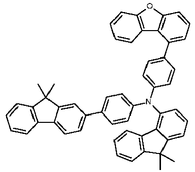 | 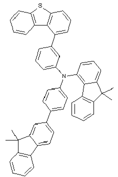 |
| #550 | #551 | #552 |
| 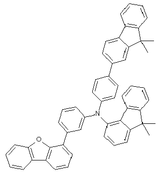 | 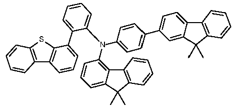 | 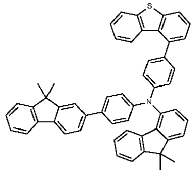 |
| #553 | #554 | #555 |
| 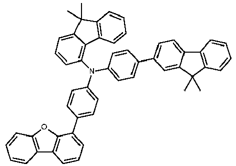 | 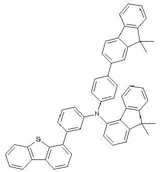 | 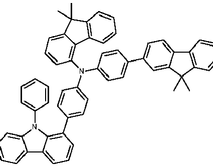 |
| #556 | #557 | #558 |
| 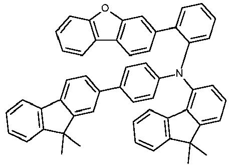 | 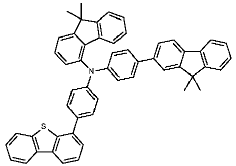 | 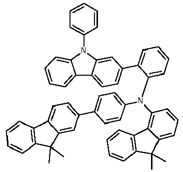 |
| #559 | #560 | #561 |
| 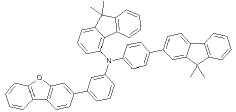 | 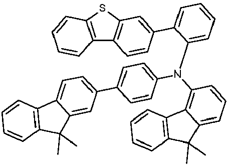 | 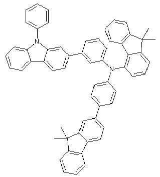 |
| #562 | #563 | #564 |
| 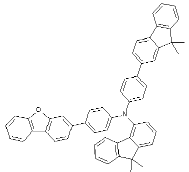 | 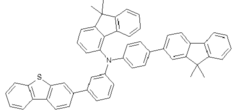 | 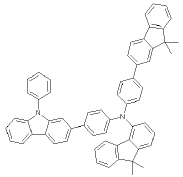 |
| #565 | #566 | #567 |
| 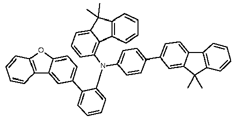 | 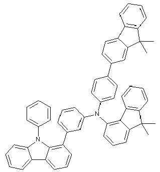 | 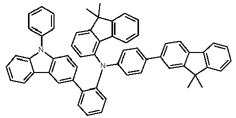 |
| #568 | #569 | #570 |
| 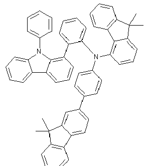 | 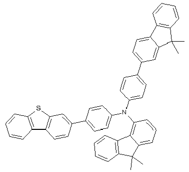 | 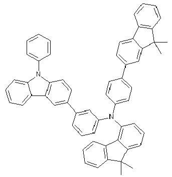 |
| #571 | #572 | #573 |
| 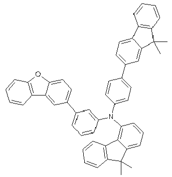 | 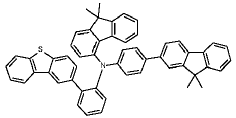 | 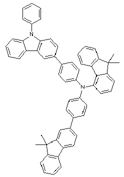 |
| #574 | #575 | #576 |
| 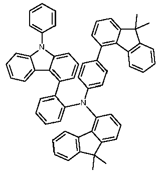 | 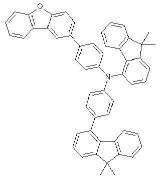 | 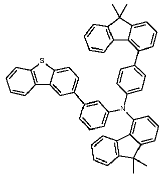 |
| #577 | #578 | #579 |
| 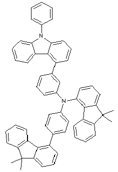 | 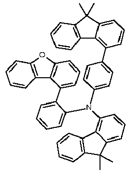 | 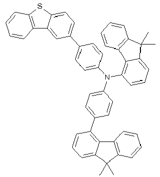 |
| #580 | #581 | #582 |
| 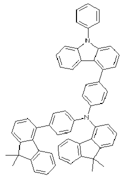 | 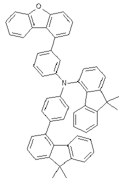 | 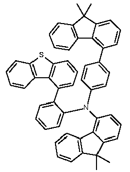 |
| #583 | #584 | #585 |
| 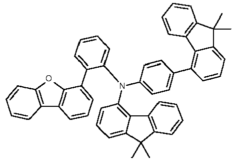 | 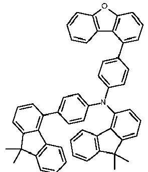 | 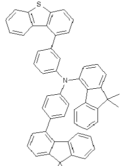 |
| #586 | #587 | #588 |
| 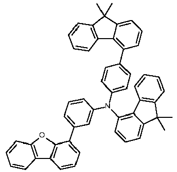 | 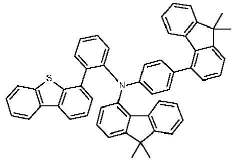 | 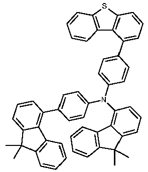 |
| #589 | #590 | #591 |
| 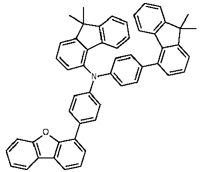 | 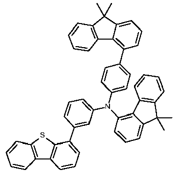 | 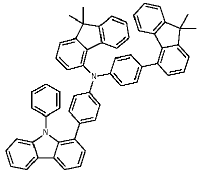 |
| #592 | #593 | #594 |
| 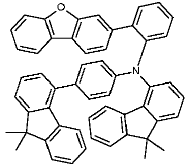 | 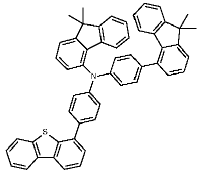 | 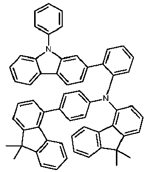 |
| #595 | #596 | #597 |
| 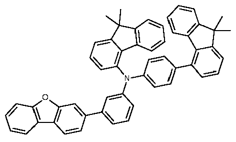 | 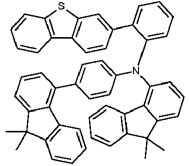 | 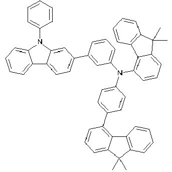 |
| #598 | #599 | #600 |
| 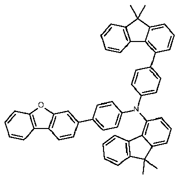 | 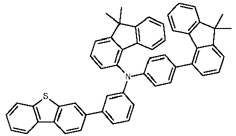 | 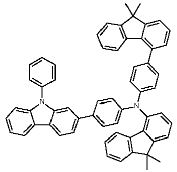 |
| #601 | #602 | #603 |
| 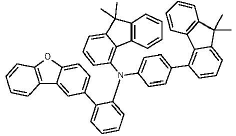 | 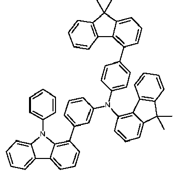 | 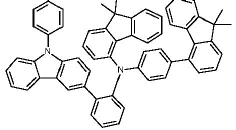 |
| #604 | #605 | #606 |
| 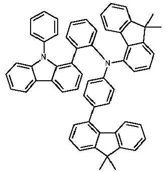 | 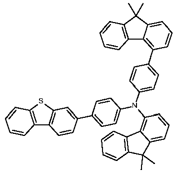 | 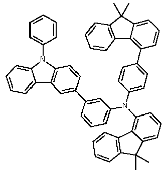 |
| #607 | #608 | #609 |
| 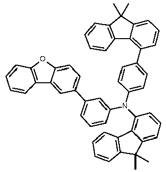 | 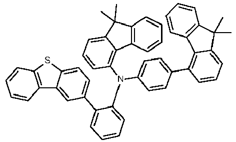 | 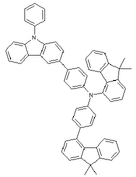 |
| #610 | #611 | #612 |
| 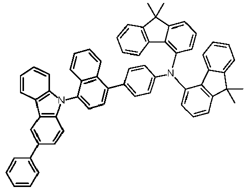 | 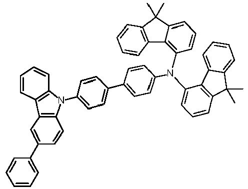 | 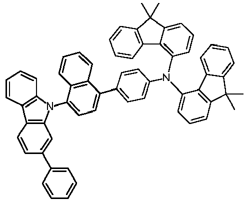 |
| 613 | 614 | 615 |
| 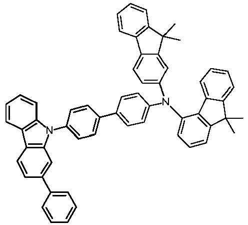 | 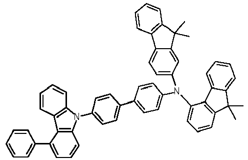 | 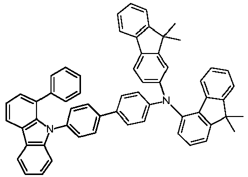 |
| #616 | #617 | #618 |
| 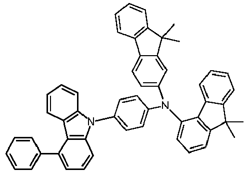 | 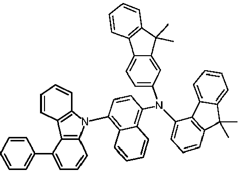 | 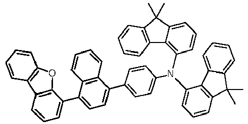 |
| #619 | #620 | 621 |
| 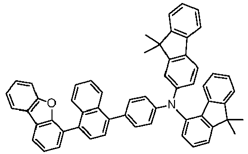 | 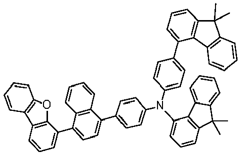 | 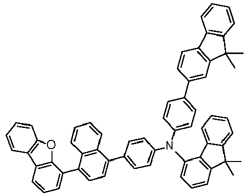 |
| #622 | #623 | #624 |
| 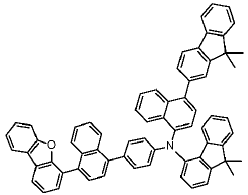 | 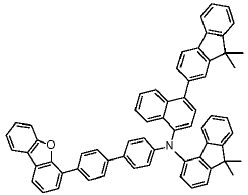 | 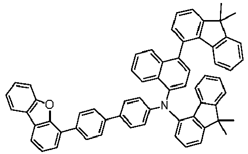 |
| #625 | #626 | #627 |
| 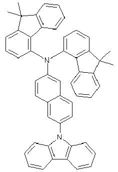 | 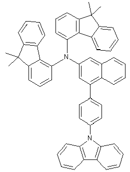 | 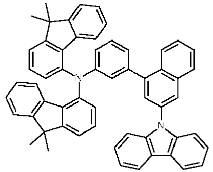 |
| 628 | 629 | 630 |
| 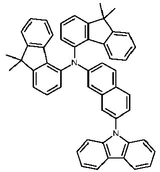 | 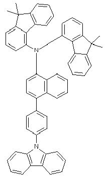 | 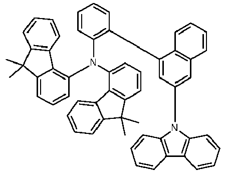 |
| 631 | 632 | 633 |
| 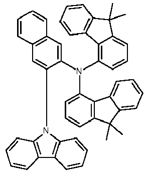 | 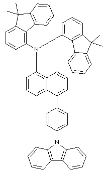 | 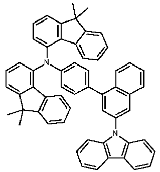 |
| 634 | 635 | 636 |
| 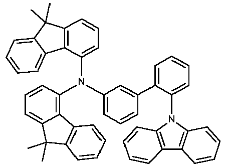 | 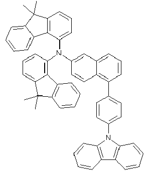 | 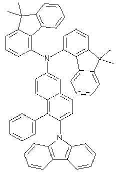 |
| 637 | 638 | 639 |
| 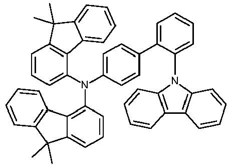 | 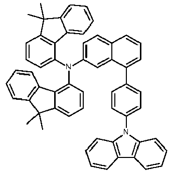 | 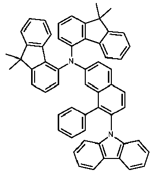 |
| 640 | 641 | 642 |
| 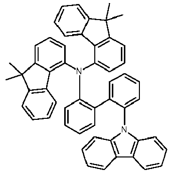 | 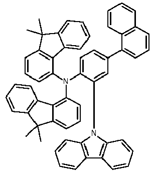 | 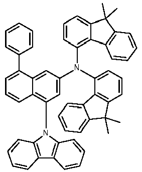 |
| 643 | 644 | 645 |
| 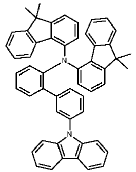 | 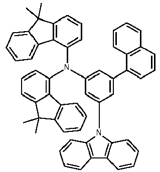 | 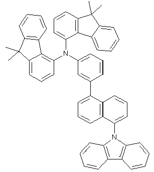 |
| 646 | 647 | 648 |
| 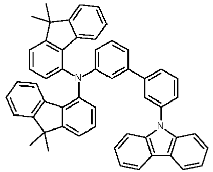 | 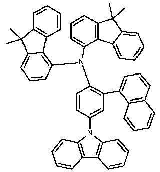 | 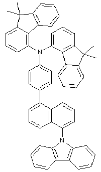 |
| 649 | 650 | 651 |
| 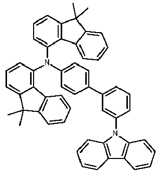 | 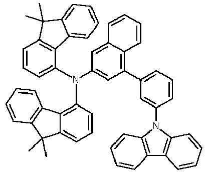 | 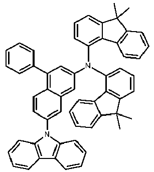 |
| 652 | 653 | 654 |
| 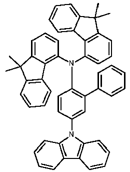 | 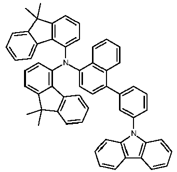 | 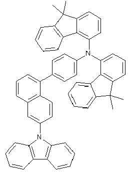 |
| 655 | 656 | 657 |
| 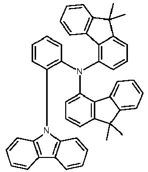 | 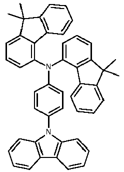 | 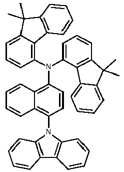 |
| 658 | 659 | 660 |
| 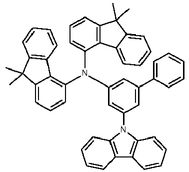 | 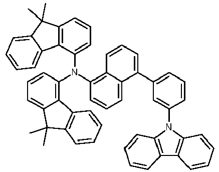 | 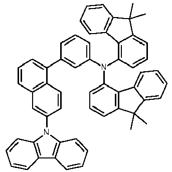 |
| 661 | 662 | 663 |
| 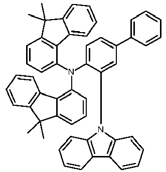 | 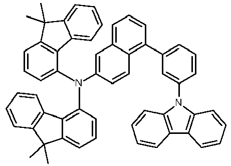 | 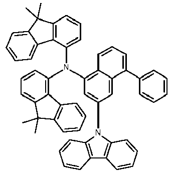 |
| 664 | 665 | 666 |
| 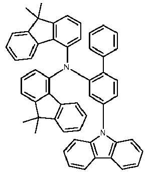 | 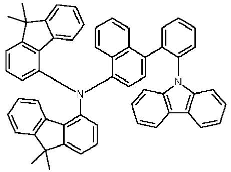 | 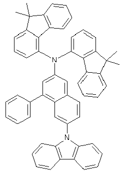 |
| 667 | 668 | 669 |
| 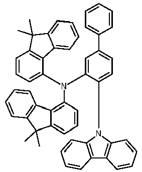 | 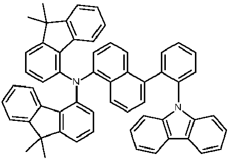 | 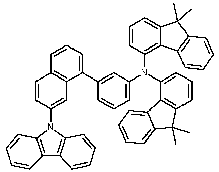 |
| 670 | 671 | 672 |
| 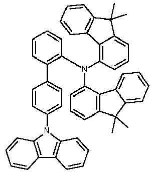 | 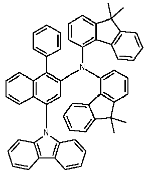 | 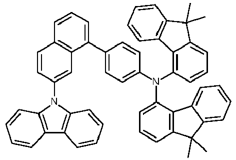 |
| 673 | 674 | 675 |
| 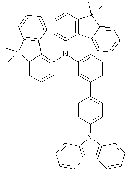 | 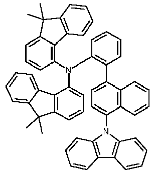 | 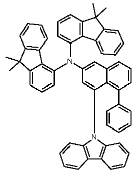 |
| 676 | 677 | 678 |
| 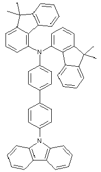 | 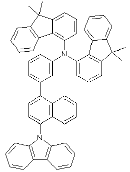 | 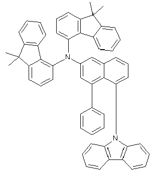 |
| 679 | 680 | 681 |
| 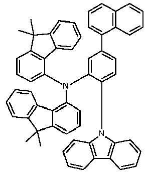 | 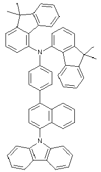 | 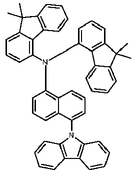 |
| 682 | 683 | 684 |
| 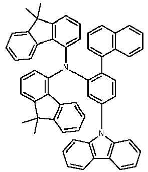 | 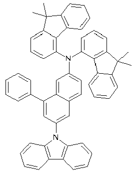 | 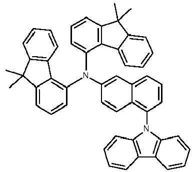 |
| 685 | 686 | 687 |
| 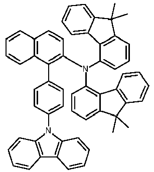 | 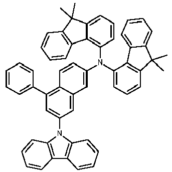 | 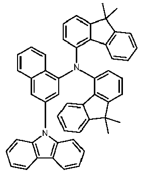 |
| 688 | 689 | 690 |
| 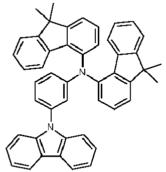 | 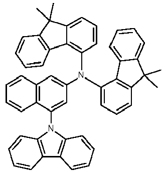 | 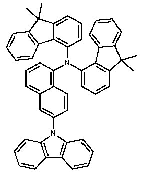 |
| 691 | 692 | 693 |
| 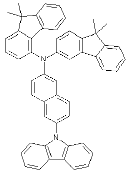 | 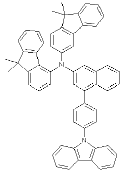 | 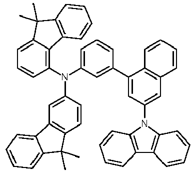 |
| #694 | #695 | #696 |
| 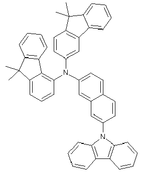 | 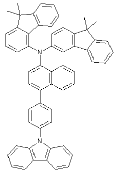 | 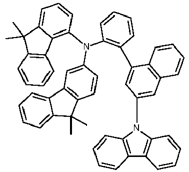 |
| **#697** | **#698** | **#699** |
| 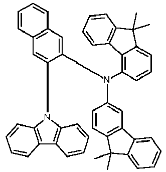 | 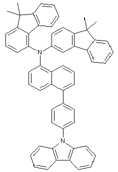 | 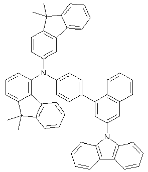 |
| **#700** | **#701** | **#702** |
| 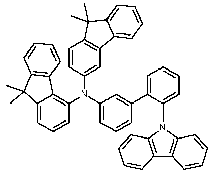 | 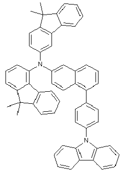 | 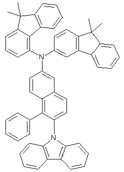 |
| **#703** | **#704** | **#705** |
| 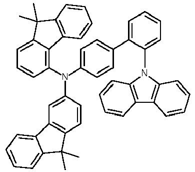 | 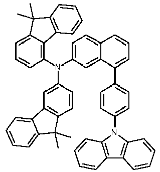 | 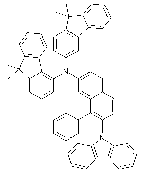 |
| **#706** | **#707** | **#708** |
| 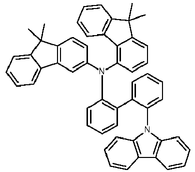 | 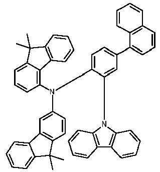 | 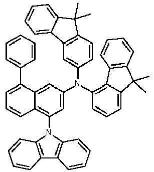 |
| **#709** | **#710** | **#711** |
| 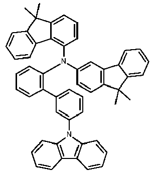 | 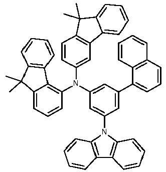 | 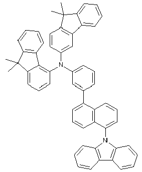 |
| **#712** | **#713** | **#714** |
| 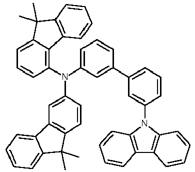 | 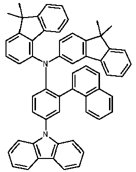 | 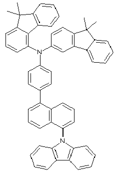 |
| **#715** | **#716** | **#717** |
| 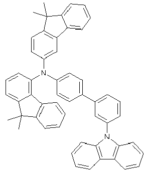 | 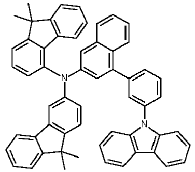 | 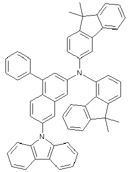 |
| #718 | **#719** | **#720** |
| 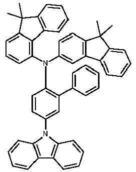 | 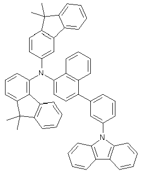 | 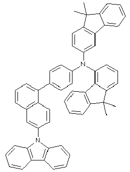 |
| **#721** | **#722** | **#723** |
| 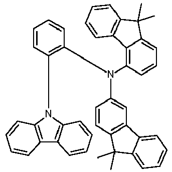 | 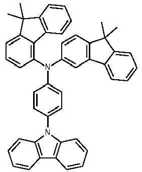 | 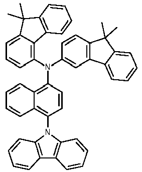 |
| **#724** | **#725** | **#726** |
| 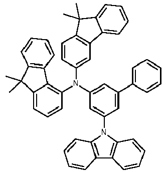 | 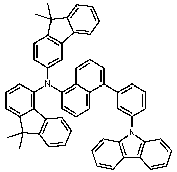 | 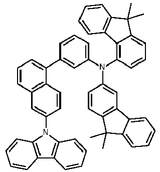 |
| **#727** | **#728** | **#729** |
| 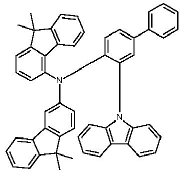 | 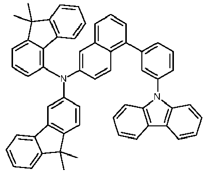 | 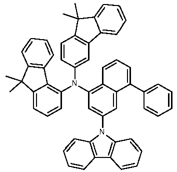 |
| **#730** | **#731** | **#732** |
| 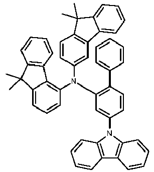 | 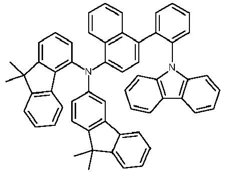 | 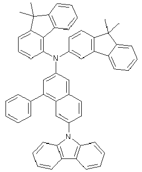 |
| **#733** | **#734** | **#735** |
| 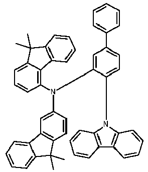 | 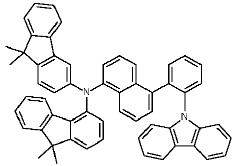 | 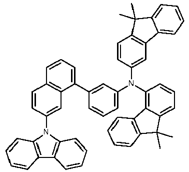 |
| **#736** | **#737** | **#738** |
| 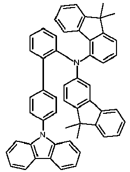 | 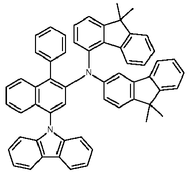 | 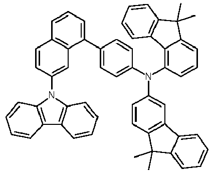 |
| **#739** | **#740** | **#741** |
| 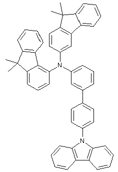 | 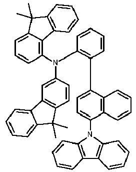 | 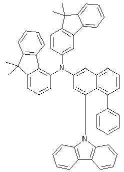 |
| **#742** | **#743** | **#744** |
| 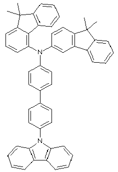 | 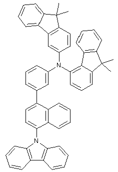 | 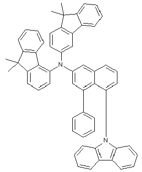 |
| **#745** | **#746** | **#747** |
| 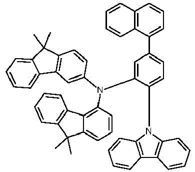 | 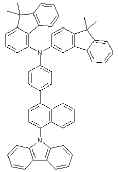 | 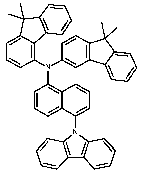 |
| **#748** | **#749** | **#750** |
| 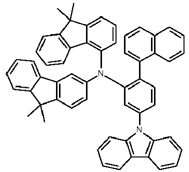 | 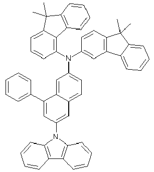 | 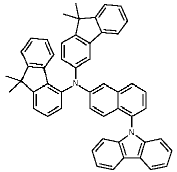 |
| **#751** | **#752** | **#753** |
| 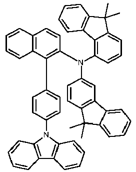 | 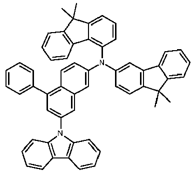 | 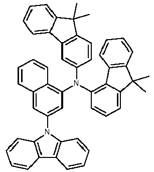 |
| **#754** | **#755** | **#756** |
| 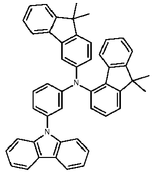 | 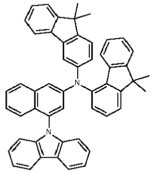 | 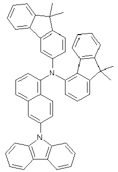 |
| **#757** | **#758** | **#759** |
| 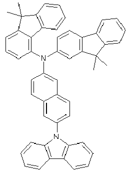 | 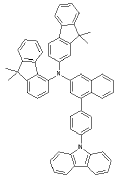 | 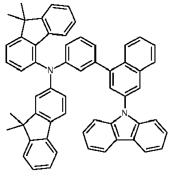 |
| **#760** | **#761** | **#762** |
| 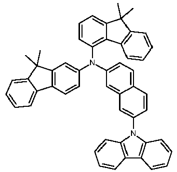 | 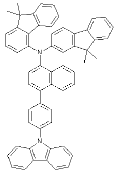 | 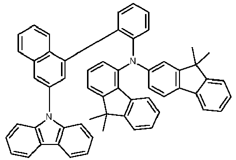 |
| **#763** | **#764** | **#765** |
| 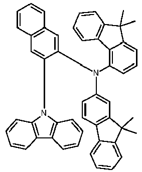 | 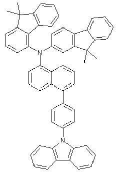 | 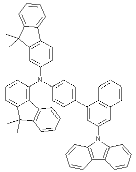 |
| **#766** | **#767** | **#768** |
| 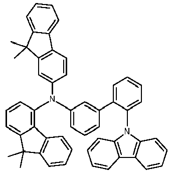 | 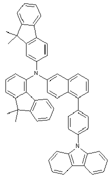 | 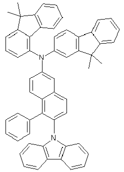 |
| **#769** | **#770** | **#771** |
| 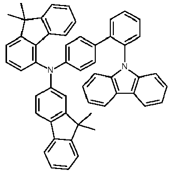 | 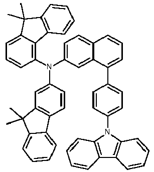 | 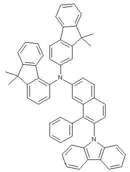 |
| **#772** | **#773** | **#774** |
| 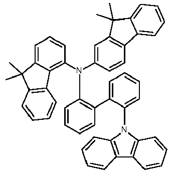 | 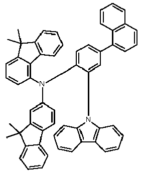 | 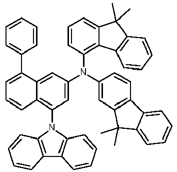 |
| **#775** | **#776** | **#777** |
| 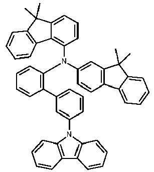 | 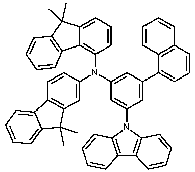 | 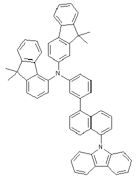 |
| **#778** | **#779** | **#780** |
| 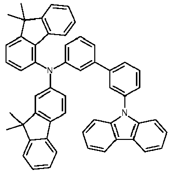 | 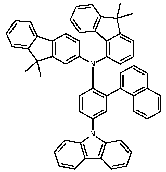 | 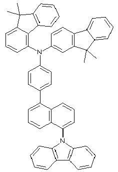 |
| **#781** | **#782** | **#783** |
| 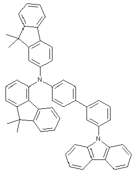 | 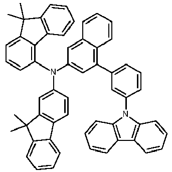 | 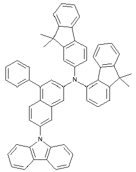 |
| **#784** | **#785** | **#786** |
| 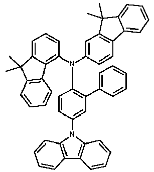 | 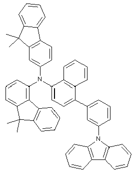 | 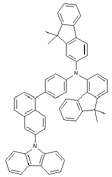 |
| **#787** | **#788** | **#789** |
| 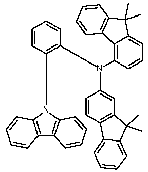 | 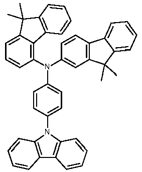 | 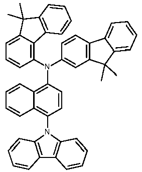 |
| **#790** | **#791** | **#792** |
| 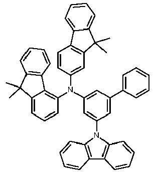 | 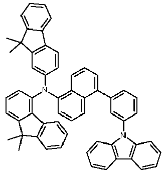 | 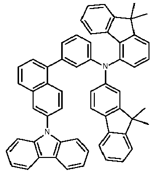 |
| **#793** | **#794** | **#795** |
| 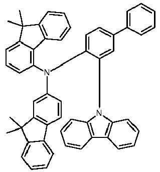 | 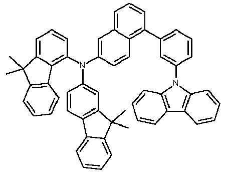 | 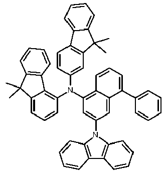 |
| **#796** | **#797** | **#798** |
| 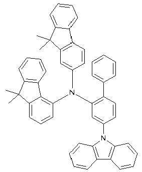 | 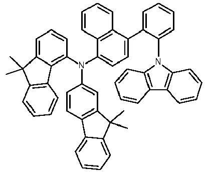 | 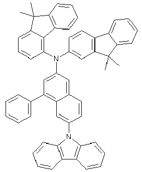 |
| **#799** | **#800** | **#801** |
| 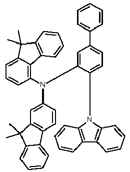 | 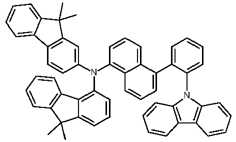 | 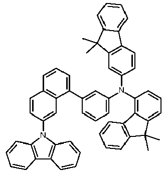 |
| **#802** | **#803** | **#804** |
| 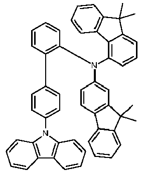 | 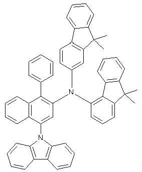 | 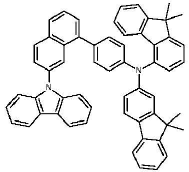 |
| **#805** | **#806** | **#807** |
| 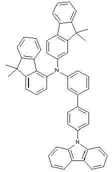 | 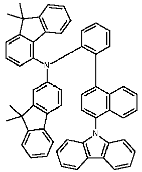 | 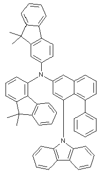 |
| **#808** | **#809** | **#810** |
| 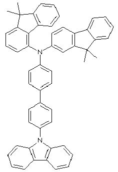 | 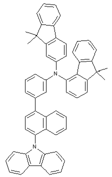 | 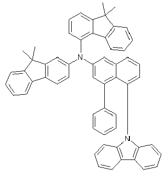 |
| **#811** | **#812** | **#813** |
| 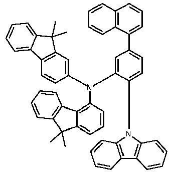 | 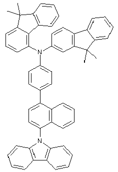 | 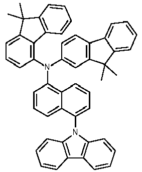 |
| **#814** | **#815** | **#816** |
| 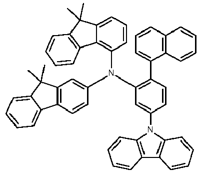 | 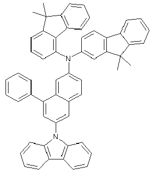 | 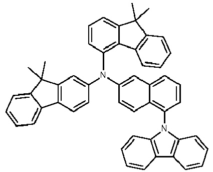 |
| **#817** | **#818** | **#819** |
| 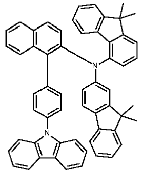 | 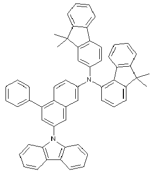 | 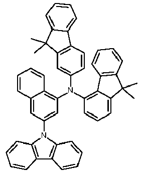 |
| **#820** | **#821** | **#822** |
| 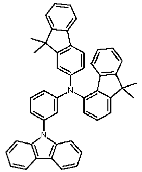 | 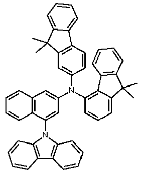 | 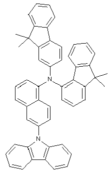 |
| **#823** | **#824** | **#825** |
| 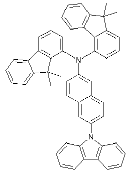 | 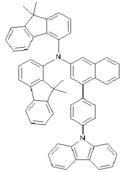 | 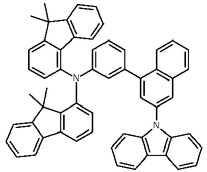 |
| **#826** | **#827** | **#828** |
| 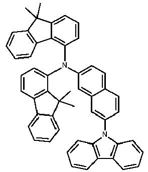 | 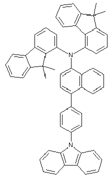 | 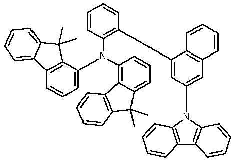 |
| **#829** | **#830** | **#831** |
| 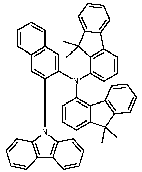 | 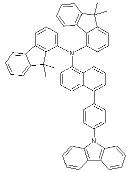 | 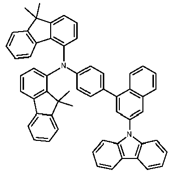 |
| **#832** | **#833** | **#834** |
| 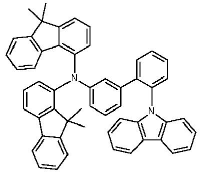 | 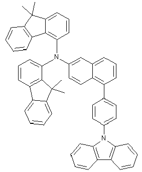 | 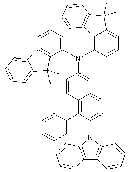 |
| **#835** | **#836** | **#837** |
| 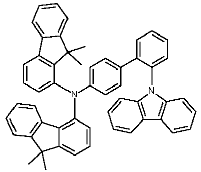 | 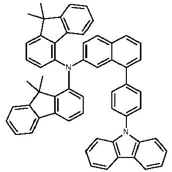 | 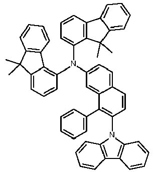 |
| **#838** | **#839** | **#840** |
| 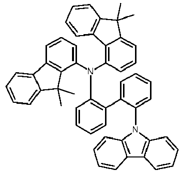 | 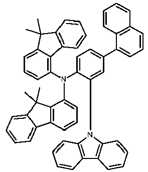 | 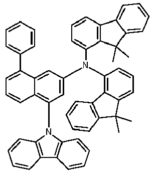 |
| **#841** | **#842** | **#843** |
| 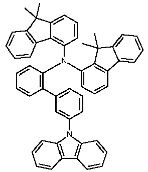 | 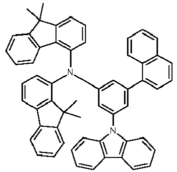 | 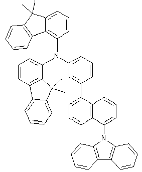 |
| **#844** | **#845** | **#846** |
| 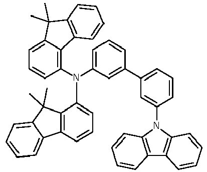 | 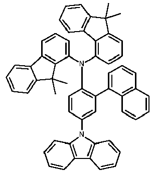 | 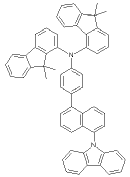 |
| **#847** | **#848** | **#849** |
| 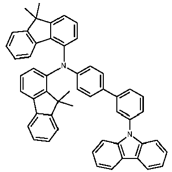 | 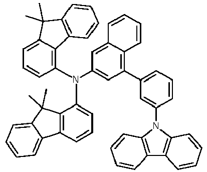 | 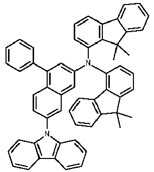 |
| **#850** | **#851** | **#852** |
| 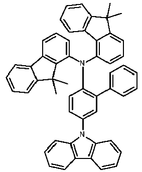 | 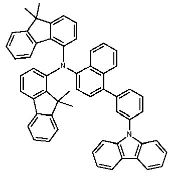 | 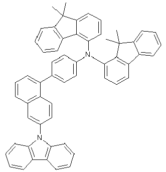 |
| **#853** | **#854** | **#855** |
| 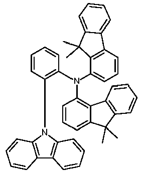 | 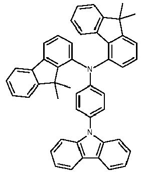 | 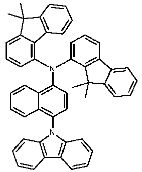 |
| **#856** | **#857** | **#858** |
| 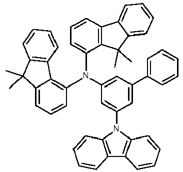 | 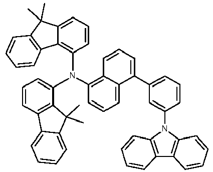 | 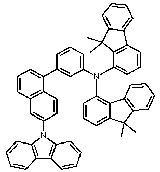 |
| **#859** | **#860** | **#861** |
| 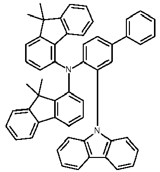 | 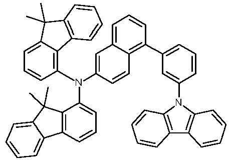 | 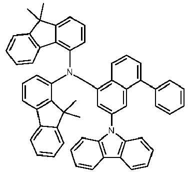 |
| **#862** | **#863** | **#864** |
| 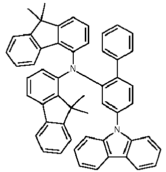 | 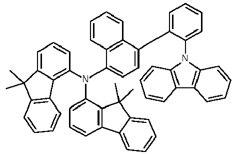 | 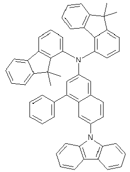 |
| **#865** | **#866** | **#867** |
| 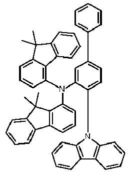 | 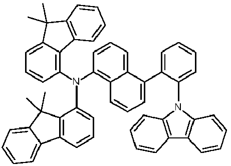 | 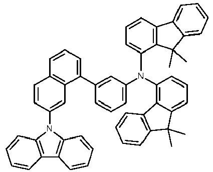 |
| **#868** | **#869** | **#870** |
| 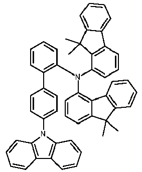 | 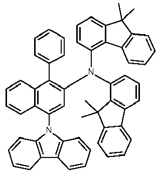 | 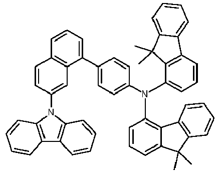 |
| **#871** | **#872** | **#873** |
| 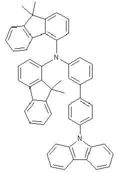 | 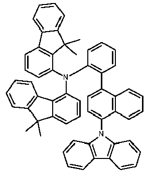 | 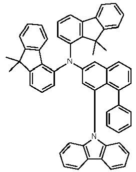 |
| **#874** | **#875** | **#876** |
| 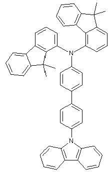 | 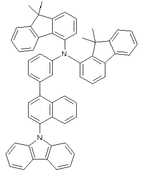 | 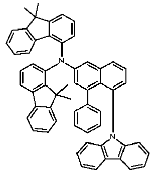 |
| **#877** | **#878** | **#879** |
| 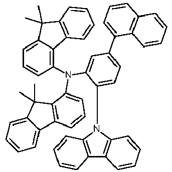 | 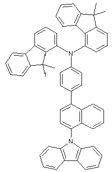 | 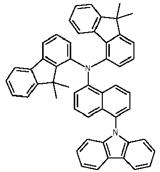 |
| **#880** | **#881** | **#882** |
| 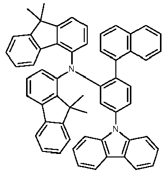 | 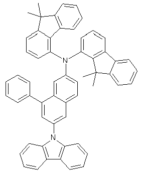 | 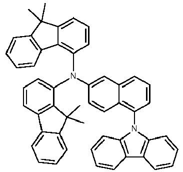 |
| **#883** | **#884** | **#885** |
| 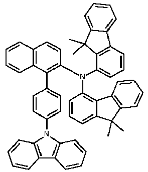 | 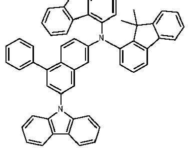 | 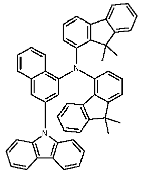 |
| **#886** | **#887** | **#888** |
| 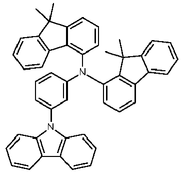 | 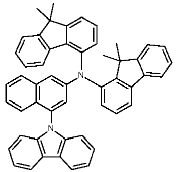 | 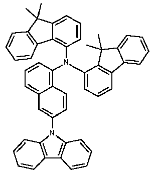 |
| **#889** | **#890** | **#891** |

Mit # gekennzeichnete Verbindungen in der obenstehenden Tabelle und im Folgenden sind nicht Teil der Erfindung.

Die Verbindungen der Formel (I-A-4) können mittels bekannter Reaktionen der organischen Chemie hergestellt werden, insbesondere mittels Buchwald-Kupplungsreaktionen.

Ein bevorzugter Syntheseweg zur Herstellung von Verbindungen der Formel (I-A-4) ist im untenstehenden Schema 1 gezeigt.

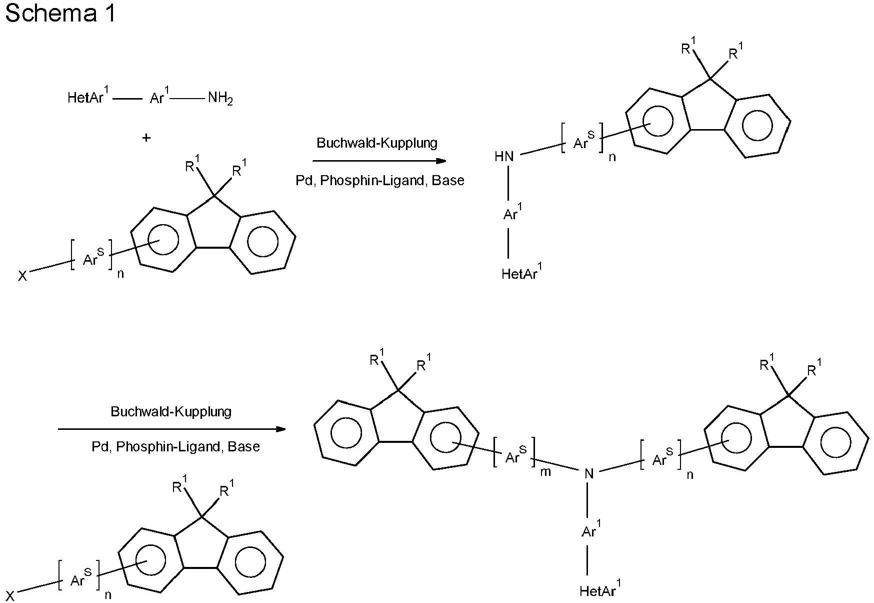

Dabei sind die auftretenden Variablen definiert wie für Formel (I-A-4), wobei m und n in Schema 1 0 sind, und X ist gewählt aus reaktiven Gruppen, bevorzugt aus Cl, Br und I.

In Schema 1 wird das primäre Amin der Formel HetAr¹-Ar¹-NH₂ als Ausgangsmaterial eingesetzt. Dessen Synthese ist in vielen Fällen im Stand der Technik bekannt. In den anderen Fällen kann es mittels bekannter Syntheseverfahren hergestellt werden. Das genannte primäre Amin wird in einer Buchwald-Kupplungsreaktion mit einem Fluorenyl-Derivat umgesetzt, das eine reaktive Gruppe X trägt. Das erhaltene Intermediat, ein sekundäres Amin, wird in einer zweiten Buchwald-Kupplungsreaktion mit einem anderen Fluorenyl-Derivat umgesetzt. Dabei wird die Verbindung der Formel (I-A-4) erhalten.

Ein alternatives, ebenfalls bevorzugtes Verfahren zur Herstellung von Verbindungen der Formel (I-A-4) ist im untenstehenden Schema 2 gezeigt. In diesem Verfahren wird das primäre Amin HetAr¹-Ar¹-NH₂ in einer einzigen Buchwald-Kupplungsreaktion zu einer Verbindung der Formel (I-A-4) umgesetzt. Dabei werden mehr Äquivalente des Fluorenyl-Derivats, das eine reaktive Gruppe trägt, eingesetzt, so dass in einem Schritt aus dem primären direkt das tertiäre Amin erhalten wird.

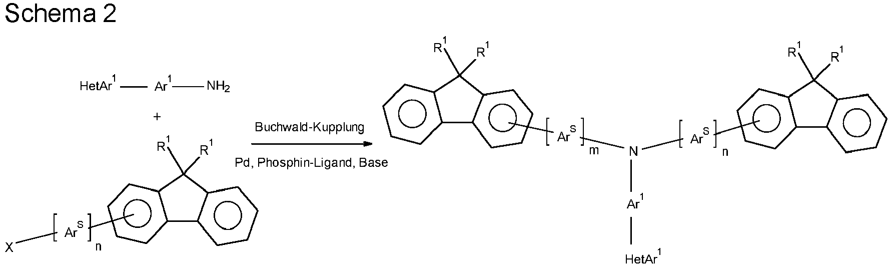

Gegenstand der vorliegenden Anmeldung ist ein Verfahren zur Herstellung einer Verbindung der Formel (I-A-4), dadurch gekennzeichnet, dass eine Verbindung HetAr¹-Ar¹-NH₂, wobei die auftretenden Variablen definiert sind wie für Formel (I-A-4), wobei m und n in Schema 2 0 sind, in einer Buchwald-Kupplungsreaktion mit einem Fluoren umgesetzt wird, das eine reaktive Gruppe X aufweist.

Bevorzugt ist die reaktive Gruppe X gewählt aus Cl, Br und I. Gemäß einer bevorzugten Ausführungsform wird die Verbindung der Formel (I-A-4) aus der Verbindung HetAr¹-Ar¹-NH₂ in einem einzigen Schritt erhalten, indem in einem Schritt eine zweifache Kupplungsreaktion erfolgt. Gemäß einer alternativen bevorzugten Ausführungsform wird die Verbindung der Formel (I-A-4) in zwei aufeinander folgenden Schritten erhalten, indem zunächst die Verbindung HetAr¹-Ar¹-NH₂ an einer der beiden N-H-Bindungen des primären Amins in einer ersten Buchwald-Kupplungsreaktion mit einem Fluoren umgesetzt wird, das eine reaktive Gruppe trägt. Anschließend wird das erhaltene Intermediat, das ein sekundäres Amin ist, an der verbliebenen N-H-Bindung in einer zweiten Buchwald-Kupplungsreaktion mit einem weiteren Fluoren umgesetzt, das eine reaktive Gruppe trägt, wobei die Verbindung der Formel (I-A-4) erhalten wird.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I-A-4), wobei die Bindung(en) zum Polymer, Oligomer oder Dendrimer an beliebigen, in Formel (I-A-4) mit R¹, R², R⁴ oder R⁵ substituierten Positionen lokalisiert sein können. Je nach Verknüpfung der Verbindung ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I-A-4) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I-A-4) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I-A-4) in Oligomeren, Dendrimeren und Polymeren gelten dieselben Bevorzugungen wie oben für Verbindungen gemäß Formel (I-A-4) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I-A-4) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I-A-4) sowie mindestens ein Lösungsmittel, bevorzugt ein organisches Lösungsmittel. Wie solche Lösungen hergestellt werden können, ist dem Fachmann bekannt.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I-A-4) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I-A-4). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine lochtransportierende Schicht oder eine andere Schicht sein kann, mindestens eine Verbindung gemäß Formel (I-A-4) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen.

Die Abfolge der Schichten der organischen Elektrolumineszenzvorrichtung enthaltend die Verbindung der Formel (I-A-4) ist bevorzugt die folgende: Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen. Die erfindungsgemäßen Verbindungen sind dabei bevorzugt in einer Lochtransportschicht, Lochinjektionsschicht, Elektronenblockierschicht, und/oder emittierenden Schicht vorhanden, besonders bevorzugt in einer emittierenden Schicht als Matrixmaterial, und/oder in einer Elektronenblockierschicht.

Es ist erfindungsgemäß bevorzugt, wenn die Verbindung gemäß Formel (I-A-4) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, und/oder einer emittierenden Schicht enthalten sein. Besonders bevorzugt ist sie in einer Elektronenblockierschicht oder in einer emittierenden Schicht in Kombination mit einer phosphoreszierenden emittierenden Verbindung enthalten. In letzterem Fall ist die phosphoreszierende emittierende Verbindung bevorzugt gewählt aus rot oder grün phosphoreszierenden emittierenden Verbindungen. Ganz besonders bevorzugt ist sie in einer Elektronenblockierschicht enthalten.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I-A-4) in organischen Elektrolumineszenzvorrichtungen einsetzen. Weitere Beispiele sind in der folgenden Tabelle aufgeführt:

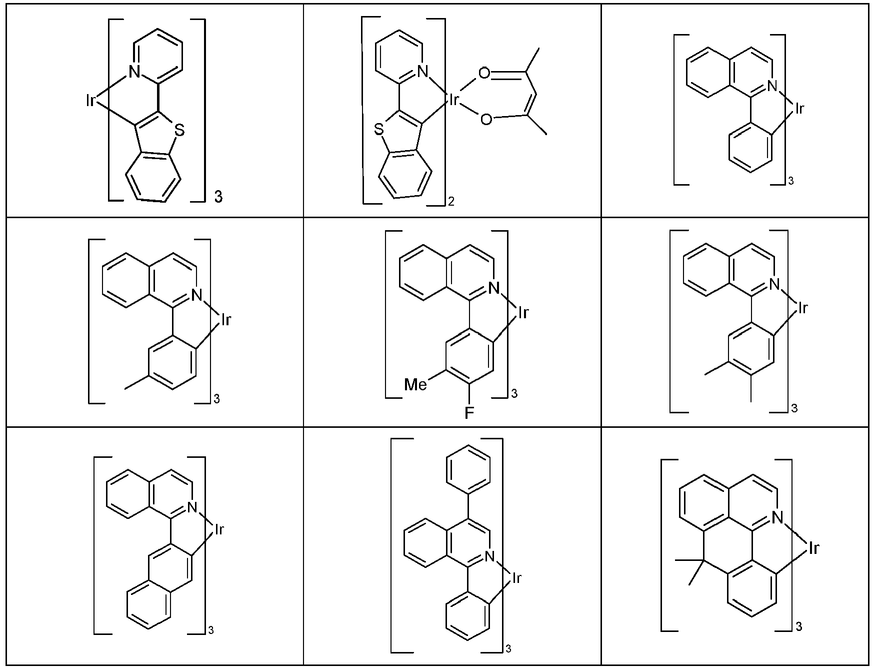

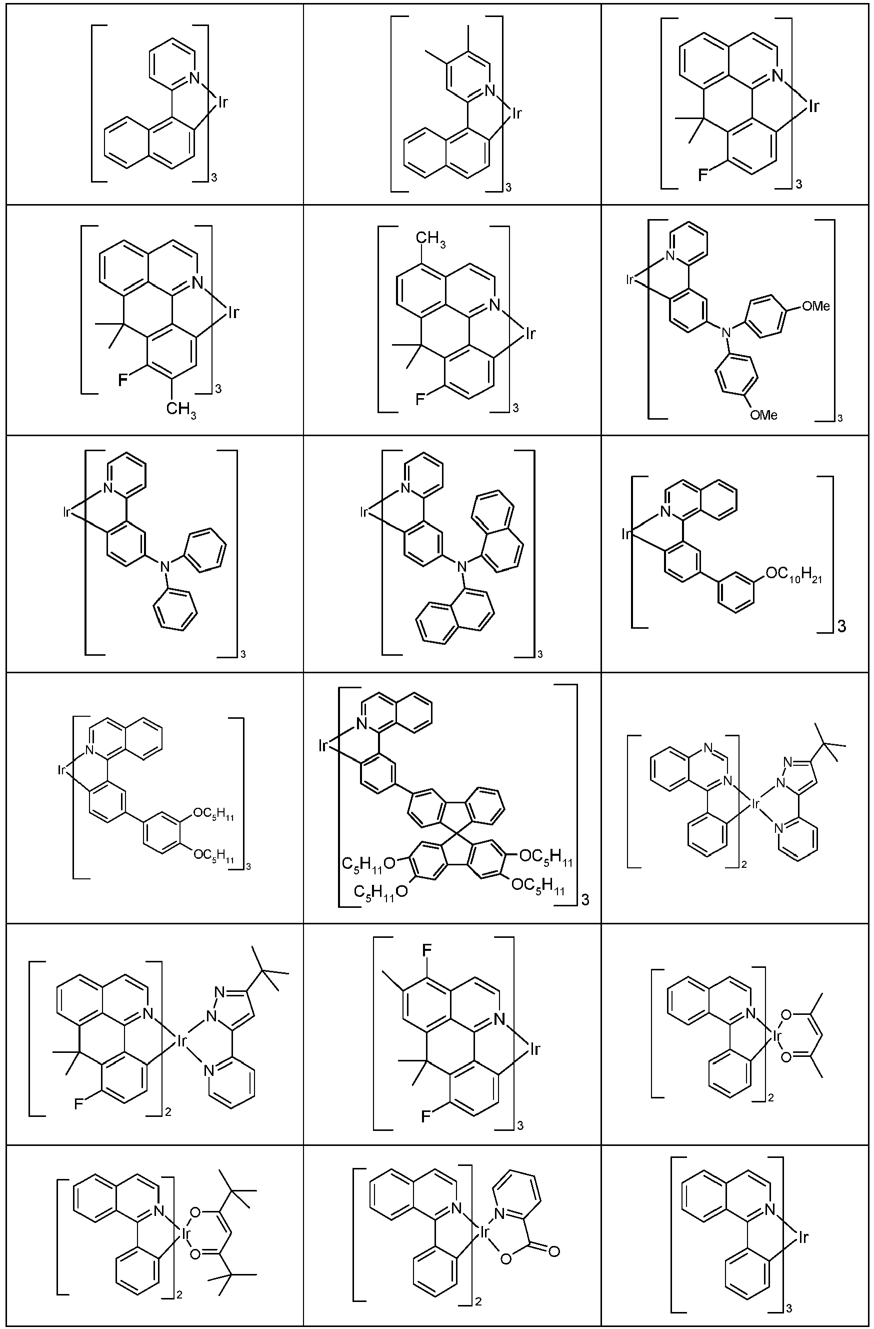

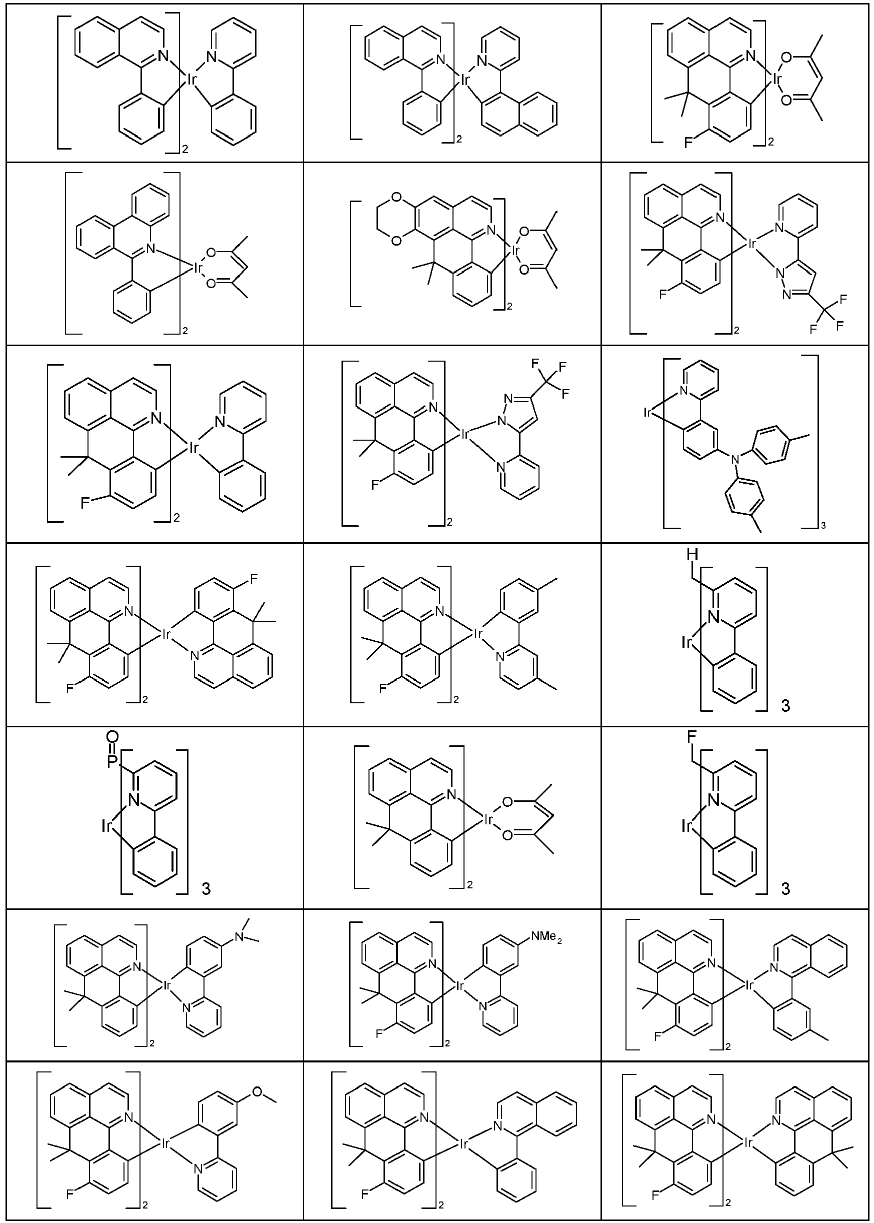

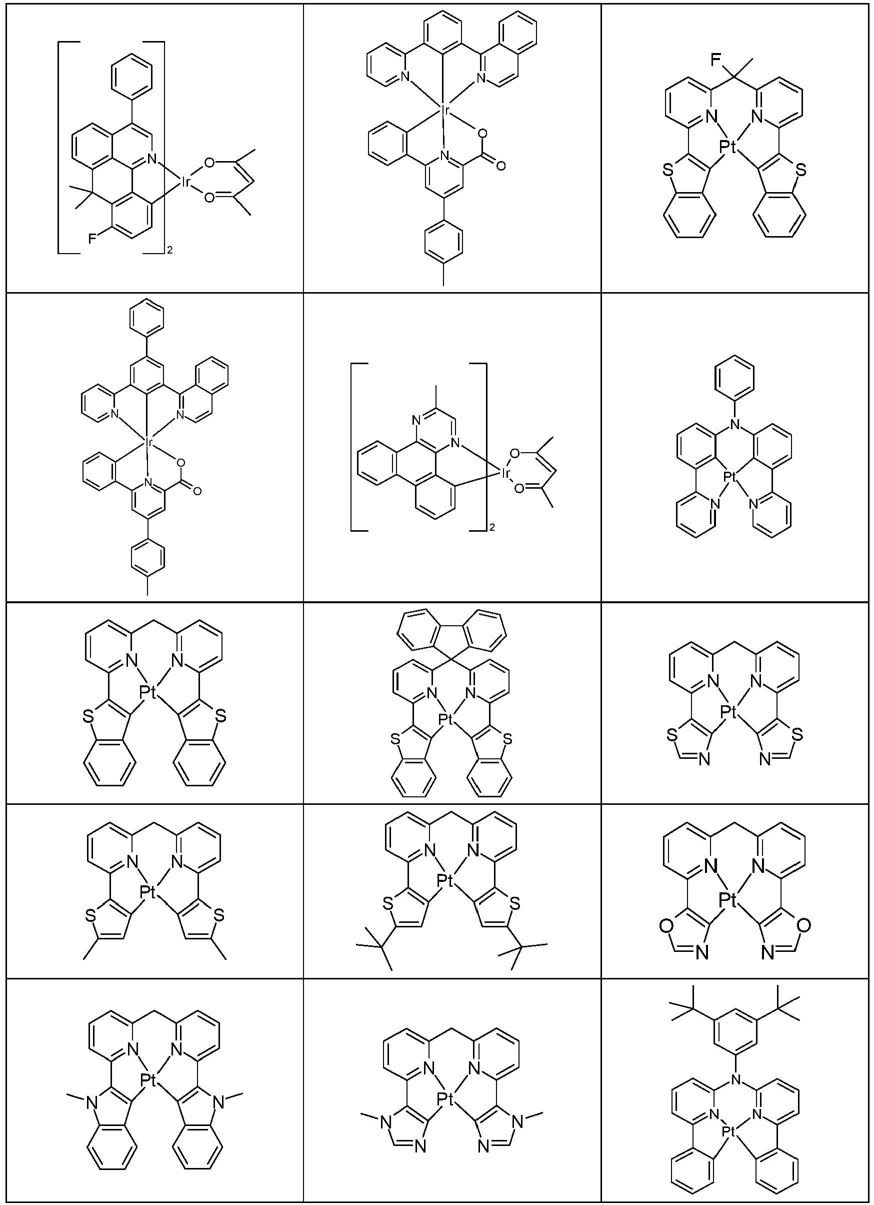

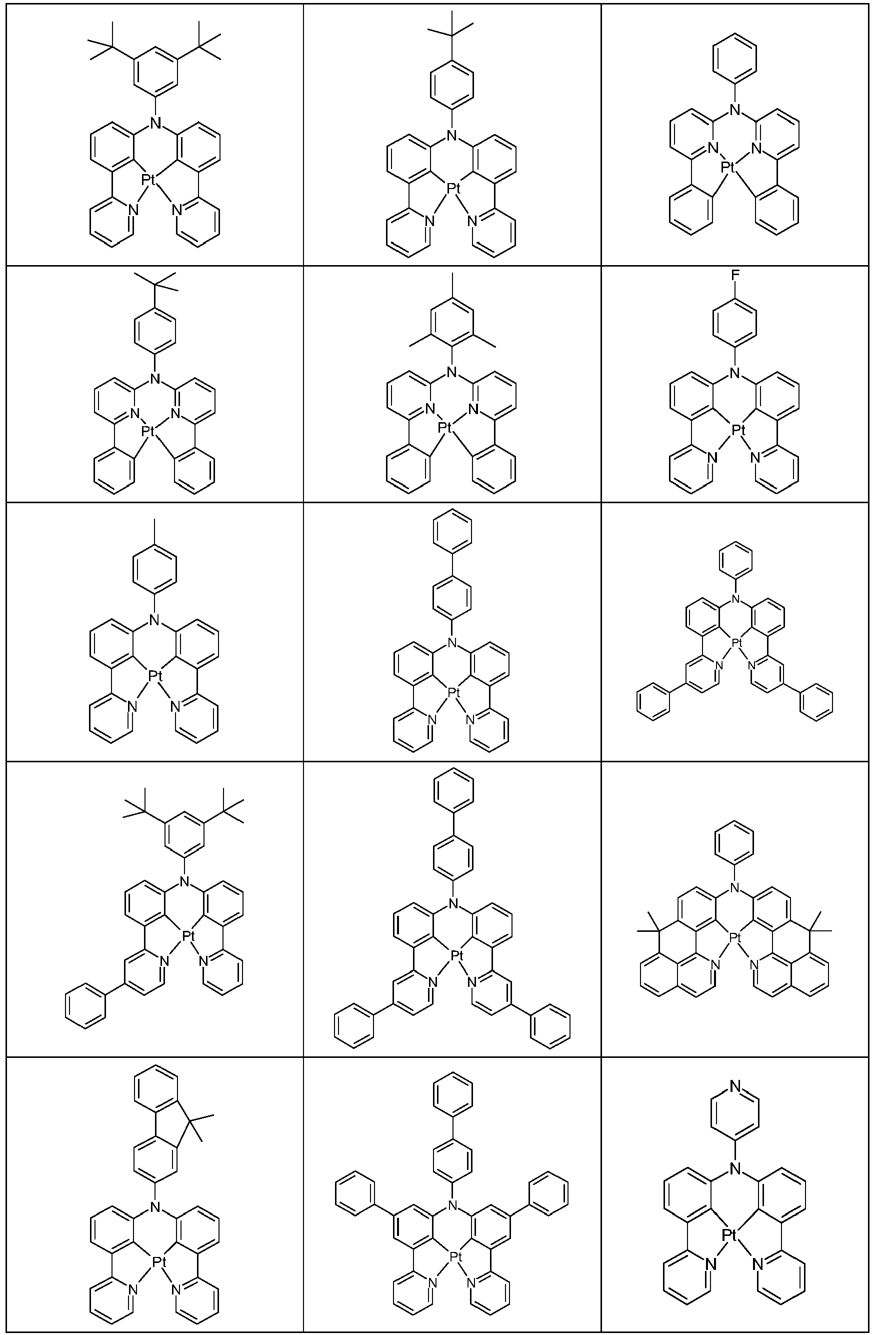

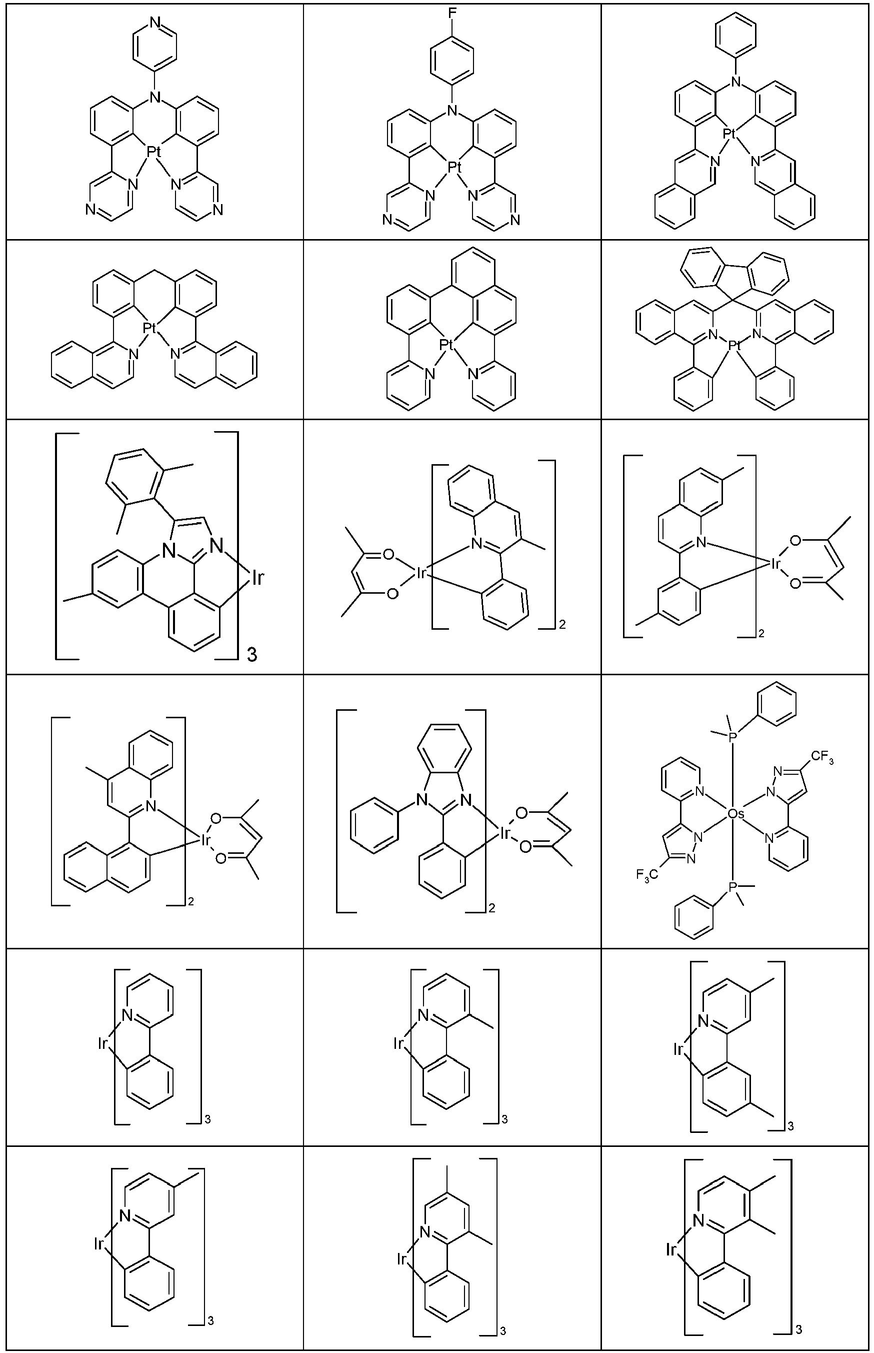

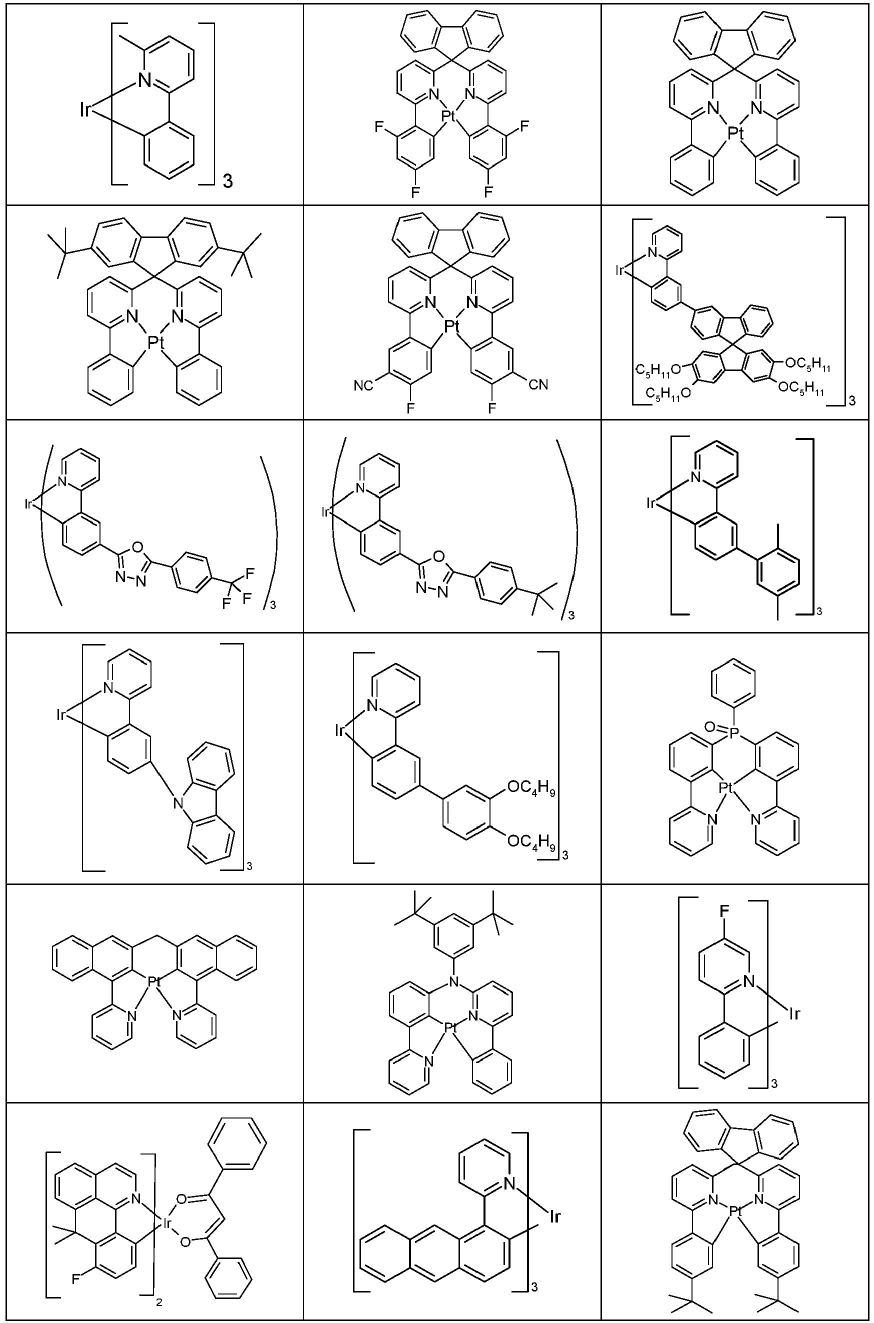

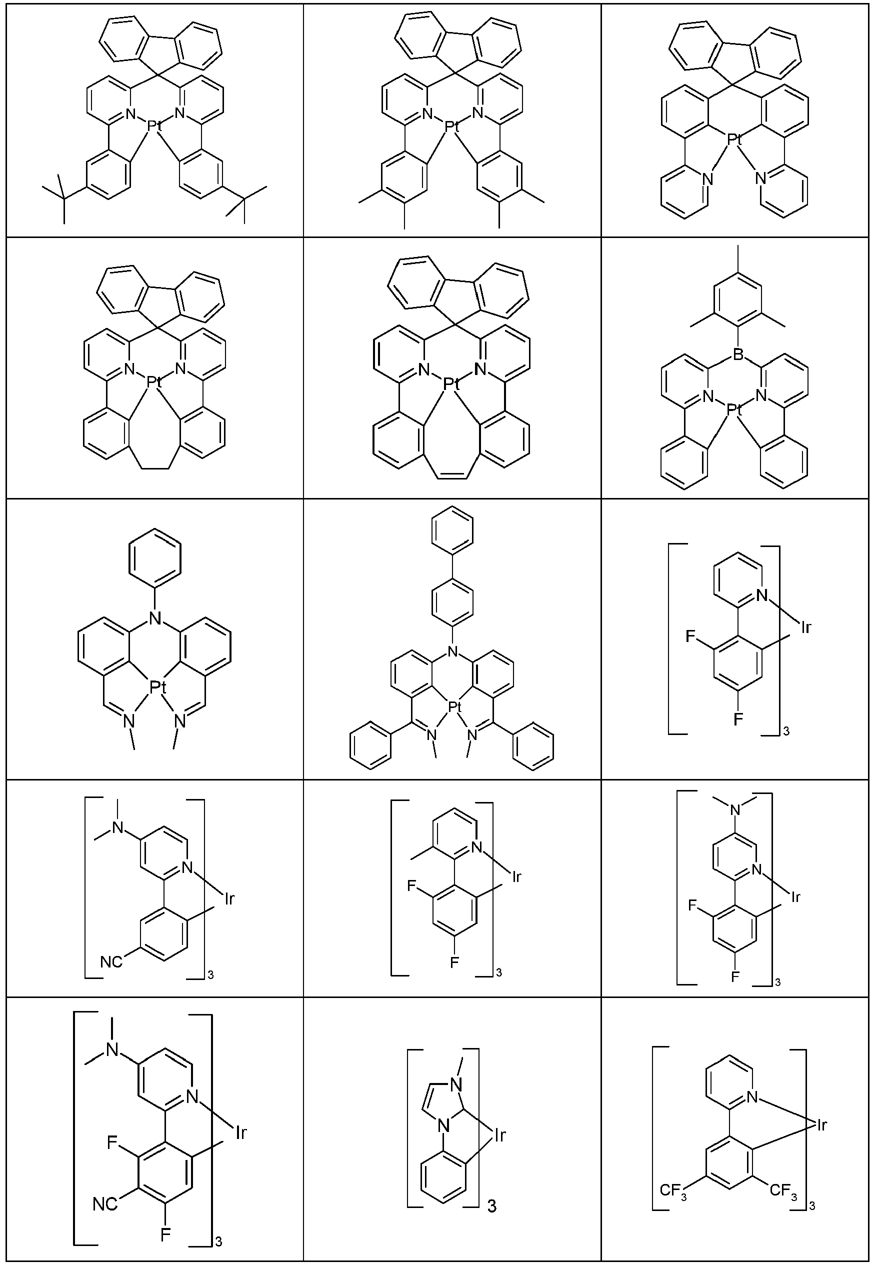

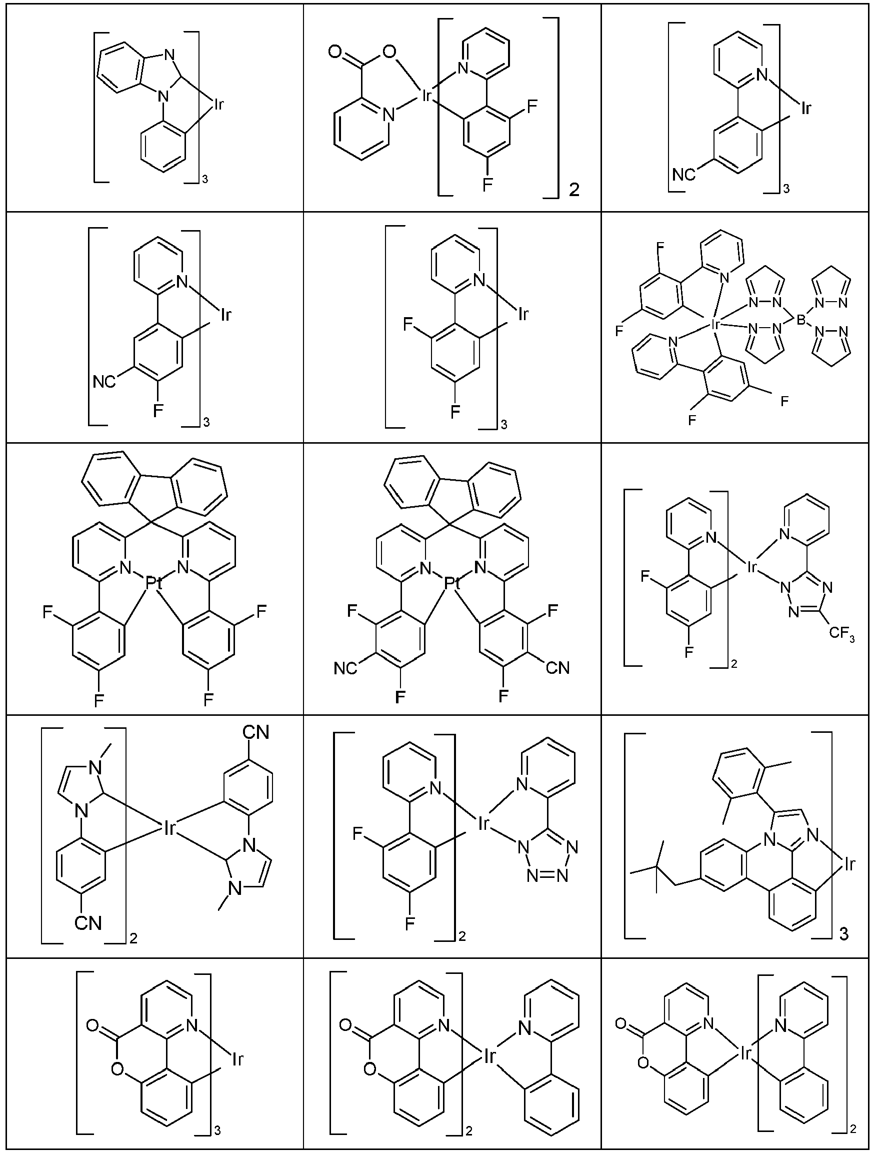

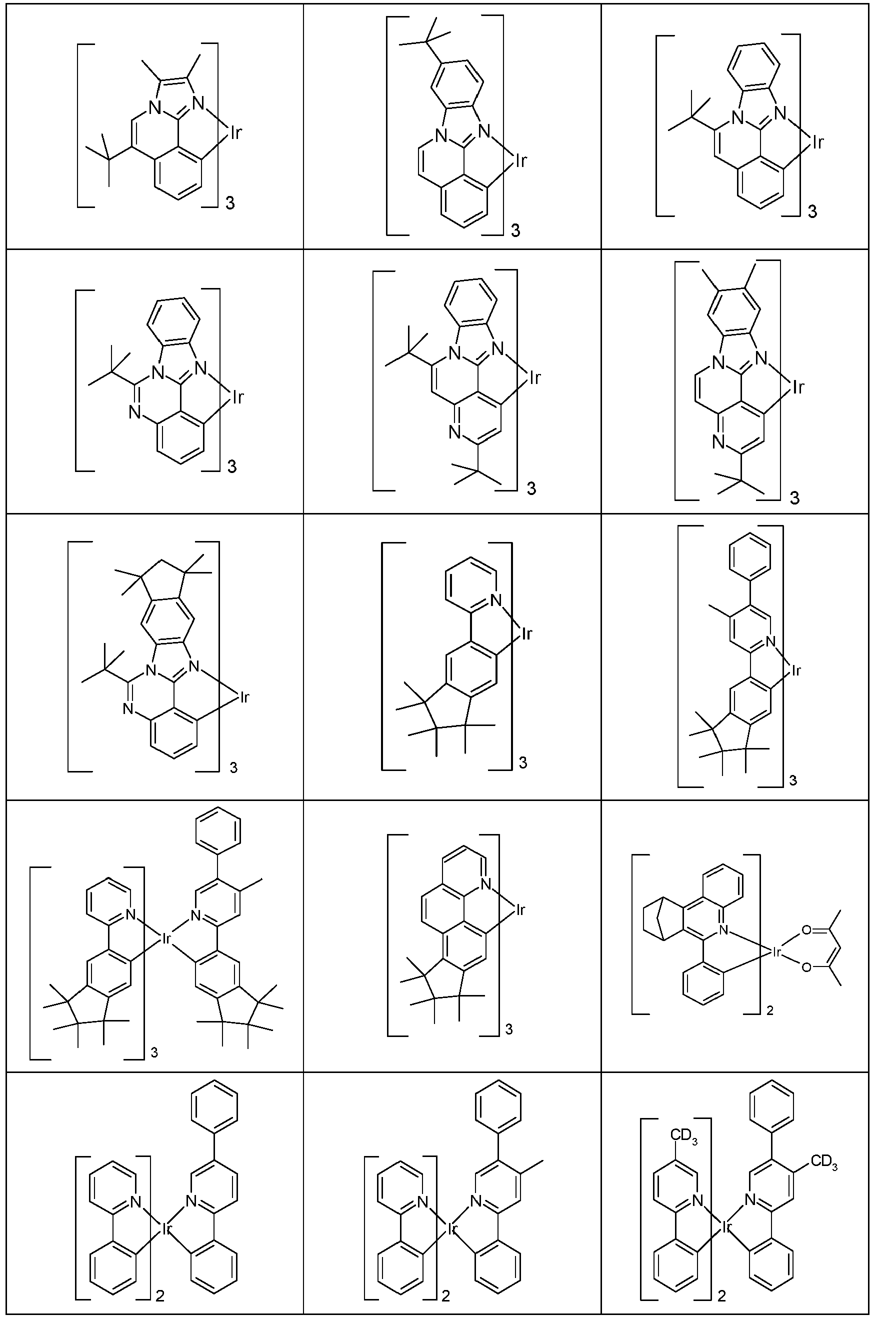

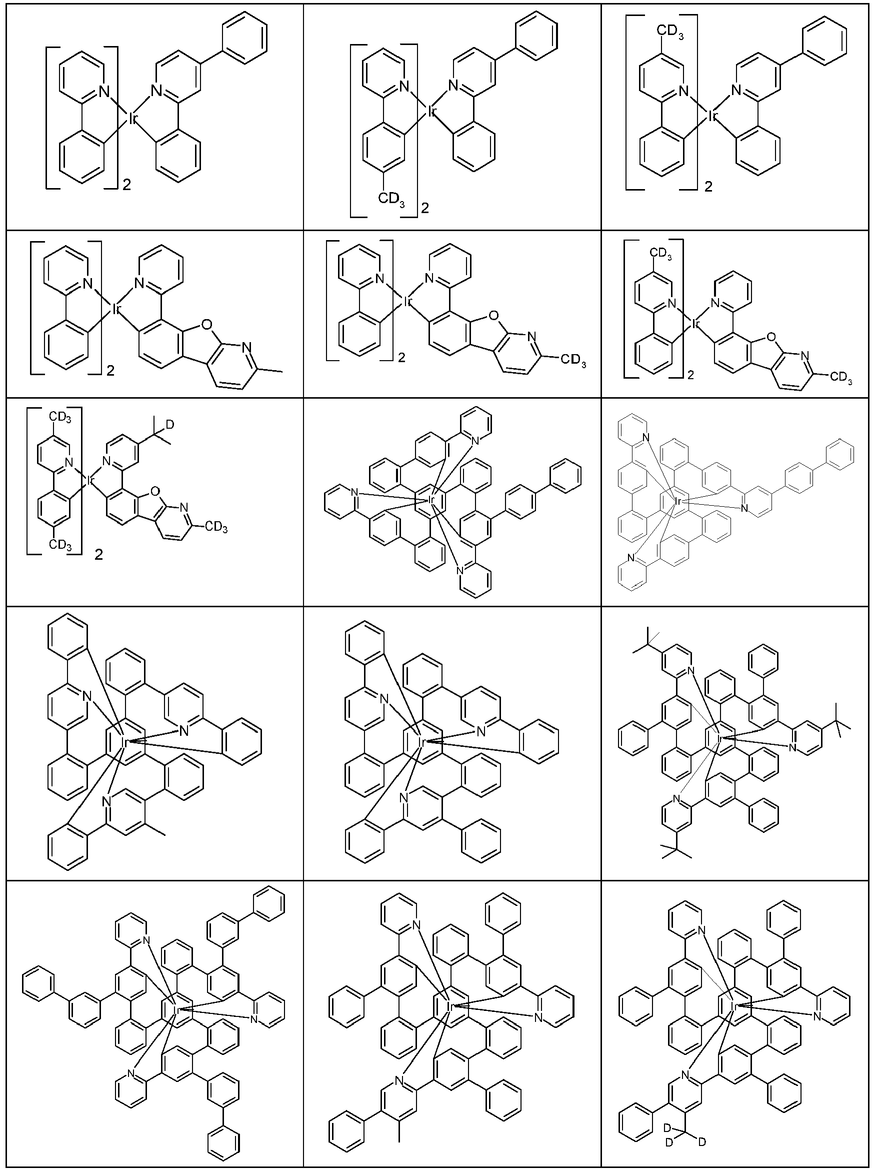

Des Weiteren können verwendet werden:

| | | | |
|---|---|---|---|
| CAS-1269508-30-6 | CAS-1989601-68-4 | CAS-1989602-19-8 | CAS-1989602-70-1 |
| CAS-1215692-34-4 | CAS-1989601-69-5 | CAS-1989602-20-1 | CAS-1989602-71-2 |
| CAS-1370364-40-1 | CAS-1989601-70-8 | CAS-1989602-21-2 | CAS-1989602-72-3 |
| CAS-1370364-42-3 | CAS-1989601-71-9 | CAS-1989602-22-3 | CAS-1989602-73-4 |

| | | | |
|---|---|---|---|
| CAS-1989600-74-9 | CAS-1989601-72-0 | CAS-1989602-23-4 | CAS-1989602-74-5 |
| CAS-1989600-75-0 | CAS-1989601-73-1 | CAS-1989602-24-5 | CAS-1989602-75-6 |
| CAS-1989600-77-2 | CAS-1989601-74-2 | CAS-1989602-25-6 | CAS-1989602-76-7 |
| CAS-1989600-78-3 | CAS-1989601-75-3 | CAS-1989602-26-7 | CAS-1989602-77-8 |
| CAS-1989600-79-4 | CAS-1989601-76-4 | CAS-1989602-27-8 | CAS-1989602-78-9 |
| CAS-1989600-82-9 | CAS-1989601-77-5 | CAS-1989602-28-9 | CAS-1989602-79-0 |
| CAS-1989600-83-0 | CAS-1989601-78-6 | CAS-1989602-29-0 | CAS-1989602-80-3 |
| CAS-1989600-84-1 | CAS-1989601-79-7 | CAS-1989602-30-3 | CAS-1989602-82-5 |
| CAS-1989600-85-2 | CAS-1989601-80-0 | CAS-1989602-31-4 | CAS-1989602-84-7 |
| CAS-1989600-86-3 | CAS-1989601-81-1 | CAS-1989602-32-5 | CAS-1989602-85-8 |
| CAS-1989600-87-4 | CAS-1989601-82-2 | CAS-1989602-33-6 | CAS-1989602-86-9 |
| CAS-1989600-88-5 | CAS-1989601-83-3 | CAS-1989602-34-7 | CAS-1989602-87-0 |
| CAS-1989600-89-6 | CAS-1989601-84-4 | CAS-1989602-35-8 | CAS-1989602-88-1 |
| CAS-1989601-11-7 | CAS-1989601-85-5 | CAS-1989602-36-9 | CAS-1989604-00-3 |
| CAS-1989601-23-1 | CAS-1989601-86-6 | CAS-1989602-37-0 | CAS-1989604-01-4 |
| CAS-1989601-26-4 | CAS-1989601-87-7 | CAS-1989602-38-1 | CAS-1989604-02-5 |
| CAS-1989601-28-6 | CAS-1989601-88-8 | CAS-1989602-39-2 | CAS-1989604-03-6 |
| CAS-1989601-29-7 | CAS-1989601-89-9 | CAS-1989602-40-5 | CAS-1989604-04-7 |
| CAS-1989601-33-3 | CAS-1989601-90-2 | CAS-1989602-41-6 | CAS-1989604-05-8 |
| CAS-1989601-40-2 | CAS-1989601-91-3 | CAS-1989602-42-7 | CAS-1989604-06-9 |
| CAS-1989601-41-3 | CAS-1989601-92-4 | CAS-1989602-43-8 | CAS-1989604-07-0 |
| CAS-1989601-42-4 | CAS-1989601-93-5 | CAS-1989602-44-9 | CAS-1989604-08-1 |
| CAS-1989601-43-5 | CAS-1989601-94-6 | CAS-1989602-45-0 | CAS-1989604-09-2 |
| CAS-1989601-44-6 | CAS-1989601-95-7 | CAS-1989602-46-1 | CAS-1989604-10-5 |
| CAS-1989601-45-7 | CAS-1989601-96-8 | CAS-1989602-47-2 | CAS-1989604-11-6 |
| CAS-1989601-46-8 | CAS-1989601-97-9 | CAS-1989602-48-3 | CAS-1989604-13-8 |
| CAS-1989601-47-9 | CAS-1989601-98-0 | CAS-1989602-49-4 | CAS-1989604-14-9 |
| CAS-1989601-48-0 | CAS-1989601-99-1 | CAS-1989602-50-7 | CAS-1989604-15-0 |
| CAS-1989601-49-1 | CAS-1989602-00-7 | CAS-1989602-51-8 | CAS-1989604-16-1 |
| CAS-1989601-50-4 | CAS-1989602-01-8 | CAS-1989602-52-9 | CAS-1989604-17-2 |
| CAS-1989601-51-5 | CAS-1989602-02-9 | CAS-1989602-53-0 | CAS-1989604-18-3 |
| CAS-1989601-52-6 | CAS-1989602-03-0 | CAS-1989602-54-1 | CAS-1989604-19-4 |
| CAS-1989601-53-7 | CAS-1989602-04-1 | CAS-1989602-55-2 | CAS-1989604-20-7 |
| CAS-1989601-54-8 | CAS-1989602-05-2 | CAS-1989602-56-3 | CAS-1989604-21-8 |
| CAS-1989601-55-9 | CAS-1989602-06-3 | CAS-1989602-57-4 | CAS-1989604-22-9 |
| CAS-1989601-56-0 | CAS-1989602-07-4 | CAS-1989602-58-5 | CAS-1989604-23-0 |
| CAS-1989601-57-1 | CAS-1989602-08-5 | CAS-1989602-59-6 | CAS-1989604-24-1 |
| CAS-1989601-58-2 | CAS-1989602-09-6 | CAS-1989602-60-9 | CAS-1989604-25-2 |
| CAS-1989601-59-3 | CAS-1989602-10-9 | CAS-1989602-61-0 | CAS-1989604-26-3 |
| CAS-1989601-60-6 | CAS-1989602-11-0 | CAS-1989602-62-1 | CAS-1989604-27-4 |
| CAS-1989601-61-7 | CAS-1989602-12-1 | CAS-1989602-63-2 | CAS-1989604-28-5 |
| CAS-1989601-62-8 | CAS-1989602-13-2 | CAS-1989602-64-3 | CAS-1989604-29-6 |
| CAS-1989601-63-9 | CAS-1989602-14-3 | CAS-1989602-65-4 | CAS-1989604-30-9 |
| CAS-1989601-64-0 | CAS-1989602-15-4 | CAS-1989602-66-5 | CAS-1989604-31-0 |
| CAS-1989601-65-1 | CAS-1989602-16-5 | CAS-1989602-67-6 | CAS-1989604-32-1 |
| CAS-1989601-66-2 | CAS-1989602-17-6 | CAS-1989602-68-7 | CAS-1989604-33-2 |
| CAS-1989601-67-3 | CAS-1989602-18-7 | CAS-1989602-69-8 | CAS-1989604-34-3 |
| CAS-1989604-35-4 | CAS-1989604-88-7 | CAS-1989605-52-8 | CAS-1989606-07-6 |
| CAS-1989604-36-5 | CAS-1989604-89-8 | CAS-1989605-53-9 | CAS-1989606-08-7 |
| CAS-1989604-37-6 | CAS-1989604-90-1 | CAS-1989605-54-0 | CAS-1989606-09-8 |
| CAS-1989604-38-7 | CAS-1989604-92-3 | CAS-1989605-55-1 | CAS-1989606-10-1 |
| CAS-1989604-39-8 | CAS-1989604-93-4 | CAS-1989605-56-2 | CAS-1989606-11-2 |
| CAS-1989604-40-1 | CAS-1989604-94-5 | CAS-1989605-57-3 | CAS-1989606-12-3 |
| CAS-1989604-41-2 | CAS-1989604-95-6 | CAS-1989605-58-4 | CAS-1989606-13-4 |
| CAS-1989604-42-3 | CAS-1989604-96-7 | CAS-1989605-59-5 | CAS-1989606-14-5 |
| CAS-1989604-43-4 | CAS-1989604-97-8 | CAS-1989605-61-9 | CAS-1989606-15-6 |
| CAS-1989604-45-6 | CAS-1989605-09-5 | CAS-1989605-62-0 | CAS-1989606-16-7 |
| CAS-1989604-46-7 | CAS-1989605-10-8 | CAS-1989605-63-1 | CAS-1989606-17-8 |
| CAS-1989604-47-8 | CAS-1989605-11-9 | CAS-1989605-64-2 | CAS-1989606-18-9 |
| CAS-1989604-48-9 | CAS-1989605-13-1 | CAS-1989605-65-3 | CAS-1989606-19-0 |
| CAS-1989604-49-0 | CAS-1989605-14-2 | CAS-1989605-66-4 | CAS-1989606-20-3 |
| CAS-1989604-50-3 | CAS-1989605-15-3 | CAS-1989605-67-5 | CAS-1989606-21-4 |
| CAS-1989604-52-5 | CAS-1989605-16-4 | CAS-1989605-68-6 | CAS-1989606-22-5 |
| CAS-1989604-53-6 | CAS-1989605-17-5 | CAS-1989605-69-7 | CAS-1989606-23-6 |
| CAS-1989604-54-7 | CAS-1989605-18-6 | CAS-1989605-70-0 | CAS-1989606-24-7 |
| CAS-1989604-55-8 | CAS-1989605-19-7 | CAS-1989605-71-1 | CAS-1989606-26-9 |
| CAS-1989604-56-9 | CAS-1989605-20-0 | CAS-1989605-72-2 | CAS-1989606-27-0 |
| CAS-1989604-57-0 | CAS-1989605-21-1 | CAS-1989605-73-3 | CAS-1989606-28-1 |
| CAS-1989604-58-1 | CAS-1989605-22-2 | CAS-1989605-74-4 | CAS-1989606-29-2 |
| CAS-1989604-59-2 | CAS-1989605-23-3 | CAS-1989605-75-5 | CAS-1989606-30-5 |
| CAS-1989604-60-5 | CAS-1989605-24-4 | CAS-1989605-76-6 | CAS-1989606-31-6 |
| CAS-1989604-61-6 | CAS-1989605-25-5 | CAS-1989605-77-7 | CAS-1989606-32-7 |
| CAS-1989604-62-7 | CAS-1989605-26-6 | CAS-1989605-78-8 | CAS-1989606-33-8 |
| CAS-1989604-63-8 | CAS-1989605-27-7 | CAS-1989605-79-9 | CAS-1989606-34-9 |
| CAS-1989604-64-9 | CAS-1989605-28-8 | CAS-1989605-81-3 | CAS-1989606-35-0 |
| CAS-1989604-65-0 | CAS-1989605-29-9 | CAS-1989605-82-4 | CAS-1989606-36-1 |
| CAS-1989604-66-1 | CAS-1989605-30-2 | CAS-1989605-83-5 | CAS-1989606-37-2 |
| CAS-1989604-67-2 | CAS-1989605-31-3 | CAS-1989605-84-6 | CAS-1989606-38-3 |
| CAS-1989604-68-3 | CAS-1989605-32-4 | CAS-1989605-85-7 | CAS-1989606-39-4 |
| CAS-1989604-69-4 | CAS-1989605-33-5 | CAS-1989605-86-8 | CAS-1989606-40-7 |
| CAS-1989604-70-7 | CAS-1989605-34-6 | CAS-1989605-87-9 | CAS-1989606-41-8 |
| CAS-1989604-71-8 | CAS-1989605-35-7 | CAS-1989605-88-0 | CAS-1989606-42-9 |
| CAS-1989604-72-9 | CAS-1989605-36-8 | CAS-1989605-89-1 | CAS-1989606-43-0 |
| CAS-1989604-73-0 | CAS-1989605-37-9 | CAS-1989605-90-4 | CAS-1989606-44-1 |
| CAS-1989604-74-1 | CAS-1989605-38-0 | CAS-1989605-91-5 | CAS-1989606-45-2 |
| CAS-1989604-75-2 | CAS-1989605-39-1 | CAS-1989605-92-6 | CAS-1989606-46-3 |
| CAS-1989604-76-3 | CAS-1989605-40-4 | CAS-1989605-93-7 | CAS-1989606-48-5 |
| CAS-1989604-77-4 | CAS-1989605-41-5 | CAS-1989605-94-8 | CAS-1989606-49-6 |
| CAS-1989604-78-5 | CAS-1989605-42-6 | CAS-1989605-95-9 | CAS-1989606-53-2 |
| CAS-1989604-79-6 | CAS-1989605-43-7 | CAS-1989605-96-0 | CAS-1989606-55-4 |
| CAS-1989604-80-9 | CAS-1989605-44-8 | CAS-1989605-97-1 | CAS-1989606-56-5 |
| CAS-1989604-81-0 | CAS-1989605-45-9 | CAS-1989605-98-2 | CAS-1989606-61-2 |
| CAS-1989604-82-1 | CAS-1989605-46-0 | CAS-1989605-99-3 | CAS-1989606-62-3 |
| CAS-1989604-83-2 | CAS-1989605-47-1 | CAS-1989606-00-9 | CAS-1989606-63-4 |
| CAS-1989604-84-3 | CAS-1989605-48-2 | CAS-1989606-01-0 | CAS-1989606-67-8 |
| CAS-1989604-85-4 | CAS-1989605-49-3 | CAS-1989606-04-3 | CAS-1989606-69-0 |
| CAS-1989604-86-5 | CAS-1989605-50-6 | CAS-1989606-05-4 | CAS-1989606-70-3 |
| CAS-1989604-87-6 | CAS-1989605-51-7 | CAS-1989606-06-5 | CAS-1989606-74-7 |
| CAS-1989658-39-0 | CAS-2088184-56-7 | CAS-2088185-07-1 | CAS-2088185-66-2 |
| CAS-1989658-41-4 | CAS-2088184-57-8 | CAS-2088185-08-2 | CAS-2088185-67-3 |
| CAS-1989658-43-6 | CAS-2088184-58-9 | CAS-2088185-09-3 | CAS-2088185-68-4 |
| CAS-1989658-47-0 | CAS-2088184-59-0 | CAS-2088185-10-6 | CAS-2088185-69-5 |
| CAS-1989658-49-2 | CAS-2088184-60-3 | CAS-2088185-11-7 | CAS-2088185-70-8 |
| CAS-2088184-07-8 | CAS-2088184-61-4 | CAS-2088185-12-8 | CAS-2088185-71-9 |
| CAS-2088184-08-9 | CAS-2088184-62-5 | CAS-2088185-13-9 | CAS-2088185-72-0 |
| CAS-2088184-09-0 | CAS-2088184-63-6 | CAS-2088185-14-0 | CAS-2088185-73-1 |
| CAS-2088184-10-3 | CAS-2088184-64-7 | CAS-2088185-15-1 | CAS-2088185-74-2 |
| CAS-2088184-11-4 | CAS-2088184-65-8 | CAS-2088185-16-2 | CAS-2088185-75-3 |
| CAS-2088184-13-6 | CAS-2088184-66-9 | CAS-2088185-17-3 | CAS-2088185-76-4 |
| CAS-2088184-14-7 | CAS-2088184-67-0 | CAS-2088185-18-4 | CAS-2088185-77-5 |
| CAS-2088184-15-8 | CAS-2088184-68-1 | CAS-2088185-19-5 | CAS-2088185-78-6 |
| CAS-2088184-16-9 | CAS-2088184-69-2 | CAS-2088185-20-8 | CAS-2088185-79-7 |
| CAS-2088184-17-0 | CAS-2088184-70-5 | CAS-2088185-21-9 | CAS-2088185-80-0 |
| CAS-2088184-18-1 | CAS-2088184-71-6 | CAS-2088185-22-0 | CAS-2088185-81-1 |
| CAS-2088184-19-2 | CAS-2088184-72-7 | CAS-2088185-23-1 | CAS-2088185-82-2 |
| CAS-2088184-20-5 | CAS-2088184-73-8 | CAS-2088185-32-2 | CAS-2088185-83-3 |
| CAS-2088184-21-6 | CAS-2088184-74-9 | CAS-2088185-33-3 | CAS-2088185-84-4 |
| CAS-2088184-22-7 | CAS-2088184-75-0 | CAS-2088185-34-4 | CAS-2088185-85-5 |
| CAS-2088184-23-8 | CAS-2088184-76-1 | CAS-2088185-35-5 | CAS-2088185-86-6 |
| CAS-2088184-24-9 | CAS-2088184-77-2 | CAS-2088185-36-6 | CAS-2088185-87-7 |
| CAS-2088184-25-0 | CAS-2088184-78-3 | CAS-2088185-37-7 | CAS-2088185-88-8 |
| CAS-2088184-26-1 | CAS-2088184-79-4 | CAS-2088185-38-8 | CAS-2088185-89-9 |
| CAS-2088184-27-2 | CAS-2088184-80-7 | CAS-2088185-39-9 | CAS-2088185-90-2 |
| CAS-2088184-28-3 | CAS-2088184-81-8 | CAS-2088185-40-2 | CAS-2088185-91-3 |
| CAS-2088184-29-4 | CAS-2088184-82-9 | CAS-2088185-41-3 | CAS-2088185-92-4 |
| CAS-2088184-30-7 | CAS-2088184-83-0 | CAS-2088185-42-4 | CAS-2088185-93-5 |
| CAS-2088184-32-9 | CAS-2088184-84-1 | CAS-2088185-43-5 | CAS-2088185-94-6 |
| CAS-2088184-34-1 | CAS-2088184-85-2 | CAS-2088185-44-6 | CAS-2088185-95-7 |
| CAS-2088184-35-2 | CAS-2088184-86-3 | CAS-2088185-45-7 | CAS-2088185-96-8 |
| CAS-2088184-36-3 | CAS-2088184-87-4 | CAS-2088185-46-8 | CAS-2088185-97-9 |
| CAS-2088184-37-4 | CAS-2088184-88-5 | CAS-2088185-47-9 | CAS-2088185-98-0 |
| CAS-2088184-38-5 | CAS-2088184-89-6 | CAS-2088185-48-0 | CAS-2088185-99-1 |
| CAS-2088184-39-6 | CAS-2088184-90-9 | CAS-2088185-49-1 | CAS-2088186-00-7 |
| CAS-2088184-40-9 | CAS-2088184-91-0 | CAS-2088185-50-4 | CAS-2088186-01-8 |
| CAS-2088184-41-0 | CAS-2088184-92-1 | CAS-2088185-51-5 | CAS-2088186-02-9 |
| CAS-2088184-42-1 | CAS-2088184-93-2 | CAS-2088185-52-6 | CAS-2088195-88-2 |
| CAS-2088184-43-2 | CAS-2088184-94-3 | CAS-2088185-53-7 | CAS-2088195-89-3 |
| CAS-2088184-44-3 | CAS-2088184-95-4 | CAS-2088185-54-8 | CAS-2088195-90-6 |
| CAS-2088184-45-4 | CAS-2088184-96-5 | CAS-2088185-55-9 | CAS-2088195-91-7 |
| CAS-2088184-46-5 | CAS-2088184-97-6 | CAS-2088185-56-0 | CAS-861806-70-4 |
| CAS-2088184-47-6 | CAS-2088184-98-7 | CAS-2088185-57-1 | CAS-1269508-30-6 |
| CAS-2088184-48-7 | CAS-2088184-99-8 | CAS-2088185-58-2 | |
| CAS-2088184-49-8 | CAS-2088185-00-4 | CAS-2088185-59-3 | |
| CAS-2088184-50-1 | CAS-2088185-01-5 | CAS-2088185-60-6 | |
| CAS-2088184-51-2 | CAS-2088185-02-6 | CAS-2088185-61-7 | |
| CAS-2088184-52-3 | CAS-2088185-03-7 | CAS-2088185-62-8 | |
| CAS-2088184-53-4 | CAS-2088185-04-8 | CAS-2088185-63-9 | |
| CAS-2088184-54-5 | CAS-2088185-05-9 | CAS-2088185-64-0 | |
| CAS-2088184-55-6 | CAS-2088185-06-0 | CAS-2088185-65-1 | |

In einer bevorzugten Ausführungsform der Erfindung werden die Verbindungen gemäß Formel (I-A-4) als lochtransportierendes Material eingesetzt. Die Verbindungen liegen dann bevorzugt in einer lochtransportierenden Schicht vor. Bevorzugte Ausführungsformen von lochtransportierenden Schichten sind Lochtransportschichten, Elektronenblockierschichten und Lochinjektionsschichten. Wenn die Verbindung der Formel (I-A-4) in einer lochtransportierenden Schicht vorliegt, ist diese bevorzugt eine elektronenblockierende Schicht. Diese grenzt bevorzugt anodenseitig direkt an die emittierende Schicht an.

Eine Lochtransportschicht gemäß der vorliegenden Anmeldung ist eine Schicht mit lochtransportierender Funktion, welche sich zwischen Anode und emittierender Schicht befindet. Insbesondere ist es eine lochtransportierende Schicht, die keine Lochinjektionsschicht und keine Elektronenblockierschicht ist.

Lochinjektionsschichten und Elektronenblockierschichten werden im Sinne der vorliegenden Anmeldung als spezielle Ausführungsformen von lochtransportierenden Schichten verstanden. Eine Lochinjektionsschicht ist dabei im Fall von mehreren lochtransportierenden Schichten zwischen Anode und emittierender Schicht eine lochtransportierende Schicht, welche sich direkt an die Anode anschließt oder nur durch eine einzelne Beschichtung der Anode von ihr getrennt ist. Eine Elektronenblockierschicht ist im Fall von mehreren lochtransportierenden Schichten zwischen Anode und emittierender Schicht diejenige lochtransportierende Schicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I-A-4) enthält, besonders bevorzugt genau eine oder zwei eine Verbindung gemäß Formel (I-A-4) enthalten.

Wird die Verbindung gemäß Formel (I-A-4) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I-A-4) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 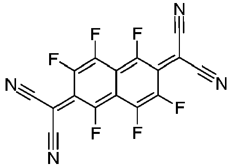 | 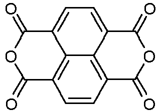 | 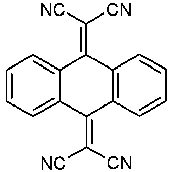 |
| (D-1) | (D-2) | (D-3) |
| 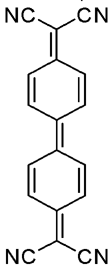 | 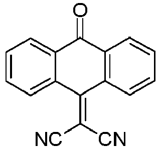 | 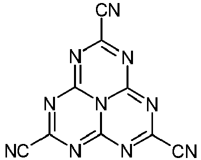 |
| (D-4) | (D-5) | (D-6) |
| 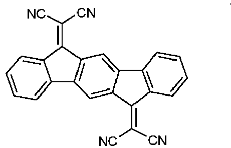 | 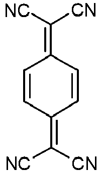 | 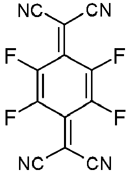 |
| (D-7) | (D-8) | (D-9) |
| 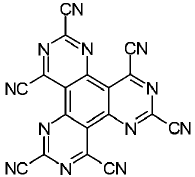 | 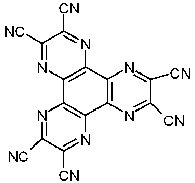 | 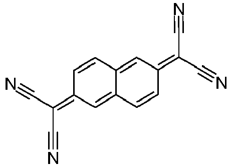 |
| (D-10) | (D-11) | (D-12) |
| 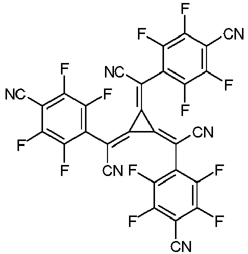 | 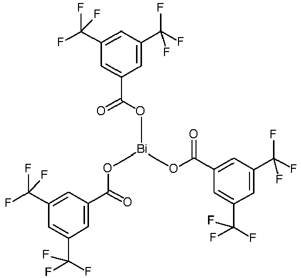 | |
| (D-13) | (D-14) | |

In einer weiteren bevorzugten Ausführungsform der Erfindung wird die Verbindung gemäß Formel (I-A-4) als Lochtransportmaterial in Kombination mit einem Hexaazatriphenylenderivat in einer OLED verwendet. Besonders bevorzugt wird das Hexaazatriphenylenderivat dabei in einer separaten Schicht eingesetzt.

In einer bevorzugten Ausführungsform der vorliegenden Erfindung wird die Verbindung der Formel (I-A-4) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I-A-4) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I-A-4) stellt dabei bevorzugt das Matrixmaterial mit lochtransportierenden Eigenschaften dar. Entsprechend ist, wenn die Verbindung der Formel (I-A-4) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen, darunter insbesondere aus denjenigen, die elektronentransportierende Eigenschaften aufweisen. Besonders bevorzugte Matrixmaterialien, die in Kombination mit den erfindungsgemäßen Verbindungen als Matrixkomponenten eines Mixed-Matrix-Systems verwendet werden können, sind die folgenden Materialien:

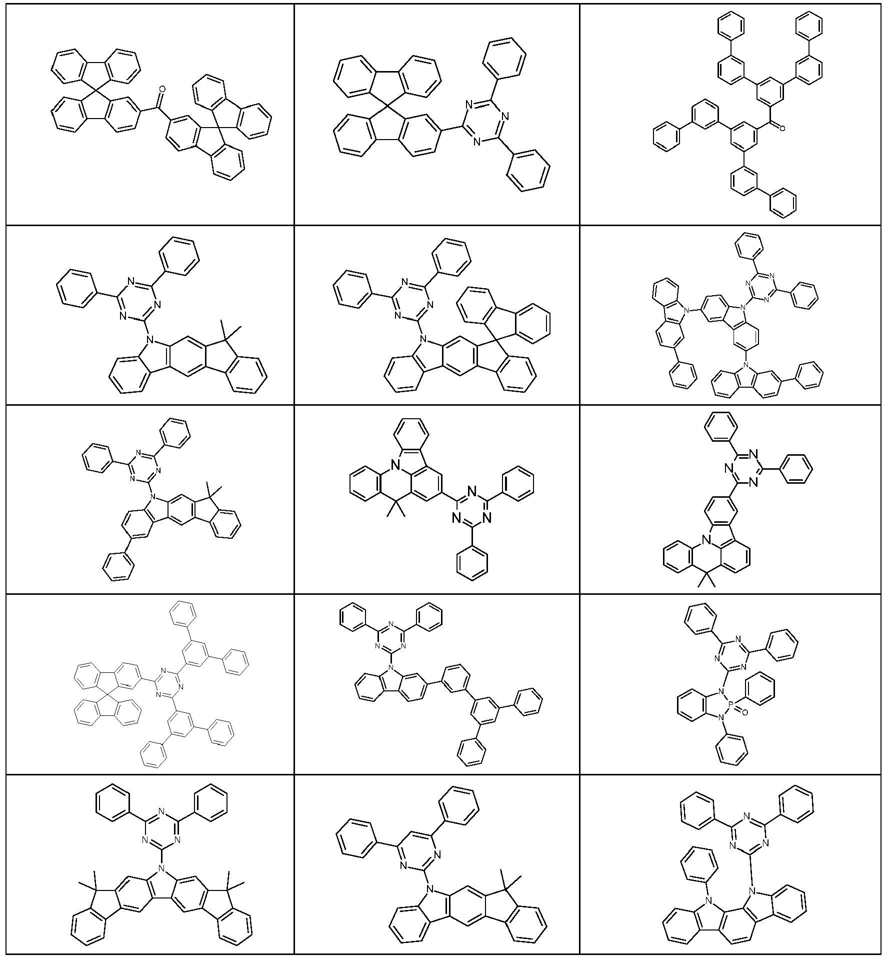

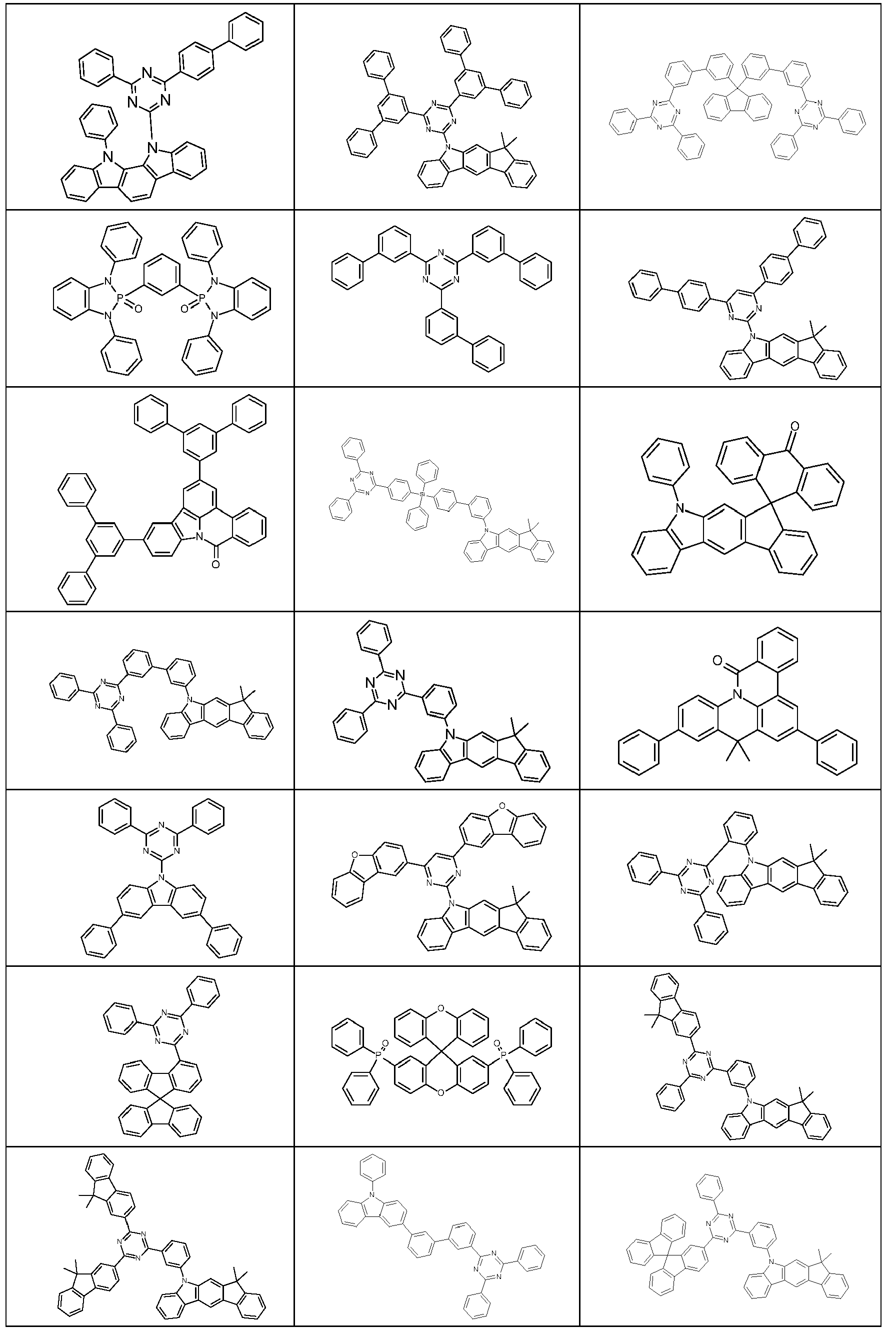

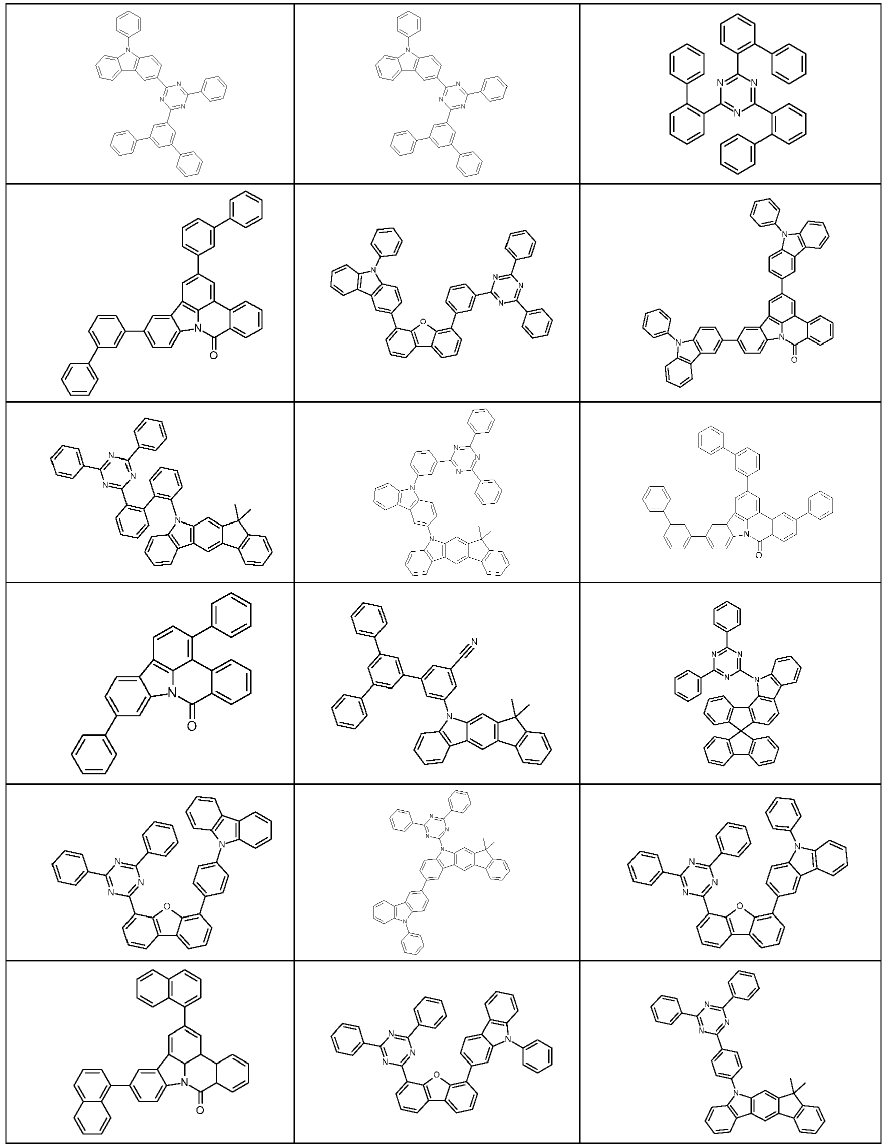

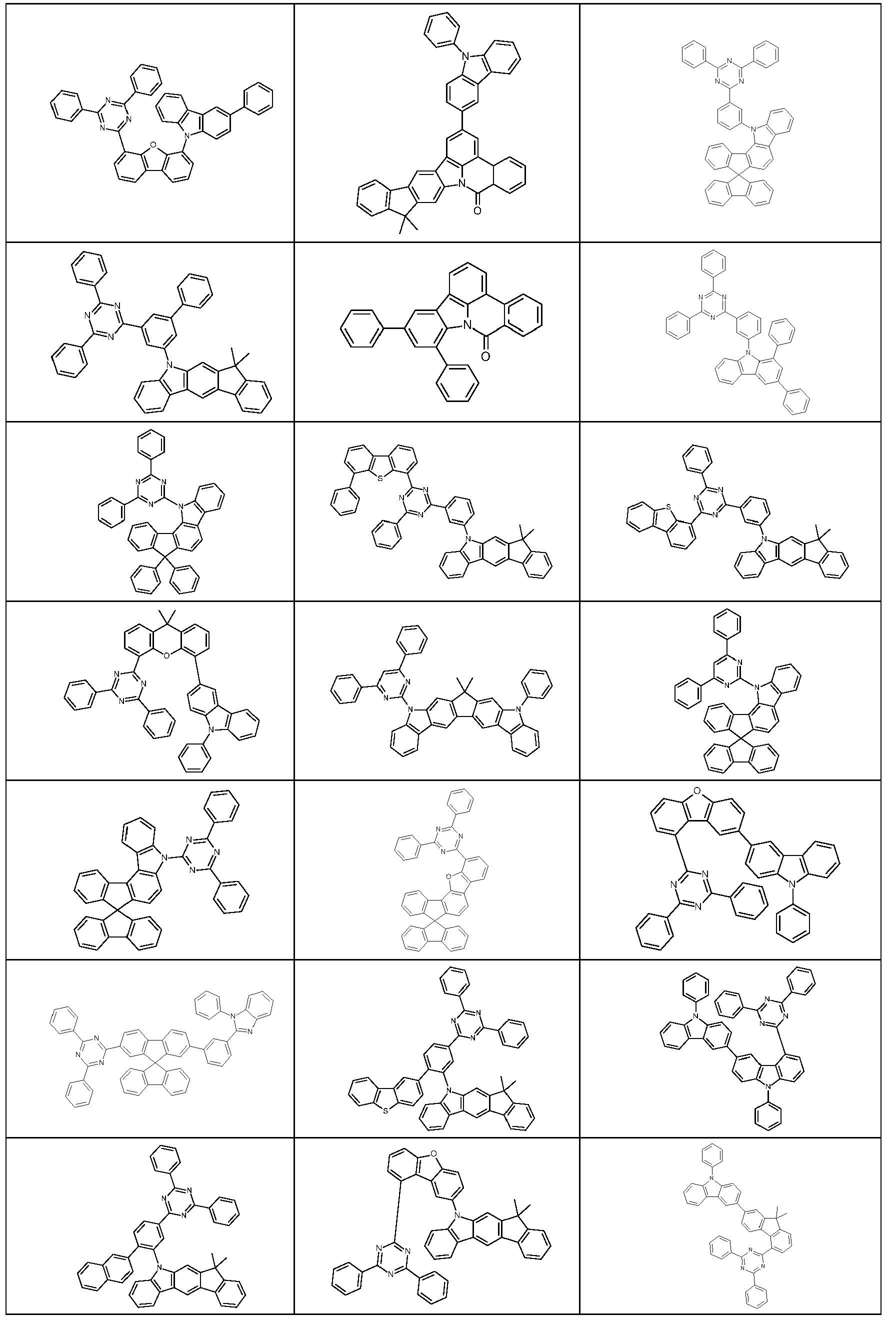

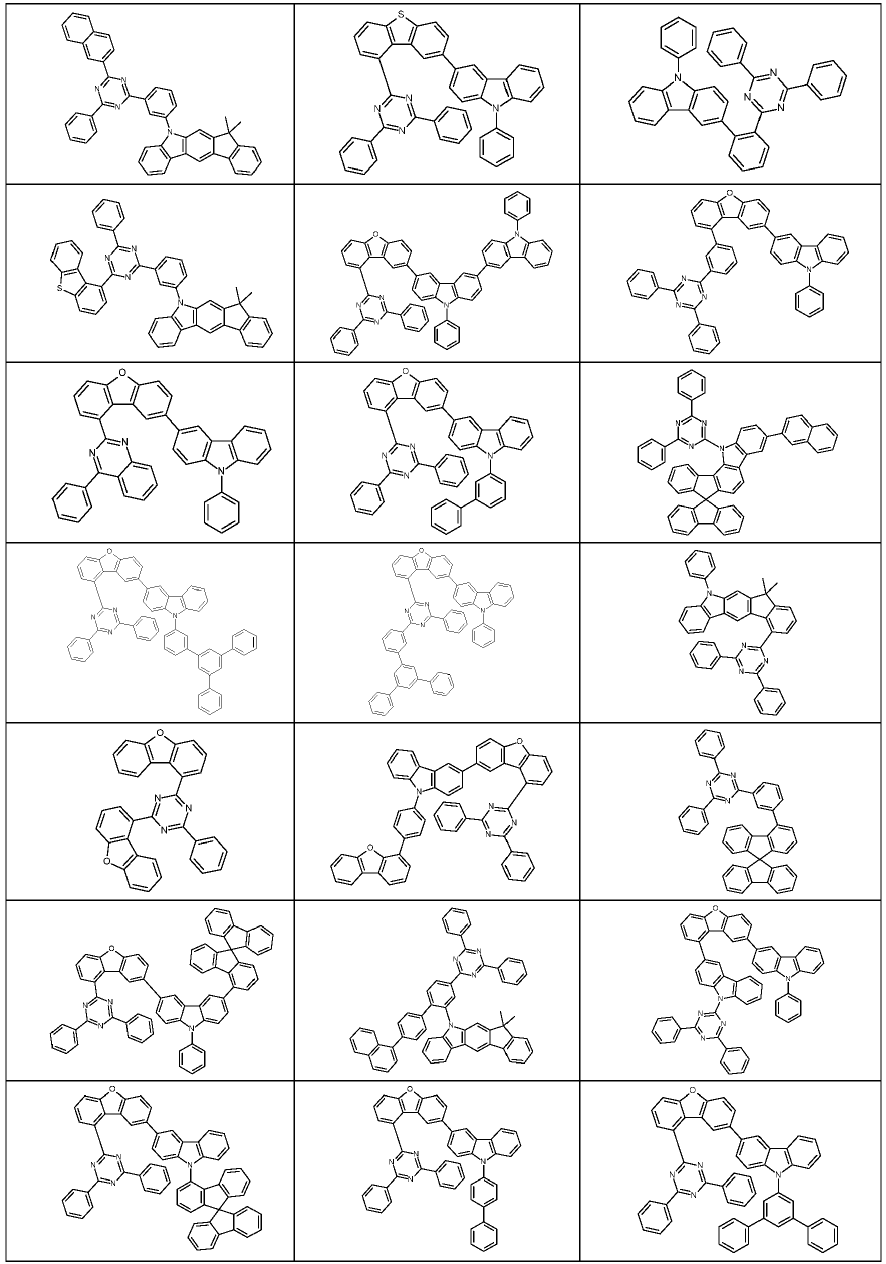

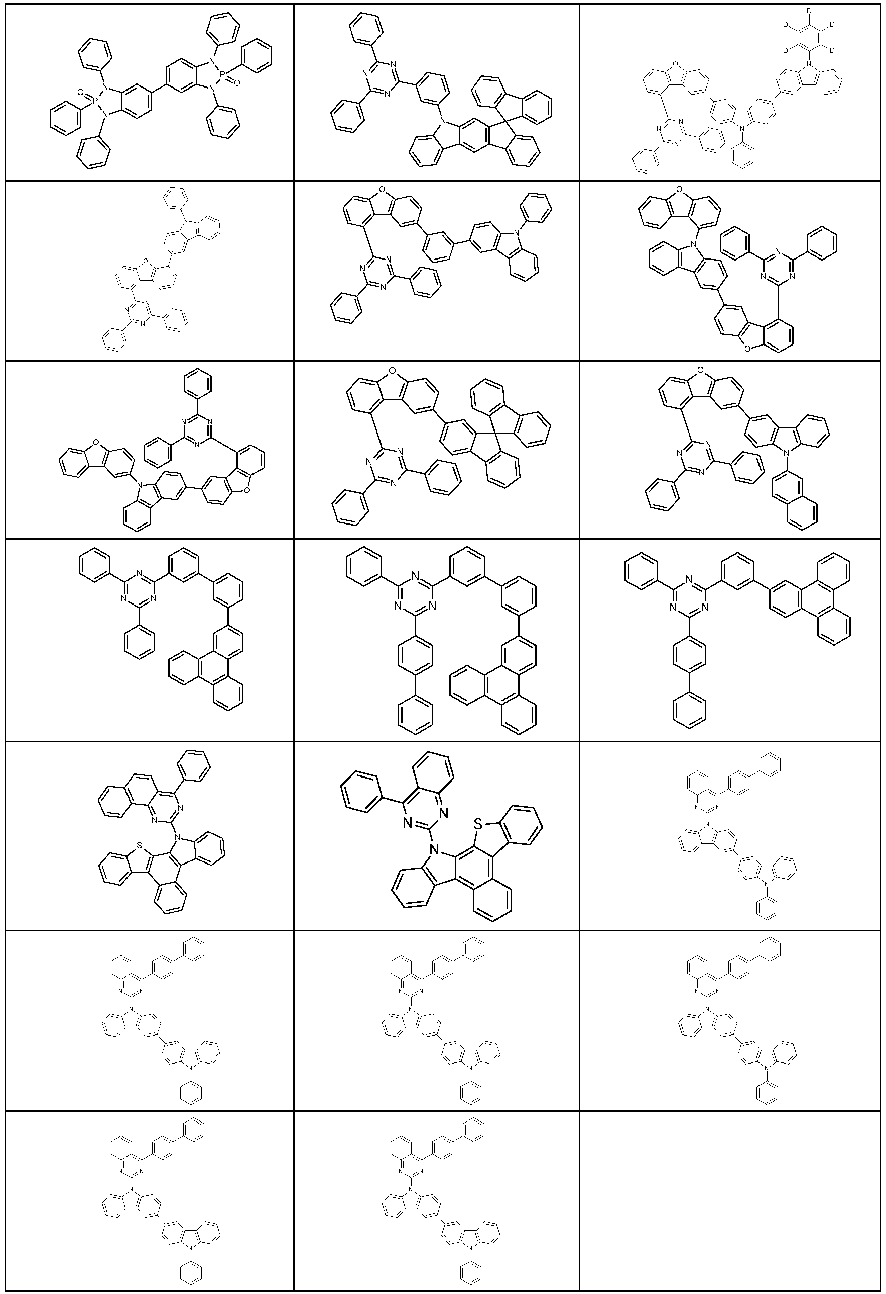

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine, Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren oder Dinaphthylanthracen), insbesondere der Oligoarylene enthaltend kondensierte aromatische Gruppen, der Oligoarylenvinylene (z. B. DPVBi oder Spiro-DPVBi), der polypodalen Metallkomplexe, der lochleitenden Verbindungen, der elektronenleitenden Verbindungen, insbesondere Ketone, Phosphinoxide und Sulfoxide, der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende emittierende Verbindungen sind neben den Verbindungen der Formel (I-A-4) aromatische Ketone, aromatische Phosphinoxide oder aromatische Sulfoxide oder Sulfone, Triarylamine, Carbazolderivate, Indolocarbazolderivate, Indenocarbazolderivate, Azacarbazolderivate, bipolare Matrixmaterialien, Silane, Azaborole oder Boronester, Triazinderivate, Zinckomplexe, Diazasilol- bzw. Tetraazasilol-Derivate, Diazaphosphol-Derivate, überbrückte Carbazol-Derivate, Triphenylenderivate und Lactame.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I-A-4) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugte Materialien für lochtransportierende Schichten der OLEDs sind die folgenden Materialien:

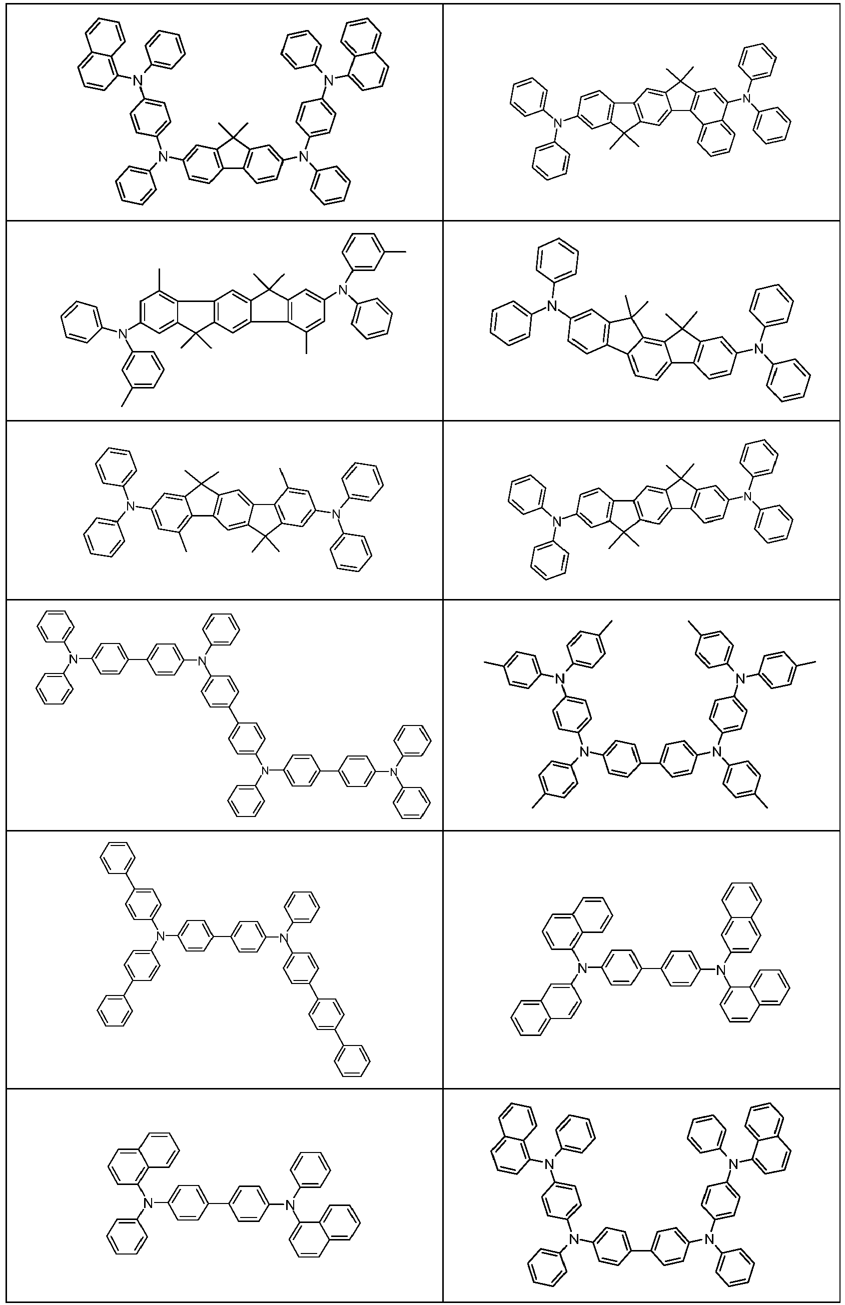

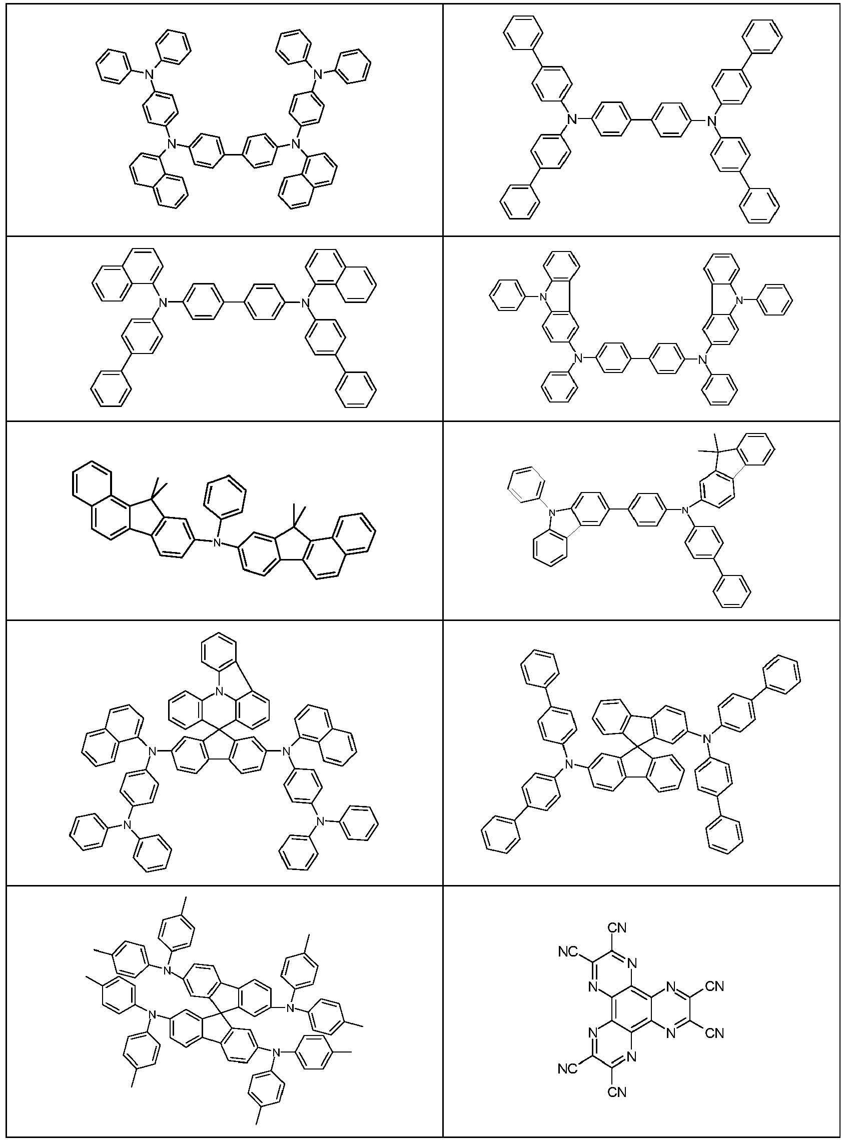

Bevorzugt umfasst die erfindungsgemäße OLED zwei oder mehr unterschiedliche lochtransportierende Schichten. Die Verbindung der Formel (I-A-4) kann dabei in einer oder in mehreren oder in allen lochtransportierenden Schichten eingesetzt werden. Gemäß einer bevorzugten Ausführungsform wird die Verbindung der Formel (I-A-4) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I-A-4) bevorzugt in lochtransportierenden Schichten der erfindungsgemäßen OLEDs eingesetzt werden, sind insbesondere Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Besonders bevorzugt sind die in der folgenden Tabelle gezeigten Verbindungen:

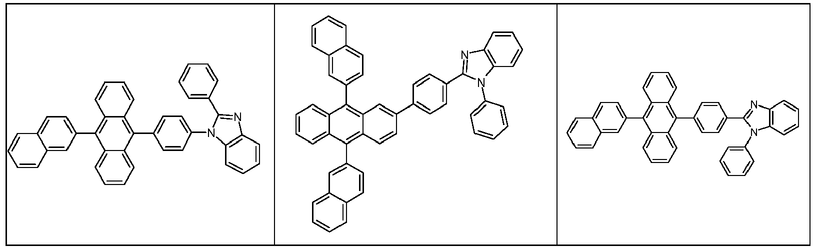

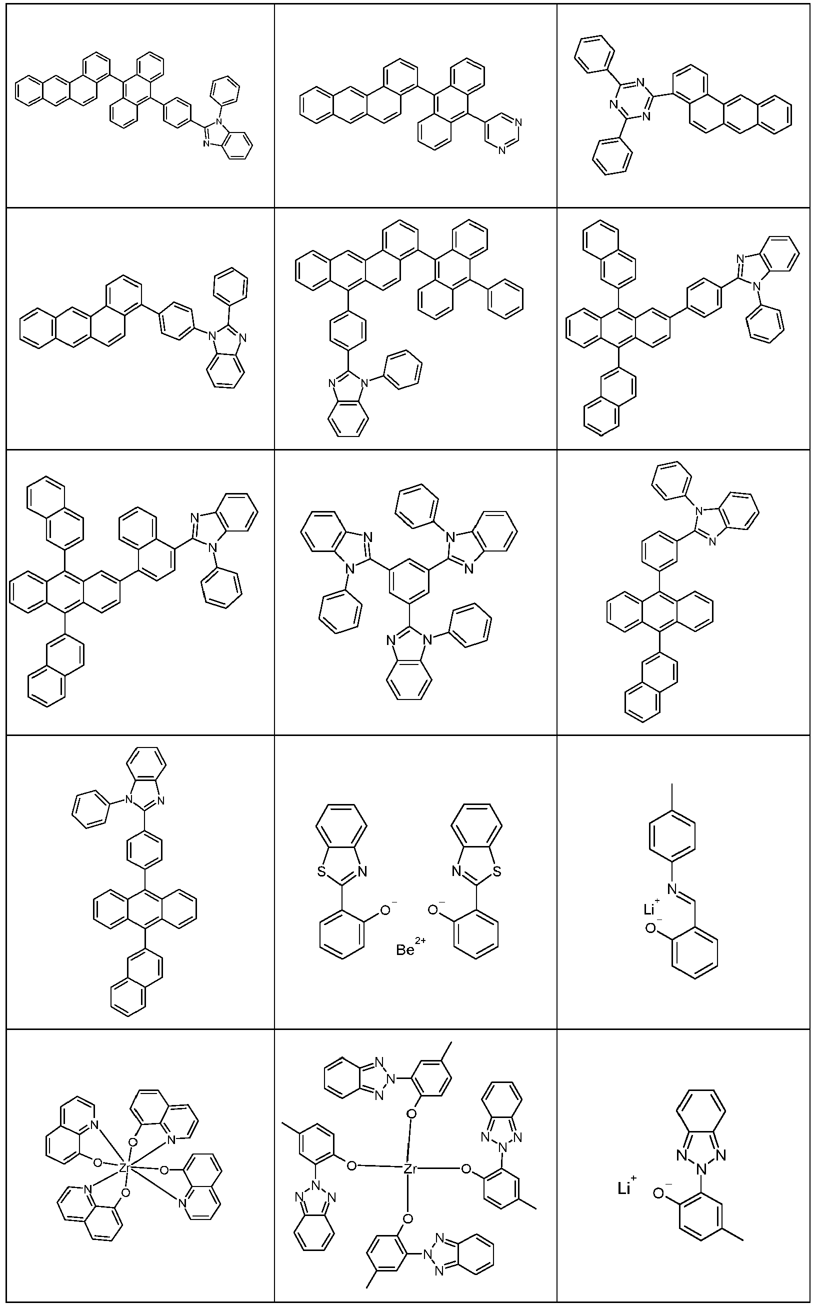

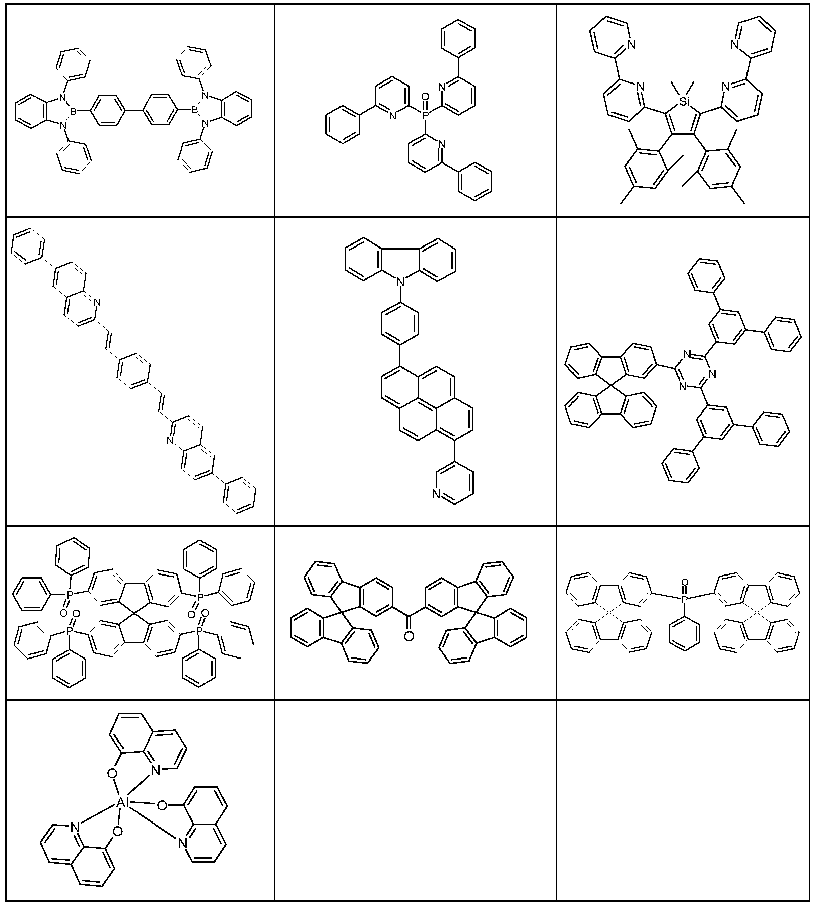

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I-A-4) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I-A-4) in Displays, als Lichtquellen in Beleuchtungsanwendungen sowie als Lichtquellen in medizinischen und/oder kosmetischen Anwendungen eingesetzt werden.

### Beispiele

### A) Synthesebeispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### A-1) Darstellung der Synthone:

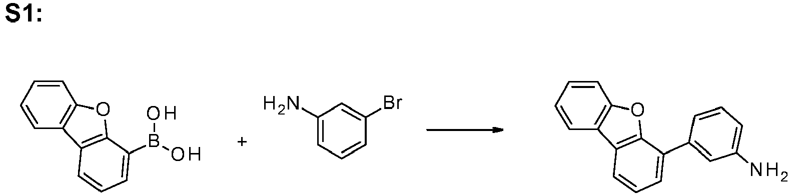

4-Dibenzofuranboronsäure [CAS-100124-06-9] (70.10 g; 330.7 mmol), 3-Bromanilin [CAS-591-19-5] (52.00 g; 302.2 mmol) und Natronlauge 20%ig w/w (180 mL; 1.37 mol) werden in Tetrahydrofuran (750 mL) und Wasser (100 mL) vogelegt und 45 min mit Argon gesättigt. Danach werden Tris(dibenzylidenaceton)-dipalladium(0) [CAS-51364-51-3], (276 mg; 0.30 mmol) und Tri-o-tolylphosphin (920 mg; 3.02 mmol) eingetragen und die Reaktionsmischung 10 h unter Rückfluss gerührt. Nach Abkühlen des Ansatzes wird dieser mit Eisessig auf pH-Wert 7 gestellt, die organische Phase im Scheidetrichter abgetrennt und isoliert. Anschließend wird die organische Phase über eine Fritte mit vorgeschlämmten Kieselgel in Ethylacetat filtriert. Es wird zweimal mit Ethylacetat (je 150 mL) nachgewaschen, das Filtrat wird über Na₂SO₄ getrocknet und anschließend zur Trockene eingeengt. Das Rohprodukt wird aus Ethylacetat umkristallsiert. Ausbeute: 66.5 g (244 mmol), 81%; Reinheit: > 95 %ig nach ¹H-NMR.

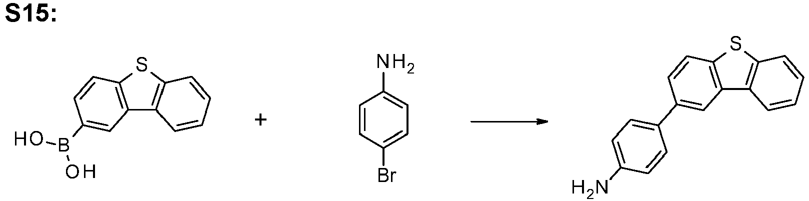

Durchführung analog der Versuchsbeschreibung zu S1, nur dass statt 4-Dibenzofuranboronsäure 2-Dibenzothiophenboronsäure [CAS-668983-97-9] und statt 3-Bromanilin 4-Bromanilin [CAS-106-40-1] verwendet wird. Die Aufarbeitung erfolgt analog. Das Rohprodukt wird aus n-Butanol umkristallisiert. Ausbeute 73.5 g (267 mmol, 88%); Reinheit > 97 %ig nach ¹H-NMR.

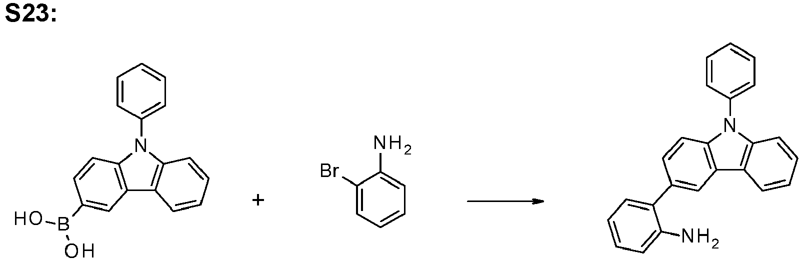

Durchführung analog der Versuchsbeschreibung zu S1, nur dass statt 4-Dibenzofuranboronsäure 3-N-Phenylcarbazolboronsäure [CAS-854952-58-2] und statt 3-Bromanilin 2-Bromanilin [CAS-615-36-1] verwendet wird. Die Aufarbeitung erfolgt analog. Das Rohprodukt wird über Säulenchromatographie aufgereinigt. Ausbeute 48.0 g (144 mmol, 47%); Reinheit > 95 %ig nach ¹H-NMR.

Analog können die folgende Verbindungen dargestellt werden. Hierbei kann zur Aufreinigung auch Säulenchromatographie verwendet werden, oder zur Umkristallisation oder Heißextraktion andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid; N-Methylpyrrolidon etc. verwendet werden. Die Ausbeuten liegen typischerweise im Bereich zwischen 40% und 90%.

| | | |
|---|---|---|
| Edukt 1 | Edukt 2 | Produkt |
| 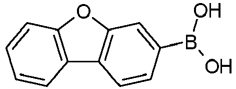 | 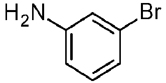 | 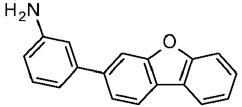 |
| CAS-395087-89-5 | CAS-591-19-5 | S2 |
| 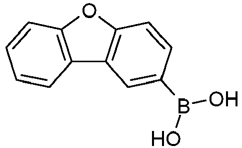 | 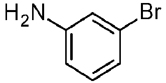 | 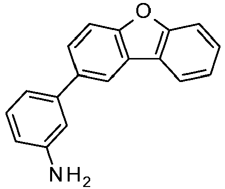 |
| CAS-402936-15-6 | CAS-591-19-5 | S3 |
| 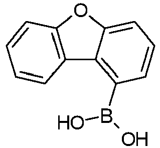 | 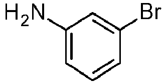 | 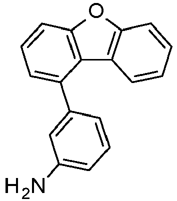 |
| CAS-162607-19-4 | CAS-591-19-5 | S4 |
| 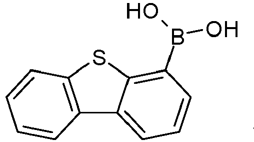 | 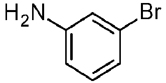 | 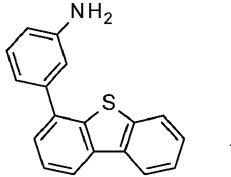 |
| CAS-108847-20-7 | CAS-591-19-5 | S5 |
| 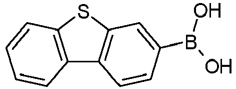 | 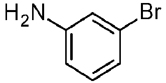 | 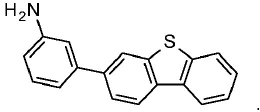 |
| CAS-108847-24-1 | CAS-591-19-5 | S6 |
| 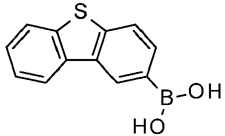 | 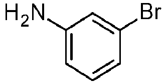 | 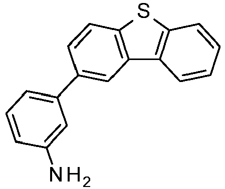 |
| CAS-668983-97-9 | CAS-591-19-5 | S7 |
| 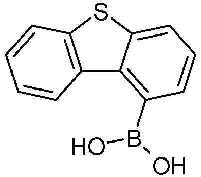 | 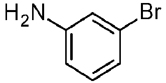 | 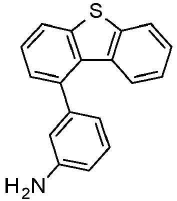 |
| CAS-1245943-60-5 | CAS-591-19-5 | S8 |
| 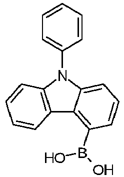 | 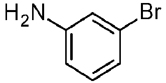 | 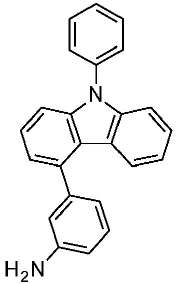 |
| CAS-1370555-65-9 | CAS-591-19-5 | S9 |
| 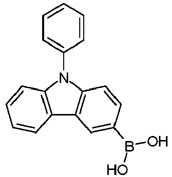 | 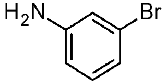 | 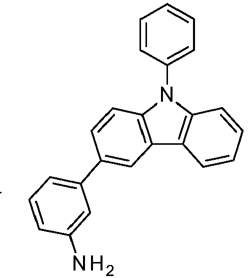 |
| CAS-854952-58-2 | CAS-591-19-5 | S10 |
| 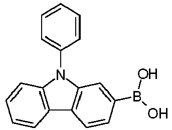 | 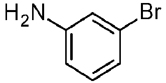 | 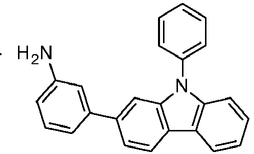 |
| CAS-1001911-63-2 | CAS-591-19-5 | S11 |
| 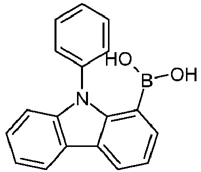 | 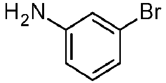 | 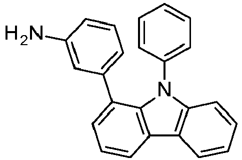 |
| CAS-1333002-41-7 | CAS-591-19-5 | S12 |
| 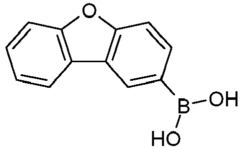 | 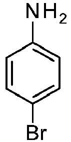 | 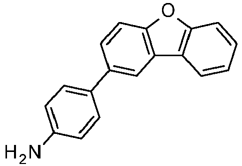 |
| CAS-402936-15-6 | CAS-106-40-1 | S13 |
| 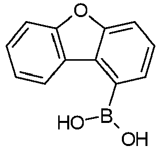 | 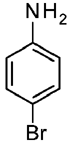 | 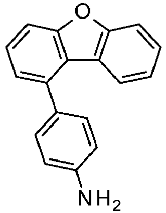 |
| CAS-162607-19-4 | CAS-106-40-1 | S14 |
| 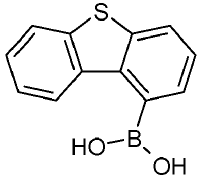 | 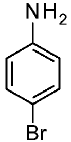 | 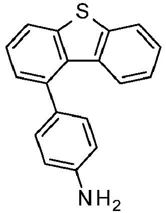 |
| CAS-1245943-60-5 | CAS-106-40-1 | S16 |
| 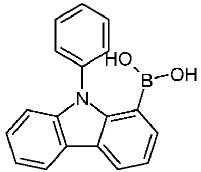 | 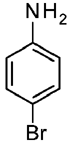 | 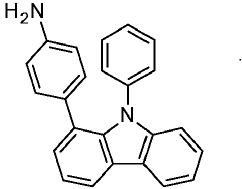 |
| CAS-1333002-41-7 | CAS-106-40-1 | S17 |
| 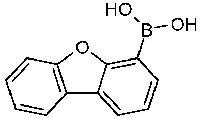 | 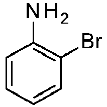 | 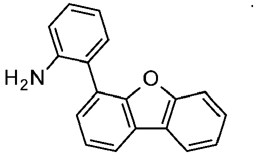 |
| CAS-100124-06-9 | CAS-615-36-1 | S18 |
| 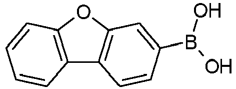 | 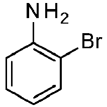 | 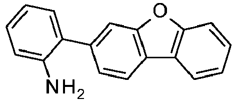 |
| CAS-395087-89-5 | CAS-615-36-1 | S19 |
| 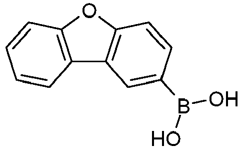 | 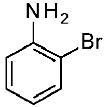 | 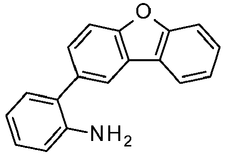 |
| CAS-402936-15-6 | CAS-615-36-1 | S20 |
| 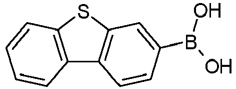 | 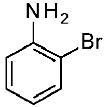 | 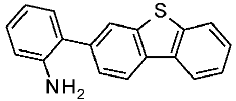 |
| CAS-108847-24-1 | CAS-615-36-1 | S21 |
| 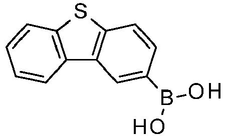 | 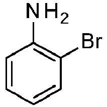 | 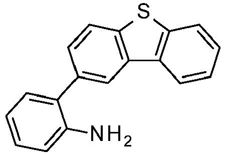 |
| CAS-668983-97-9 | CAS-615-36-1 | S22 |
| 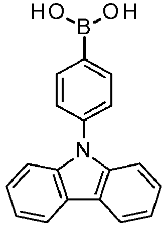 | 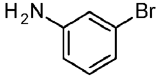 | 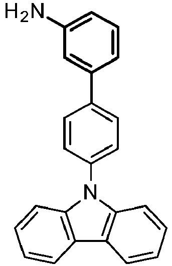 |
| CAS-419536-33-7 | CAS-591-19-5 | S24 |
| 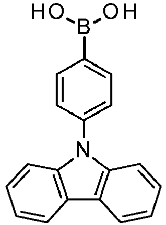 | 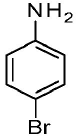 | 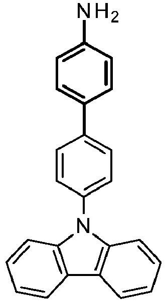 |
| CAS-419536-33-7 | CAS-106-40-1 | S25 |
| 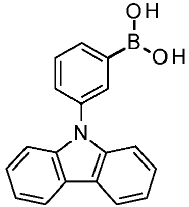 | 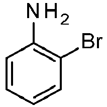 | 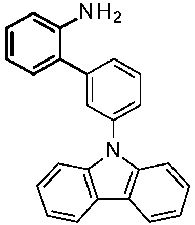 |
| CAS-864377-33-3 | CAS-615-36-1 | S26 |
| 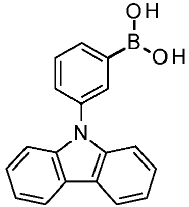 | 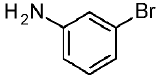 | 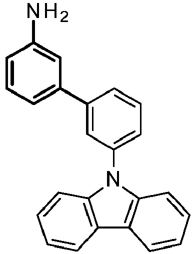 |
| CAS-864377-33-3 | CAS-591-19-5 | S27 |
| 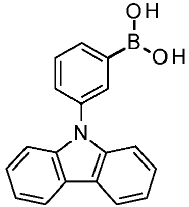 | 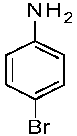 | 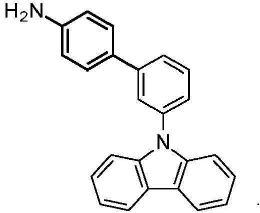 |
| CAS-864377-33-3 | CAS-106-40-1 | S28 |
| 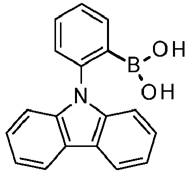 | 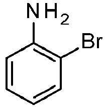 | 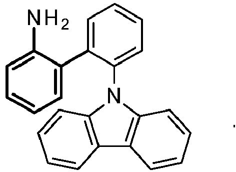 |
| CAS-1189047-28-6 | CAS-615-36-1 | S29 |
| 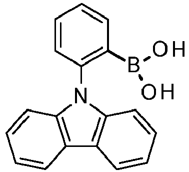 | 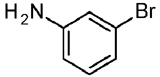 | 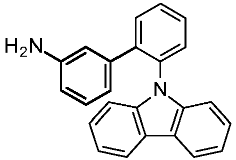 |
| CAS-1189047-28-6 | CAS-591-19-5 | S30 |
| 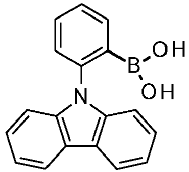 | 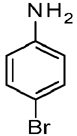 | 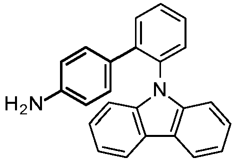 |
| CAS-1189047-28-6 | CAS-106-40-1 | S31 |
| 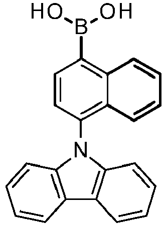 | 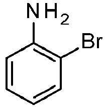 | 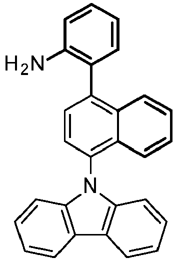 |
| CAS-1246021-50-0 | CAS-615-36-1 | S32 |
| 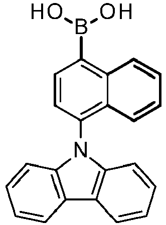 | 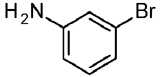 | 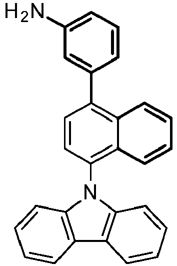 |
| CAS-1246021-50-0 | CAS-591-19-5 | S33 |
| 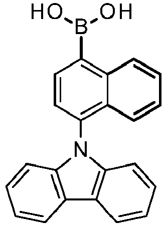 | 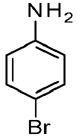 | 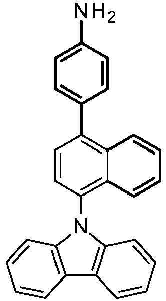 |
| CAS-1246021-50-0 | CAS-106-40-1 | S34 |
| 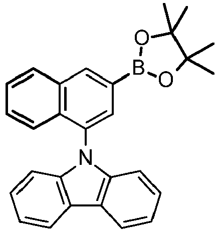 | 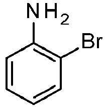 | 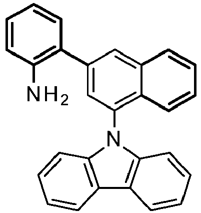 |
| CAS-1819346-26-3 | CAS-615-36-1 | S35 |
| 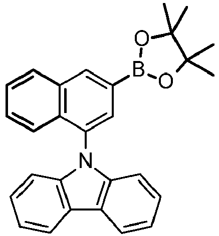 | 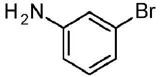 | 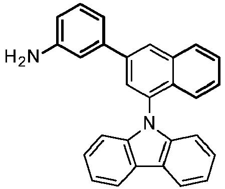 |
| CAS-1819346-26-3 | CAS-591-19-5 | S36 |
| 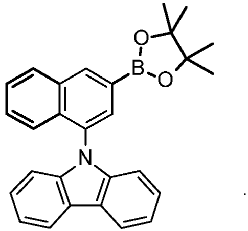 | 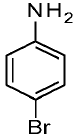 | 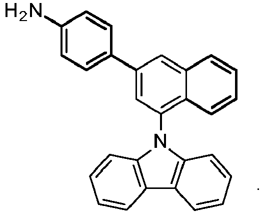 |
| CAS-1819346-26-3 | CAS-106-40-1 | S37 |
| 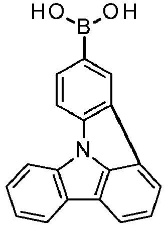 | 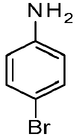 | 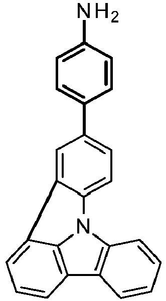 |
| CAS-1174032-92-8 | CAS-106-40-1 | S38 |
| 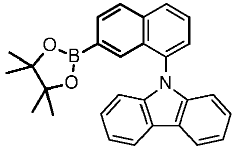 | 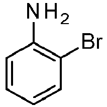 | 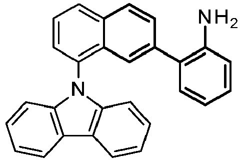 |
| CAS-1221685-92-2 | CAS-615-36-1 | S39 |
| 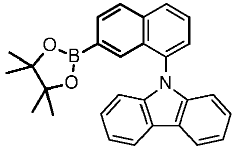 | 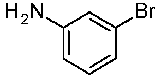 | 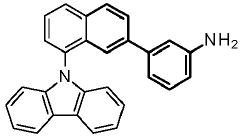 |
| CAS-1221685-92-2 | CAS-591-19-5 | S40 |
| 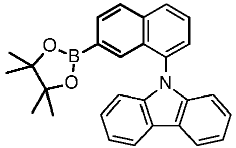 | 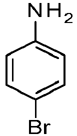 | 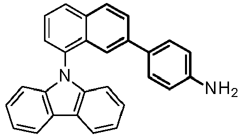 |
| CAS-1221685-92-2 | CAS-106-40-1 | S41 |
| 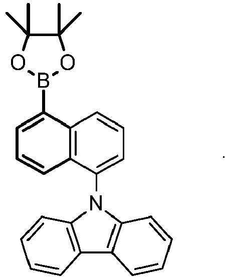 | 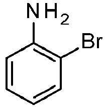 | 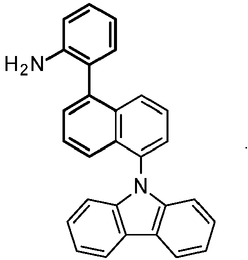 |
| CAS-1820789-13-6 | CAS-615-36-1 | S42 |
| 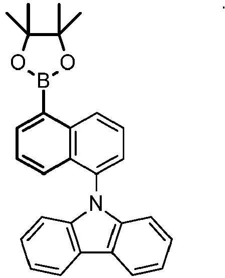 | 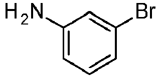 | 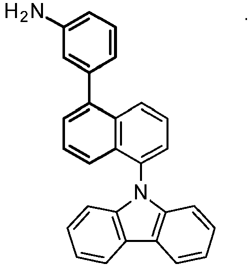 |
| | CAS-591-19-5 | S43 |
| 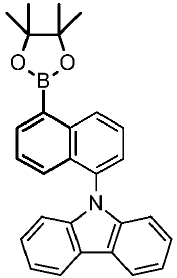 | 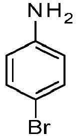 | 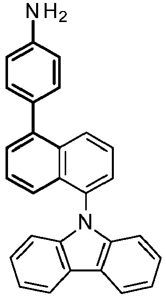 |
| | CAS-106-40-1 | S44 |
| 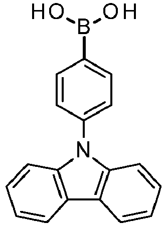 | 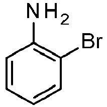 | 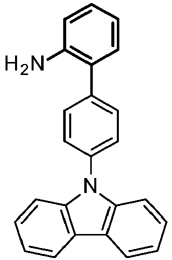 |
| CAS-419536-33-7 | CAS-615-36-1 | S45 |

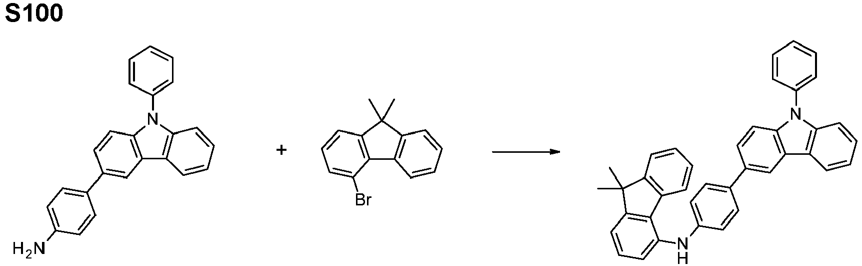

4-(9-Phenylcarbazol-3-yl)anilin [CAS-1370034-59-5] (16.72 g; 50.0 mmol), 4-Bromo-9,9-dimethyl-9H-fluoren [CAS-942615-32-9 ] (14.34 g; 52.5 mmol) und Caesiumcarbonat (32.58 g; 100.0 mmol) werden in o-Xylol (300 mL) vogelegt und 45 min mit Argon gesättigt. Danach wird 1,1-Bis(diphenylphosphino)ferrocen-dichloropalladium(II) Komplex mit DCM [CAS-95464-05-4] (1.22 g; 1.5 mmol) eingetragen und die Reaktionsmischung 24 h unter Rückfluss gerührt. Nach Abkühlen des Ansatzes wird dieser mit 400 ml Toluol erweitert, und die organische Phase mit Wasser (2 x 400 mL) gewaschen. Die organische Phase wird über ein Celitebett vorgeschlämmt, mit Toluol filtriert, das Filtrat über Na₂SO₄ getrocknet und zur Trockene eingeengt. Der Rückstand wird aus Cyclohexan umkristallisiert. Ausbeute: 20.4 g (38.7 mmol), 77%; Reinheit: > 98 %ig nach ¹H-NMR.

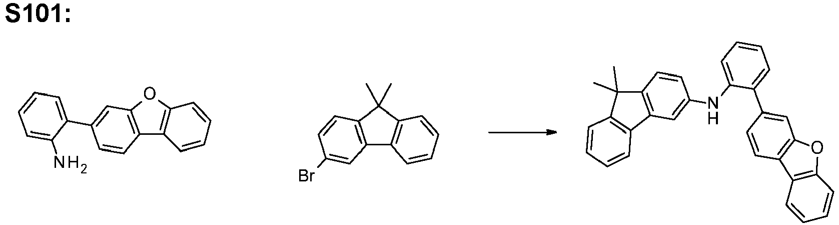

Durchführung analog der Versuchsbeschreibung zu S100, nur dass statt 4-(9-Phenylcarbazol-3-yl)anilin S19 und statt 4-Bromo-9,9-dimethyl-9H-fluoren 3-Bromo-9,9-dimethyl-9*H*-fluoren [CAS-1190360-23-6] verwendet wird. Das Rohprodukt wird über Säulenchromatographie aufgereinigt. Ausbeute: 10.9 g (24.2 mmol), 48%; Reinheit: > 97 %ig nach ¹H-NMR.

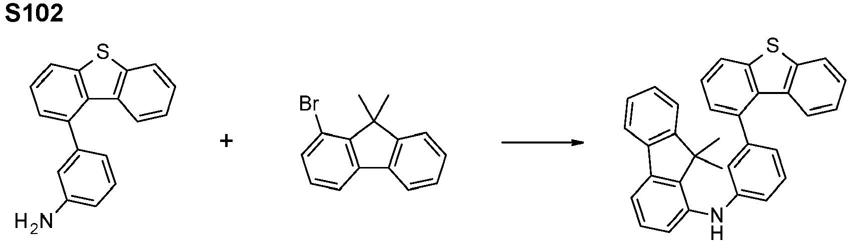

Durchführung analog der Versuchsbeschreibung zu S100, nur dass statt 4-(9-Phenylcarbazol-3-yl)anilin S8 und statt 4-Bromo-9,9-dimethyl-9H-fluoren 1-Bromo-9,9-dimethyl-9H-fluoren [CAS-1225053-54-2] verwendet wird. Das Rohprodukt wird aus Acetonitril umkristallisiert. Ausbeute: 15.9 g (33.9 mmol), 68%; Reinheit: > 97 %ig nach ¹H-NMR.

Analog können folgende Verbindungen dargestellt werden. Hierbei kann als Katalysatorsystem statt 1,1-Bis(diphenylphosphino)ferrocen-dichloropalladium(II) Komplex mit DCM [CAS-95464-05-4] auch Tris(dibenzylideneacetone)dipalladium(0) [CAS-51364-51-3], (0.02 equiv.) und S-Phos [CAS- 657408-07-6] (0.04 equiv) verwendet werden. Hierbei kann zur Aufreinigung auch Säulenchromatographie verwendet werden, oder zur Umkristallisation oder Heißextraktion andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid; N-Methylpyrrolidon etc. verwendet werden. Die Ausbeuten liegen typischerweise im Bereich zwischen 40% und 85%.

| Edukt 1 | Edukt 2 | Produkt |
|---|---|---|
| 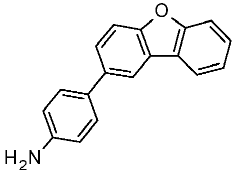 | 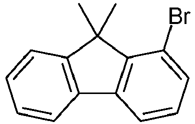 | 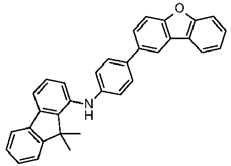 |
| CAS- 1178274-17-3 | CAS-1225053-54-2 | S103 |
| 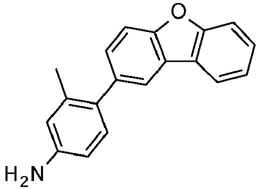 | 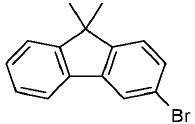 | 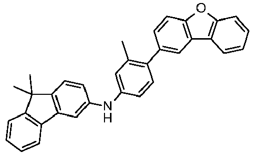 |
| | CAS-1190360-23-6 | S104 |
| CAS-1184428-04-3 | | |
| 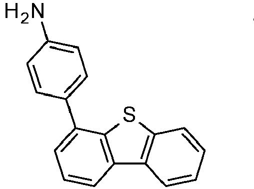 | 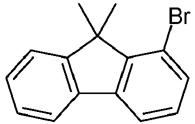 | 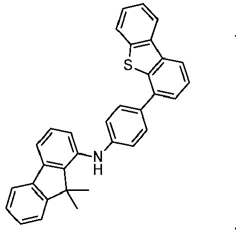 |
| CAS-1370034-50-6 | CAS-1225053-54-2 | S105 |
| 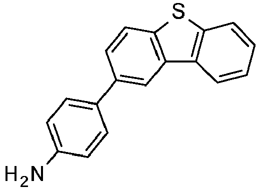 | 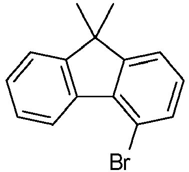 | 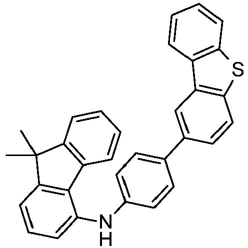 |
| CAS-1574121-68-8 | CAS-942615-32-9 | S106 |
| 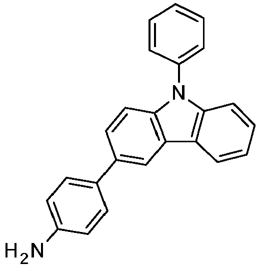 | 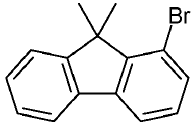 | 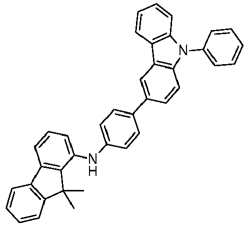 |
| CAS-1370034-59-5 | CAS-1225053-54-2 | S107 |
| 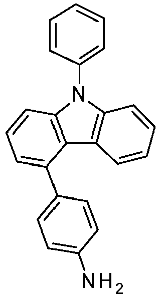 | 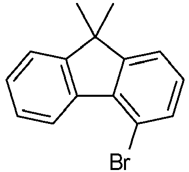 | 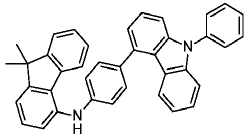 |
| CAS-1629995-08-9 | CAS-942615-32-9 | S108 |
| 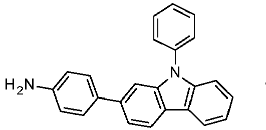 | 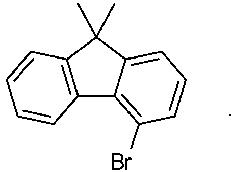 | 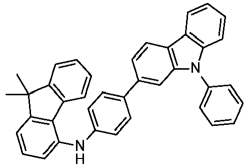 |
| CAS-1911626-45-3 | CAS-942615-32-9 | S109 |
| 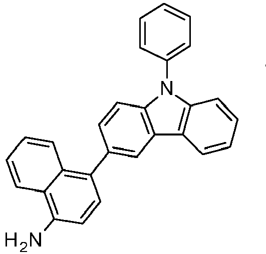 | 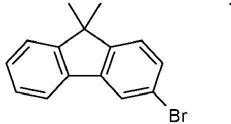 | 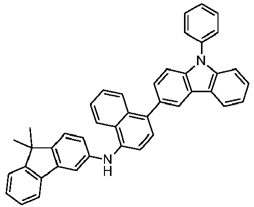 |
| CAS-1699765-82-6 | CAS-1190360-23-6 | S110 |
| 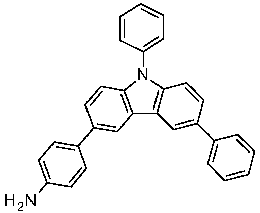 | 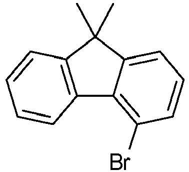 | 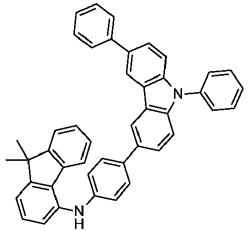 |
| CAS-1458611-14-7 | CAS-942615-32-9 | S111 |
| 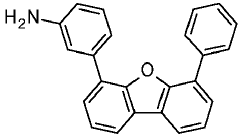 | 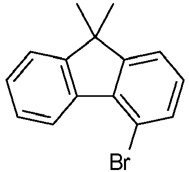 | 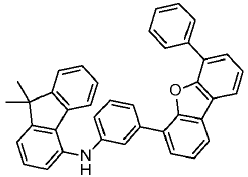 |
| CAS-2101611-03-2 | CAS-942615-32-9 | S112 |
| 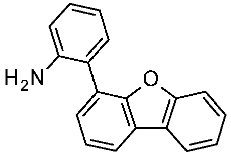 | 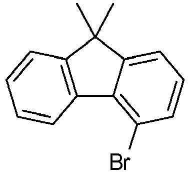 | 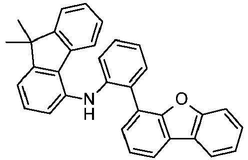 |
| CAS-1559070-70-0 | CAS-942615-32-9 | S113 |
| 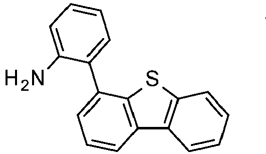 | 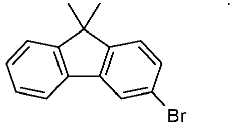 | 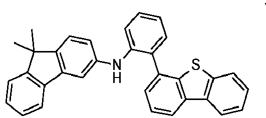 |
| CAS-530403-06-6 | CAS-1190360-23-6 | S114 |
| 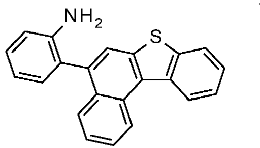 | 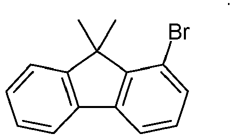 | 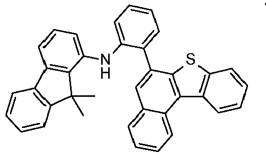 |
| CAS-2044846-62-8 | CAS-1225053-54-2 | S115 |
| 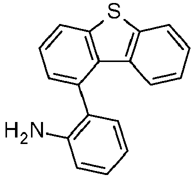 | 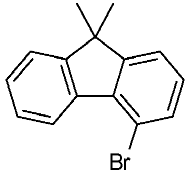 | 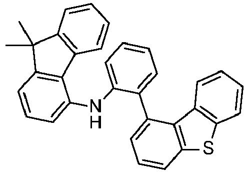 |
| CAS-1850406-53-9 | CAS-942615-32- | S116 |
| 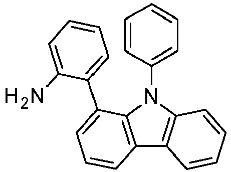 | 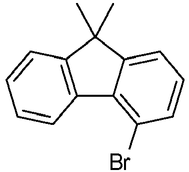 | 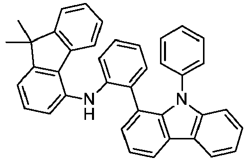 |
| CAS-1850406-99-3 | CAS-942615-32- | S117 |
| 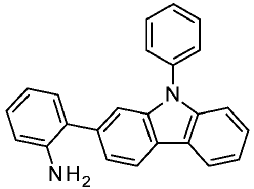 | 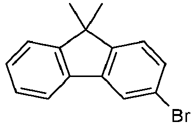 | 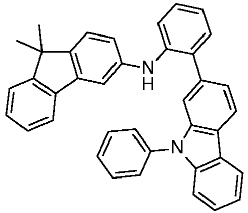 |
| CAS 1850406-91-5 | CAS-1190360-23-6 | S118 |
| 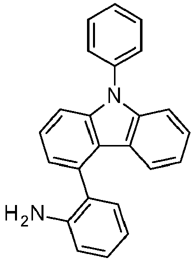 | 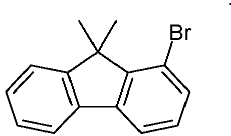 | 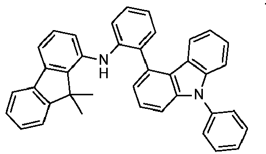 |
| CAS-1850406-35-7 | CAS-1225053-54-2 | S119 |
| 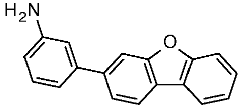 | 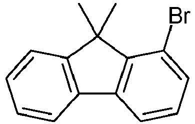 | 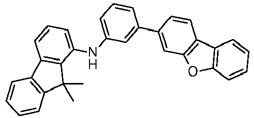 |
| S2 | CAS-1225053-54-2 | S120 |
| 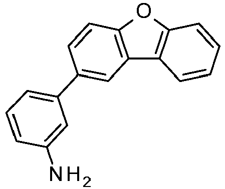 | 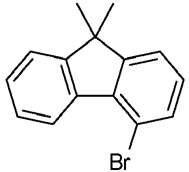 | 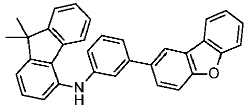 |
| S3 | CAS-942615-32- | S121 |
| 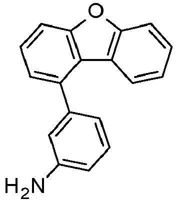 | 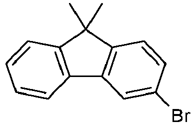 | 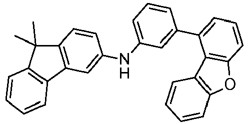 |
| S4 | CAS-1190360-23-6 | S122 |
| 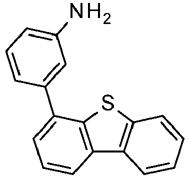 | 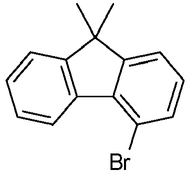 | 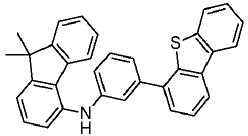 |
| S5 | CAS-942615-32 | S123 |
| 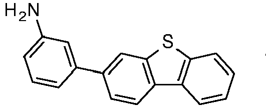 | 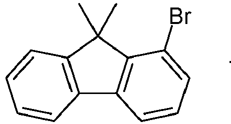 | 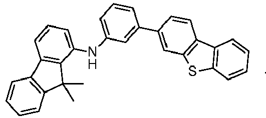 |
| S6 | CAS-1225053-54-2 | S124 |
| 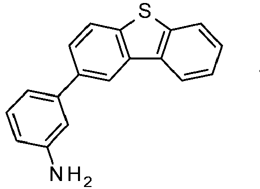 | 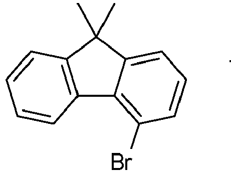 | 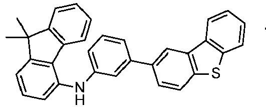 |
| S7 | CAS-942615-32 | S125 |
| 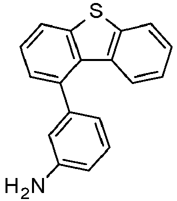 | 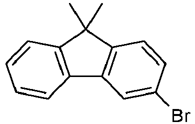 | 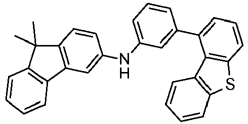 |
| S8 | CAS-1190360-23-6 | S126 |
| 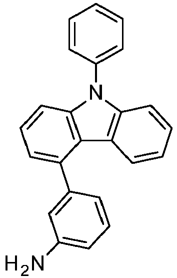 | 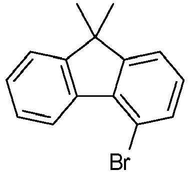 | 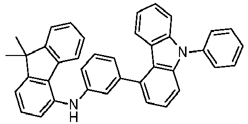 |
| S9 | CAS-942615-32 | S127 |
| 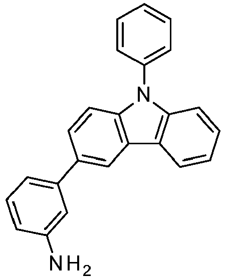 | 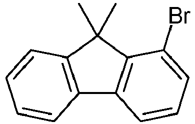 | 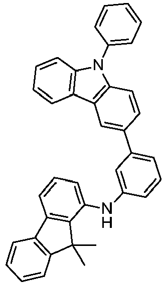 |
| S10 | CAS-1225053-54-2 | S128 |
| 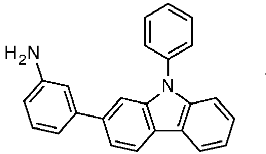 | 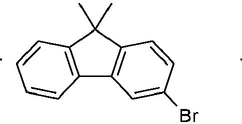 | 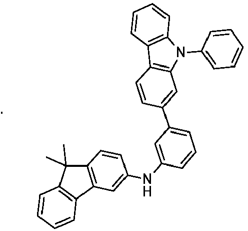 |
| S11 | CAS-1190360-23-6 | S129 |
| 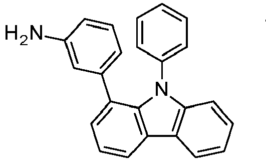 | 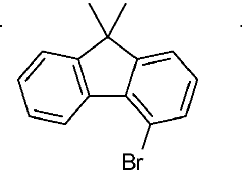 | 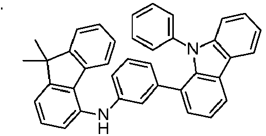 |
| S12 | CAS-942615-32 | S130 |
| 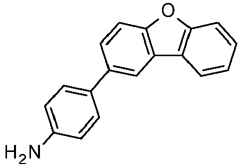 | 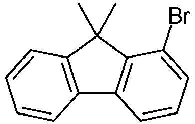 | 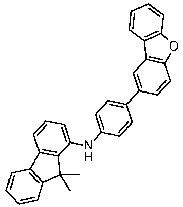 |
| S13 | CAS-1225053-54-2 | S131 |
| 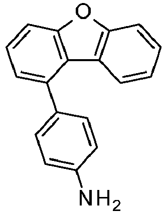 | 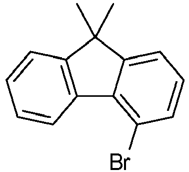 | 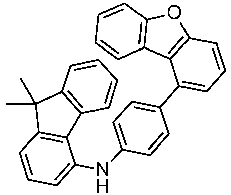 |
| S14 | CAS-942615-32 | S132 |
| 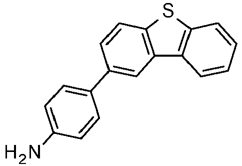 | 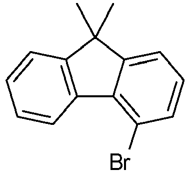 | 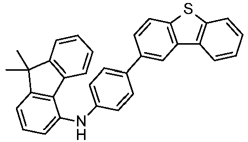 |
| S15 | CAS-942615-32 | S133 |
| 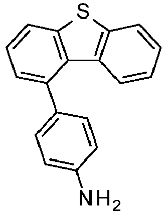 | 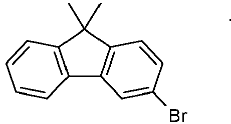 | 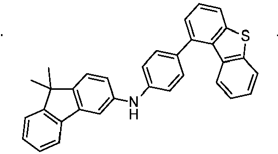 |
| S16 | CAS-1190360-23-6 | S134 |
| 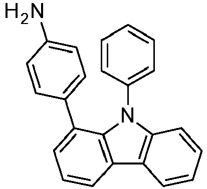 | 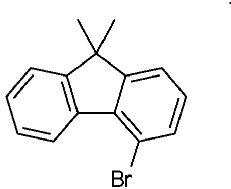 | 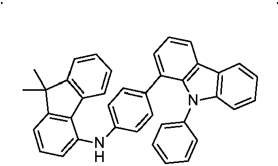 |
| S17 | CAS-942615-32 | S135 |
| 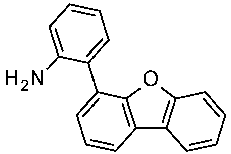 | 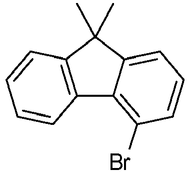 | 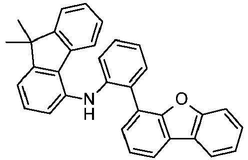 |
| S18 | CAS-942615-32 | S136 |
| 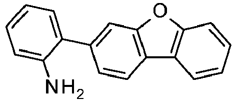 | 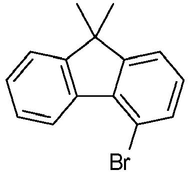 | 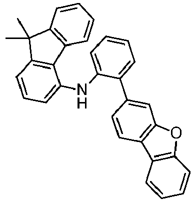 |
| S19 | CAS-942615-32 | S137 |
| 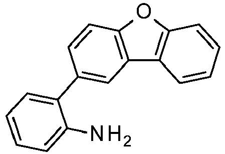 | 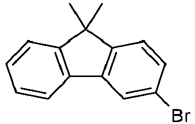 | 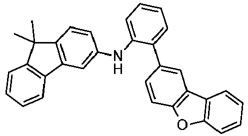 |
| S20 | CAS-1190360-23-6 | S138 |
| 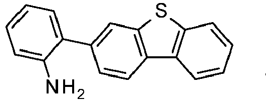 | 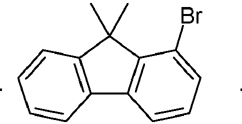 | 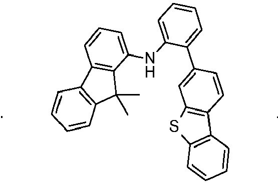 |
| S21 | CAS-1225053-54-2 | S139 |
| 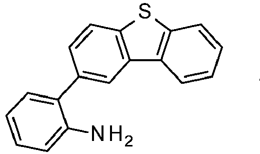 | 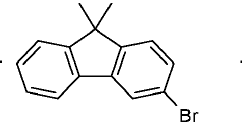 | 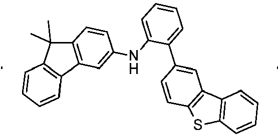 |
| S22 | CAS-1190360-23-6 | S140 |
| 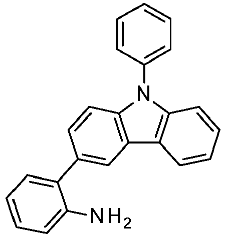 | 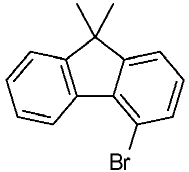 | 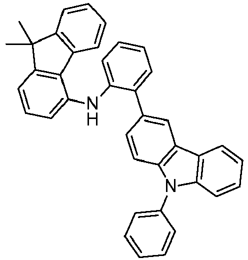 |
| S23 | CAS-942615-32 | S141 |
| 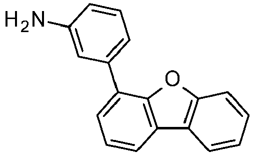 | 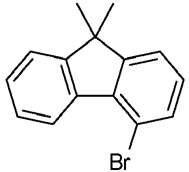 | 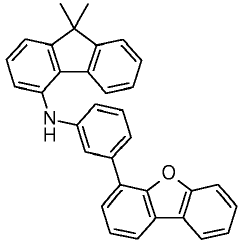 |
| S1 | CAS-942615-32 | S142 |
| 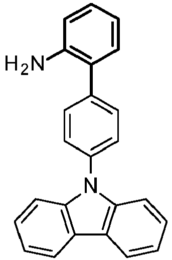 | 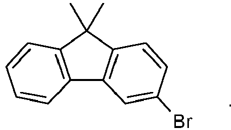 | 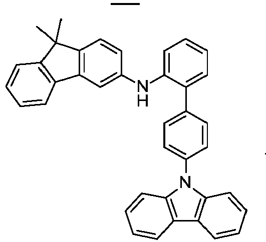 |
| S45 | CAS-1190360-23-6 | S143 |
| 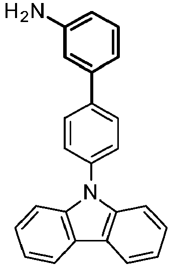 | 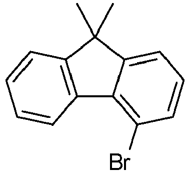 | 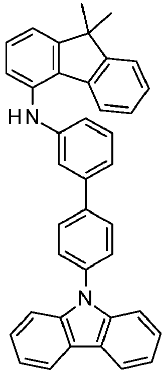 |
| S24 | CAS-942615-32 | S144 |
| 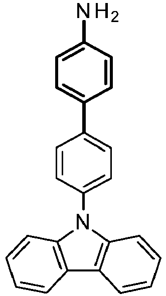 | 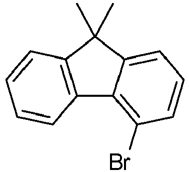 | 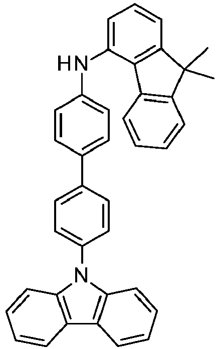 |
| S25 | CAS-942615-32 | S145 |
| 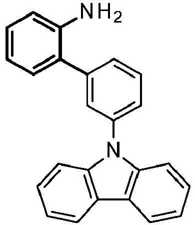 | 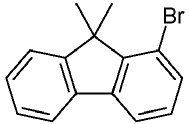 | 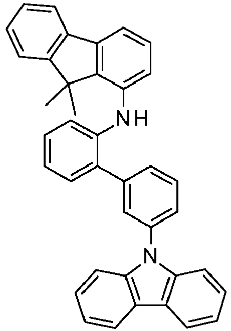 |
| S26 | CAS-1225053-54-2 | S146 |
| 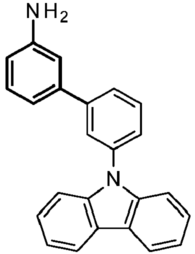 | 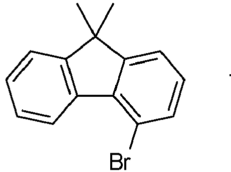 | 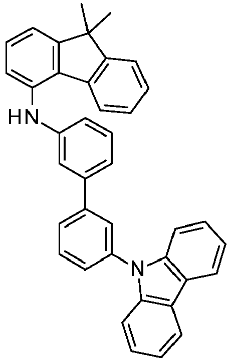 |
| S27 | CAS-942615-32 | S147 |
| 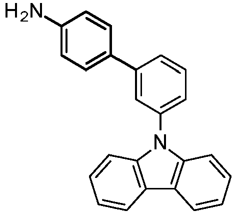 | 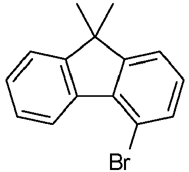 | 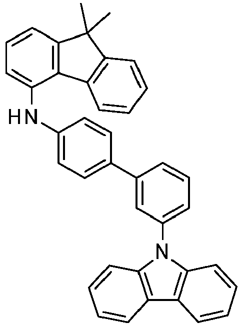 |
| S28 | CAS-942615-32 | S148 |
| 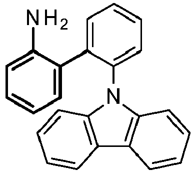 | 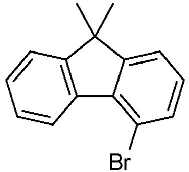 | 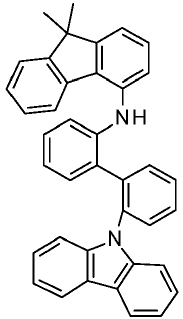 |
| S29 | CAS-942615-32 | S149 |
| 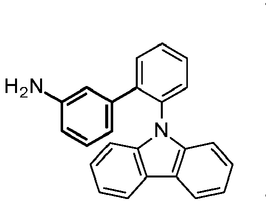 S30 | 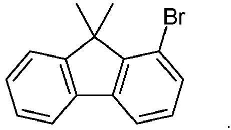 | 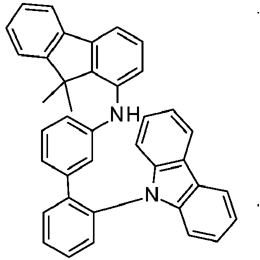 S150 |
| 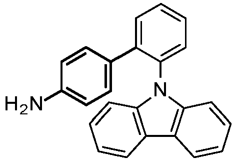 | 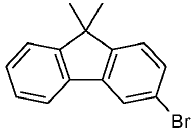 | 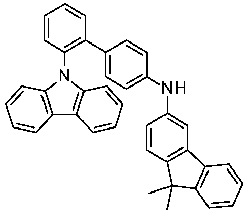 |
| S31 | CAS-1190360-23-6 | S151 |
| 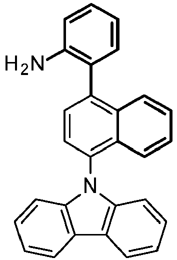 | 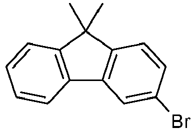 | 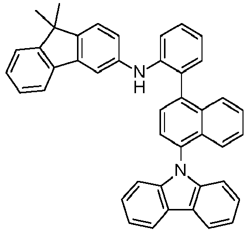 |
| S32 | CAS-1190360-23-6 | S152 |
| 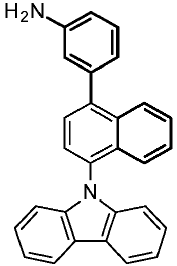 | 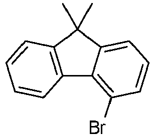 | 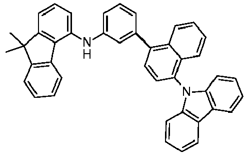 |
| S33 | CAS-942615-32 | S153 |
| 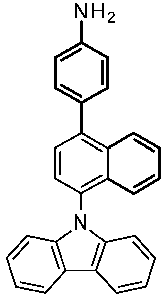 | 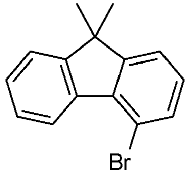 | 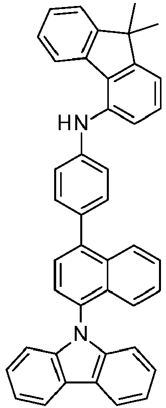 |
| S34 | CAS-942615-32 | S154 |
| 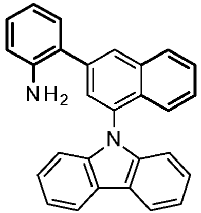 | 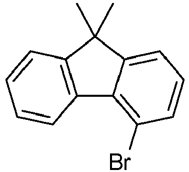 | 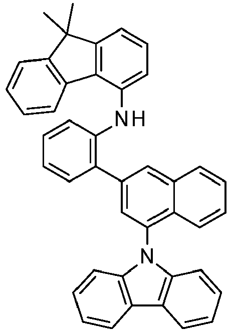 |
| S35 | CAS-942615-32 | S155 |
| 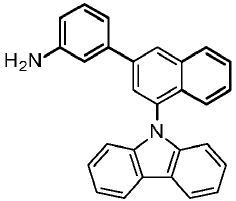 | 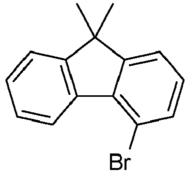 | 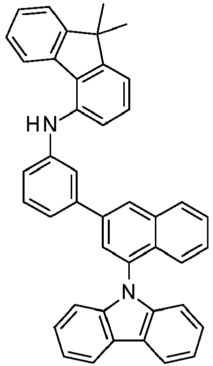 |
| S36 | CAS-942615-32 | S156 |
| 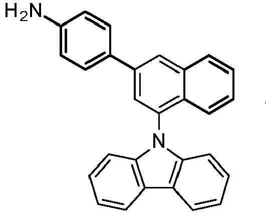 | 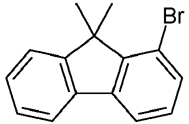 | 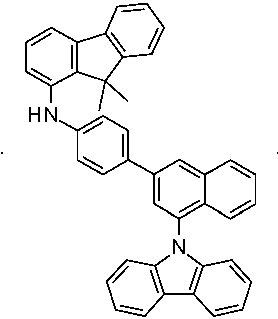 |
| S37 | CAS-1225053-54-2 | S157 |
| 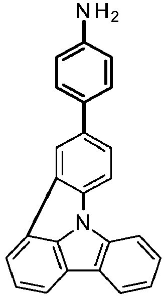 | 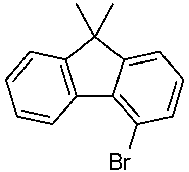 | 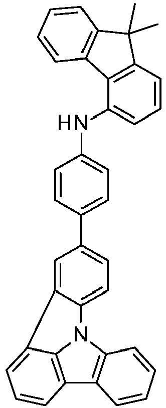 |
| S38 | CAS-942615-32 | S158 |
| 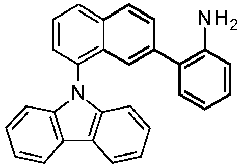 | 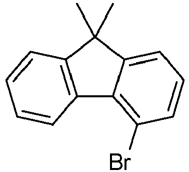 | 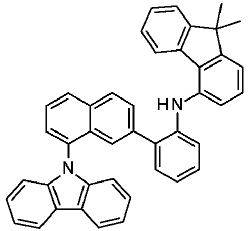 |
| S39 | CAS-942615-32 | S159 |
| 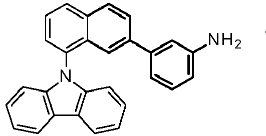 | 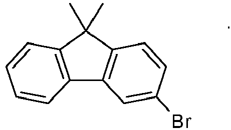 | 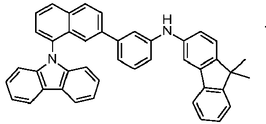 |
| S40 | CAS-1190360-23-6 | S160 |
| 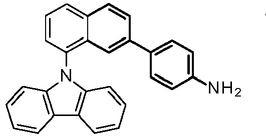 | 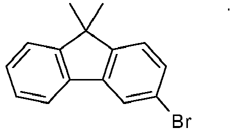 | 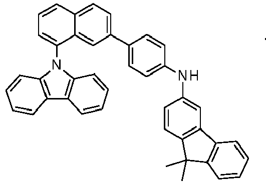 |
| S41 | CAS-1190360-23-6 | S161 |
| 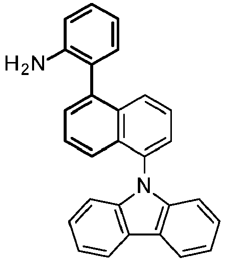 | 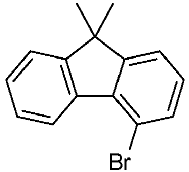 | 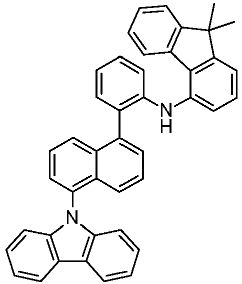 |
| S42 | CAS-942615-32 | S162 |
| 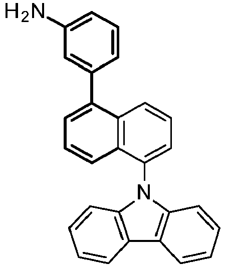 | 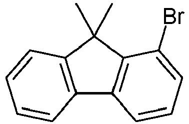 | 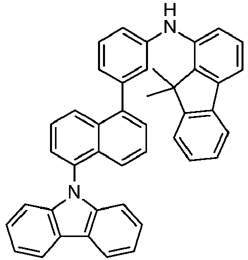 |
| S43 | CAS-1225053-54-2 | S163 |
| 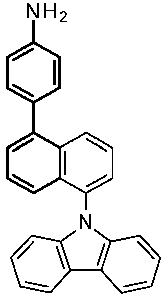 | 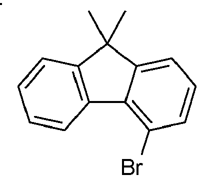 | 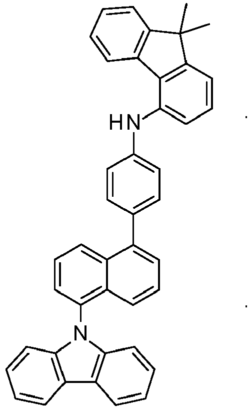 |
| S44 | CAS-942615-32 | S164 |
| 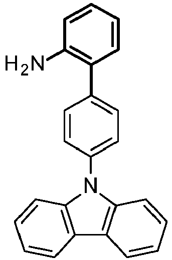 | 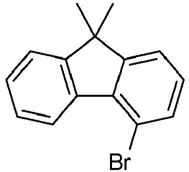 | 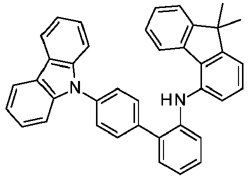 |
| S45 | CAS-942615-32 | S165 |
| 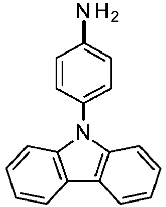 | 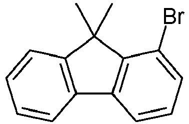 | 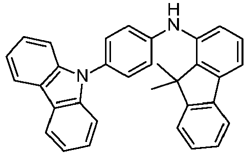 |
| CAS-52708-37-9 | CAS-1225053-54-2 | S166 |
| 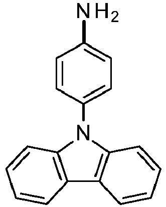 | 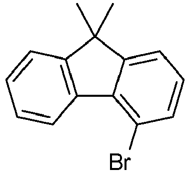 | 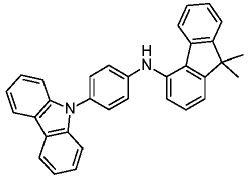 |
| CAS-52708-37-9 | CAS-942615-32 | S167 |
| 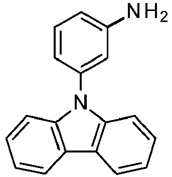 | 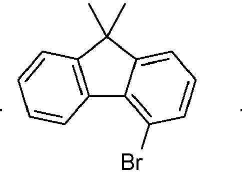 | 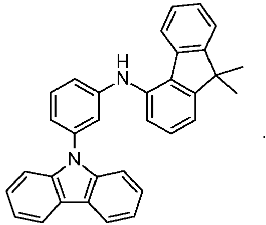 |
| CAS-1023659-21-3 | CAS-942615-32 | S168 |
| 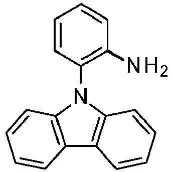 | 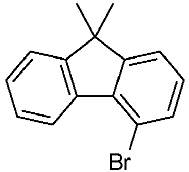 | 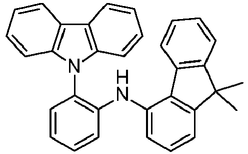 |
| CAS-101716-43-2 | CAS-942615-32 | S169 |
| 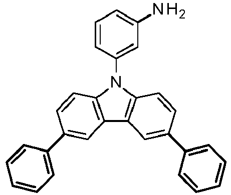 | 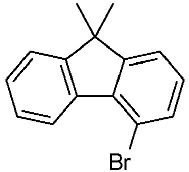 | 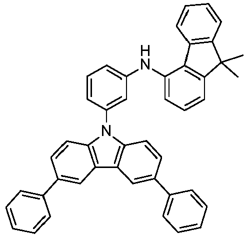 |
| CAS-1708110-65-9 | CAS-942615-32 | S170 |
| 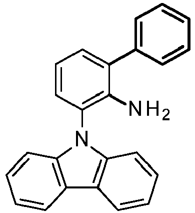 | 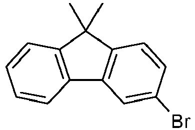 | 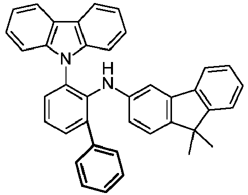 |
| CAS-1673542-71-6 | CAS-1190360-23-6 | S171 |

### A-2) Darstellung der Endstufen

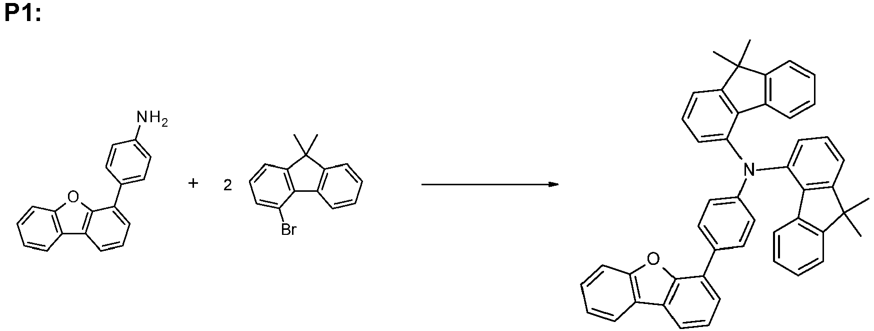

4-Dibenzofuran-4-yl-phenylamin [CAS- 578027-21-1] (12.00 g; 46.26 mmol), 4-Bromo-9,9-dimethyl-9H-fluoren [CAS-942615-32-9 ] (26.55 g; 97.2 mmol) und Natrium-tert-butylat (13.34 g; 138.83 mmol) werden in Toluol (500 mL) vogelegt und 45 min mit Argon gesättigt. Danach wird Palladium(II)-acetat [3375-31-3] (519 mg; 2.31 mmol) und Tri-tert-butylphosphin-Lösung 1.0 M in Toluol [13716-12-6] (4.63 ml; 4.63 mmol) eingetragen und 16 h unter Rückfluss gerührt. Nach Abkühlen des Ansatzes wird dieser mit 200 ml *n*-Heptan erweitert, der ausgefallene Feststoff abgesaugt, dieser mit 500 mL Wasser verrührt, der Feststoff erneut abgesaugt und mit Ethanol (4 x 50 mL) gewaschen. Das Rohprodukt wird zweimal mit Toluol/*n*-Heptan über Alox basisch heißextrahiert, anschließend zweimal aus o-Xylol umkristallisiert und abschließend im Hochvakuum sublimiert.

Ausbeute: 16.0 g (24.9 mmol), 54%; Reinheit: > 99.9 %ig nach HPLC. 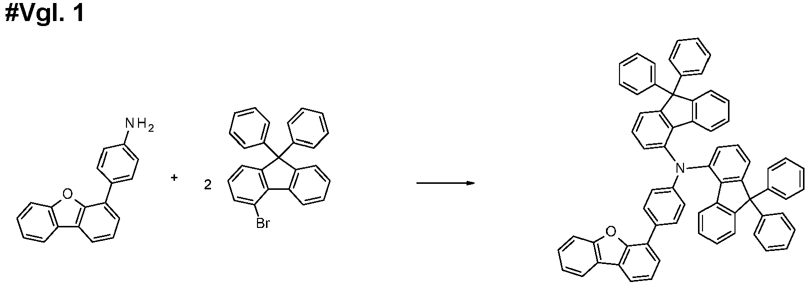 Durchführung analog der Versuchsbeschreibung zu P1, wobei statt 4-Bromo-9,9-dimethyl-9H-fluoren 4-Bromo-9,9-diphenyl-9H-fluoren [CAS-713125-22-5] verwendet wird. Zur Aufreinigung wird zweimal mit Toluol über Alox basisch heißextrahiert, anschließend einmal aus o-Xylol umkristallisiert und abschließend im Hochvakuum sublimiert. Ausbeute 26.4 g (29.6 mmol, 64%); Reinheit > 99.9 %ig nach HPLC 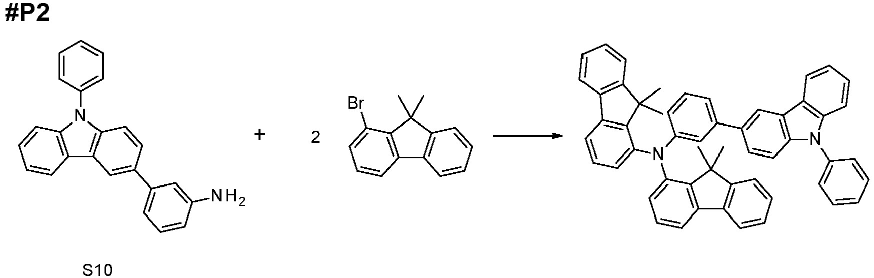 Durchführung analog der Versuchsbeschreibung zu P1, nur dass statt 4-Dibenzofuran-4-yl-phenylamin S10 (46.26 mmol) und statt 4-Bromo-9,9-dimethyl-9H-fluoren 1-Bromo-9,9-dimethyl-9H-fluoren [CAS-1225053-54-2] verwendet wird. Zur Aufreinigung wird zweimal mit Toluol/n-Heptan über Alox basisch heißextrahiert, anschließend zweimal aus n-Butylacetat umkristallisiert und abschließend im Hochvakuum sublimiert. Ausbeute 6.6 g (9.1 mmol, 20%); Reinheit > 99.9 %ig nach HPLC 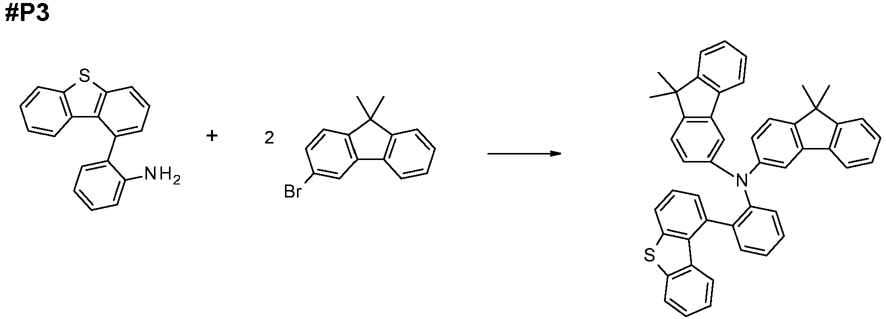 Durchführung analog der Versuchsbeschreibung zu P1, nur dass statt 4-Dibenzofuran-4-yl-phenylamin 1-Dibenzothiophen-2-yl-phenylamin [CAS-1850406-53-9] (46.26 mmol) und statt 4-Dibenzofuran-4-yl-phenylamin 3-Bromo-9,9-dimethyl-9H-fluoren (97.2 mmol) [CAS-1190360-23-6] verwendet wird. Als Katalysatorsystem werden statt Palladium(II)acetat und Tri-tert-butylphosphin, Tris(dibenzylideneacetone)dipalladium(0) [CAS-51364-51-3] (0.01 equiv) und S-Phos [CAS- 657408-07-6] (0.03 equiv) verwendet. Zur Aureinigung wird zweimal mit Toluol/n-Heptan über Alox basisch heißextrahiert, anschließend einmal ausToluol umkristallisiert und abschließend im Hochvakuum sublimiert. Ausbeute: 10.1 g (15.3 mmol, 33%); Reinheit > 99.9 nach HPLC

Analog können folgende Verbindungen dargestellt werden. Hierbei kann zur Aufreinigung auch Säulenchromatographie verwendet werden, oder zur Umkristallisation oder Heißextraktion andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid; N-Methylpyrrolidon etc. verwendet werden. Die Ausbeuten liegen typischerweise im Bereich zwischen 15% und 75%.

| Edukt 1 | Edukt 2 | Produkt |
|---|---|---|
| 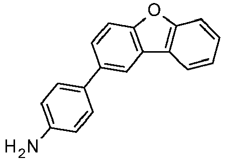 | 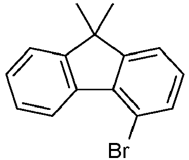 | 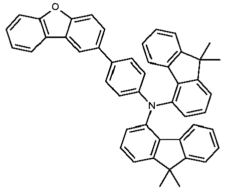 |
| CAS- 1178274-17-3 | CAS-942615-32-9 | P4 |
| 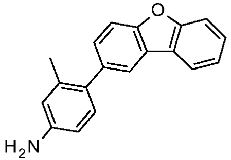 | 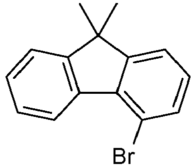 | 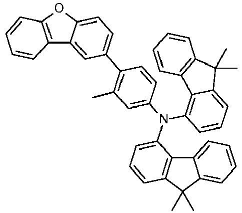 |
| CAS-1184428-04-3 | CAS-942615-32-9 | P5 |
| 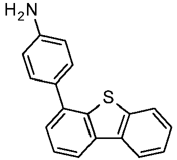 | 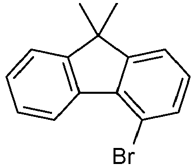 | 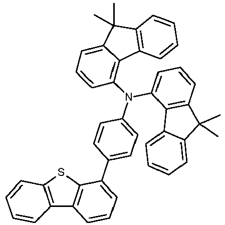 |
| CAS-1370034-50-6 | CAS-942615-32-9 | P6 |
| 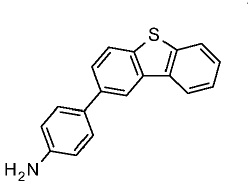 | 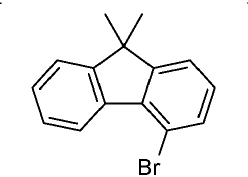 | 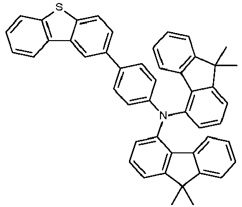 |
| CAS-1574121-68-8 | CAS-942615-32-9 | P7 |
| 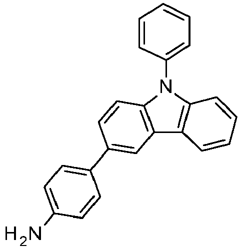 | 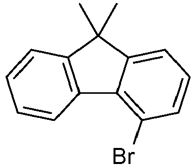 | 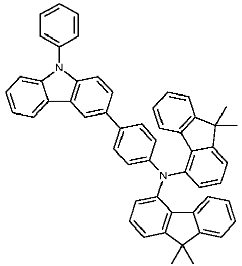 |
| CAS-1370034-59-5 | CAS-942615-32-9 | P8 |
| 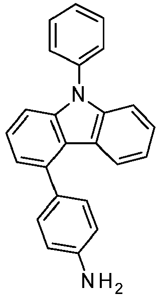 | 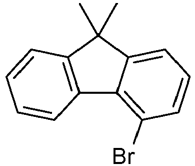 | 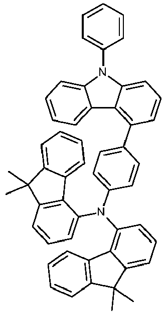 |
| CAS-1629995-08-9 | CAS-942615-32-9 | P9 |
| 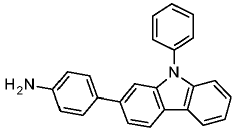 | 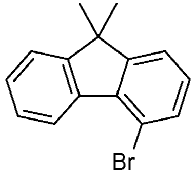 | 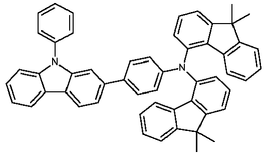 |
| CAS-1911626-45-3 | CAS-942615-32-9 | P10 |
| 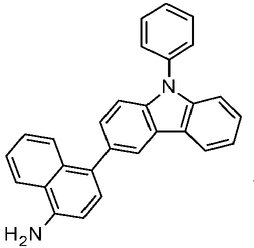 | 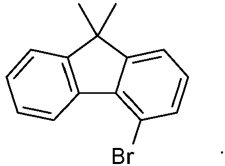 | 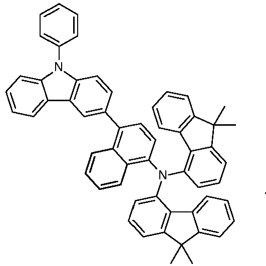 |
| CAS-1699765-82-6 | CAS-942615-32-9 | P11 |
| 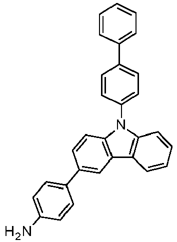 | 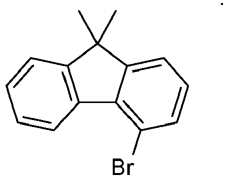 | 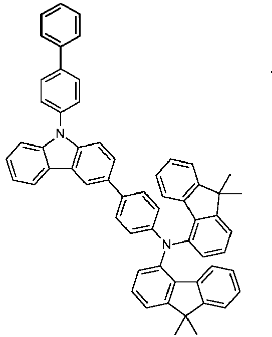 |
| CAS-1401003-44-8 | CAS-942615-32-9 | P12 |
| 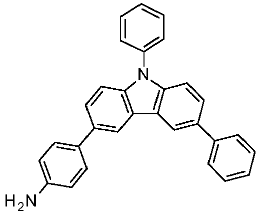 | 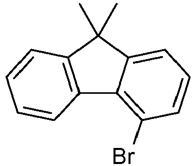 | 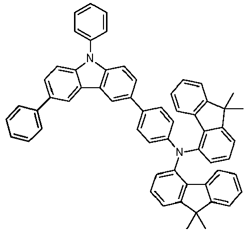 |
| CAS-1458611-14-7 | CAS-942615-32-9 | P13 |
| 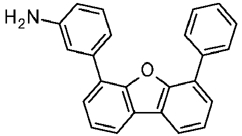 | 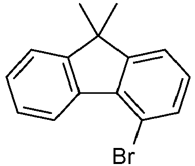 | 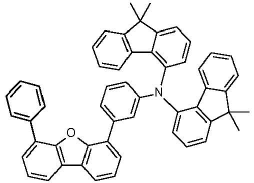 |
| CAS-2101611-03-2 | CAS-942615-32-9 | P14 |
| 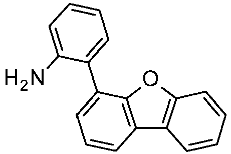 | 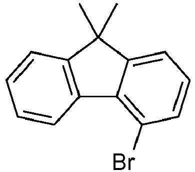 | 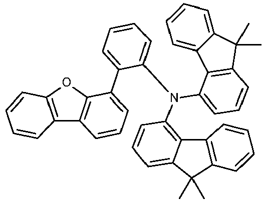 |
| CAS-1559070-70-0 | CAS-942615-32-9 | P15 |
| 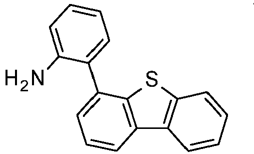 | 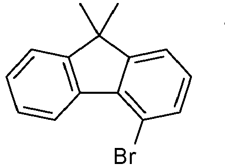 | 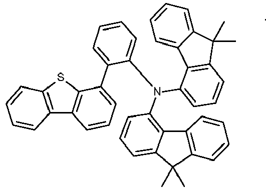 |
| CAS-530403-06-6 | CAS-942615-32-9 | P16 |
| 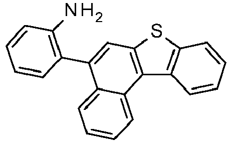 | 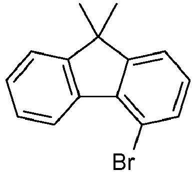 | 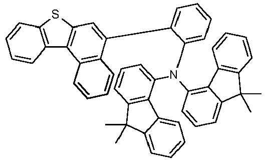 |
| CAS-2044846-62-8 | CAS-942615-32-9 | P17 |
| 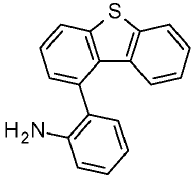 | 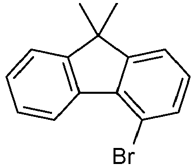 | 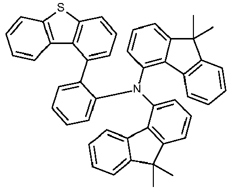 |
| CAS-1850406-53-9 | CAS-942615-32-9 | P18 |
| 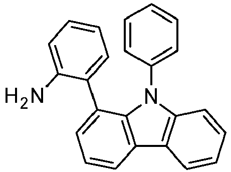 | 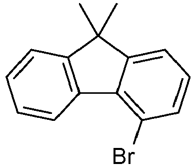 | 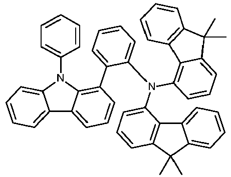 |
| CAS-1850406-99-3 | CAS-942615-32-9 | P19 |
| 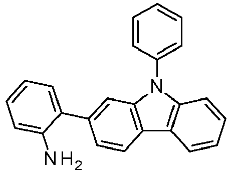 | 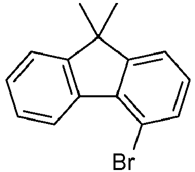 | 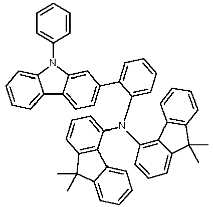 |
| CAS 1850406-91-5 | CAS-942615-32-9 | P20 |
| 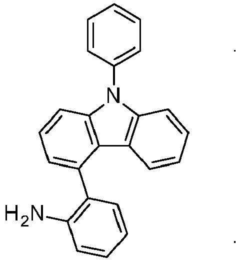 | 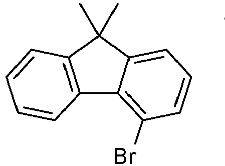 | 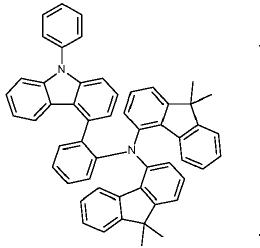 |
| CAS-1850406-35-7 | CAS-942615-32-9 | P21 |
| 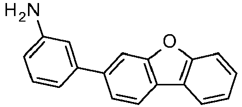 | 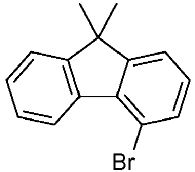 | 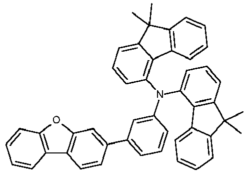 |
| S2 | CAS-942615-32-9 | P22 |
| 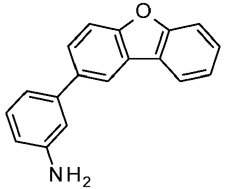 | 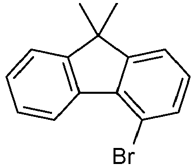 | 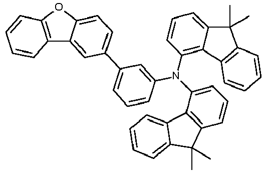 |
| S3 | CAS-942615-32-9 | P23 |
| 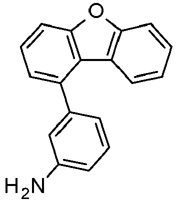 | 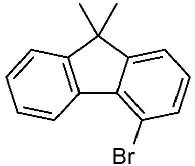 | 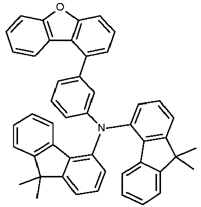 |
| S4 | CAS-942615-32-9 | P24 |
| 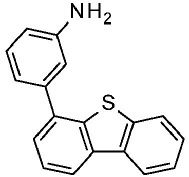 | 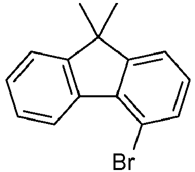 | 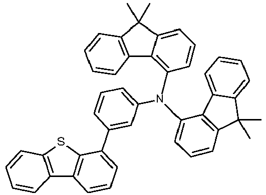 |
| S5 | CAS-942615-32-9 | P25 |
| 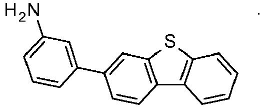 | 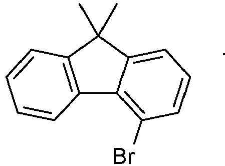 | 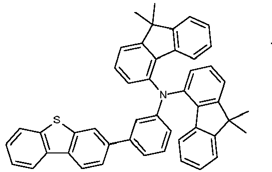 |
| S6 | CAS-942615-32-9 | P26 |
| 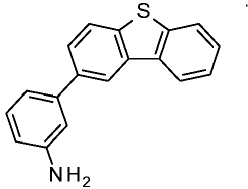 | 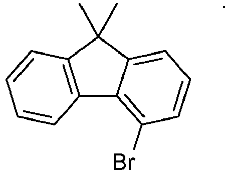 | 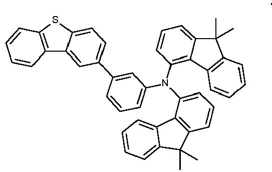 |
| S7 | CAS-942615-32-9 | P27 |
| 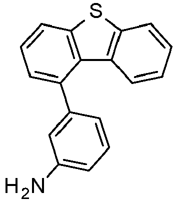 | 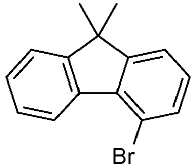 | 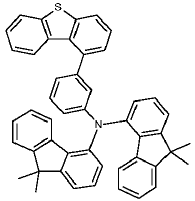 |
| S8 | CAS-942615-32-9 | P28 |
| 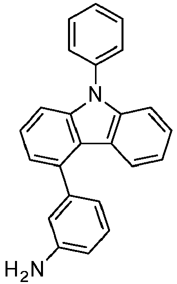 | 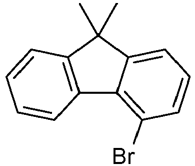 | 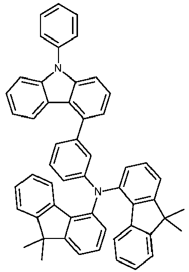 |
| S9 | CAS-942615-32-9 | P29 |
| 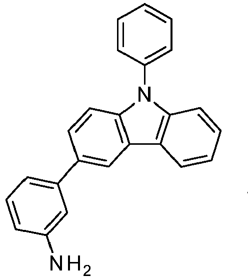 | 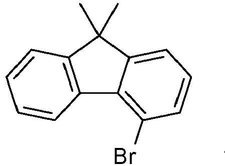 | 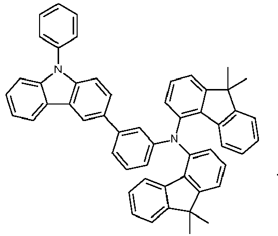 |
| S10 | CAS-942615-32-9 | P30 |
| 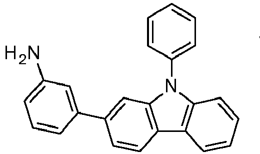 | 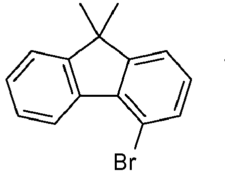 | 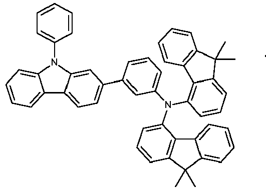 |
| S11 | CAS-942615-32-9 | P31 |
| 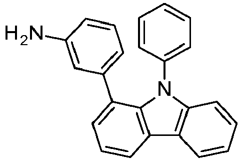 | 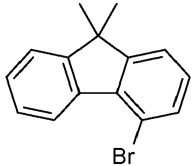 | 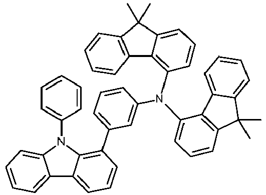 |
| S12 | CAS-942615-32-9 | P32 |
| 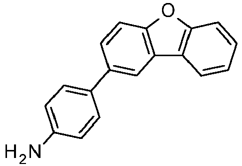 | 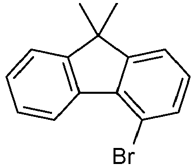 | 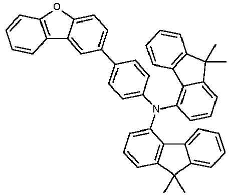 |
| S13 | CAS-942615-32-9 | P33 |
| 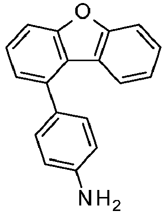 | 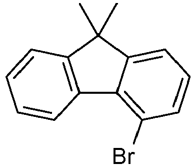 | 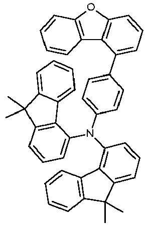 |
| S14 | CAS-942615-32-9 | P34 |
| 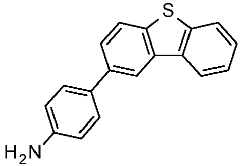 | 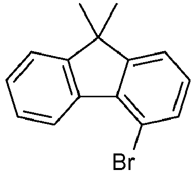 | 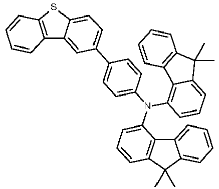 |
| S15 | CAS-942615-32-9 | P35 |
| 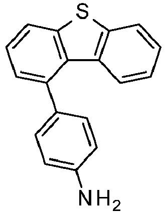 | 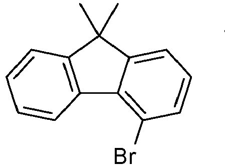 | 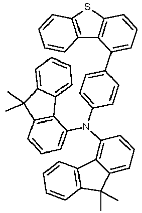 |
| S16 | CAS-942615-32-9 | P36 |
| 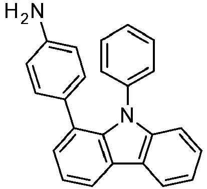 | 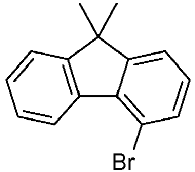 | 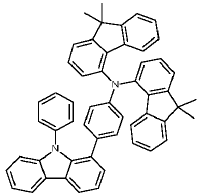 |
| S17 | CAS-942615-32-9 | P37 |
| 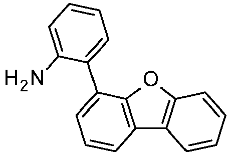 | 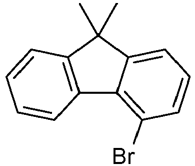 | 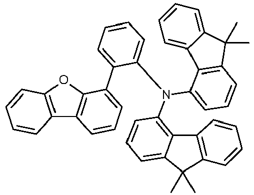 |
| S18 | CAS-942615-32-9 | P38 |
| 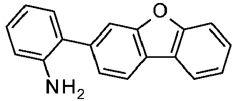 | 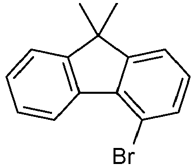 | 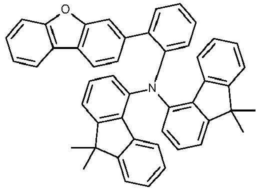 |
| S19 | CAS-942615-32-9 | P39 |
| 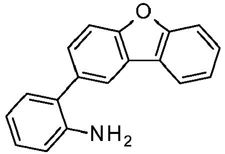 | 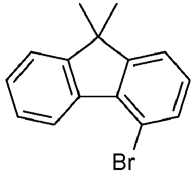 | 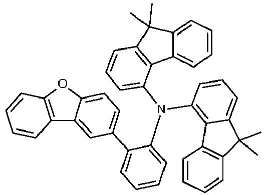 |
| S20 | CAS-942615-32-9 | P40 |
| 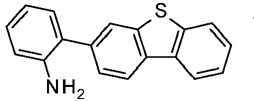 | 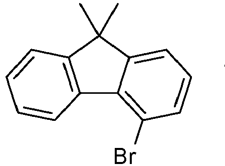 | 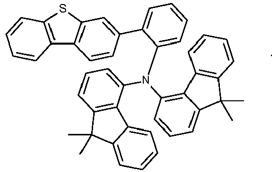 |
| S21 | CAS-942615-32-9 | P41 |
| 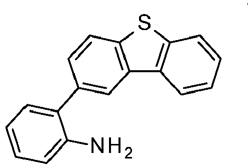 | 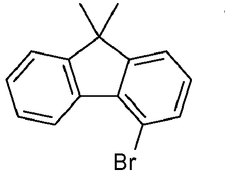 | 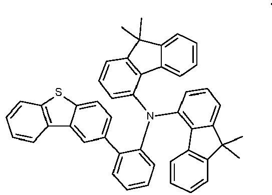 |
| S22 | CAS-942615-32-9 | P42 |
| 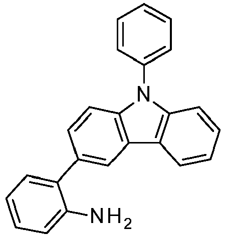 | 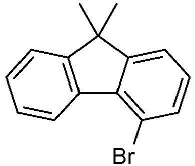 | 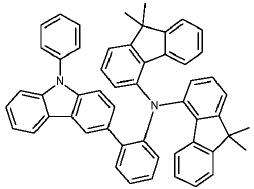 |
| S23 | CAS-942615-32-9 | P43 |
| 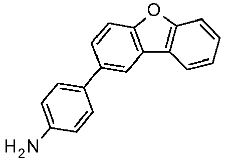 | 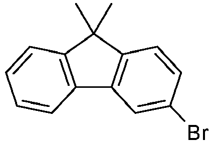 | 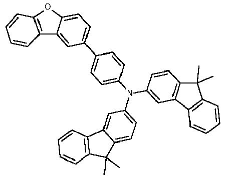 |
| CAS- 1178274-17-3 | CAS-1190360-23-6 | #P44 |
| 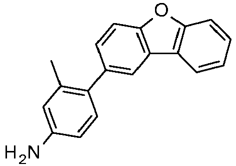 | 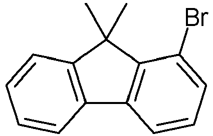 | 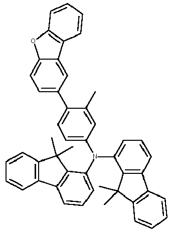 |
| CAS-1184428-04-3 | CAS-1225053-54-2 | #P45 |
| 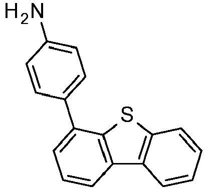 | 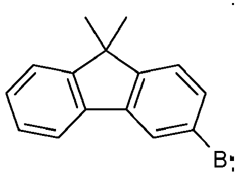 | 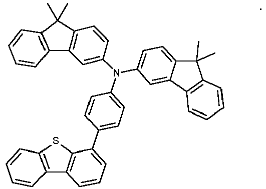 |
| CAS-1370034-50-6 | CAS-1190360-23-6 | #P46 |
| 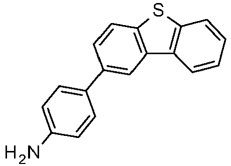 | 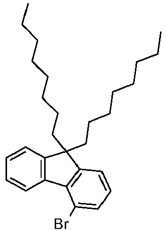 | 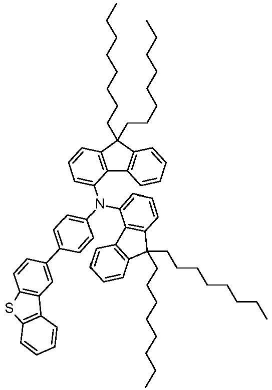 |
| CAS-1574121-68-8 | CAS-1469898-60-9 | P47 |
| 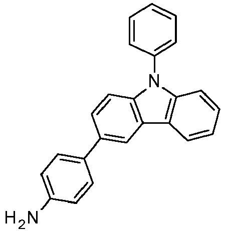 | 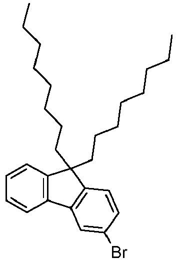 | 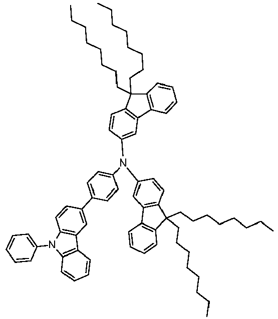 |
| CAS-1370034-59-5 | CAS-1609186-23-3 | #P48 |
| 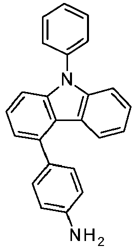 | 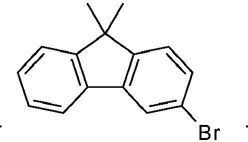 | 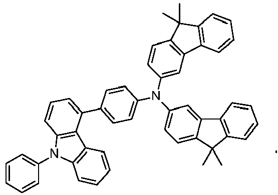 |
| CAS-1629995-08-9 | CAS-1190360-23-6 | #P49 |
| 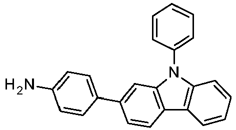 | 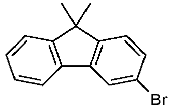 | 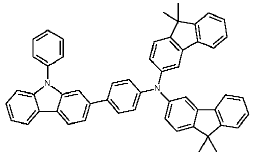 |
| CAS-1911626-45-3 | CAS-1190360-23-6 | #P50 |
| 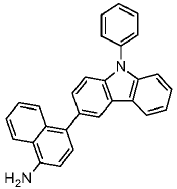 | 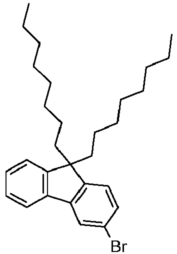 | 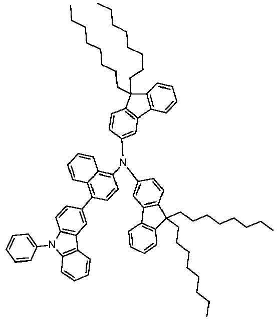 |
| CAS-1699765-82-6 | CAS-1609186-23-3 | #P51 |
| 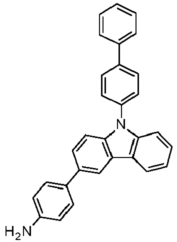 | 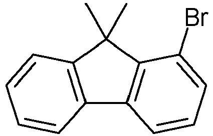 | 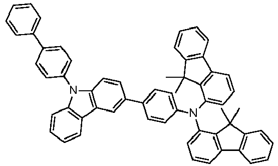 |
| CAS-1401003-44-8 | CAS-1225053-54-2 | #P52 |
| 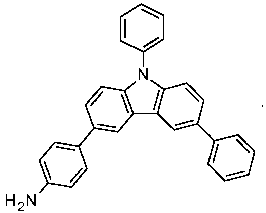 | 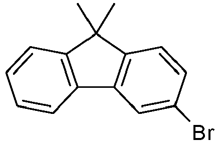 | 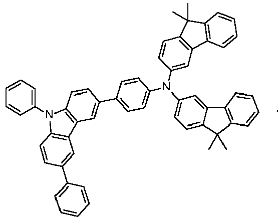 |
| CAS-1458611-14-7 | CAS-1190360-23-6 | #P53 |
| 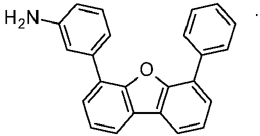 | 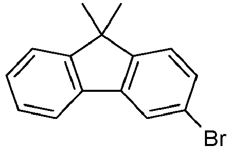 | 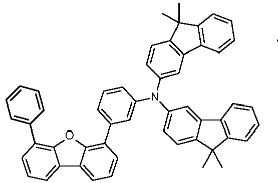 |
| CAS-2101611-03-2 | CAS-1190360-23-6 | #P54 |
| 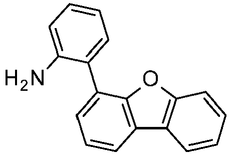 | 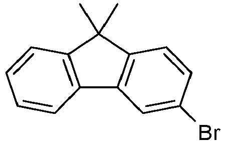 | 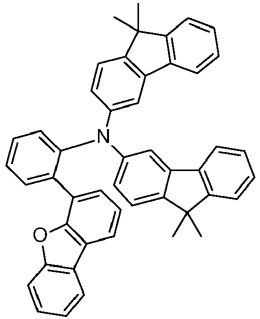 |
| CAS-1559070-70-0 | CAS-1190360-23-6 | #P55 |
| 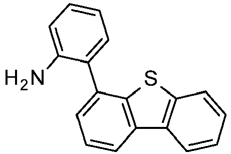 | 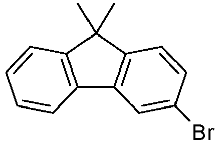 | 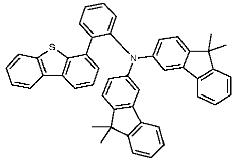 |
| CAS-530403-06-6 | CAS-1190360-23-6 | #P56 |
| 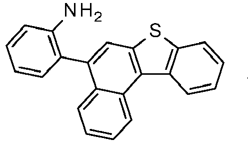 | 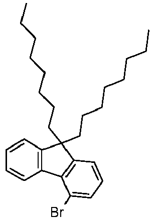 | 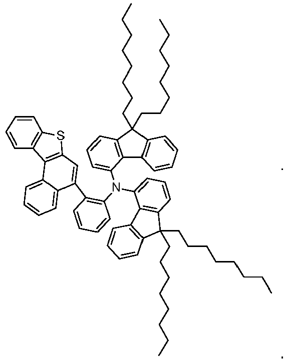 |
| CAS-2044846-62-8 | CAS-1469898-60-9 | P57 |
| 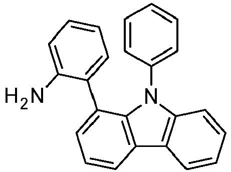 | 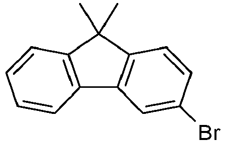 | 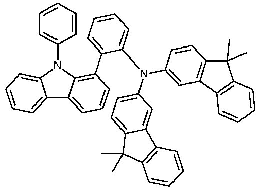 |
| CAS-1850406-99-3 | CAS-1190360-23-6 | #P58 |
| 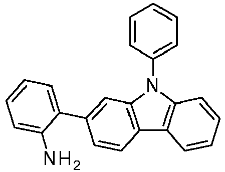 | 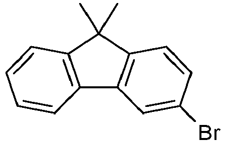 | 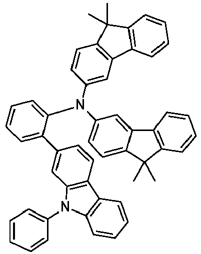 |
| CAS1850406-91-5 | CAS-1190360-23-6 | #P59 |
| 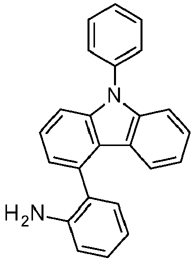 | 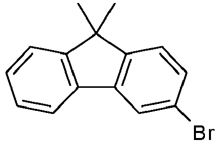 | 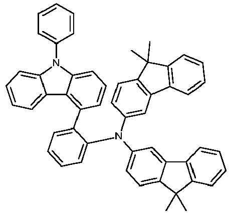 |
| CAS-1850406-35-7 | CAS-1190360-23-6 | #P60 |
| 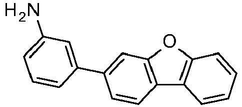 | 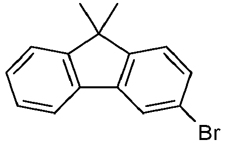 | 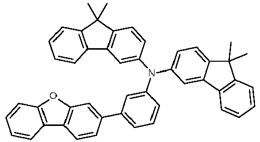 |
| S2 | CAS-1190360-23-6 | #P61 |
| 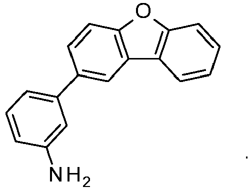 | 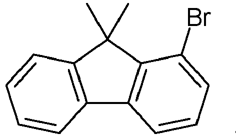 | 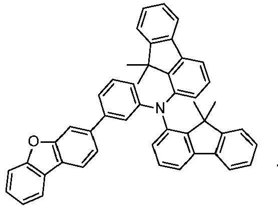 |
| S3 | CAS-1225053-54-2 | #P62 |
| 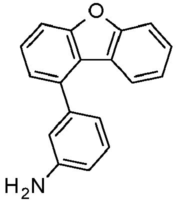 | 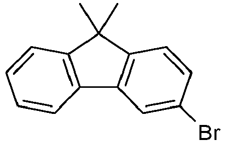 | 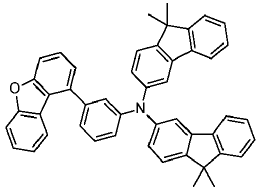 |
| S4 | CAS-1190360-23-6 | #P63 |
| 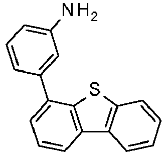 | 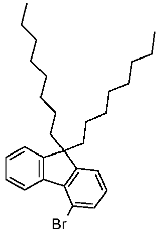 | 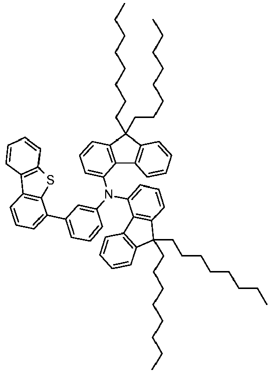 |
| S5 | CAS-1469898-60-9 | P64 |
| 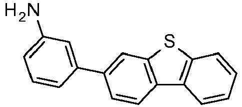 | 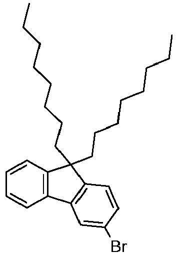 | 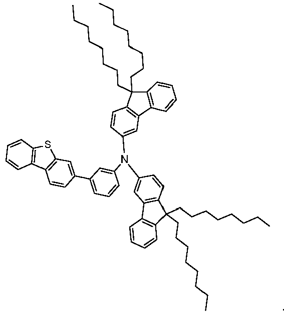 |
| S6 | CAS-1609186-23-3 | #P65 |
| 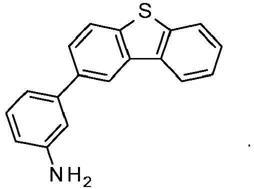 | 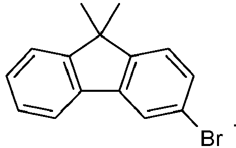 | 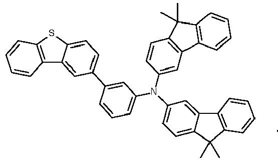 |
| S7 | CAS-1190360-23-6 | #P66 |
| 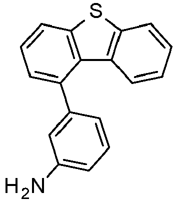 | 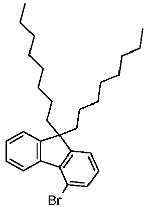 | 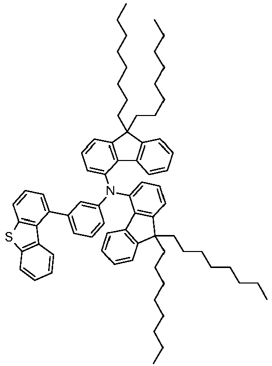 |
| S8 | CAS-1469898-60-9 | P67 |
| 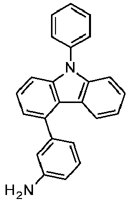 | 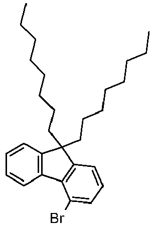 | 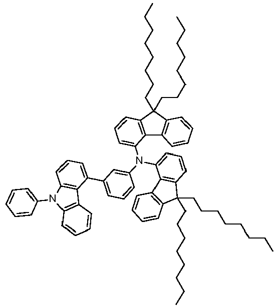 |
| S9 | CAS-1469898-60-9 | P68 |
| 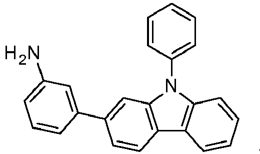 | 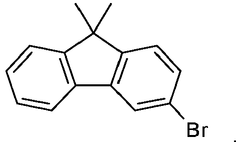 | 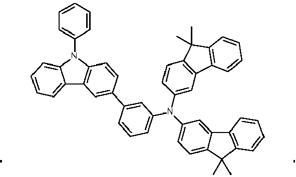 |
| S11 | CAS-1190360-23-6 | #P69 |
| 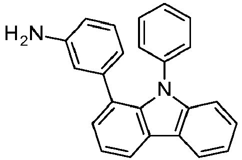 | 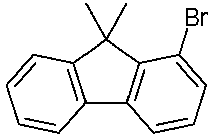 | 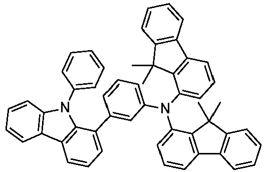 |
| S12 | CAS-1225053-54-2 | #P70 |
| 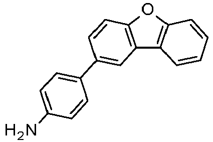 | 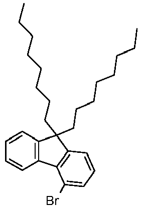 | 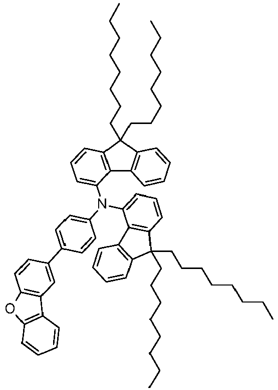 |
| S13 | CAS-1469898-60-9 | P71 |
| 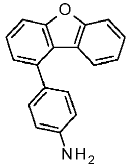 | 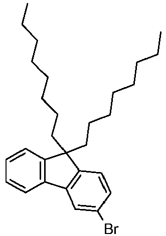 | 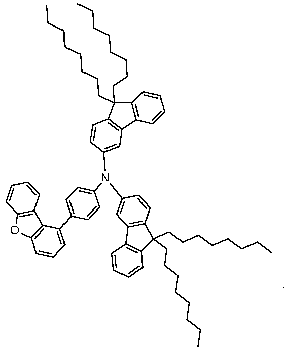 |
| S14 | CAS-1609186-23-3 | #P72 |
| 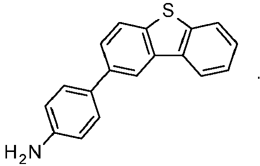 | 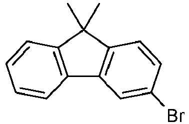 | 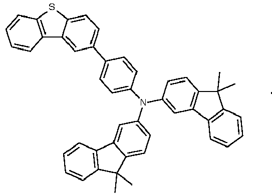 |
| S15 | CAS-1190360-23-6 | #P73 |
| 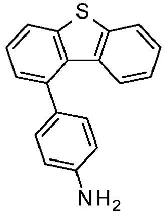 | 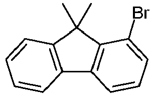 | 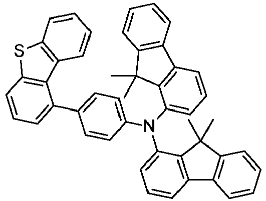 |
| S16 | CAS-1225053-54-2 | #P74 |
| 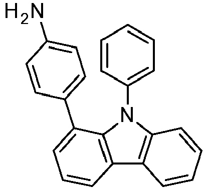 | 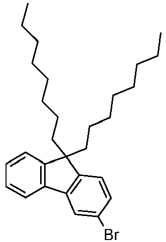 | 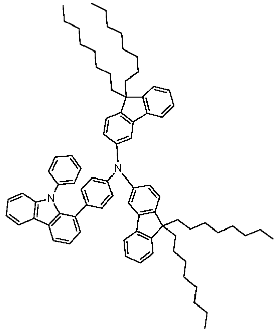 |
| S17 | CAS-1609186-23-3 | #P75 |
| 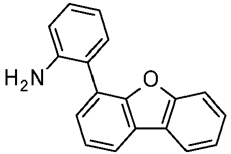 | 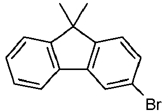 | 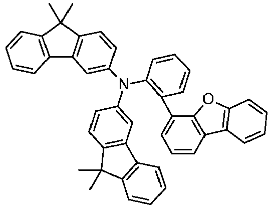 |
| S18 | CAS-1190360-23-6 | #P76 |
| 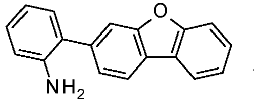 | 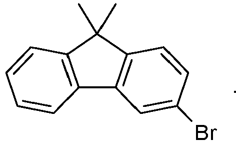 | 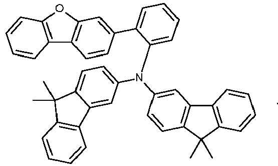 |
| S19 | CAS-1190360-23-6 | #P77 |
| 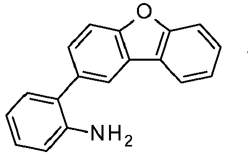 | 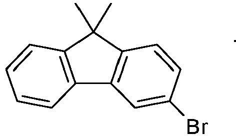 | 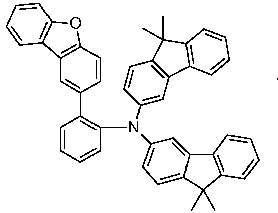 |
| S20 | CAS-1190360-23-6 | #P78 |
| 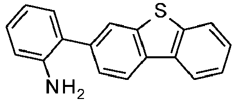 | 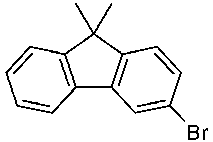 | 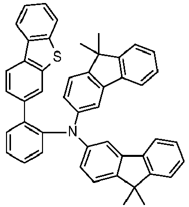 |
| S21 | CAS-1190360-23-6 | #P79 |
| 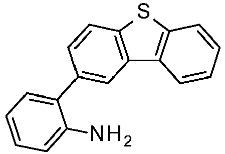 | 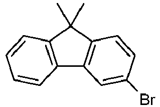 | 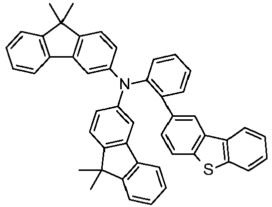 |
| S22 | CAS-1190360-23-6 | #P80 |
| 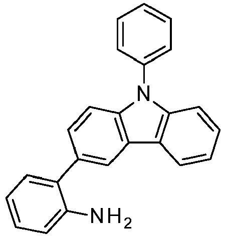 | 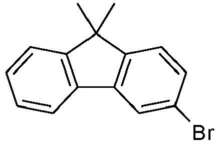 | 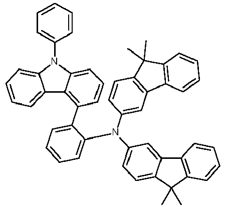 |
| S23 | CAS-1190360-23-6 | #P81 |
| 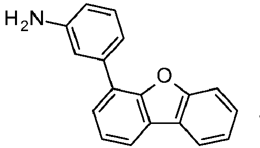 | 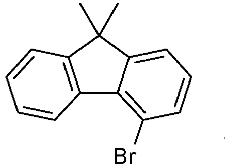 | 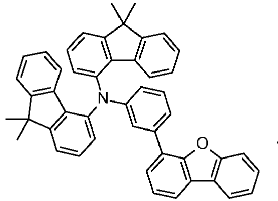 |
| S1 | CAS-942615-32-9 | P82 |
| 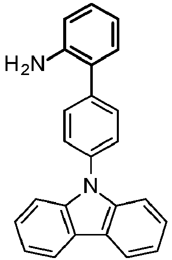 | 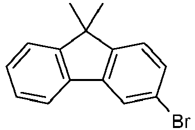 | 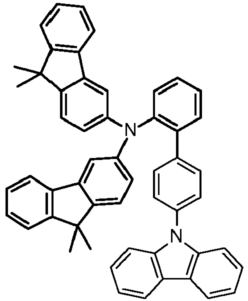 |
| S45 | CAS-1190360-23-6 | #P83 |
| 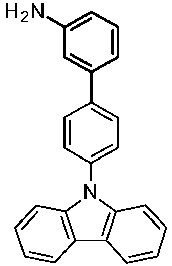 | 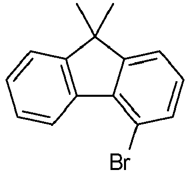 | 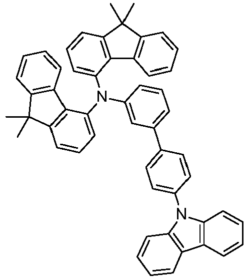 |
| S24 | CAS-942615-32 | P84 |
| 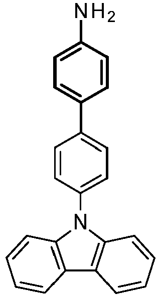 | 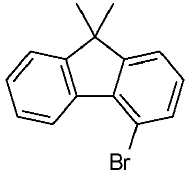 | 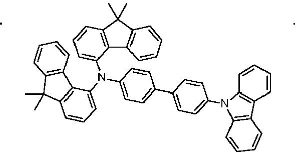 |
| S25 | CAS-942615-32 | P85 |
| 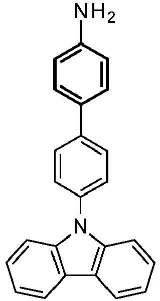 | 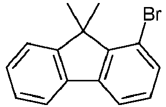 | 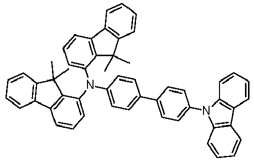 |
| S25 | CAS-1225053-54-2 | #P86 |
| 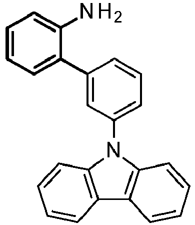 | 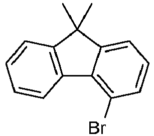 | 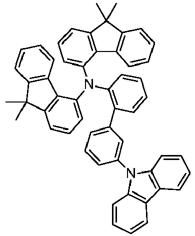 |
| S26 | CAS-942615-32 | P87 |
| 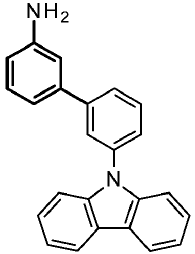 | 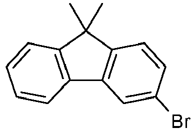 | 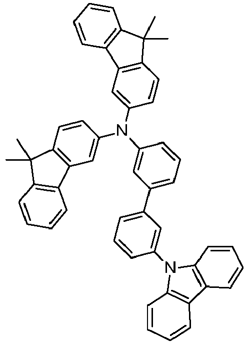 |
| S27 | CAS-1190360-23-6 | #P88 |
| 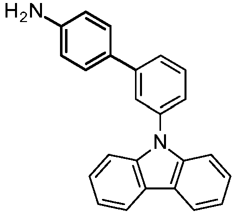 | 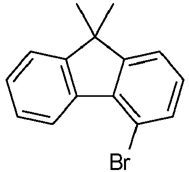 | 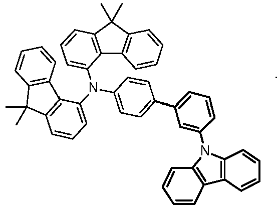 |
| S28 | CAS-942615-32 | P89 |
| 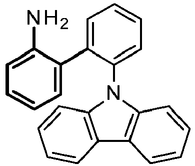 | 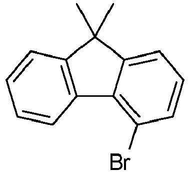 | 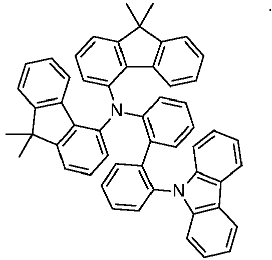 |
| S29 | CAS-942615-32 | P90 |
| 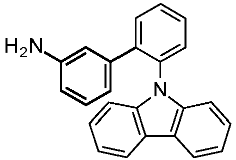 | 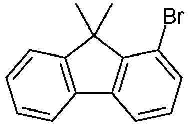 | 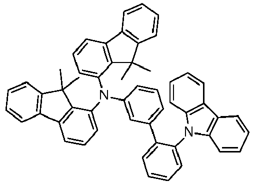 |
| S30 | CAS-1225053-54-2 | #P91 |
| 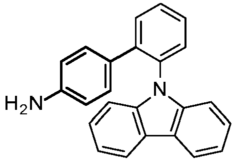 | 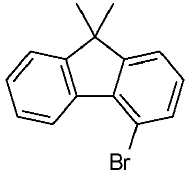 | 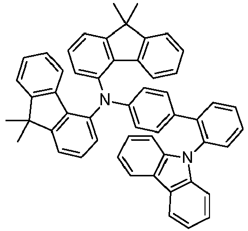 |
| S31 | CAS-942615-32 | P92 |
| 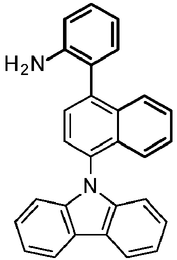 | 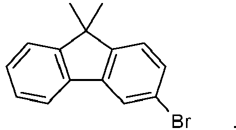 | 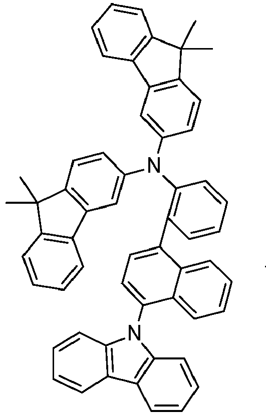 |
| S32 | CAS-1190360-23-6 | #P93 |
| 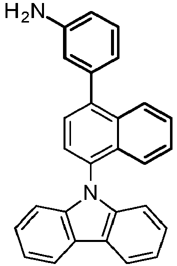 | 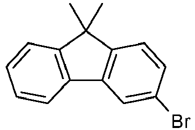 | 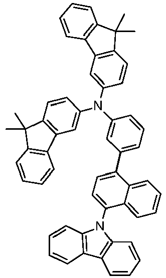 |
| S33 | CAS-1190360-23-6 | #P94 |
| 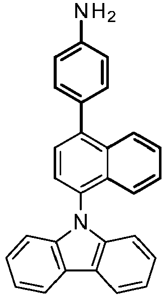 | 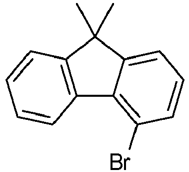 | 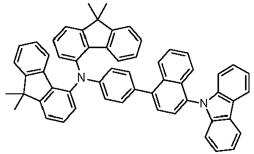 |
| S34 | CAS-942615-32 | P95 |
| 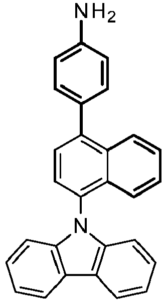 | 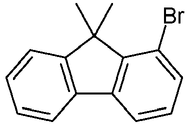 | 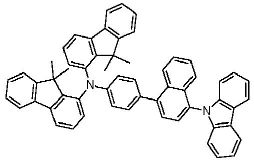 |
| S34 | CAS-1225053-54-2 | #P96 |
| 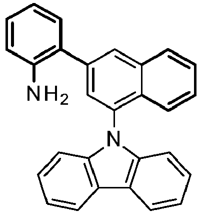 | 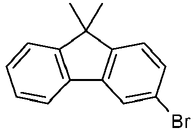 | 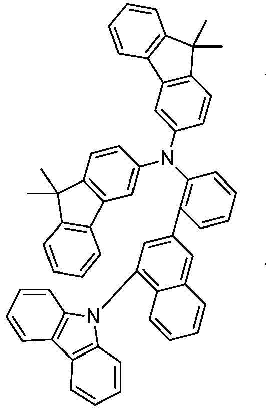 |
| S35 | CAS-1190360-23-6 | #P97 |
| 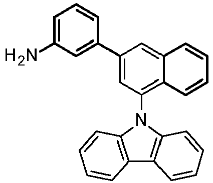 | 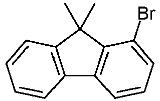 | 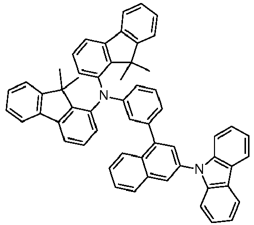 |
| S36 | CAS-1225053-54-2 | #P98 |
| 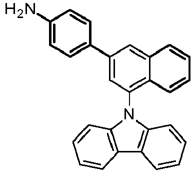 | 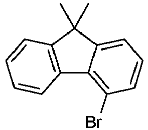 | 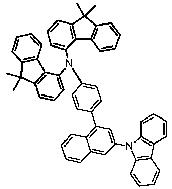 |
| S37 | CAS-942615-32 | P99 |
| 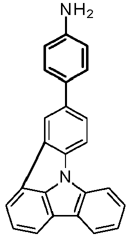 | 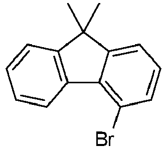 | 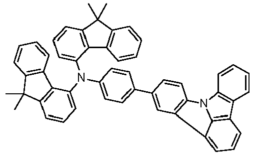 |
| S38 | CAS-942615-32 | P100 |
| 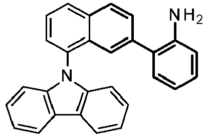 | 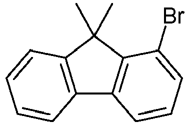 | 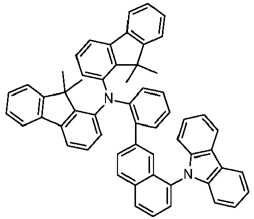 |
| S39 | CAS-1225053-54-2 | #P101 |
| 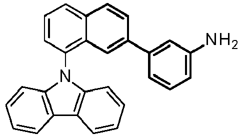 | 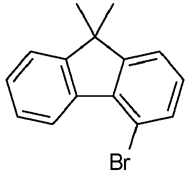 | 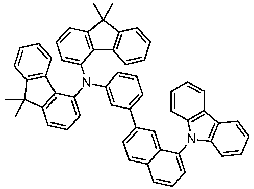 |
| S40 | CAS-942615-32 | P102 |
| 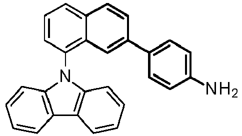 | 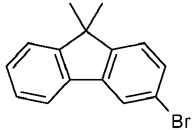 | 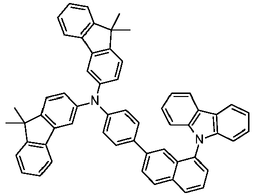 |
| S41 | CAS-1190360-23-6 | #P103 |
| 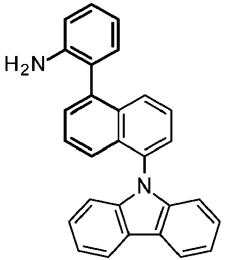 | 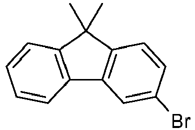 | 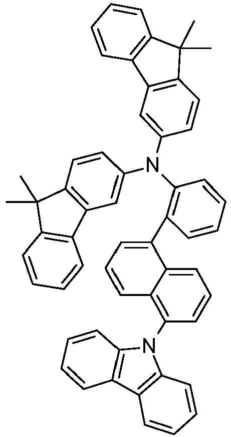 |
| S42 | CAS-1190360-23-6 | #P104 |
| 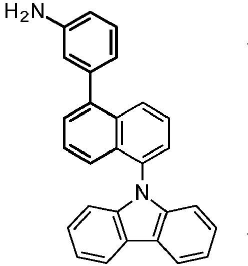 | 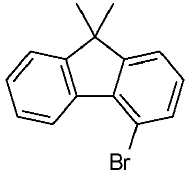 | 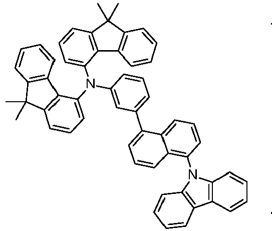 |
| S43 | CAS-942615-32 | P105 |
| 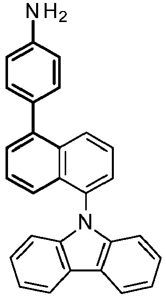 | 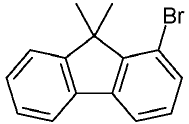 | 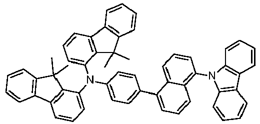 |
| S44 | CAS-1225053-54-2 | #P106 |
| 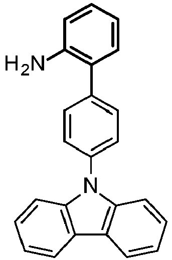 | 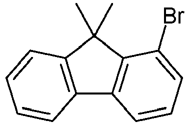 | 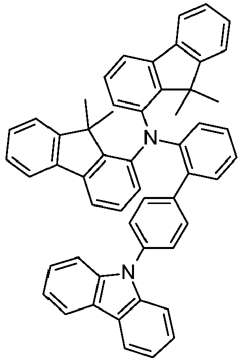 |
| S45 | CAS-1225053-54-2 | #P107 |
| 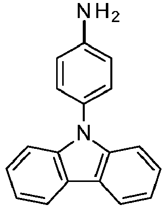 | 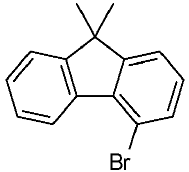 | 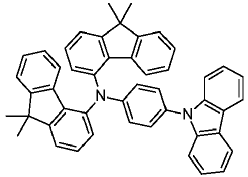 |
| CAS-52708-37-9 | CAS-942615-32 | P108 |
| 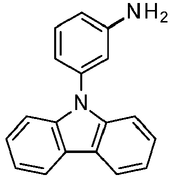 | 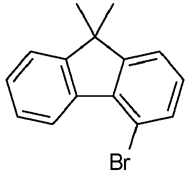 | 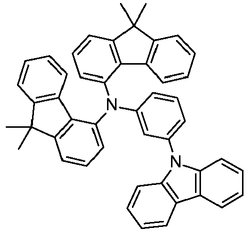 |
| CAS-1023659-21-3 | CAS-942615-32 | P109 |
| 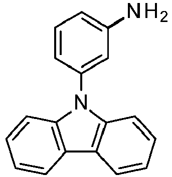 | 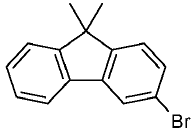 | 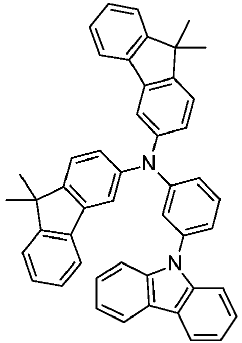 |
| CAS-1023659-21-3 | CAS-1190360-23-6 | #P110 |
| 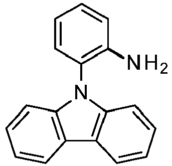 | 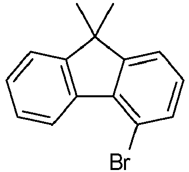 | 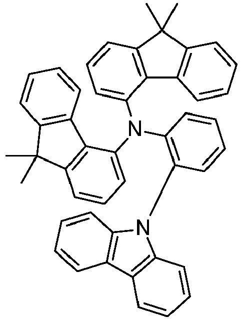 |
| CAS-101716-43-2 | CAS-942615-32 | P111 |
| 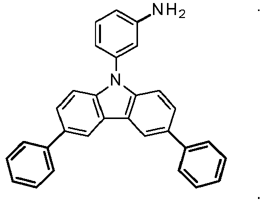 | 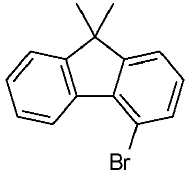 | 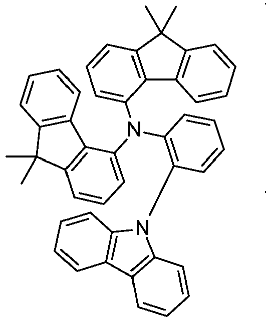 |
| CAS-1708110-65-9 | CAS-942615-32 | P112 |
| 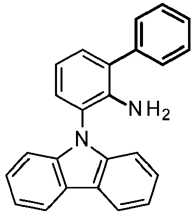 | 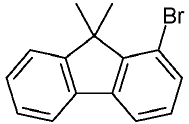 | 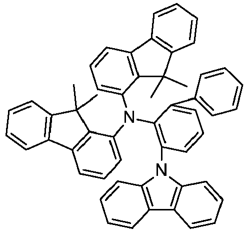 |
| CAS-1673542-71-6 | CAS-1225053-54-2 | #P113 |

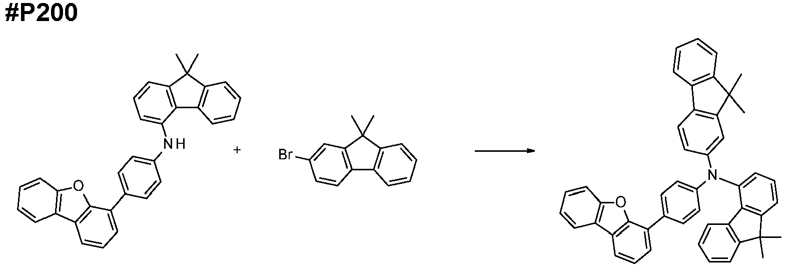

N-(4-(Dibenzo[b,d]furan-4-yl)phenyl)-9,9-dimethyl-9H-fluoren-4-amin [CAS-1933454-49-9] (20.4 g; 45.27 mmol), 2-Bromo-9,9-dimethyl-9H-fluoren [CAS-28320-31-2] (14.1 g; 50.0 mmol) und Natrium-*tert*-butylat (6.78 g; 70.55 mmol) werden in Toluol (350 mL) vogelegt und 45 min mit Argon gesättigt. Danach werden Tris(dibenzy-lideneacetone)dipalladium(0) [CAS-51364-51-3] (831 mg; 0.91 mmol) und S-Phos [CAS- 657408-07-6] (745 mg; 1.91 mmol) eingetragen und die Reaktionsmischung 16 h unter Rückfluss gerührt. Nach Abkühlen des Ansatzes wird dieser mit Wasser (300 mL) erweitert und im Scheidetrichter extraktiv aufgearbeitet. Die organische Phase wir 2 x mit Wasser (je 300 mL) gewaschen, über Na₂SO₄ getrocknet und zur Trockene eingeengt. Das Rohprodukt wird zweimal mit Toluol/*n*-Heptan über Alox basisch heißextrahiert, anschließend zweimal aus *n*-Butanol umkristallisiert und abschließend im Hochvakuum sublimiert. Ausbeute: 13.6 g (19.6 mmol), 43%; Reinheit: > 99.9 %ig nach HPLC.

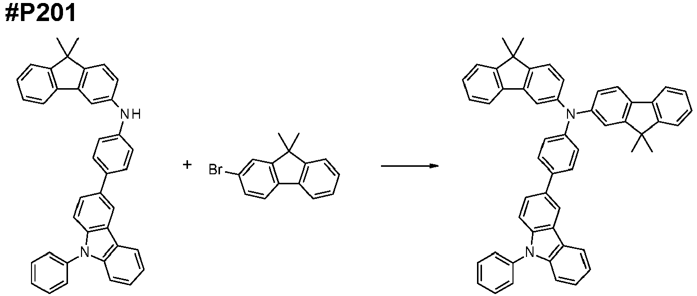

Durchführung analog der Versuchsbeschreibung zu P200, nur dass statt N-(4-(dibenzo[b,d]furan-4-yl)phenyl)-9,9-dimethyl-9H-fluoren-4-amin 9,9-dimethyl-N-[4-(9-phenylcarbazol-3-yl)phenyl]fluoren-3-amin [CAS-2110513-13-6] verwendet wird. Das Rohprodukt wird zweimal mit Toluol/*n*-Heptan über Alox basisch heißextrahiert, anschließend zweimal aus Ethylacetat umkristallisiert und abschließend im Hochvakuum sublimiert. Ausbeute: 12.4 g (17.2 mmol), 38%; Reinheit: > 99.9 %ig nach HPLC.

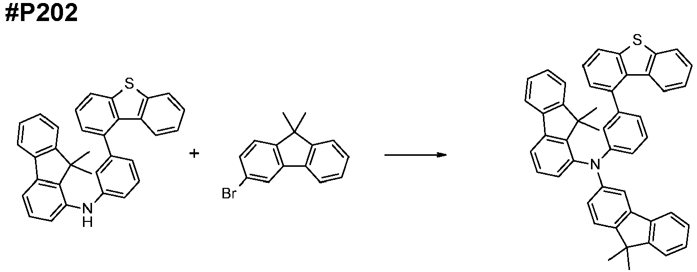

Durchführung analog der Veruschbeschreibung zu P200, nur dass statt N-(4-(dibenzo[b,d]furan-4-yl)phenyl)-9,9-dimethyl-9H-fluoren-4-amin S102 und statt 2-Bromo-9,9-dimethyl-9H-fluoren 3-Bromo-9,9-dimethyl-9H-fluoren [CAS-1190360-23-6] verwendet wird.Das Rohprodukt wird zweimal mit Toluol/*n*-Heptan über Alox basisch heißextrahiert, anschließend zweimal aus n-Butylacetat umkristallisiert und abschließend im Hochvakuum sublimiert. Ausbeute: 14.1 g (21.3 mmol), 47%; Reinheit: > 99.9 %ig nach HPLC.

Analog können folgende Verbindungen dargestellt werden. Hierbei kann als Katalysatorsystem statt Tris(dibenzylidenacetone)dipalladium(O) [CAS-51364-51-3], und S-Phos [CAS-657408-07-6] auch Palladium(II)-acetat [3375-31-3] (0.02 equiv.) und Tri-*tert*-butylphosphin-Lösung 1.0 M in Toluol [13716-12-6] (0.05 equiv.) verwendet werden. Hierbei kann zur Aufreinigung auch Säulenchromatographie verwendet werden, oder zur Umkristallisation oder Heißextraktion andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid; N-Methylpyrrolidon etc. Die Ausbeuten liegen typischerweise im Bereich zwischen 15% und 75%.

| Edukt 1 | Edukt 2 | Produkt |
|---|---|---|
| 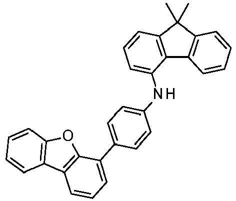 | 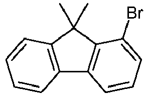 | 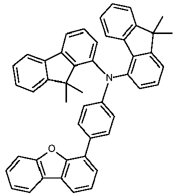 |
| CAS-1933454-49-9 | CAS-1225053-54-2 | #P203 |
| 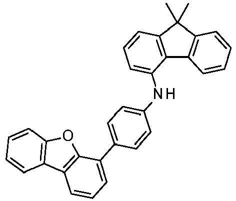 | 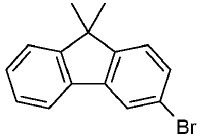 CAS-1190360-23-6 | 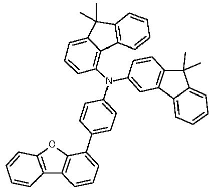 |
| CAS-1933454-49-9 | | #P204 |
| 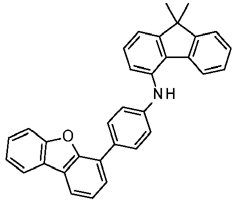 | 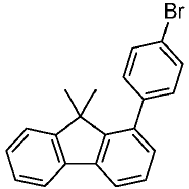 | 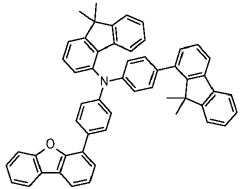 |
| CAS-1933454-49-9 | CAS-1786416-87-2 | #P205 |
| 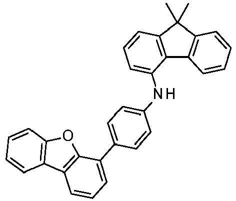 | 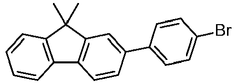 | 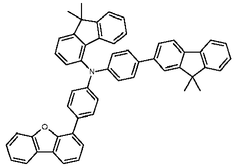 |
| CAS-1933454-49-9 | CAS-574750-94-0 | #P206 |
| 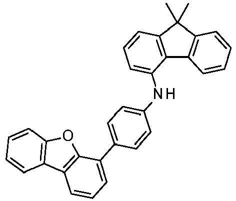 | 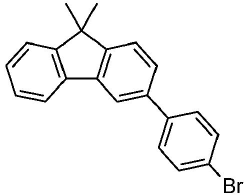 | 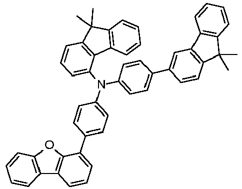 |
| CAS-1933454-49-9 | CAS-1421789-06-1 | #P207 |
| 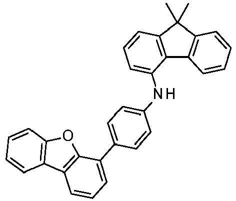 | 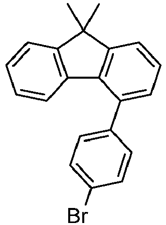 | 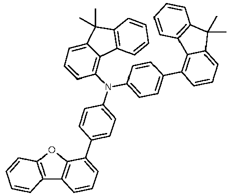 |
| CAS-1933454-49-9 | CAS-1860896-38-3 | #P208 |
| 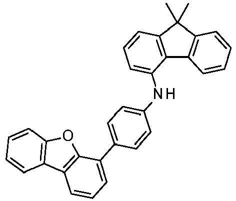 | 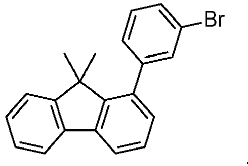 | 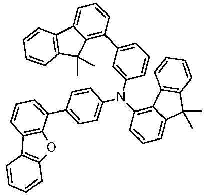 |
| CAS-1933454-49-9 | CAS-2035081-41-3 | #P209 |
| 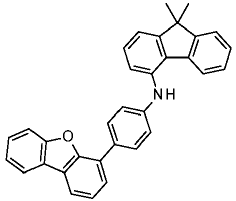 | 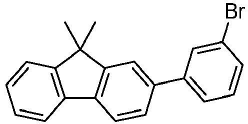 | 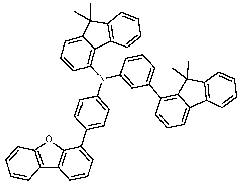 |
| CAS-1933454-49-9 | CAS-881912-14-7 | #P210 |
| 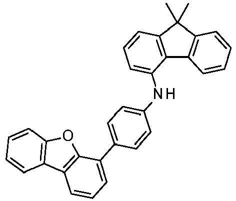 | 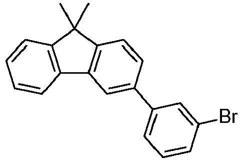 | 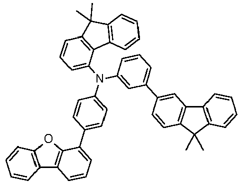 |
| CAS-1933454-49-9 | CAS-1417161-08-0 | #P211 |
| 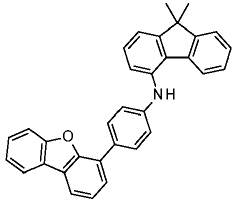 | 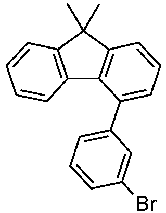 | 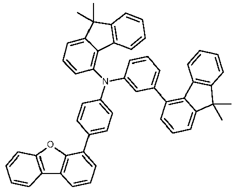 |
| CAS-1933454-49-9 | CAS-1860896-40-7 | #P213 |
| 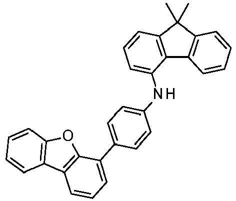 | 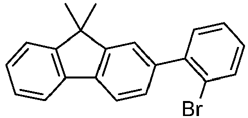 | 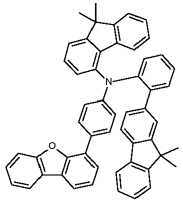 |
| CAS-1933454-49-9 | CAS-881912-12-5 | #P214 |
| 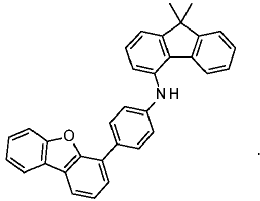 | 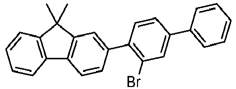 | 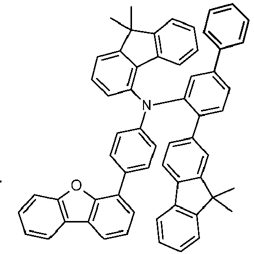 |
| CAS-1933454-49-9 | CAS-881912-16-9 | #P215 |
| 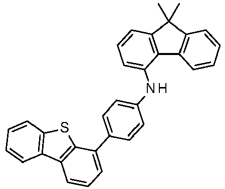 | 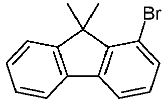 | 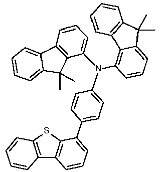 |
| CAS-2089116-67-4 | CAS-1225053-54-2 | #P216 |
| 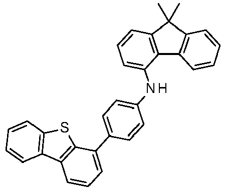 | 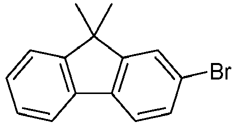 | 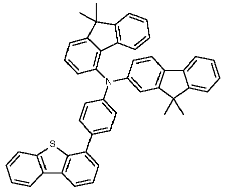 |
| CAS-2089116-67-4 | CAS-28320-31-2 | #P217 |
| 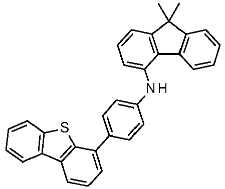 | 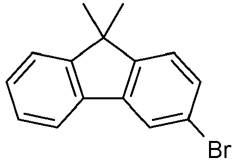 | 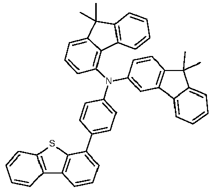 |
| CAS-2089116-67-4 | CAS-1190360-23-6 | #P218 |
| 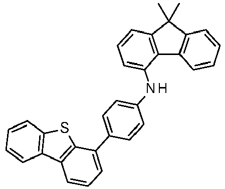 | 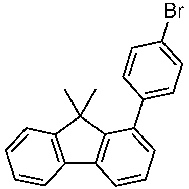 | 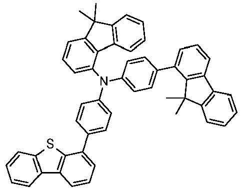 |
| CAS-2089116-67-4 | CAS-1786416-87-2 | #P219 |
| 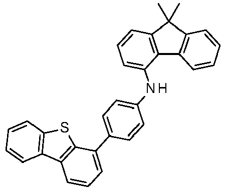 | 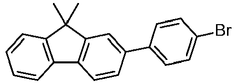 | 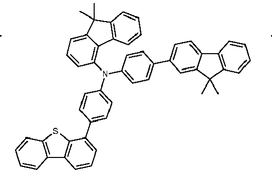 |
| CAS-2089116-67-4 | CAS-574750-94-0 | #P220 |
| 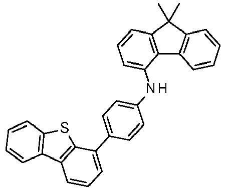 | 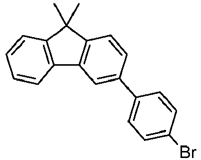 | 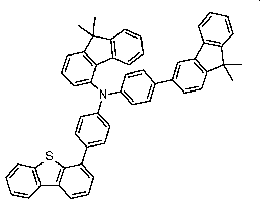 |
| CAS-2089116-67-4 | CAS-1421789-06-1 | #P221 |
| 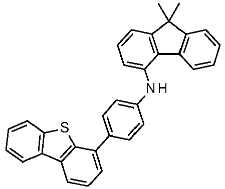 | 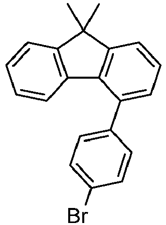 | 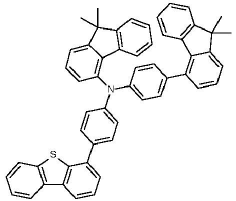 |
| CAS-2089116-67-4 | CAS-1860896-38-3 | #P222 |
| 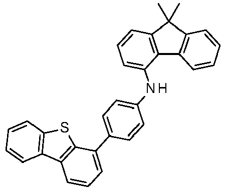 | 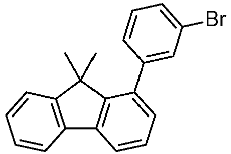 | 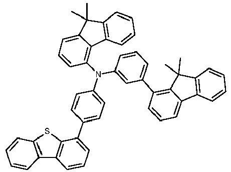 |
| CAS-2089116-67-4 | CAS-2035081-41-3 | #P223 |
| 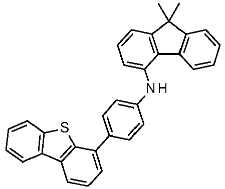 | 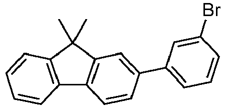 | 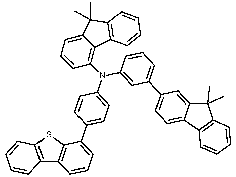 |
| CAS-2089116-67-4 | CAS-881912-14-7 | #P224 |
| 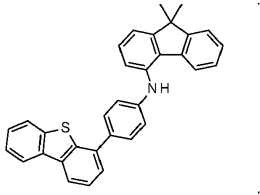 | 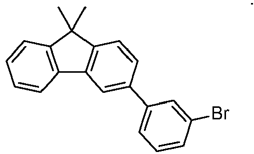 | 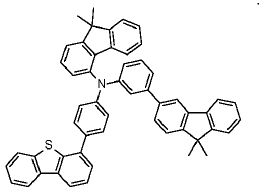 |
| CAS-2089116-67-4 | CAS-1417161-08-0 | #P225 |
| 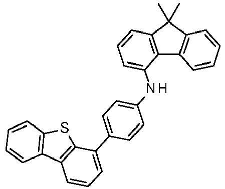 | 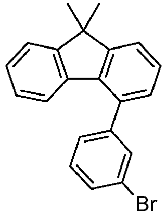 | 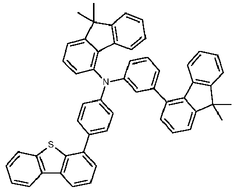 |
| CAS-2089116-67-4 | CAS-1860896-40-7 | #P226 |
| 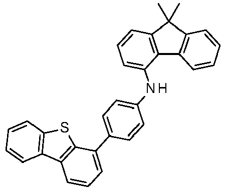 | 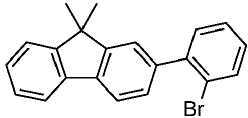 | 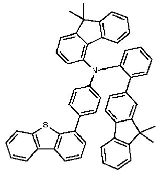 |
| CAS-2089116-67-4 | CAS-881912-12-5 | #P227 |
| 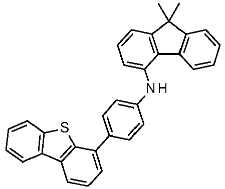 | 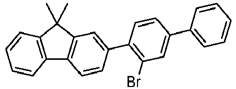 | 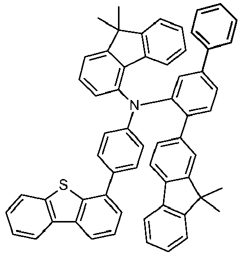 |
| CAS-2089116-67-4 | CAS-881912-16-9 | #P228 |
| 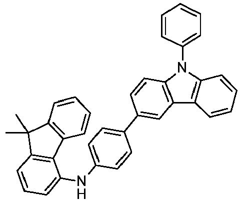 | 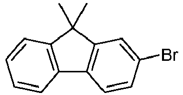 | 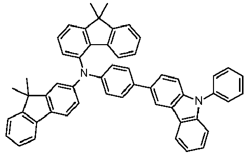 |
| S100 | CAS-28320-31-2 | #P229 |
| 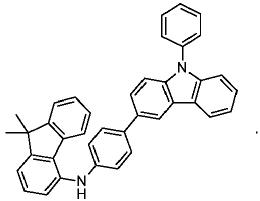 | 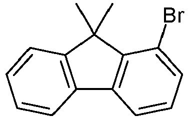 | 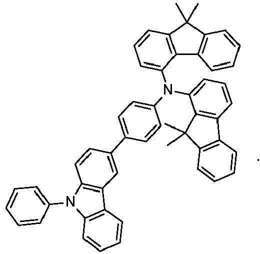 |
| S100 | CAS-1225053-54-2 | #P230 |
| 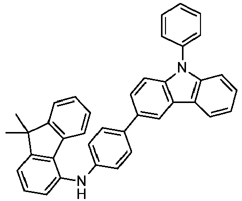 | 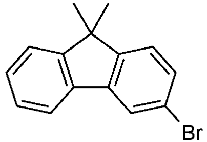 | 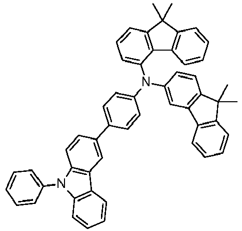 |
| S100 | CAS-1190360-23-6 | #P231 |
| 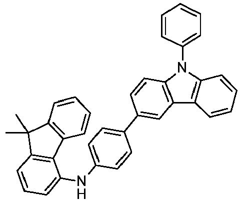 | 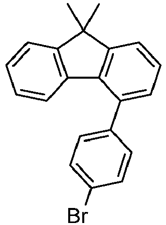 | 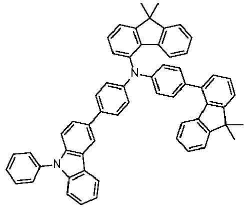 |
| S100 | CAS-1860896-38-3 | #P232 |
| 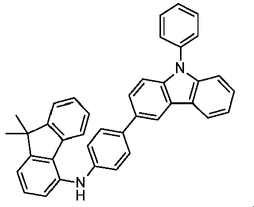 | 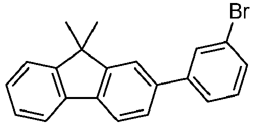 | 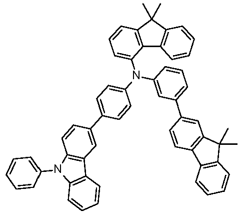 |
| S100 | CAS-881912-14-7 | #P233 |
| 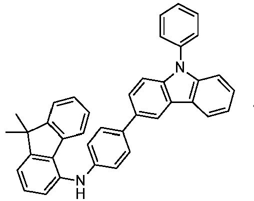 | 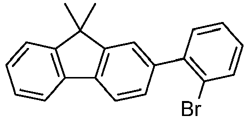 | 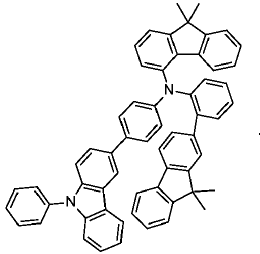 |
| S100 | CAS-881912-12-5 | #P234 |
| 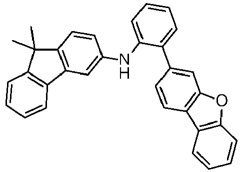 S101 | 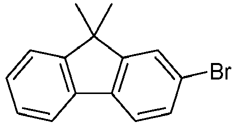 | 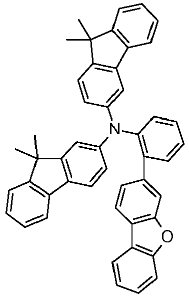 |
| | CAS-28320-31-2 | #P235 |
| 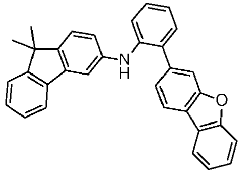 | 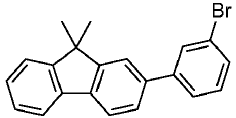 | 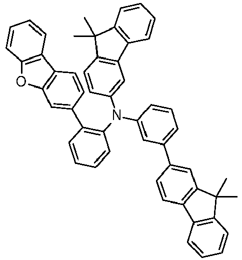 |
| S101 | CAS-881912-14-7 | #P236 |
| 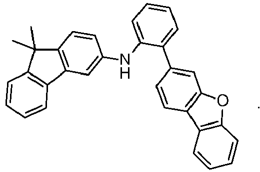 | 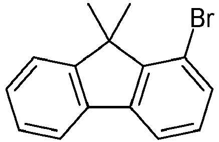 | 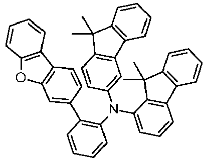 |
| S101 | CAS-1225053-54-2 | #P237 |
| 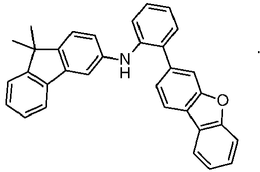 | 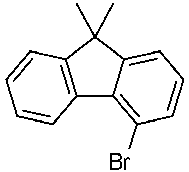 | 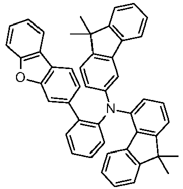 |
| S101 | CAS-942615-32-9 | #P238 |
| 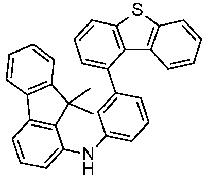 | 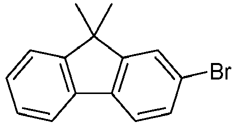 | 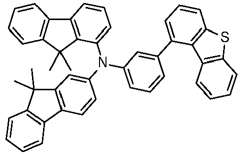 |
| S102 | CAS-28320-31-2 | #P239 |
| 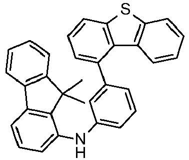 | 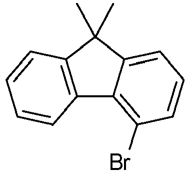 | 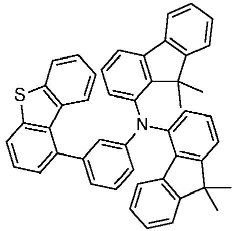 |
| S102 | CAS-942615-32-9 | #P240 |
| 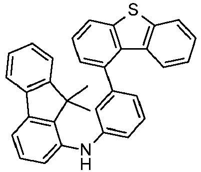 | 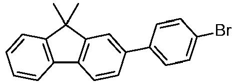 | 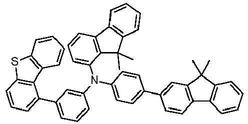 |
| S102 | CAS-574750-94-0 | #P241 |
| 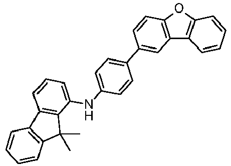 | 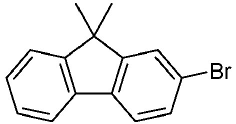 | 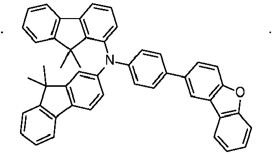 |
| S103 | CAS-28320-31-2 | #P242 |
| 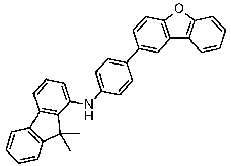 | 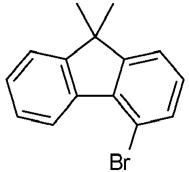 | 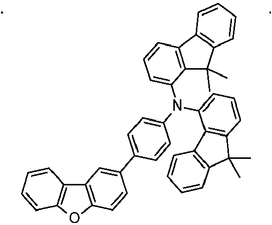 |
| S103 | CAS-942615-32-9 | #P243 |
| 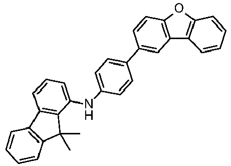 | 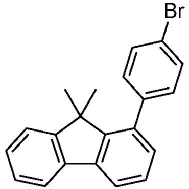 | 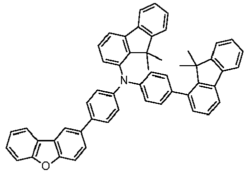 |
| S103 | CAS-1786416-87-2 | #P244 |
| 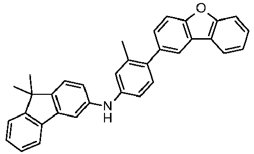 | 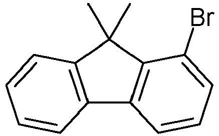 | 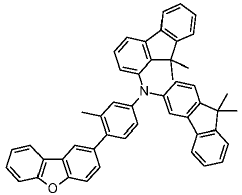 |
| S104 | CAS-1225053-54-2 | #P245 |
| 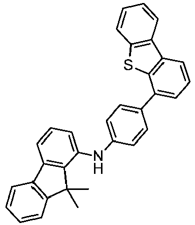 | 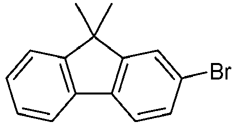 | 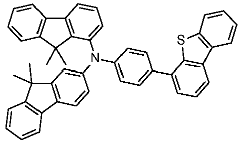 |
| S105 | CAS-28320-31-2 | #P246 |
| 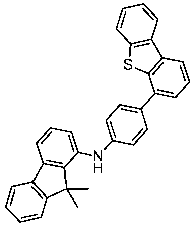 | 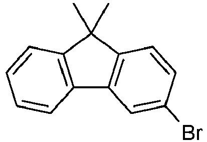 | 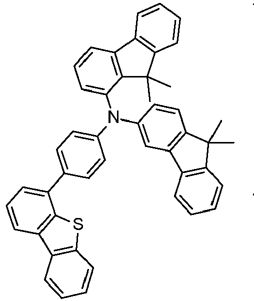 |
| S105 | CAS-1190360-23-6 | #P247 |
| 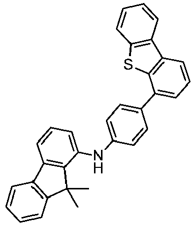 | 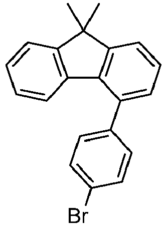 | 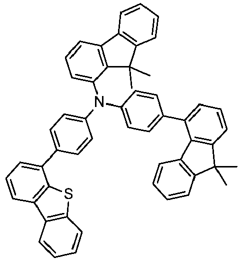 |
| S105 | CAS-1860896-38-3 | #P248 |
| 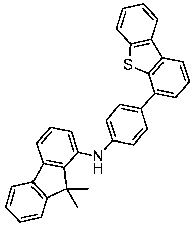 | 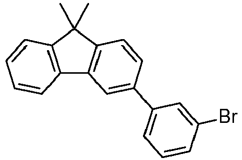 | 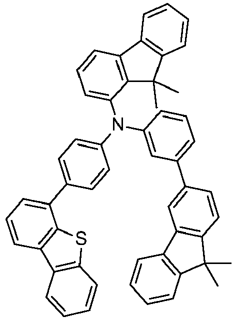 |
| S105 | CAS-1417161-08-0 | #P249 |
| 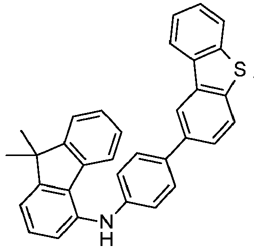 | 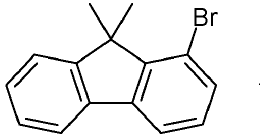 | 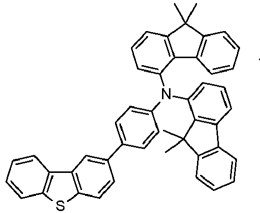 |
| S106 | CAS-1225053-54-2 | #P250 |
| 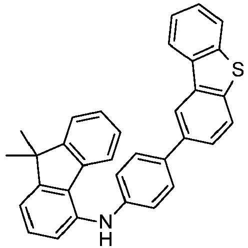 | 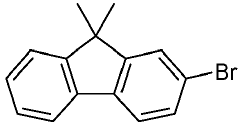 | 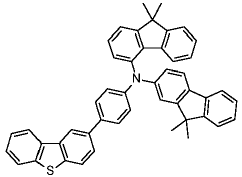 |
| S106 | CAS-28320-31-2 | #P251 |
| 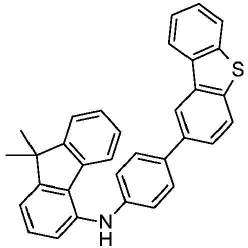 | 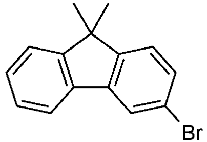 | 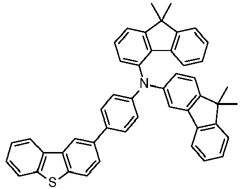 |
| S106 | CAS-1190360-23-6 | #P252 |
| 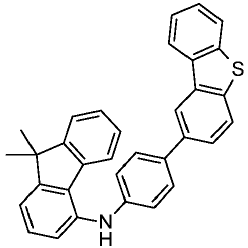 | 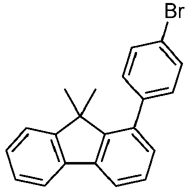 | 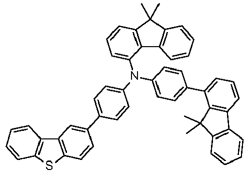 |
| S106 | CAS-1786416-87-2 | #P253 |
| 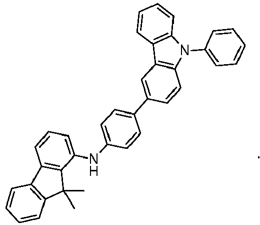 | 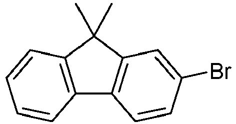 | 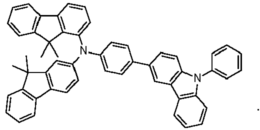 |
| S107 | CAS-28320-31-2 | #P254 |
| 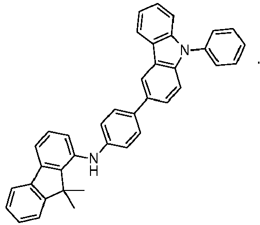 | 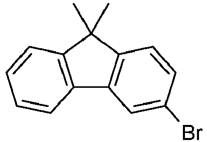 | 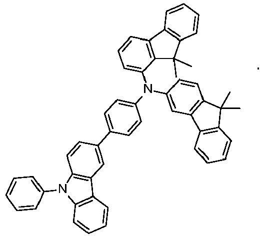 |
| S107 | CAS-1190360-23-6 | #P255 |
| 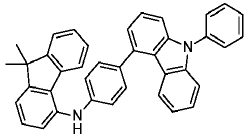 | 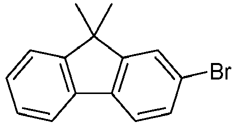 | 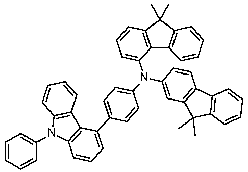 |
| S108 | CAS-28320-31-2 | #P256 |
| 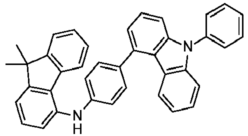 | 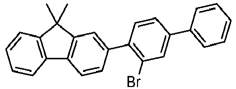 | 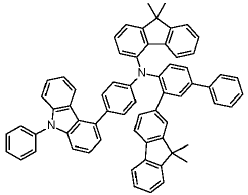 |
| S108 | CAS-881912-16-9 | #P257 |
| 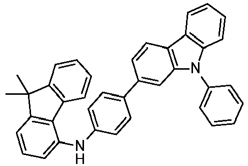 | 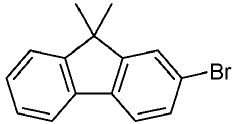 | 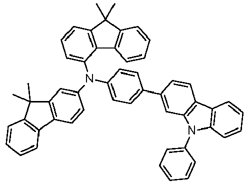 |
| S109 | CAS-28320-31-2 | #P258 |
| 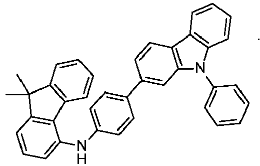 | 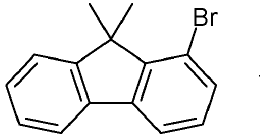 | 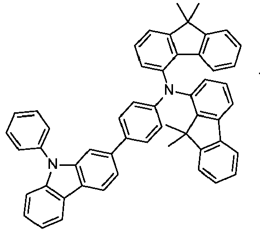 |
| S109 | CAS-1225053-54-2 | #P259 |
| 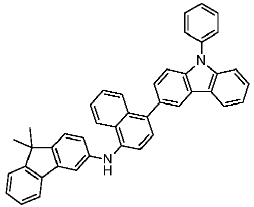 | 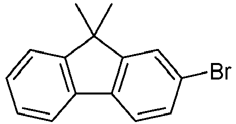 | 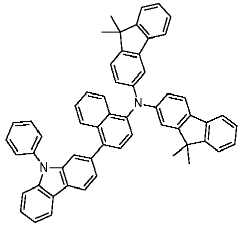 |
| S110 | CAS-28320-31-2 | #P260 |
| 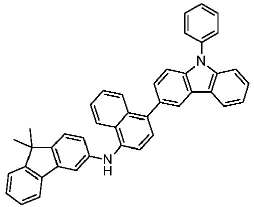 | 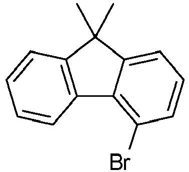 | 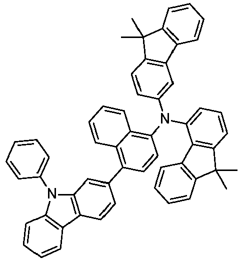 |
| S110 | CAS-942615-32-9 | #P261 |
| 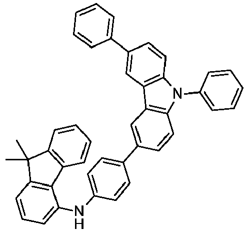 | 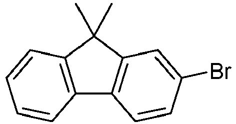 | 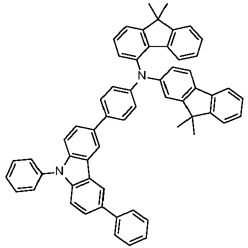 |
| S111 | CAS-28320-31-2 | #P262 |
| 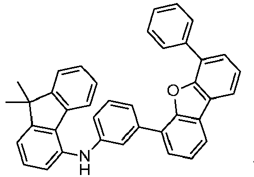 | 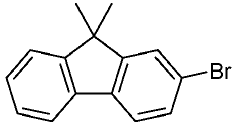 | 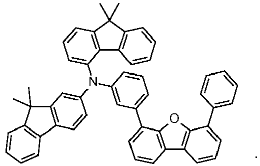 |
| S112 | CAS-28320-31-2 | #P263 |
| 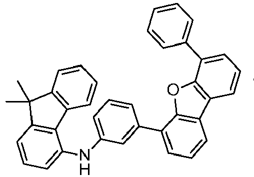 | 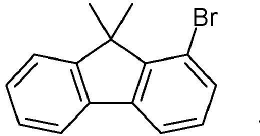 | 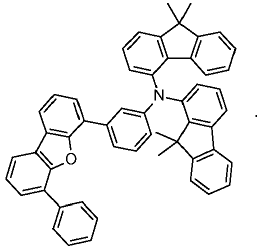 |
| S112 | CAS-1225053-54-2 | #P264 |
| 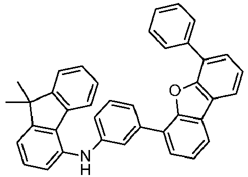 | 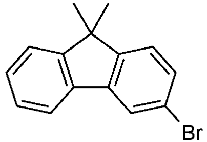 | 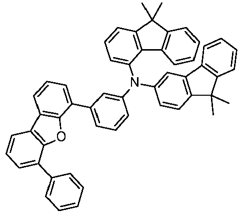 |
| S112 | CAS-1190360-23-6 | #P265 |
| 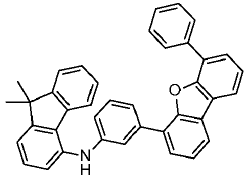 | 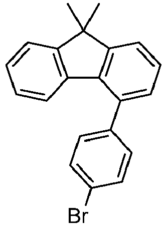 | 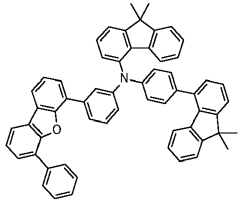 |
| S112 | CAS-1860896-38-3 | #P266 |
| 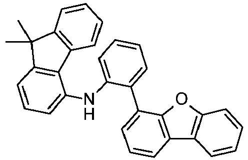 | 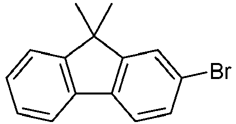 | 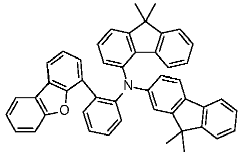 |
| S113 | CAS-28320-31-2 | #P267 |
| 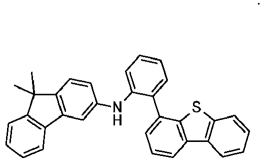 | 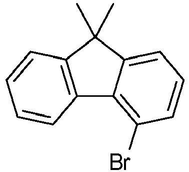 | 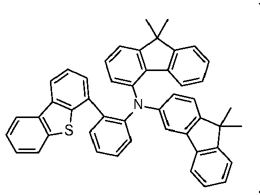 |
| S114 | CAS-942615-32-9 | #P268 |
| 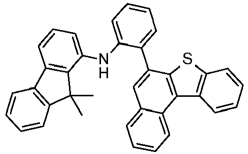 | 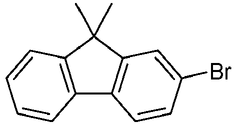 | 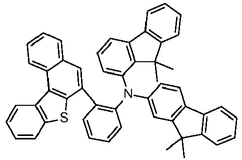 |
| S115 | CAS-28320-31-2 | #P269 |
| 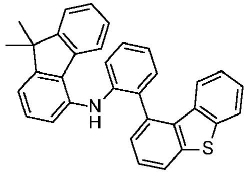 | 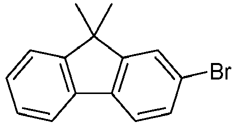 | 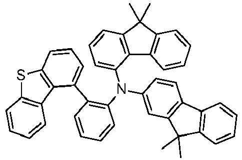 |
| S116 | CAS-28320-31-2 | #P270 |
| 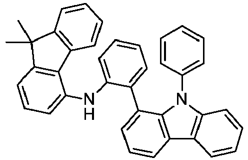 | 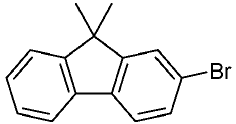 | 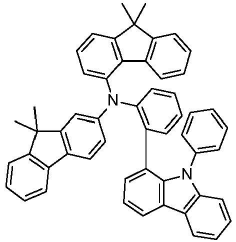 |
| S117 | CAS-28320-31-2 | #P271 |
| 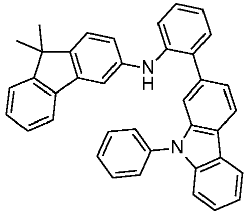 | 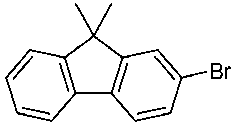 | 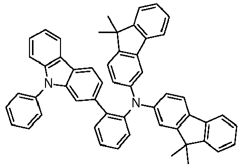 |
| S118 | CAS-28320-31-2 | #P272 |
| 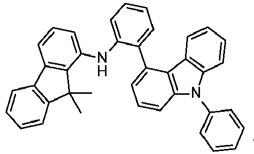 | 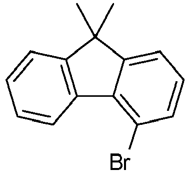 | 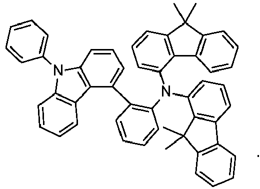 |
| S119 | CAS-942615-32-9 | #P273 |
| 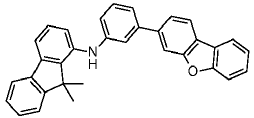 | 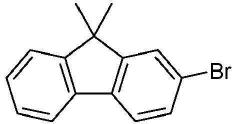 | 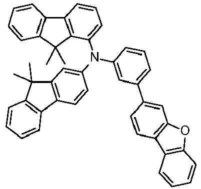 |
| S120 | CAS-28320-31-2 | #P274 |
| 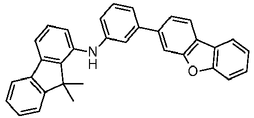 | 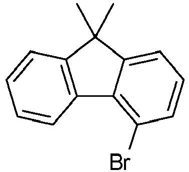 | 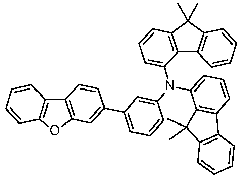 |
| S120 | CAS-942615-32-9 | #P275 |
| 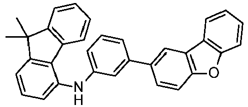 | 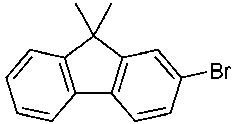 | 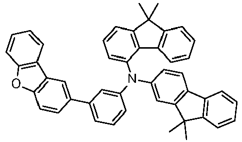 |
| S121 | CAS-28320-31-2 | #P276 |
| 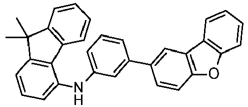 | 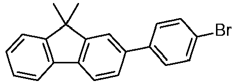 | 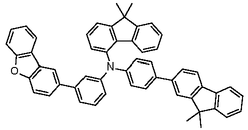 |
| S121 | CAS-574750-94-0 | #P277 |
| 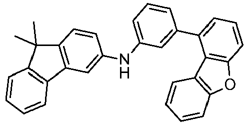 | 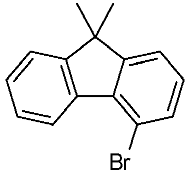 | 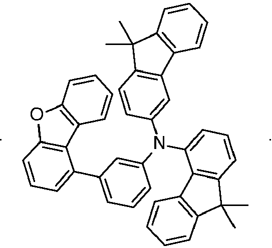 |
| S122 | CAS-942615-32-9 | #P278 |
| 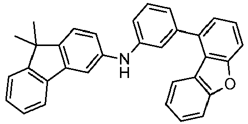 | 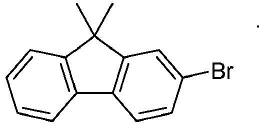 | 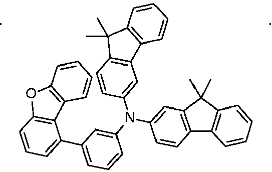 |
| S122 | CAS-28320-31-2 | #P279 |
| 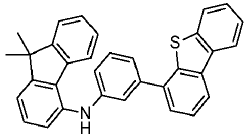 | 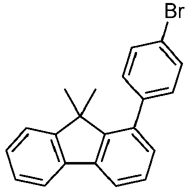 | 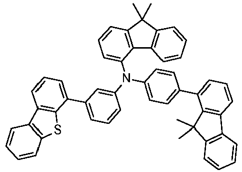 |
| S123 | CAS-1786416-87-2 | #P280 |
| 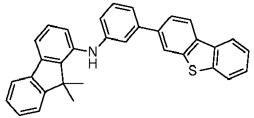 | 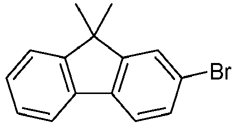 | 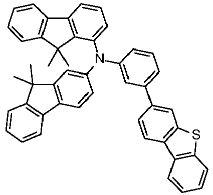 |
| S124 | CAS-28320-31-2 | #P281 |
| 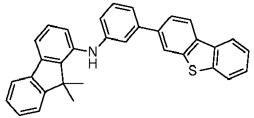 | 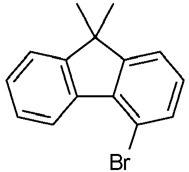 | 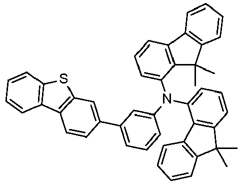 |
| S124 | CAS-942615-32-9 | #P282 |
| 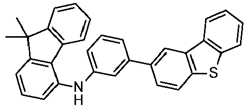 | 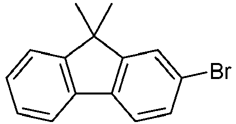 | 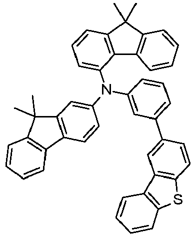 |
| S125 | CAS-28320-31-2 | #P283 |
| 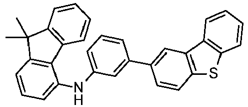 | 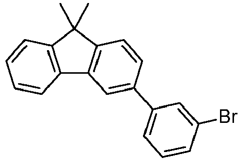 | 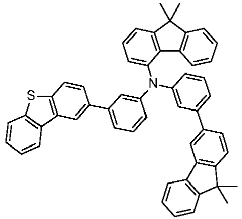 |
| S125 | CAS-1417161-08-0 | #P284 |
| 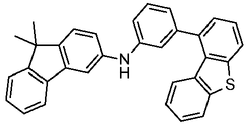 | 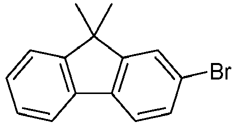 | 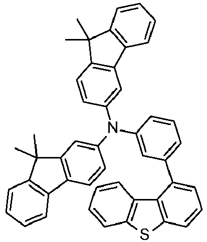 |
| S126 | CAS-28320-31-2 | #P285 |
| 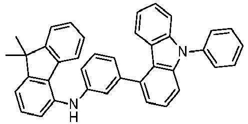 | 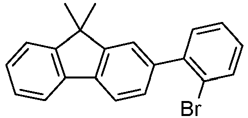 | 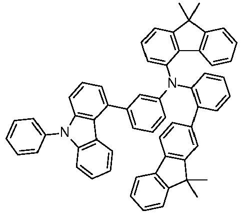 |
| S127 | CAS-881912-12-5 | #P286 |
| 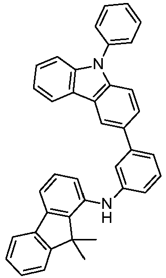 | 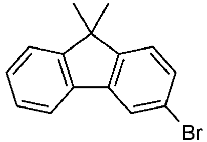 | 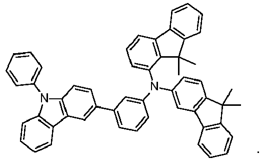 |
| S128 | CAS-1190360-23-6 | #P287 |
| 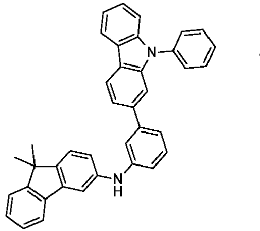 | 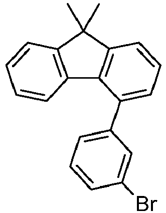 | 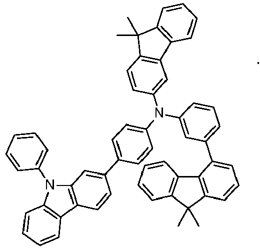 |
| S129 | CAS-1860896-40-7 | #P288 |
| 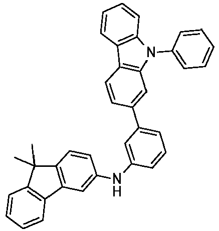 | 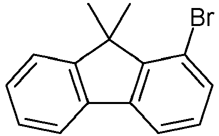 | 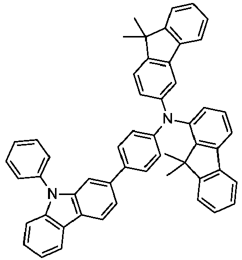 |
| S129 | CAS-1225053-54-2 | #P289 |
| 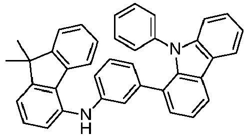 | 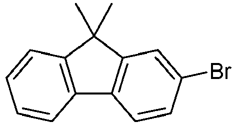 | 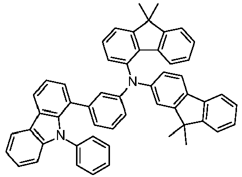 |
| S130 | CAS-28320-31-2 | #P290 |
| 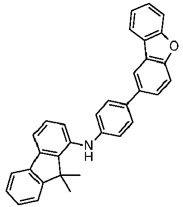 | 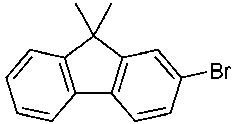 | 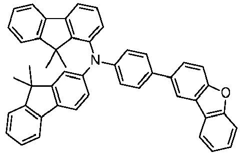 |
| S131 | CAS-28320-31-2 | #P291 |
| 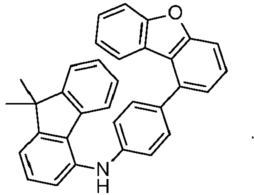 | 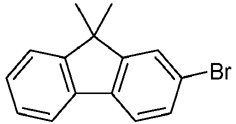 | 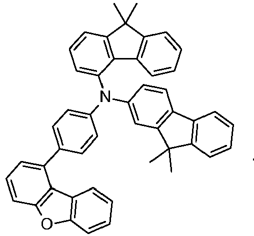 |
| S132 | CAS-28320-31-2 | #P292 |
| 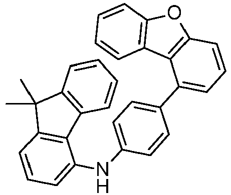 | 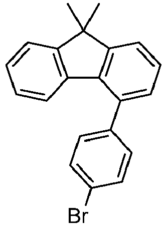 | 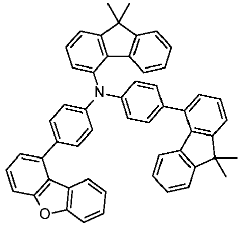 |
| S132 | CAS-1860896-38-3 | #P293 |
| 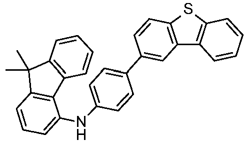 | 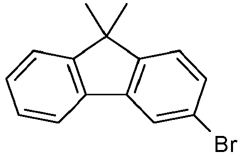 | 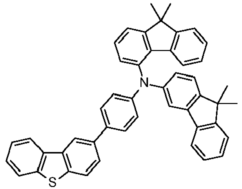 |
| S133 | CAS-1190360-23-6 | #P294 |
| 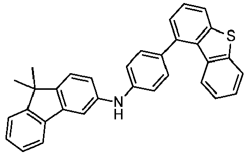 | 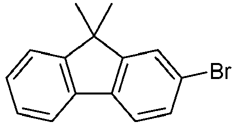 | 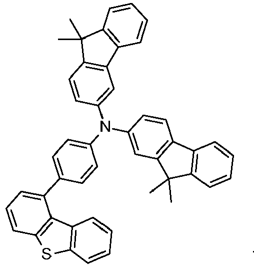 |
| S134 | CAS-28320-31-2 | #P295 |
| 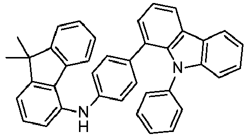 | 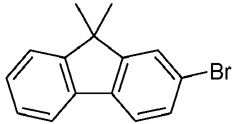 | 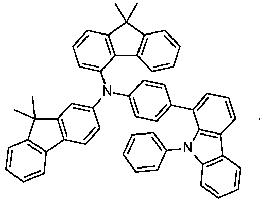 |
| S135 | CAS-28320-31-2 | #P296 |
| 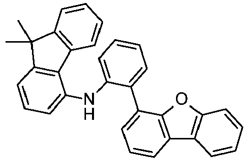 | 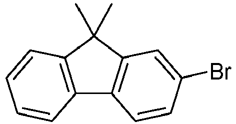 | 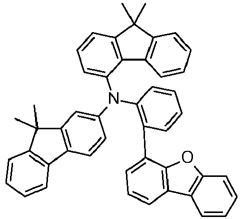 |
| S136 | CAS-28320-31-2 | #P297 |
| 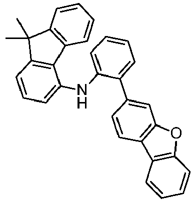 | 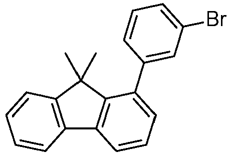 | 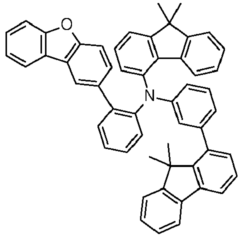 |
| S137 | CAS-2035081-41-3 | #P298 |
| 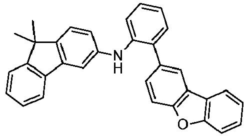 | 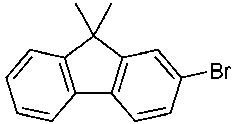 | 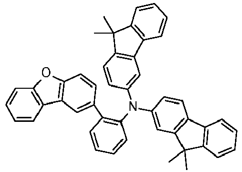 |
| S138 | CAS-28320-31-2 | #P299 |
| 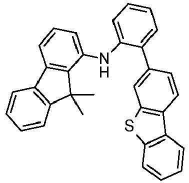 | 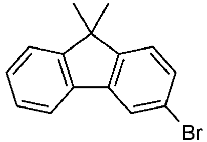 | 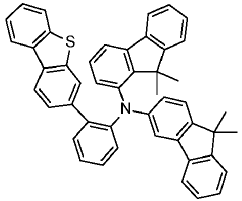 |
| S139 | CAS-1190360-23-6 | #P300 |
| 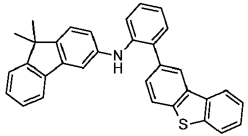 | 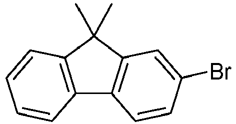 | 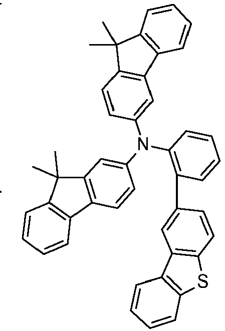 |
| S140 | CAS-28320-31-2 | #P301 |
| 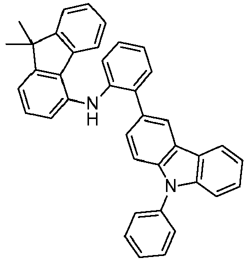 | 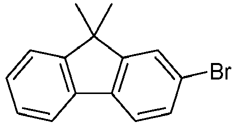 | 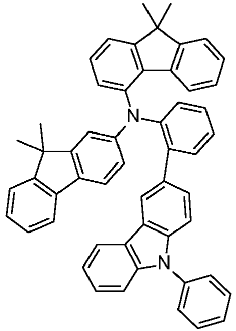 |
| S141 | CAS-28320-31-2 | #P302 |
| 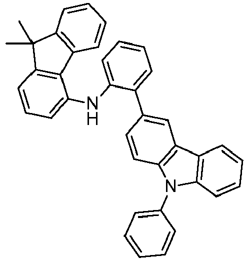 | 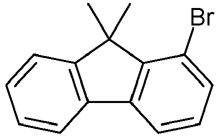 | 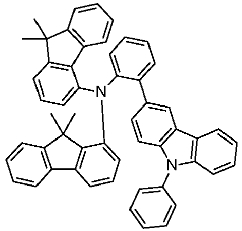 |
| S141 | CAS-1225053-54-2 | #P303 |
| 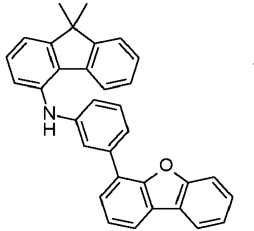 | 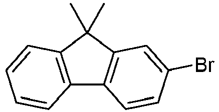 | 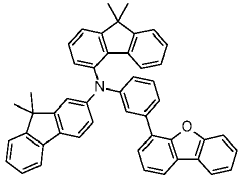 |
| S142 | CAS-28320-31-2 | #P304 |
| 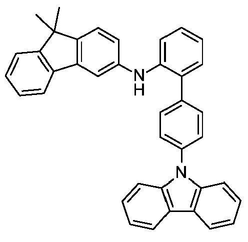 | 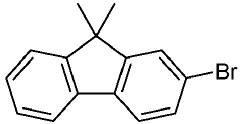 | 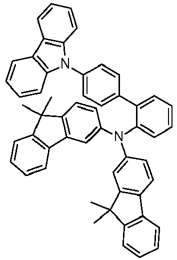 |
| S143 | CAS-28320-31-2 | #P305 |
| 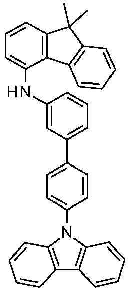 | 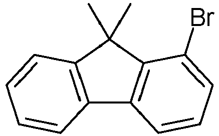 | 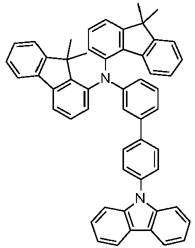 |
| S144 | CAS-1225053-54-2 | P306 |
| 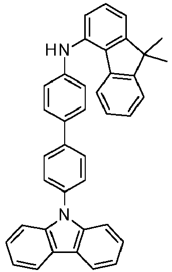 | 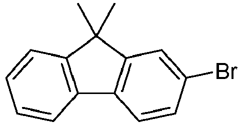 | 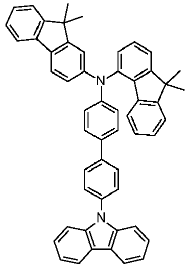 |
| S145 | CAS-28320-31-2 | #P307 |
| 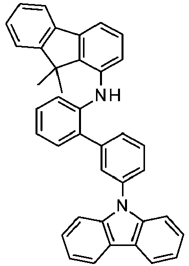 | 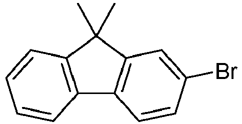 | 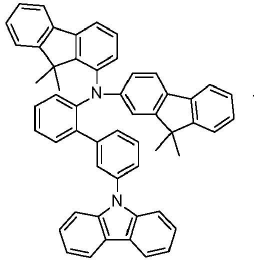 |
| S146 | CAS-28320-31-2 | #P308 |
| 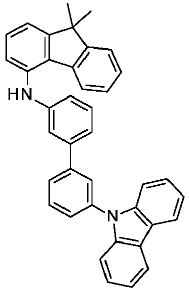 | 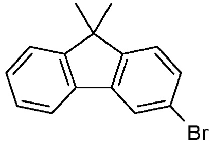 | 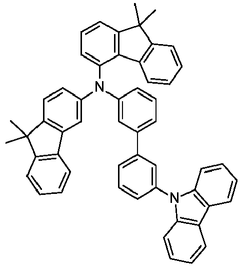 |
| S147 | CAS-1190360-23-6 | #P309 |
| 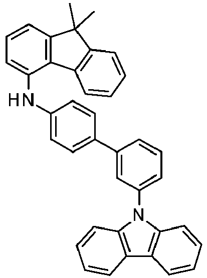 | 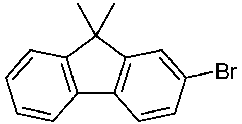 | 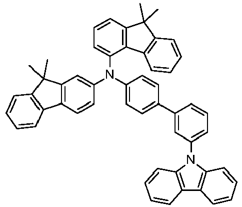 |
| S148 | CAS-28320-31-2 | #P310 |
| 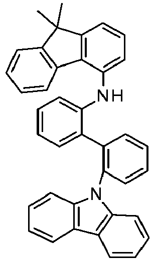 | 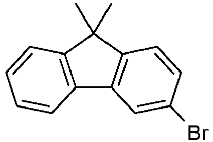 | 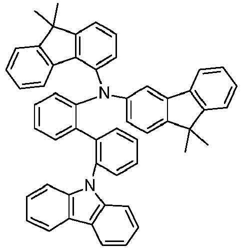 |
| S149 | CAS-1190360-23-6 | #P311 |
| 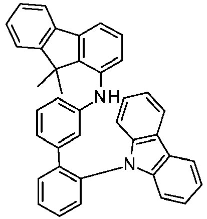 | 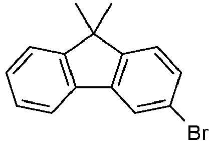 | 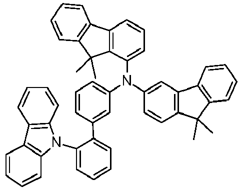 |
| S150 | CAS-1190360-23-6 | #P312 |
| 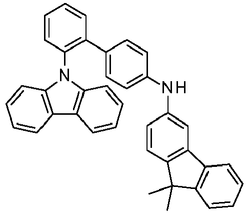 | 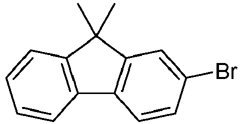 | 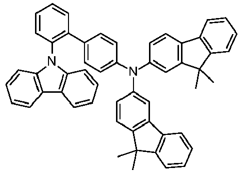 |
| S151 | CAS-28320-31-2 | #P313 |
| 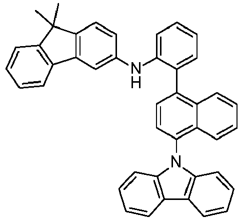 | 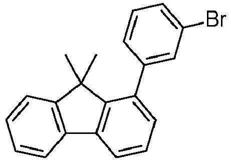 | 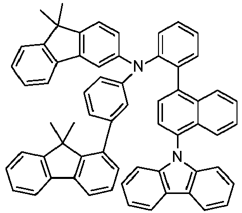 |
| S152 | CAS-2035081-41-3 | #P314 |
| 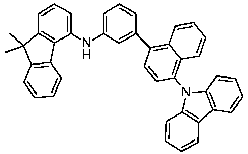 | 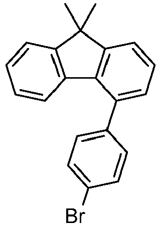 | 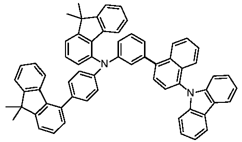 |
| S153 | CAS-1860896-38-3 | #P315 |
| 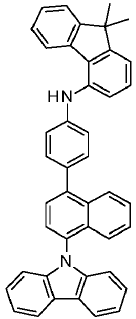 | 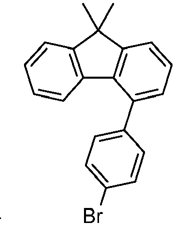 | 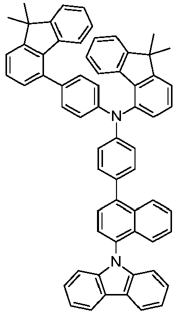 |
| S154 | CAS-1860896-38-3 | #P316 |
| 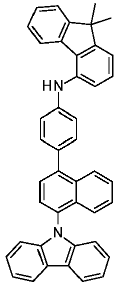 | 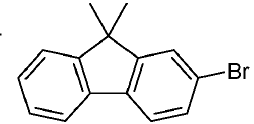 | 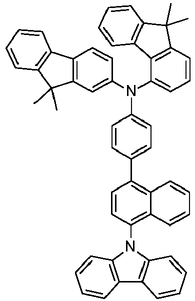 |
| S154 | CAS-28320-31-2 | #P317 |
| 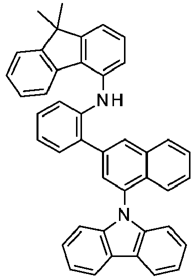 | 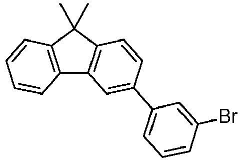 | 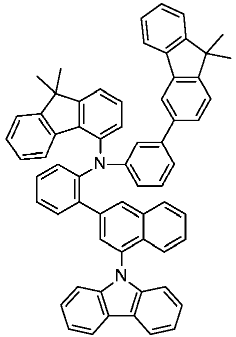 |
| S155 | CAS-1417161-08-0 | #P318 |
| 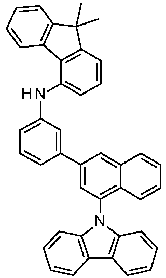 | 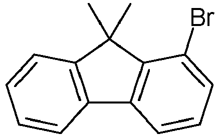 | 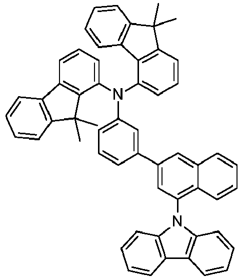 |
| S156 | CAS-1225053-54-2 | #P319 |
| 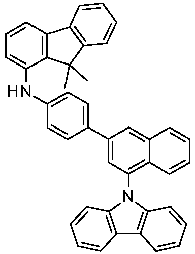 | 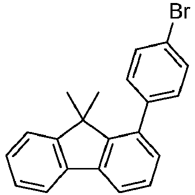 | 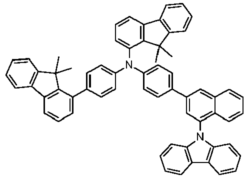 |
| S157 | CAS-1786416-87-2 | #P320 |
| 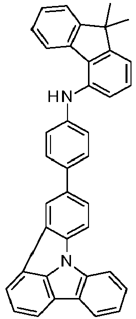 | 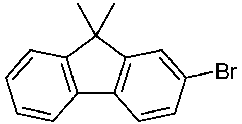 | 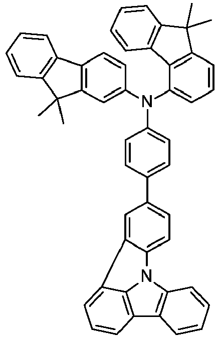 |
| S158 | CAS-28320-31-2 | #P321 |
| 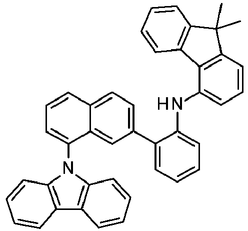 | 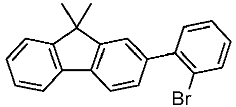 | 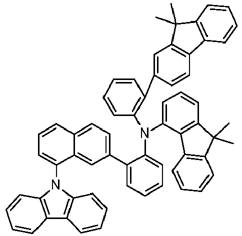 |
| S159 | CAS-881912-12-5 | #P322 |
| 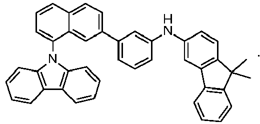 | 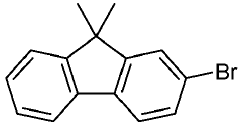 | 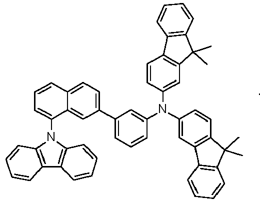 |
| S160 | CAS-28320-31-2 | #P323 |
| 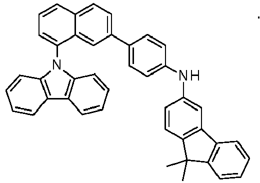 | 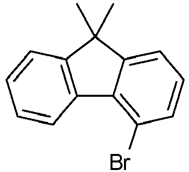 | 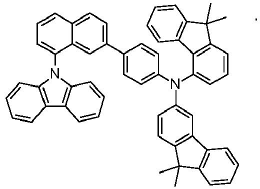 |
| S161 | CAS-942615-32-9 | #P324 |
| 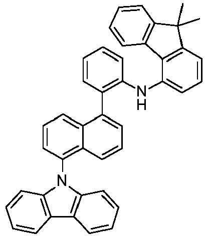 | 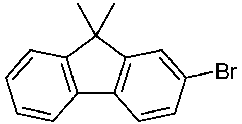 | 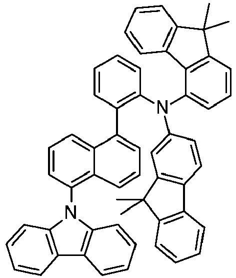 |
| S162 | CAS-28320-31-2 | #P326 |
| 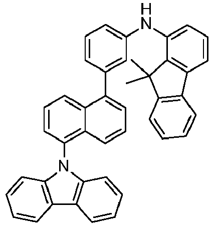 | 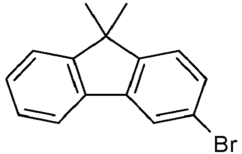 | 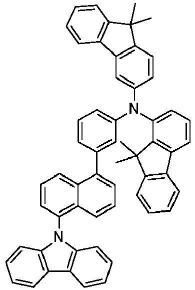 |
| S163 | CAS-1190360-23-6 | #P327 |
| 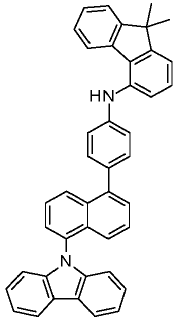 | 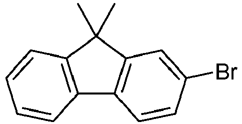 | 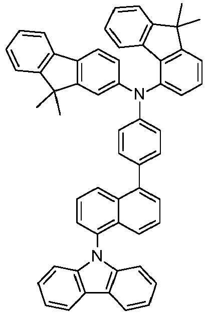 |
| S164 | CAS-28320-31-2 | #P328 |
| 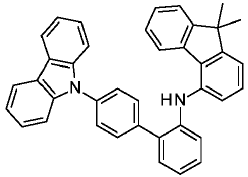 | 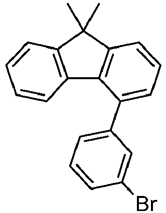 | 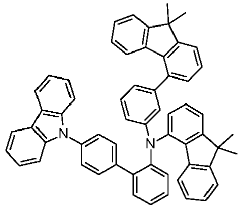 |
| S165 | CAS-1860896-40-7 | #P329 |
| 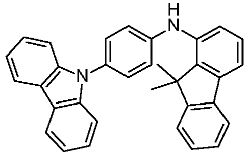 | 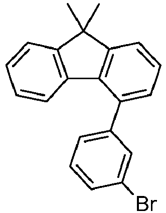 | 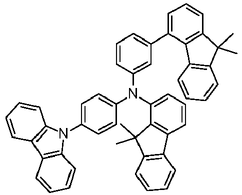 |
| S166 | CAS-1860896-40-7 | #P330 |
| 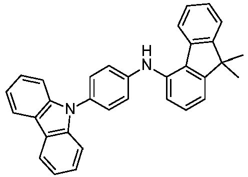 | 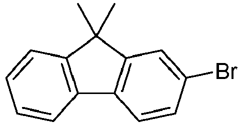 | 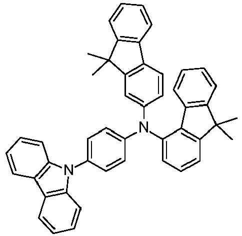 |
| S167 | CAS-28320-31-2 | #P331 |
| 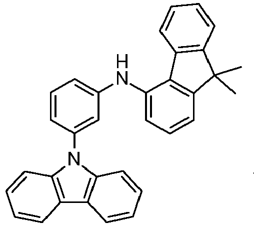 | 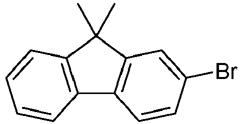 | 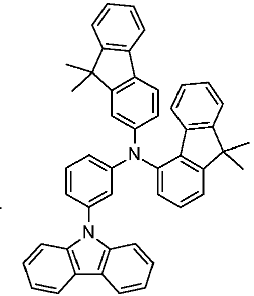 |
| S168 | CAS-28320-31-2 | #P332 |
| 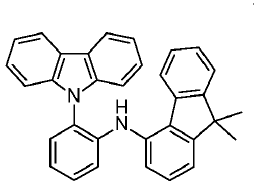 | 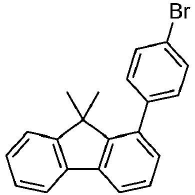 | 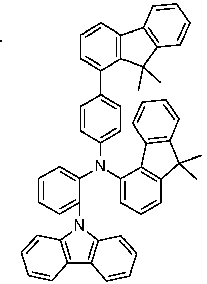 |
| S169 | CAS-1786416-87-2 | #P333 |
| 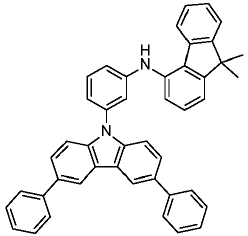 | 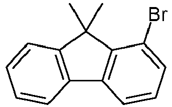 | 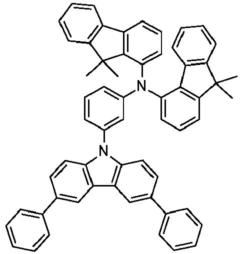 |
| S170 | CAS-1225053-54-2 | #P334 |
| 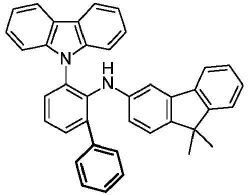 | 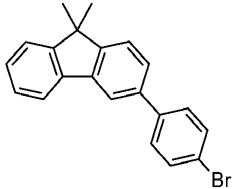 | 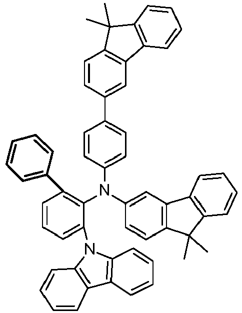 |
| S171 | CAS-1421789-06-1 | #P335 |
| 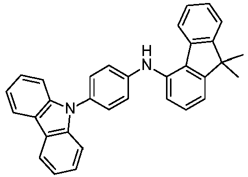 | 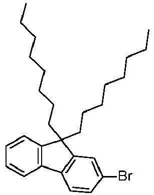 | 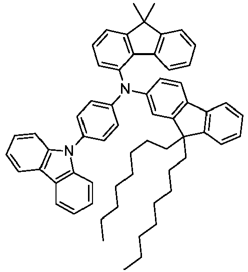 |
| S167 | CAS-302554-80-9 | #P336 |
| 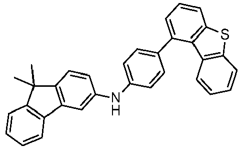 | 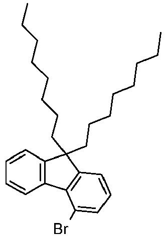 | 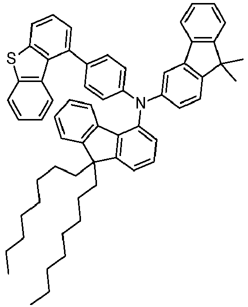 |
| S134 | CAS-1469898-60-9 | #P337 |
| 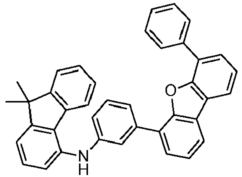 | 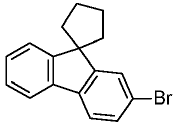 | 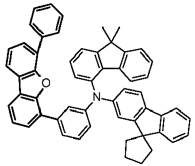 |
| S112 | CAS-797056-47-4 | #P338 |
| 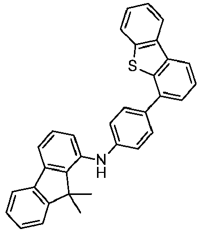 | 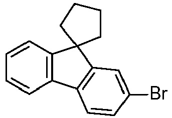 | 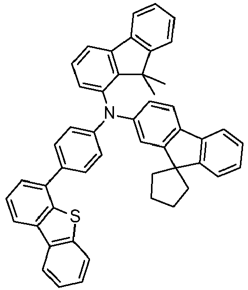 |
| S105 | CAS-797056-47-4 | #P339 |
| 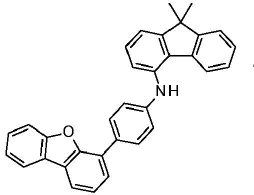 | 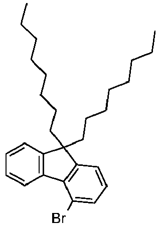 | 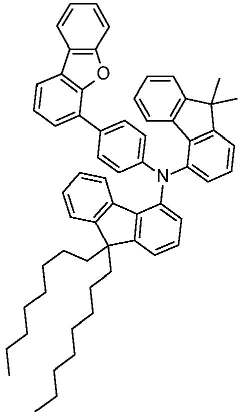 |
| CAS-1933454-49-9 | CAS-1469898-60-9 | #P340 |

### B) Device-Beispiele

Die OLEDs werden wie folgt hergestellt:
Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:EG1:TEG1 (55%:35%:10%) bedeutet hierbei, dass das Material IC1 in einem Volumenanteil von 55%, EG1 in einem Anteil von 35% und TEG1 in einem Anteil von 10% in der Schicht vorliegt. Analog besteht auch die Elektronentransportschicht aus einer Mischung von zwei Materialien.

**Tabelle 1: Aufbau der OLEDs**

| Bsp | HIL Dicke | HTL Dicke | EBL Dicke | EML Dicke | HBL Dicke | ETL Dicke |
|---|---|---|---|---|---|---|
| V1 | HATCN 5nm | SpMA1 230nm | SpMA2 20nm | IC1:SdT1:TEG1 (59%:29%:12%) 30nm | ST2 10nm | ST2:LiQ (50%:50%) 30nm |
| E2 | s.o. | s.o. | s.o. | IC1:EG1:TEG1 (59%:29%:12%) 30nm | s.o. | s.o. |
| E2 | s.o. | s.o. | s.o. | IC1:EG2:TEG1 (59%:29%:12%) 30nm | s.o. | s.o. |

**Tabelle 2: Strukturformeln der Materialien für die OLEDs**

| | |
|---|---|
| 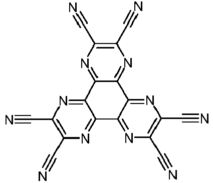 | 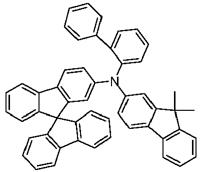 |
| HATCN | SpMA1 |
| 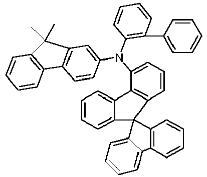 | 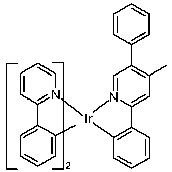 |
| SpMA2 | TEG1 |
| 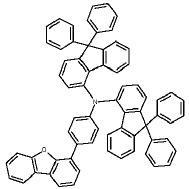 | 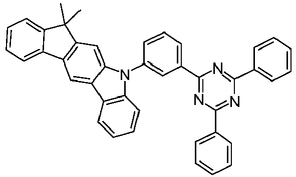 |
| SdT1 | IC1 |
| 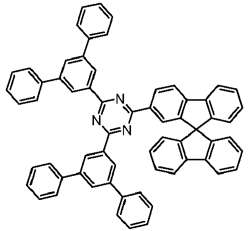 | 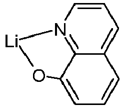 |
| ST2 | LiQ |
| 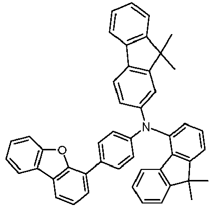 | 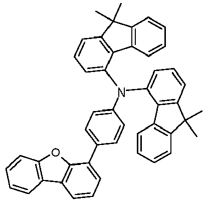 |
| #EG1 (P200) | EG2 (P1) |

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=80% in Tabelle 3 bedeutet, dass die in Spalte LD angegebene Lebensdauer der Zeit entspricht, nach der die Leuchtdichte auf 80% ihres Anfangswertes absinkt.

Die erfindungsgemäßen Verbindung EG2 wird in dem Beispiel E2 als Matrixmaterial in einer Emissionsschicht enthaltend einen grünen Triplett-Emitter eingesetzt. Dabei werden sehr gute Ergebnisse für die oben genannten Leistungsdaten erhalten (Tabelle 3a).

**Tabelle 3a: Daten der OLEDs**

| Bsp. | U1000 (V) | SE1000 (cd/A) | EQE 1000 (%) | CIE x/y bei 1000 cd/m² | j₀ (mA/cm²) | L1 (%) | LD (h) |
|---|---|---|---|---|---|---|---|
| #E1 | 3.2 | 73 | 19.5 | 0.33/0.63 | 20 | 80 | 440 |
| E2 | 3.1 | 70 | 19.0 | 0.35/0.62 | 20 | 80 | 700 |

Nach dem Vorbild des Beispiels E2 erhält man durch Verwendung von weiteren erfindungsgemäßen Verbindungen, wie in Tabelle 4 gezeigt, OLEDs mit ausgezeichneten Leistungsdaten, was die breite Anwendbarkeit der erfindungsgemäßen Verbindungen demonstriert. Die erfindungsgemäßen Verbindungen zeichnen sich in den Beispielen durch eine sehr gute Lebensdauer aus.

**Tabelle 4**

| | | |
|---|---|---|
| 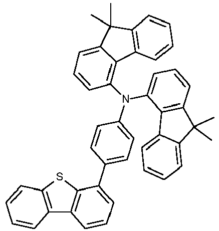 | 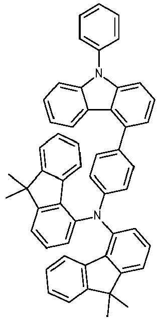 | 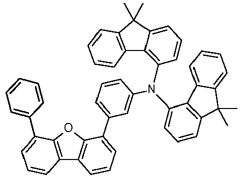 |
| P6 | P9 | P14 |
| 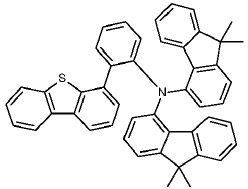 | 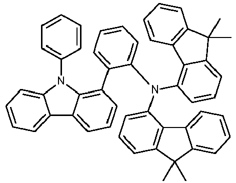 | 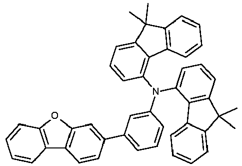 |
| P16 | P19 | P22 |
| 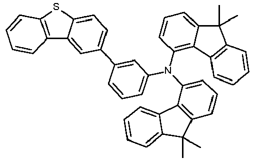 | 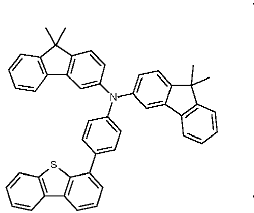 | 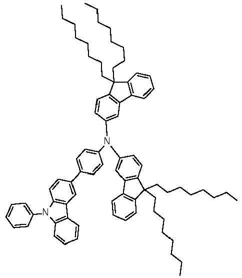 |
| P27 | #P46 | #P48 |
| 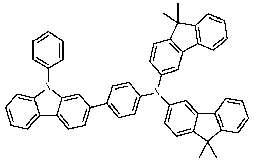 | 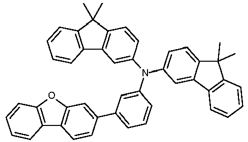 | 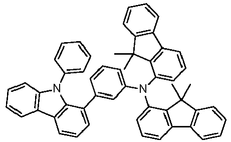 |
| #P50 | #P61 | #P70 |
| 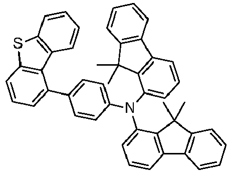 | 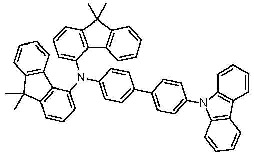 | 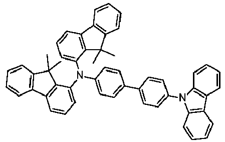 |
| #P74 | P85 | #P86 |
| 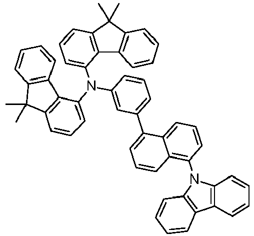 | 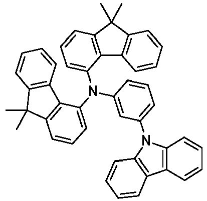 | 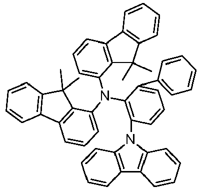 |
| P105 | P109 | #P113 |
| 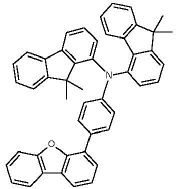 | 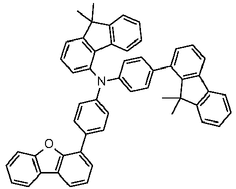 | 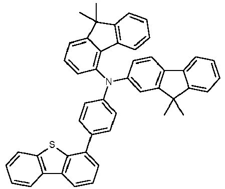 |
| #P203 | #P205 | #P217 |
| 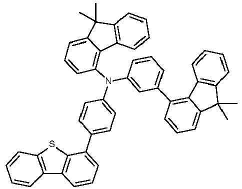 | 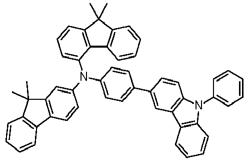 | 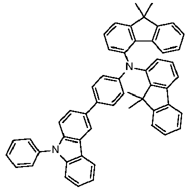 |
| #P226 | #P229 | #P230 |
| 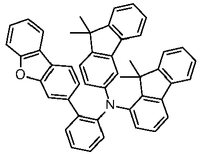 | 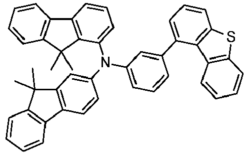 | 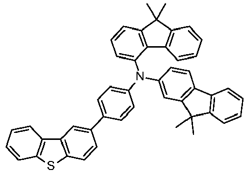 |
| #P237 | #P239 | #P251 |
| 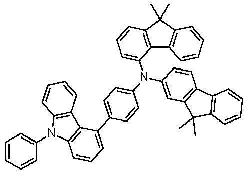 | 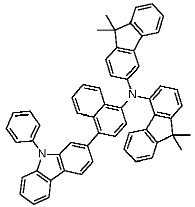 | 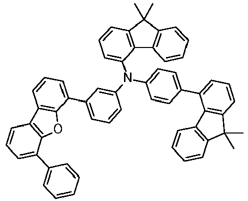 |
| #P256 | #P261 | #P266 |
| 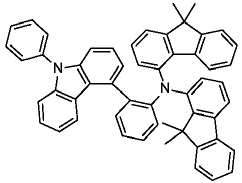 | 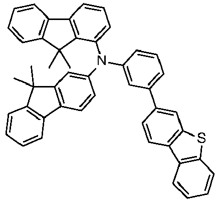 | 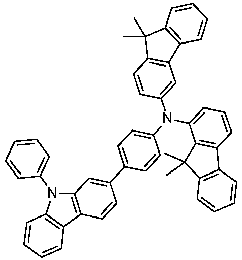 |
| #P273 | #P281 | #P289 |
| 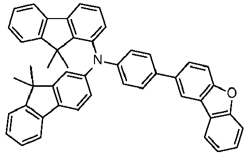 | 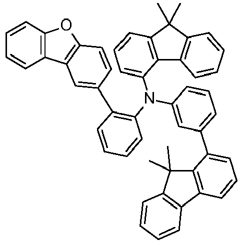 | 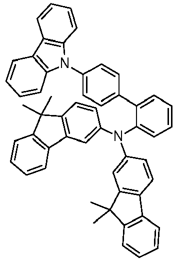 |
| #P291 | #P298 | #P305 |
| 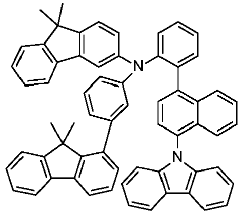 | 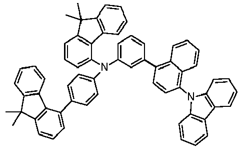 | 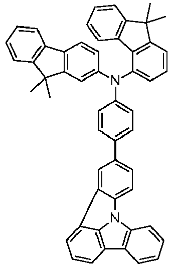 |
| #P314 | #P315 | #P321 |
| 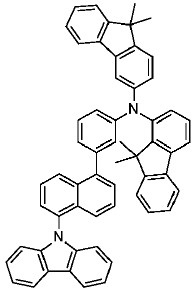 | 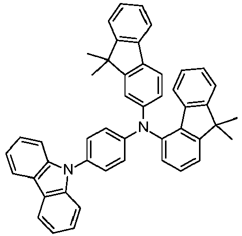 | 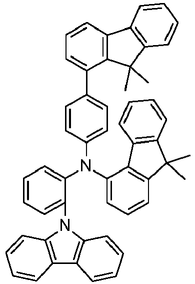 |
| #P327 | #P331 | #P333 |
| 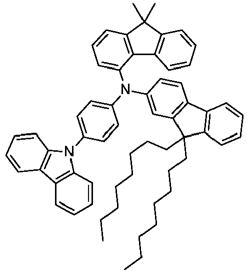 | 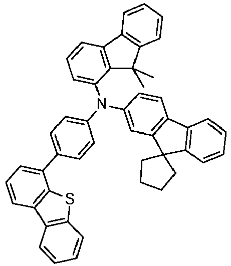 | 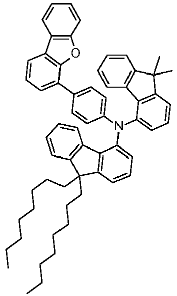 |
| #P336 | #P339 | #P340 |
| 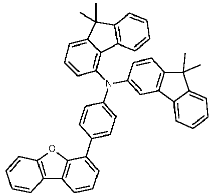 | 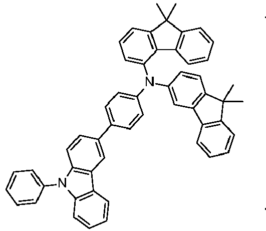 | 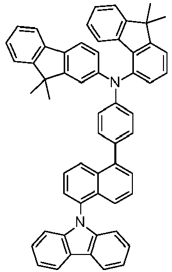 |
| #P204 | #P231 | #P328 |
| 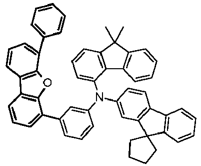 | | |
| #P338 | | |

Dabei werden ebenfalls gute Leistungsdaten, insbesondere gute Lebensdauern, erzielt.

Beim direkten Vergleich der beiden Verbindungen SdT1 und EG2 in den ansonsten identisch aufgebauten OLEDs V1 (prior art, mit SdT1) und E2 (erfindungsgemäß, mit EG2) zeigt sich, dass die OLED mit der erfindungsgemäßen Verbindung EG2 eine deutlich bessere Lebensdauer aufweist, bei leicht verringerter Betriebsspannung.

**Tabelle 3b: Daten der OLEDs**

| Bsp. | U1000 (V) | CIE x/y bei 1000 cd/m² | j₀ (mA/cm²) | L1 (%) | LD (h) |
|---|---|---|---|---|---|
| V1 | 3.3 | 0.34/0.62 | 20 | 80 | 250 |
| E2 | 3.1 | 0.35/0.62 | 20 | 80 | 700 |

| | | |
|---|---|---|
| 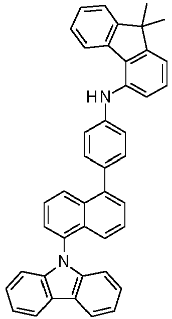 | 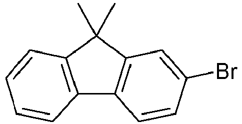 | 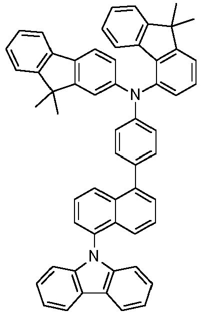 |
| S164 | CAS-28320-31-2 | P328 |
| 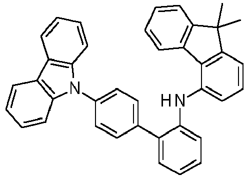 | 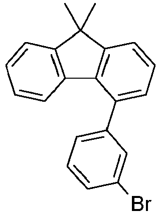 | 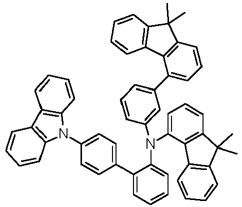 |
| S165 | CAS-1860896-40-7 | P329 |
| 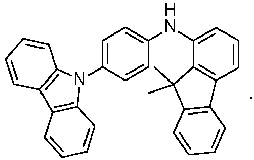 | 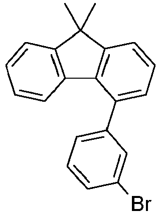 | 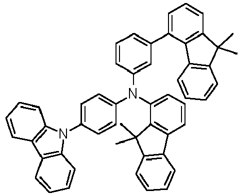 |
| S166 | CAS-1860896-40-7 | P330 |
| 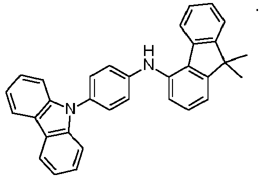 | 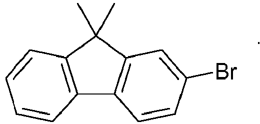 | 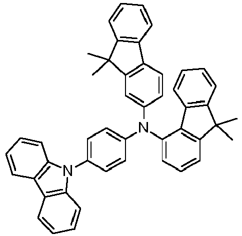 |
| S167 | CAS-28320-31-2 | P331 |
| 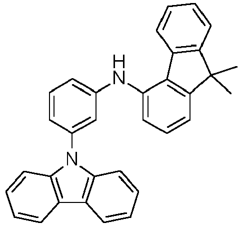 | 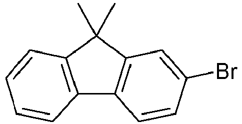 | 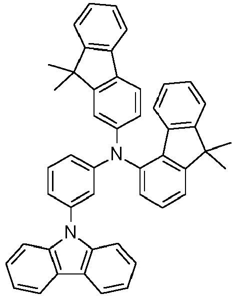 |
| S168 | CAS-28320-31-2 | P332 |
| 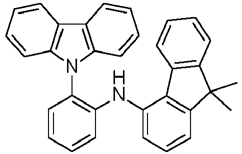 | 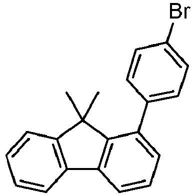 | 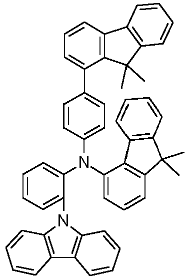 |
| S169 | CAS-1786416-87-2 | P333 |
| 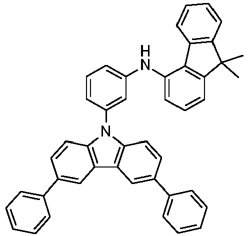 | 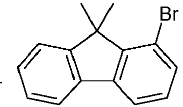 | 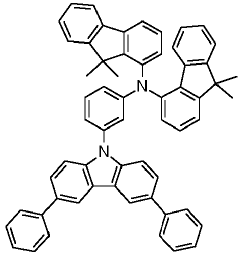 |
| S170 | CAS-1225053-54-2 | P334 |
| 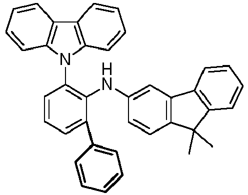 | 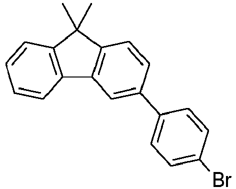 | 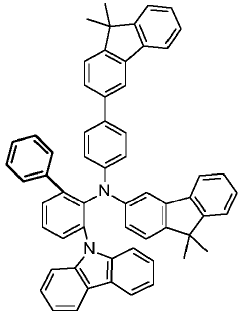 |
| S171 | CAS-1421789-06-1 | P335 |
| 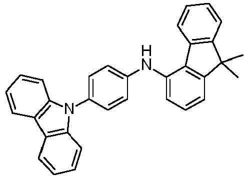 | 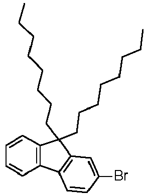 | 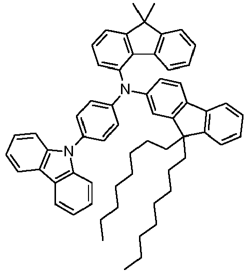 |
| S167 | CAS-302554-80-9 | P336 |
| 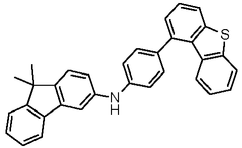 | 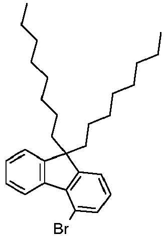 | 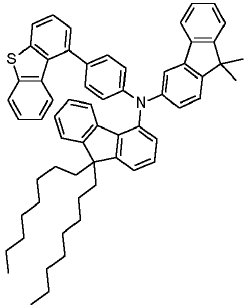 |
| S134 | CAS-1469898-60-9 | P337 |
| 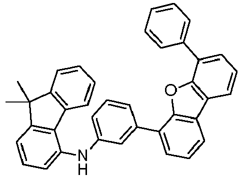 | 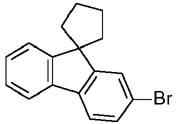 | 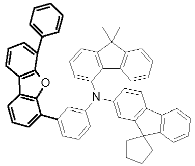 |
| S112 | CAS-797056-47-4 | P338 |
| 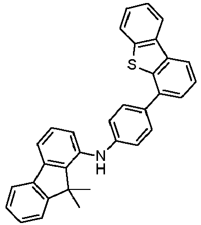 | 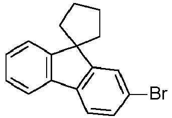 | 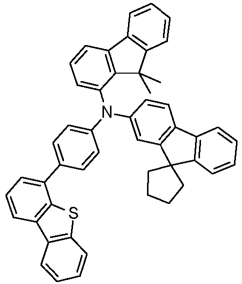 |
| S105 | CAS-797056-47-4 | P339 |
| 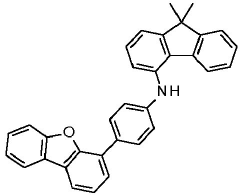 | 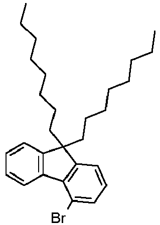 | 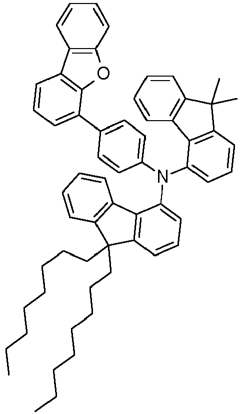 |
| CAS-1933454-49-9 | CAS-1469898-60-9 | P340 |

### B) Device-Beispiele

Die OLEDs werden wie folgt hergestellt:
Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / Lochblockierschicht (HBL) / Elektronentransportschicht (ETL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:EG1:TEG1 (55%:35%:10%) bedeutet hierbei, dass das Material IC1 in einem Volumenanteil von 55%, EG1 in einem Anteil von 35% und TEG1 in einem Anteil von 10% in der Schicht vorliegt. Analog besteht auch die Elektronentransportschicht aus einer Mischung von zwei Materialien.

**Tabelle 1: Aufbau der OLEDs**

| Bsp | HIL Dicke | HTL Dicke | EBL Dicke | EML Dicke | HBL Dicke | ETL Dicke |
|---|---|---|---|---|---|---|
| V1 | HATCN 5nm | SpMA1 230nm | SpMA2 20nm | IC1:SdT1:TEG1 (59%:29%:12%) 30nm | ST2 10nm | ST2:LiQ (50%:50%) 30nm |
| E2 | s.o. | s.o. | s.o. | IC1:EG1:TEG1 (59%:29%:12%) 30nm | s.o. | s.o. |
| E2 | s.o. | s.o. | s.o. | IC1:EG2:TEG1 (59%:29%:12%) 30nm | s.o. | s.o. |

**Tabelle 2: Strukturformeln der Materialien für die OLEDs**

| | |
|---|---|
| 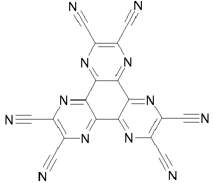 | 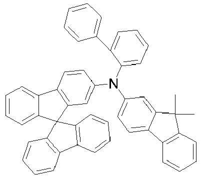 |
| HATCN | SpMA1 |
| 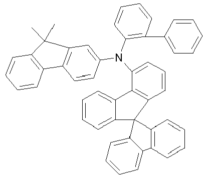 | 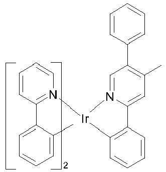 |
| SpMA2 | TEG1 |
| 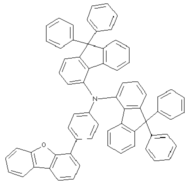 | 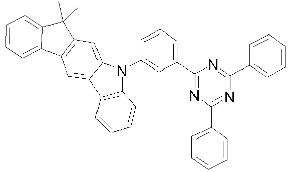 |
| SdT1 | IC1 |
| 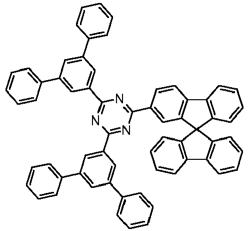 | 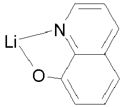 |
| ST2 | LiQ |
| 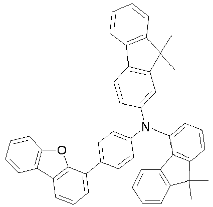 | 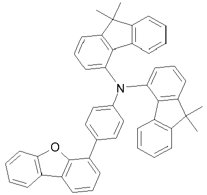 |
| EG1 (P200) | EG2 (P1) |

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=80% in Tabelle 3 bedeutet, dass die in Spalte LD angegebene Lebensdauer der Zeit entspricht, nach der die Leuchtdichte auf 80% ihres Anfangswertes absinkt.

Die erfindungsgemäßen Verbindungen EG1 und EG2 werden in den Beispielen E1 und E2 als Matrixmaterial in einer Emissionsschicht enthaltend einen grünen Triplett-Emitter eingesetzt. Dabei werden sehr gute Ergebnisse für die oben genannten Leistungsdaten erhalten (Tabelle 3a).

**Tabelle 3a: Daten der OLEDs**

| Bsp. | U1000 (V) | SE1000 (cd/A) | EQE 1000 (%) | CIE x/y bei 1000 cd/m² | j₀ (mA/cm²) | L1 (%) | LD (h) |
|---|---|---|---|---|---|---|---|
| E1 | 3.2 | 73 | 19.5 | 0.33/0.63 | 20 | 80 | 440 |
| E2 | 3.1 | 70 | 19.0 | 0.35/0.62 | 20 | 80 | 700 |

Nach dem Vorbild der Beispiele E1 bis E2 erhält man durch Verwendung von weiteren erfindungsgemäßen Verbindungen, wie in Tabelle 4 gezeigt, OLEDs mit ausgezeichneten Leistungsdaten, was die breite Anwendbarkeit der erfindungsgemäßen Verbindungen demonstriert. Die erfindungsgemäßen Verbindungen zeichnen sich in den Beispielen durch eine sehr gute Lebensdauer aus.

**Tabelle 4**

| | | |
|---|---|---|
| 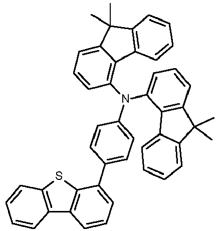 | 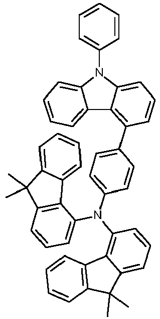 | 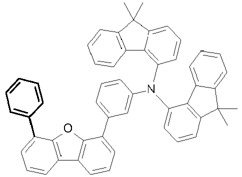 |
| P6 | P9 | P14 |
| 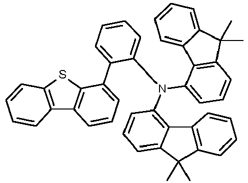 | 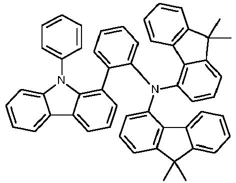 | 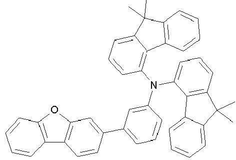 |
| P16 | P19 | P22 |
| 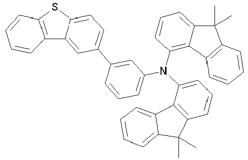 | 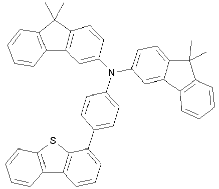 | 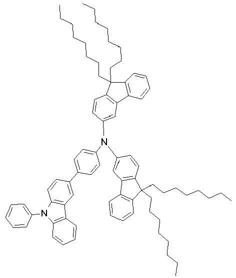 |
| P27 | P46 | P48 |
| 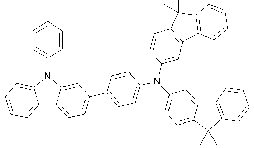 | 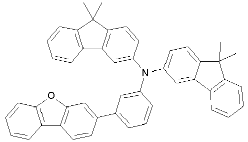 | 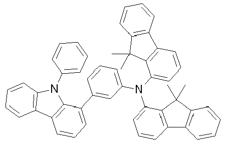 |
| P50 | P61 | P70 |
| 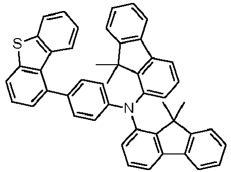 | 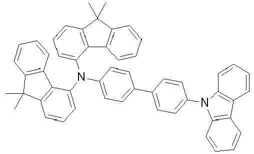 | 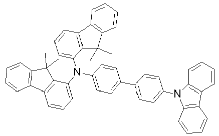 |
| P74 | P85 | P86 |
| 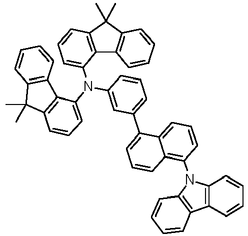 | 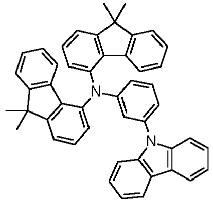 | 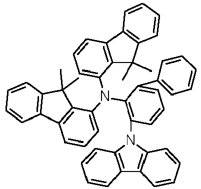 |
| P105 | P109 | P113 |
| 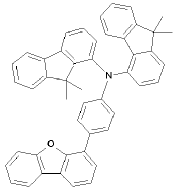 | 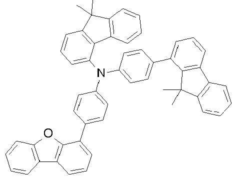 | 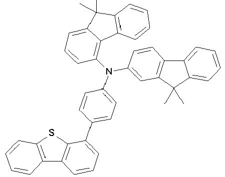 |
| P203 | P205 | P217 |
| 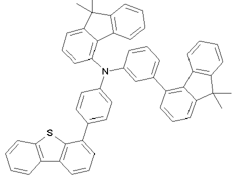 | 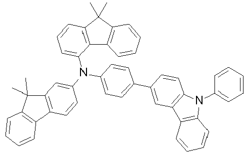 | 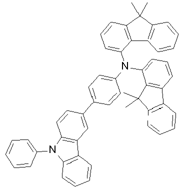 |
| P226 | P229 | P230 |
| 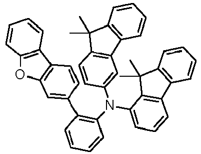 | 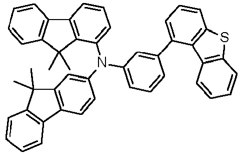 | 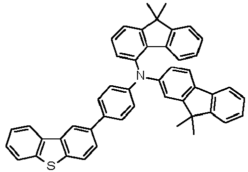 |
| P237 | P239 | P251 |
| 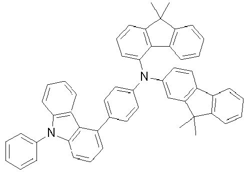 | 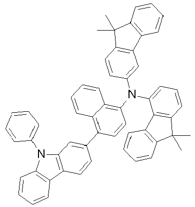 | 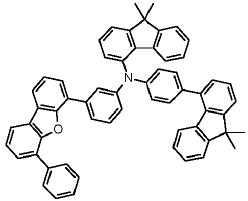 |
| P256 | P261 | P266 |
| 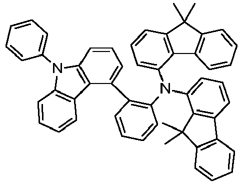 | 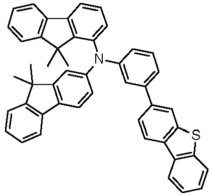 | 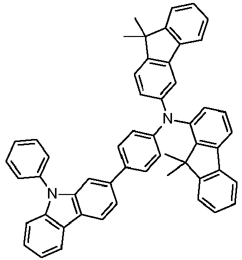 |
| P273 | P281 | P289 |
| 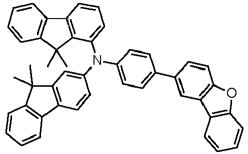 | 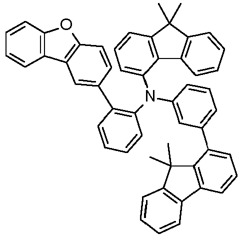 | 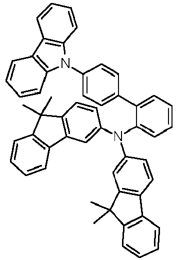 |
| P291 | P298 | P305 |
| 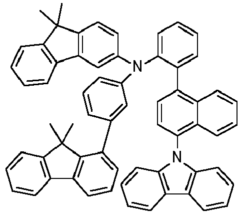 | 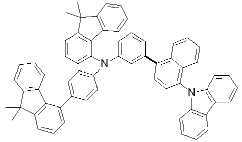 | 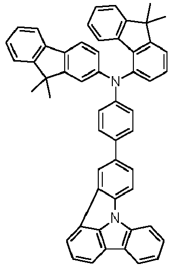 |
| P314 | P315 | P321 |
| 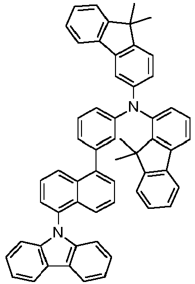 | 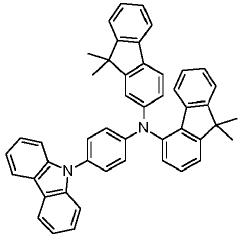 | 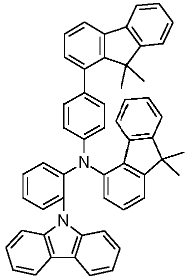 |
| P327 | P331 | P333 |
| 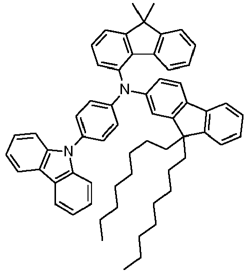 | 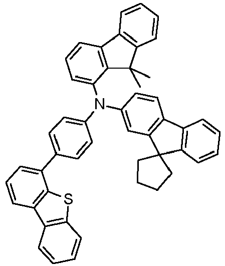 | 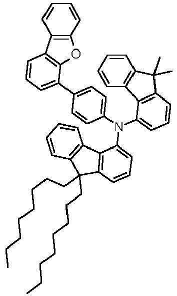 |
| P336 | P339 | P340 |
| 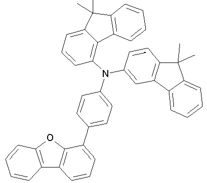 | 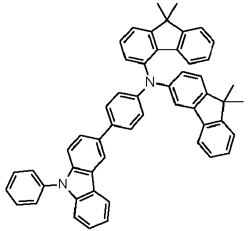 | 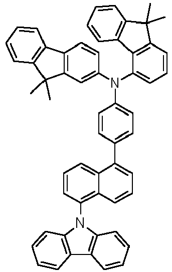 |
| P204 | P231 | P328 |
| 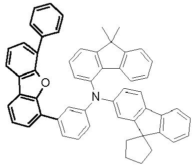 | | |
| P338 | | |

Dabei werden ebenfalls gute Leistungsdaten, insbesondere gute Lebensdauern, erzielt.

Beim direkten Vergleich der beiden Verbindungen SdT1 und EG2 in den ansonsten identisch aufgebauten OLEDs V1 (prior art, mit SdT1) und E2 (erfindungsgemäß, mit EG2) zeigt sich, dass die OLED mit der erfindungsgemäßen Verbindung EG2 eine deutlich bessere Lebensdauer aufweist, bei leicht verringerter Betriebsspannung.

**Tabelle 3b: Daten der OLEDs**

| Bsp. | U1000 (V) | CIE x/y bei 1000 cd/m² | j₀ (mA/cm²) | L1 (%) | LD (h) |
|---|---|---|---|---|---|
| V1 | 3.3 | 0.34/0.62 | 20 | 80 | 250 |
| E2 | 3.1 | 0.35/0.62 | 20 | 80 | 700 |

## Patentansprüche

1. Verbindung einer Formel (I-A-4) 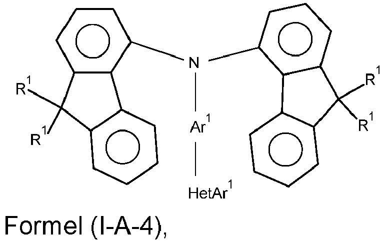 wobei die freien Positionen an den Fluorenylgruppen jeweils mit einem Rest R² substituiert sein können und wobei weiterhin gilt:
R¹ ist bei jedem Auftreten gleich oder verschieden gewählt aus geradkettigen Alkylgruppen mit 1 bis 10 C-Atomen und verzweigten oder cyclischen Alkylgruppen mit 3 bis 10 C-Atomen, wobei zwei oder mehr Reste R¹ miteinander verknüpft sein können und einen Ring bilden können, und wobei ein oder mehrere H-Atome in den Alkylgruppen durch D ersetzt sein können;
Ar¹ ist gewählt aus aromatischen Ringsystemen mit 6 bis 20 aromatischen Ringatomen, die mit einem oder mehreren Resten R⁴ substituiert sein können;
HetAr¹ ist gewählt aus heteroaromatischen Ringsystemen mit 13 bis 40 aromatischen Ringatomen, die mit einem oder mehreren Resten R⁵ substituiert sein können;
R², R⁴ sind bei jedem Auftreten gleich oder verschieden gewählt aus H, D, F, C(=O)R¹¹, CN, Si(R¹¹)₃, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste gewählt aus Resten R², und zwei oder mehr Reste gewählt aus Resten R⁴ jeweils miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹- , NR¹¹, P(=O)(R¹¹), -O-, -S-, SO oder SO₂ ersetzt sein können;
R⁵ ist bei jedem Auftreten gleich oder verschieden gewählt aus H, D, F, C(=O)R¹¹, CN, Si(R¹¹)₃, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, und aromatischen Ringsystemen mit 6 bis 40 aromatischen Ringatomen; wobei zwei oder mehr Reste gewählt aus Resten R⁵ jeweils miteinander verknüpft sein können und einen Ring bilden können; wobei die genannten Alkyl-, Alkoxy-, Alkenyl- und Alkinylgruppen und die genannten aromatischen Ringsysteme jeweils mit einem oder mehreren Resten R¹¹ substituiert sein können; und wobei eine oder mehrere CH₂-Gruppen in den genannten Alkyl-, Alkoxy-, Alkenyl- und Alkinylgruppen durch - R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können;
R¹¹ ist bei jedem Auftreten gleich oder verschieden gewählt aus H, D, F, C(=O)R²¹, CN, Si(R²¹)₃, N(R²¹)₂, P(=O)(R²¹)₂, OR²¹, S(=O)R²¹, S(=O)₂R²¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R²¹ substituiert sein können; und wobei eine oder mehrere CH₂-Gruppen in den genannten Alkyl-, Alkoxy-, Alkenyl- und Alkinylgruppen durch - R²¹C=CR²¹-, -C=C-, Si(R²¹)₂, C=O, C=NR²¹, -C(=O)O-, -C(=O)NR²¹-, NR²¹, P(=O)(R²¹), -O-, -S-, SO oder SO₂ ersetzt sein können;
R²¹ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R³ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können.

2. Verbindung nach Anspruch 1, **dadurch gekennzeichnet, dass** Ar¹ gleich Benzol ist, das jeweils mit einem oder mehreren Resten R⁴ substituiert sein kann.

3. Verbindung nach Anspruch 1 oder 2, **dadurch gekennzeichnet, dass** HetAr¹ gewählt ist aus Dibenzofuran, Dibenzothiophen und Carbazol, welche jeweils mit einem oder mehreren Resten R⁵ substituiert sein können.

4. Verbindung nach einem oder mehreren der Ansprüche 1 bis 3, **dadurch gekennzeichnet, dass** R², und R⁴ bei jedem Auftreten gleich oder verschieden gewählt sind aus H, aromatischen Ringsystemen mit 6 bis 40 aromatischen Ringatomen, und heteroaromatischen Ringsystemen mit 5 bis 40 aromatischen Ringatomen, die jeweils mit einem oder mehreren Resten R¹¹ substituiert sein können; und **dadurch gekennzeichnet, dass** R⁵ bei jedem Auftreten gleich oder verschieden gewählt ist aus H und aromatischen Ringsystemen mit 6 bis 40 aromatischen Ringatomen, die mit einem oder mehreren Resten R¹¹ substituiert sein können.

5. Verbindung nach einem oder mehreren der Ansprüche 1 bis 4, **dadurch gekennzeichnet, dass** R¹¹ bei jedem Auftreten gleich oder verschieden gewählt ist aus H, D, F, CN, Si(R²¹)₃, N(R²¹)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R²¹ substituiert sein können.

6. Verbindung nach Anspruch 1, **dadurch gekennzeichnet, dass** die Gruppe -Ar¹-HetAr¹ der Formel (H-1) oder (H-2) entspricht
| | |
|---|---|
| 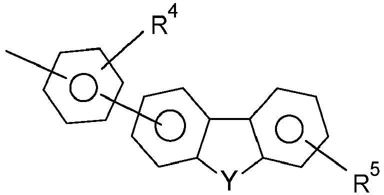 | 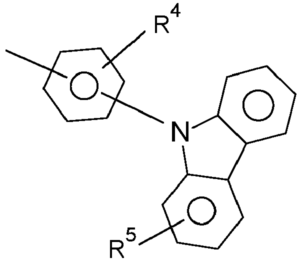 |
| Formel (H-1) | Formel (H-2) |
wobei Y gleich O, S oder NR⁵ ist; und
wobei R⁴ und R⁵ definiert sind wie in Anspruch 4;
und wobei die Gruppe über die freie Bindung an das Stickstoffatom gebunden ist.

7. Verfahren zur Herstellung einer Verbindung nach einem oder mehreren der Ansprüche 1 bis 6, **dadurch gekennzeichnet, dass** eine Verbindung HetAr¹-Ar¹-NH₂, wobei die auftretenden Variablen definiert sind wie in einem oder mehreren der Ansprüche 1 bis 6 für Formel (I-A-4), in einer Buchwald-Kupplungsreaktion mit einem Fluoren umgesetzt wird, das eine reaktive Gruppe X aufweist.

8. Oligomer, Polymer oder Dendrimer, enthaltend eine oder mehrere Verbindungen gemäß Formel (I-A-4) nach einem oder mehreren der Ansprüche 1 bis 6, wobei die Bindung(en) zum Polymer, Oligomer oder Dendrimer an beliebigen, in Formel (I-A-4) mit R¹, R², R⁴ oder R⁵ substituierten Positionen lokalisiert sein können.

9. Formulierung, enthaltend mindestens eine Verbindung nach einem oder mehreren der Ansprüche 1 bis 6 oder ein Polymer, Oligomer oder Dendrimer nach Anspruch 8, sowie mindestens ein Lösungsmittel.

10. Elektronische Vorrichtung, enthaltend mindestens eine Verbindung nach einem oder mehreren der Ansprüche 1 bis 6, oder ein Polymer, Oligomer oder Dendrimer nach Anspruch 8.

11. Elektronische Vorrichtung nach Anspruch 10, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die gewählt ist aus emittierenden Schichten und lochtransportierenden Schichten, die mindestens eine Verbindung enthält.

12. Organische Elektrolumineszenzvorrichtung nach Anspruch 11, enthaltend Anode, Kathode und mindestens eine emittierende Schicht, **dadurch gekennzeichnet, dass** die mindestens eine Verbindung in einer emittierenden Schicht in Kombination mit mindestens einem phosphoreszierenden Emitter enthalten ist.

13. Verwendung einer Verbindung nach einem oder mehreren der Ansprüche 1 bis 6 in einer elektronischen Vorrichtung.

## Claims

1. Compound of a formula (I-A-4) 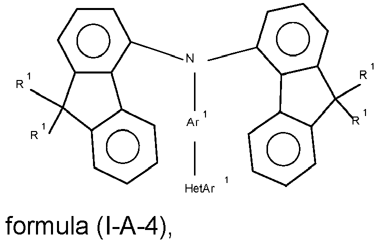 where the free positions on the fluorenyl groups may in each case be substituted by a radical R² and where the following furthermore applies:
R¹ is selected on each occurrence, identically or differently, from straight-chain alkyl groups having 1 to 10 C atoms and branched or cyclic alkyl groups having 3 to 10 C atoms, where two or more radicals R¹ may be linked to one another and may form a ring, and where one or more H atoms in the alkyl groups may be replaced by D;
Ar¹ is selected from aromatic ring systems having 6 to 20 aromatic ring atoms, which may be substituted by one or more radicals R⁴;
HetAr¹ is selected from heteroaromatic ring systems having 13 to 40 aromatic ring atoms, which may be substituted by one or more radicals R⁵;
R², R⁴ are selected on each occurrence, identically or differently, from H, D, F, C(=O)R¹¹, CN, Si(R¹¹)₃, N(R¹¹)₂, P(=O)(R¹¹)₂, OR¹¹, S(=O)R¹¹, S(=O)₂R¹¹, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals selected from radicals R², and two or more radicals selected from radicals R⁴ may in each case be linked to one another and may form a ring; where the said alkyl, alkoxy, alkenyl and alkynyl groups and the said aromatic ring systems and heteroaromatic ring systems may in each case be substituted by one or more radicals R¹¹; and where one or more CH₂ groups in the said alkyl, alkoxy, alkenyl and alkynyl groups may be replaced by -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO or SO₂;
R⁵ is selected on each occurrence, identically or differently, from H, D, F, C(=O)R¹¹, CN, Si(R¹¹)₃, N(R¹¹)₂, P(=O)(R¹¹)₂, OR¹¹, S(=O)R¹¹, S(=O)₂R¹¹, straight-chain alkyl or alkoxy groups having 1 to 20 C atoms, branched or cyclic alkyl or alkoxy groups having 3 to 20 C atoms, alkenyl or alkynyl groups having 2 to 20 C atoms, and aromatic ring systems having 6 to 40 aromatic ring atoms; where two or more radicals selected from radicals R⁵ may in each case be linked to one another and may form a ring; where the said alkyl, alkoxy, alkenyl and alkynyl groups and the said aromatic ring systems may in each case be substituted by one or more radicals R¹¹; and where one or more CH₂ groups in the said alkyl, alkoxy, alkenyl and alkynyl groups may be replaced by -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO or SO₂;
R¹¹ is selected on each occurrence, identically or differently, from H, D, F, C(=O)R²¹, CN, Si(R²¹)₃, N(R²¹)₂, P(=O)(R²¹)₂, OR²¹, S(=O)R²¹, S(=O)₂R²¹, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹¹ may be linked to one another and may form a ring; where the said alkyl, alkoxy, alkenyl and alkynyl groups and the said aromatic ring systems and heteroaromatic ring systems may in each case be substituted by one or more radicals R²¹; and where one or more CH₂ groups in the said alkyl, alkoxy, alkenyl and alkynyl groups may be replaced by -R²¹C=CR²¹-, -C=C-, Si(R²¹)₂, C=O, C=NR²¹, -C(=O)O-, -C(=O)NR²¹-, NR²¹, P(=O)(R²¹), -O-, -S-, SO or SO₂;
R²¹ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R³ may be linked to one another and may form a ring; and where the said alkyl, alkoxy, alkenyl and alkynyl groups, aromatic ring systems and heteroaromatic ring systems may be substituted by one or more radicals selected from F and CN.

2. Compound according to Claim 1, **characterised in that** Ar¹ is equal to benzene, which may in each case be substituted by one or more radicals R⁴.

3. Compound according to Claim 1 or 2, **characterised in that** HetAr¹ is selected from dibenzofuran, dibenzothiophene and carbazole, which may in each case be substituted by one or more radicals R⁵.

4. Compound according to one or more of Claims 1 to 3, **characterised in that** R² and R⁴ are selected on each occurrence, identically or differently, from H, aromatic ring systems having 6 to 40 aromatic ring atoms, and heteroaromatic ring systems having 5 to 40 aromatic ring atoms, which may in each case be substituted by one or more radicals R¹¹; and **characterised in that** R⁵ is selected on each occurrence, identically or differently, from H and aromatic ring systems having 6 to 40 aromatic ring atoms, which may be substituted by one or more radicals R¹¹.

5. Compound according to one or more of Claims 1 to 4, **characterised in that** R¹¹ is selected on each occurrence, identically or differently, from H, D, F, CN, Si(R²¹)₃, N(R²¹)₂, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R²¹.

6. Compound according to Claim 1, **characterised in that** the group -Ar¹-HetAr¹ corresponds to the formula (H-1) or (H-2)
| | |
|---|---|
| 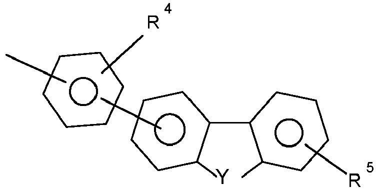 | 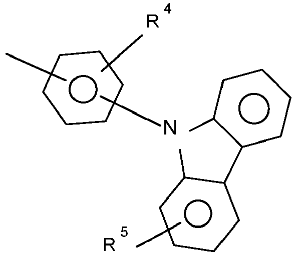 |
| formula (H-1) | formula (H-2) |
where Y is equal to O, S or NR⁵; and
where R⁴ and R⁵ are defined as in Claim 4;
and where the group is bonded to the nitrogen atom via the free bond.

7. Process for the preparation of a compound according to one or more of Claims 1 to 6, **characterised in that** a compound HetAr¹-Ar¹-NH₂, where the variables occurring are defined as in one or more of Claims 1 to 6 for formula (I-A-4), is reacted with a fluorene containing a reactive group X in a Buchwald coupling reaction.

8. Oligomer, polymer or dendrimer containing one or more compounds of the formula (I-A-4) according to one or more of Claims 1 to 6, where die bond(s) to the polymer, oligomer or dendrimer may be localised at any desired positions in formula (I-A-4) that are substituted by R¹, R², R⁴ or R⁵.

9. Formulation comprising at least one compound according to one or more of Claims 1 to 6 or a polymer, oligomer or dendrimer according to Claim 8, and at least one solvent.

10. Electronic device containing at least one compound according to one or more of Claims 1 to 6, or a polymer, oligomer or dendrimer according to Claim 8.

11. Electronic device according to Claim 10, **characterised in that** it is an organic electroluminescent device, comprising anode, cathode and at least one emitting layer, where at least one organic layer of the device is selected from emitting layers and hole-transporting layers which comprise at least one compound.

12. Organic electroluminescent device according to Claim 11, comprising anode, cathode and at least one emitting layer, **characterised in that** the at least one compound is present in an emitting layer in combination with at least one phosphorescent emitter.

13. Use of a compound according to one or more of Claims 1 to 6 in an electronic device.

## Revendications

1. Composé de formule (I-A-4) 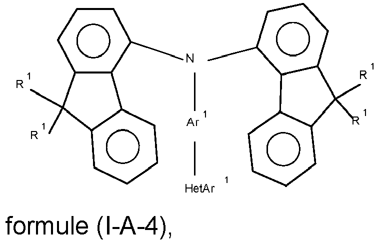 dans laquelle les positions libres sur les groupements fluorényle peuvent dans chaque cas être substituées par un radical R² et où ce qui suit s'applique également :
R¹ est choisi à chaque occurrence, de manière identique ou différente, parmi des groupements alkyle à chaîne linéaire ayant de 1 à 10 atomes de C et des groupements alkyle ramifiés ou cycliques ayant de 3 à 10 atomes de C, où deux, ou plus, radicaux R¹ peuvent être liés les uns aux autres et peuvent former un cycle, et où un ou plusieurs atomes de H dans les groupements alkyle peuvent être remplacés par D ;
Ar¹ est choisi parmi des noyaux aromatiques ayant de 6 à 20 atomes de cycle aromatique, qui peuvent être substitués par un ou plusieurs radicaux R⁴ ;
HetAr¹ est choisi parmi des noyaux hétéroaromatiques ayant de 13 à 40 atomes de cycle aromatique, qui peuvent être substitués par un ou plusieurs radicaux R⁵ ;
R², R⁴ sont choisis à chaque occurrence, de manière identique ou différente, parmi H, D, F, C(=O)R¹¹, CN, Si(R¹¹)3, N(R¹¹)2, P(=O)(R¹¹)₂, OR¹¹, S(=O)R¹¹, S(=O)₂R¹¹, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux choisis parmi les radicaux R², et deux, ou plus, radicaux choisis parmi les radicaux R⁴ peuvent dans chaque cas être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R¹¹ ; et où un ou plusieurs groupements CH₂ dans lesdits groupements alkyle, alcoxy, alcényle et alcynyle peuvent être remplacés par -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO ou SO₂ ;
R⁵ est choisi à chaque occurrence, de manière identique ou différente, parmi H, D, F, C(=O)R¹¹, CN, Si(R¹¹)3, N(R¹¹)₂, P(=O)(R¹¹)₂, OR¹¹, S(=O)R¹¹, S(=O)₂R¹¹, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, et des noyaux aromatiques ayant de 6 à 40 atomes de cycle aromatique ; où deux, ou plus, radicaux choisis parmi les radicaux R⁵ peuvent dans chaque cas être liés les uns aux autres et peuvent former un cycle ; où lesdits groupements alkyle, alcoxy, alcényle et alcynyle et lesdits noyaux aromatiques peuvent dans chaque cas être substitués par un ou plusieurs radicaux R¹¹ ; et où un ou plusieurs groupements CH₂ dans lesdits groupements alkyle, alcoxy, alcényle et alcynyle peuvent être remplacés par -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO ou SO₂ ;
R¹¹ est choisi à chaque occurrence, de manière identique ou différente, parmi H, D, F, C(=O)R²¹, CN, Si(R²¹)₃, N(R²¹)₂, P(=O)(R²¹)₂, OR²¹, S(=O)R²¹, S(=O)₂R²¹, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹¹ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R²¹ ; et où un ou plusieurs groupements CH₂ dans lesdits groupements alkyle, alcoxy, alcényle et alcynyle peuvent être remplacés par -R²¹C=CR²¹-, -C=C-, Si(R²¹)₂, C=O, C=NR²¹, -C(=O)O-, -C(=O)NR²¹-, NR²¹, P(=O)(R²¹), -O-, -S-, SO ou SO₂ ;
R²¹ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R³ peuvent être liés les uns aux autres et peuvent former un cycle ; et où lesdits groupements alkyle, alcoxy, alcényle et alcynyle, les noyaux aromatiques et les noyaux hétéroaromatiques peuvent être substitué par un ou plusieurs radicaux choisis parmi F et CN.

2. Composé selon la revendication 1, **caractérisé en ce que** Ar¹ est égal à benzène, qui peut dans chaque cas être substitué par un ou plusieurs radicaux R4.

3. Composé selon la revendication 1 ou 2, **caractérisé en ce que** HetAr¹ est choisi parmi dibenzofurane, dibenzothiophène et carbazole, qui peut dans chaque cas être substitué par un ou plusieurs radicaux R⁵.

4. Composé selon l'une ou plusieurs parmi les revendications 1 à 3, **caractérisé en ce que** R² et R⁴ sont choisis à chaque occurrence, de manière identique ou différente, parmi H, des noyaux aromatiques ayant de 6 à 40 atomes de cycle aromatique, et des noyaux hétéroaromatiques ayant de 5 à 40 atomes de cycle aromatique, qui peuvent dans chaque cas être substitués par un ou plusieurs radicaux R¹¹ ; et **caractérisé en ce que** R⁵ est choisi à chaque occurrence, de manière identique ou différente, parmi H et des noyaux aromatiques ayant de 6 à 40 atomes de cycle aromatique, qui peuvent être substitués par un ou plusieurs radicaux R¹¹.

5. Composé selon l'une ou plusieurs parmi les revendications 1 à 4, **caractérisé en ce que** R¹¹ est choisi à chaque occurrence, de manière identique ou différente, parmi H, D, F, CN, Si(R²¹)₃, N(R²¹)₂, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R²¹.

6. Composé selon la revendication 1, **caractérisé en ce que** le groupement -Ar¹-HetAr¹ correspond à la formule (H-1) ou (H-2)
| | |
|---|---|
| 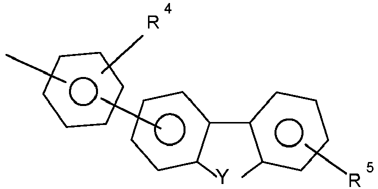 | 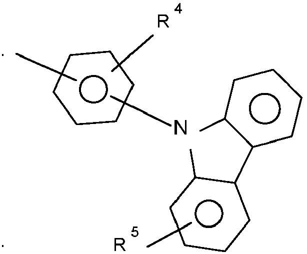 |
| formule (H-1) | formule (H-2) |
où Y est égal à O, S ou NR⁵ ; et
où R⁴ et R⁵ sont tels que définis selon la revendication 4 ;
et où le groupement est lié à l'atome d'azote via la liaison libre.

7. Procédé de préparation d'un composé selon l'une ou plusieurs parmi les revendications 1 à 6, **caractérisé en ce qu'**un composé HetAr¹-Ar¹-NH₂, où les variables présentes sont telles que définies selon l'une ou plusieurs parmi les revendications 1 à 6 pour la formule (I-A-4), est réagi avec un fluorène contenant un groupement réactif X dans une réaction de couplage de Buchwald.

8. Oligomère, polymère ou dendrimère contenant un ou plusieurs composés de formule (I-A-4) selon l'une ou plusieurs parmi les revendications 1 à 6, où la ou les liaisons au polymère, à l'oligomère ou au dendrimère peuvent être situées au niveau de positions désirées quelconques dans la formule (I-A-4) qui sont substituées par R¹, R², R⁴ ou R⁵.

9. Formulation comprenant au moins un composé selon l'une ou plusieurs parmi les revendications 1 à 6 ou un polymère, oligomère ou dendrimère selon la revendication 8, et au moins un solvant.

10. Dispositif électronique contenant au moins un composé selon l'une ou plusieurs parmi les revendications 1 à 6, ou un polymère, oligomère ou dendrimère selon la revendication 8.

11. Dispositif électronique selon la revendication 10, **caractérisé en ce qu'**il s'agit d'un dispositif électroluminescent organique, comprenant une anode, une cathode et au moins une couche émettrice, où au moins une couche organique du dispositif est choisie parmi les couches émettrices et les couches de transport de trous qui comprennent au moins un composé.

12. Dispositif électroluminescent organique selon la revendication 11, comprenant une anode, une cathode et au moins une couche émettrice, **caractérisé en ce que** le au moins un composé est présent dans une couche émettrice en combinaison avec au moins un émetteur phosphorescent.

13. Utilisation d'un composé selon l'une ou plusieurs parmi les revendications 1 à 6, dans un dispositif électronique.
